# EUROPEAN PATENT APPLICATION

(11) **EP 4 660 193 A1**
(43) Date of publication of application: **10.12.2025**
(21) Application number: 24750544.9
(22) Date of filing: 30.01.2024
(51) Int. Cl.: C07D 401/14, A61K 31/4545, A61K 31/5377, A61P 35/00, C07D 471/04

(54) **INDOLE COMPOUND DECOMPOSING IKZF2, AND USE THEREOF**

(30) Priority: 31.01.2023 KR 20230013038; 29.01.2024 KR 20240013568
(71) Applicant: Korea Research Institute of Chemical Technology, Daejeon 34114 (KR); Korea Research Institute of Bioscience and Biotechnology, Daejeon 34141 (KR)
(72) Inventor: HWANG, Jong Yeon, Daejeon 34114 (KR); CHO, Sung Yun, Daejeon 34114 (KR); HA, Jae Du, Daejeon 34114 (KR); KIM, Pil Ho, Daejeon 34114 (KR); KIM, Hyun Jin, Daejeon 34114 (KR); CHO, Yong Hee, Daejeon 34114 (KR); KIM, Jin Hwan, Daejeon 34114 (KR); CHOI, Kyoung Min, Daejeon 34114 (KR); KIM, Jeong Hoon, Daejeon 34141 (KR); KIM, Jung Ae, Daejeon 34141 (KR); PARK, Seul Ki, Daejeon 34141 (KR); PARK, Byoung Chul, Daejeon 34141 (KR); PARK, Sung Goo, Daejeon 34141 (KR); CHO, Jin Hwa, Daejeon 34141 (KR); GO, Ah Ra, Daejeon 34114 (KR)
(74) Representative: Kraus & Lederer PartGmbB
(86) International application number: PCT/KR2024/001426
(87) International publication number: WO 2024/162746

(57) **Abstract**

The present invention relates to an indole compound derivative that decomposes IKZF2 and a use thereof. More specifically, a compound of chemical formula 1 provides an agent having a novel mechanism of action against the IKZF2 protein, and thus is useful for the treatment of diseases and disorders related to the IKZF2 protein, and can in particular be effectively used for preventing or treating cancer.

## Description

### Technical Field

The present disclosure relates to an indole compound derivative for decomposing IKZF2 and use thereof and, more specifically, to an indole derivative compound that degrades IKZF2 and exhibits effects for the prevention or treatment of cancer.

### Background Art

Regulatory T (Treg) cells are a subset of CD4+ T cells that suppress excessive immune responses and play a role in maintaining immune self-tolerance and immune homeostasis. However, Treg cells exposed to cancer suppress the activity of tumor-specific effector T (Teff) cells or increase the expression of immune checkpoint receptors such as PD-1, thereby inhibiting antitumor immune responses (Padmanee Sharma et al., 2017 & Hiroyoshi Nishikawa et al., 2014). IKZF2 (Helios) is a zinc finger transcription factor belonging to the Ikaros family, which is mainly expressed in Tregs and lymphocytes and regulates lymphocyte development (Kaitlin A et al., 2021; Qi Cai et al., 2009; Angela M et al., 2010). In a mouse syngeneic model, loss of IKZF2 induced destabilization of Treg cells and promoted the secretion of pro-inflammatory cytokines, thereby enhancing antitumor immune responses (Hye-Jung Kim et al., 2015; Giuliana P. Mognol et al., 2017). In humans with IKZF2 mutations, chronic T cell hyperactivation and overproduction of pro-inflammatory cytokines by Tregs and Teffs have also been reported (Iivo Hetemaki et al., 2021; Tala Shahin et al., 2022). Furthermore, recent studies have reported that a drug degrading IKZF2 destabilized Treg cells and induced their conversion into Teff cells, thereby promoting the secretion of inflammatory cytokines such as IFNγ, and inhibited solid tumor growth when administered to humanized mouse models (Eric S. Wang et al., 2021; Jonathan Solomon et al., 2022). Therefore, degradation of IKZF2 in T cells including Tregs can induce antitumor immune responses and, acting through a mechanism different from that of conventional PD-1/PD-L1 inhibitors, may be expected to exhibit a synergistic effect when used in combination.

International Patent Publication WO 2019/038717 and International Patent Publication WO 2021/260528 disclose compounds of Chemical Formula I' and their preparation and use for the treatment of IKAROS family zinc fingeR₂ (IKZF2)-dependent diseases or disorders, or in cases where reduction in the level of IKZF2 or IKZF4 proteins may improve such diseases or disorders.

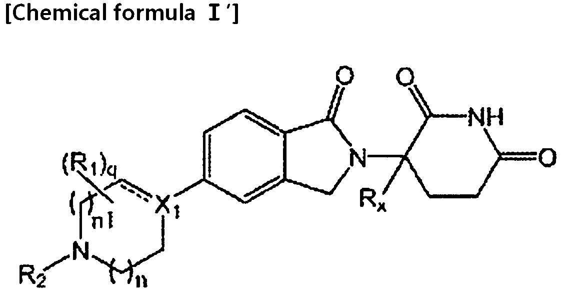

In Chemical Formula 1' of International Patent Publication No. WO 2019-038717, X₁ is CH, R₂ is (C₁-C₆) alkyl, -C(O) (C₁-C₆) alkyl, -C(O) (CH₂)₀₋₃ (C₆-C₁₀) aryl, - C(O)O (CH₂)₀₋₃ (C₆-C₁₀) aryl, (C₆-C₁₀) aryl, 5- or 6-membered heteroaryl bearing 1 to 3 heteroatoms selected from O, N, and S, (C₃-C₈) cycloalkyl or 5- to 7-membered heteroalkyl bearing 1 to 3 heteroatoms selected from O, N, and S, wherein the alkyl moiety optionally has at least R5 as a substituent; and the aryl, heteroaryl, cycloalkyl, and heterocycloalkyl moieties optionally have at least one R5 as a substituent. However, the document does not directly disclose a -C(O)indole-substituted compound in which R₂ is-C(O) (C₆-C₁₀)heteroaryl.

Leading to the present disclosure, intensive and thorough research conducted by the present inventors resulted in the finding that, compared to the compounds developed in the related art, the C (O)indole-substituted compound represented by Chemical Formula 1 of the present disclosure wherein R₂ is a -C(O) (C₆-C₁₀)heteroaryl, exhibits superior biological activity against cancer cells.

### Disclosure of Invention

### Technical Problem

The present inventors have completed the present disclosure by developing an indole derivative compound that degrades IKZF2 and exhibits effects for the prevention or treatment of cancer, and by evaluating its activity.

Accordingly, an aspect of the present disclosure is to provide a compound represented by the following Chemical Formula 1 or a pharmaceutically acceptable salt thereof.

### Solution to Problem

The present disclosure relates to a compound represented by the following Chemical Formula 1, a stereoisomer thereof, a solvate thereof, a prodrug thereof, or a pharmaceutically acceptable salt thereof. 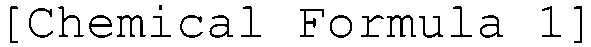 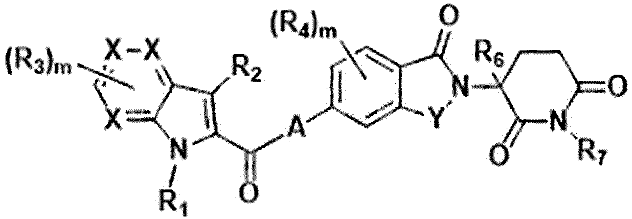 wherein,
X is independently C, CH, N, or NR₃:
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 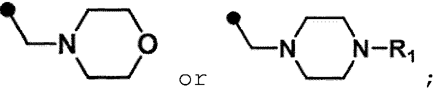
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 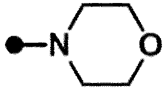 or 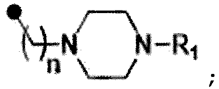
R₄ is independently substituted by a hydrogen atom, halogen, or hydroxy;
A is substituted by 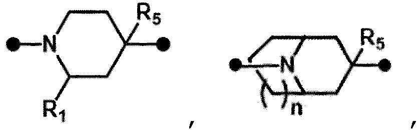 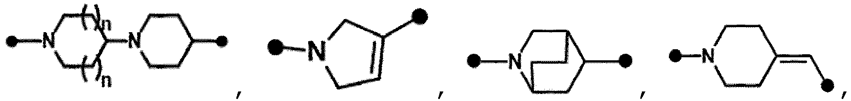 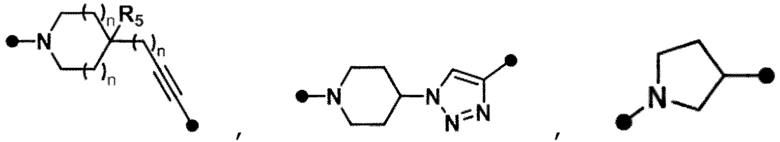 or 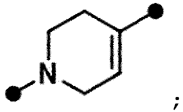
R₅ is substituted by a hydrogen atom, deuterium atom, halogen, or hydroxy;
R₆ is substituted by a hydrogen atom or deuterium atom;
R₇ is substituted by a hydrogen atom, C₁₋₅ alkyl or 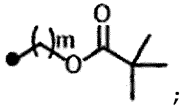
m is an integer of 1 to 3; and
n is independently an integer of 0 or 1.

The present disclosure relates to a compound represented by Chemical Formula 1, a stereoisomer thereof, a solvate thereof, a prodrug thereof, or a pharmaceutically acceptable salt thereof, wherein the Chemical Formula 1 is specified by the following Chemical Formula 2: 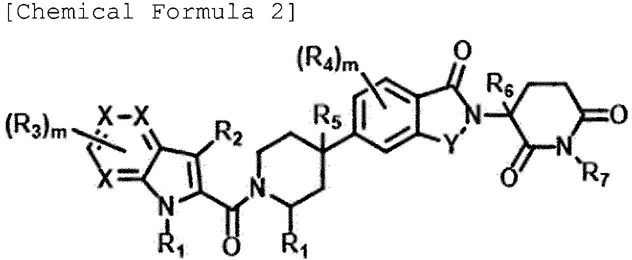 wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 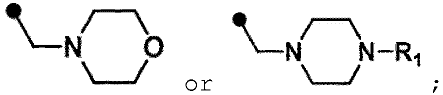
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 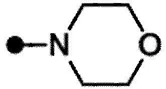 or 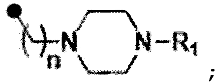
R₄ is independently substituted by a hydrogen atom, halogen, or hydroxy;
R₅ is substituted by a hydrogen atom, deuterium atom, halogen, or hydroxy;
R₆ is substituted by a hydrogen atom or deuterium atom;
R₇ is substituted by a hydrogen atom, C₁₋₅ alkyl or 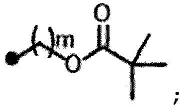
m is an integer of 1 to 3; and
n is independently an integer of 0 or 1.

The present disclosure relates to a compound represented by Chemical Formula 1, a stereoisomer thereof, a solvate thereof, a prodrug thereof, or a pharmaceutically acceptable salt thereof, wherein the Chemical Formula 1 is specified by the following Chemical Formula 3: 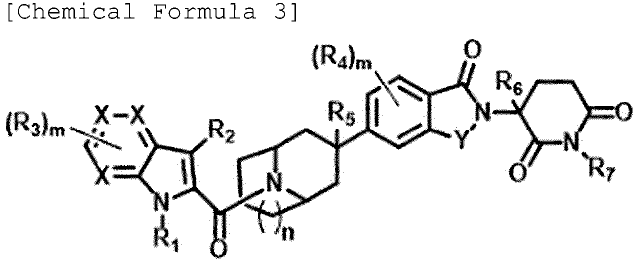 wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 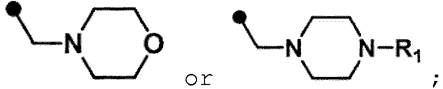
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 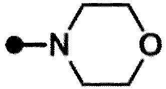 or 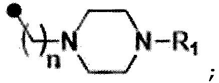
R₄ is independently substituted by a hydrogen atom, halogen, or hydroxy;
R₅ is substituted by a hydrogen atom, deuterium atom, halogen, or hydroxy;
R₆ is substituted by a hydrogen atom or deuterium atom;
R₇ is substituted by a hydrogen atom, C₁₋₅ alkyl or 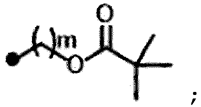
m is an integer of 1 to 3; and
n is independently an integer of 0 or 1.

The present disclosure relates to a compound represented by Chemical Formula 1, a stereoisomer thereof, a solvate thereof, a prodrug thereof, or a pharmaceutically acceptable salt thereof, wherein the Chemical Formula 1 is specified by the following Chemical Formula 4:

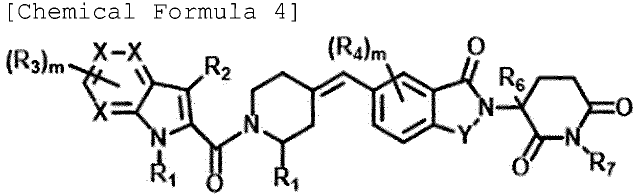

wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 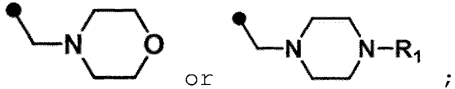
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 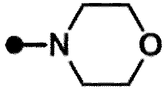 or 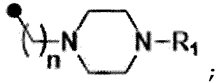
R₄ is independently substituted by a hydrogen atom, halogen, or hydroxy;
R₆ is substituted by a hydrogen atom or deuterium atom;
R₇ is substituted by a hydrogen atom, C₁₋₅ alkyl or 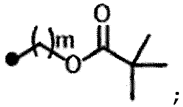
m is an integer of 1 to 3;
n is independently an integer of 0 or 1.

The present disclosure relates to a compound represented by Chemical Formula 1, a stereoisomer thereof, a solvate thereof, a prodrug thereof, or a pharmaceutically acceptable salt thereof, wherein the Chemical Formula 1 is specified by the following Chemical Formula 5:

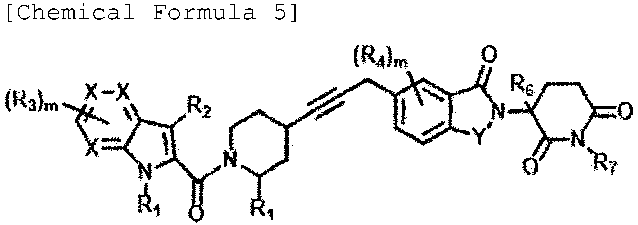

wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 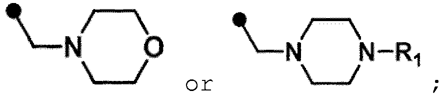
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 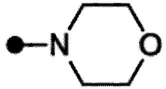 or 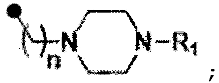
R₄ is independently substituted by a hydrogen atom, halogen, or hydroxy;
R₆ is substituted by a hydrogen atom or deuterium atom;
R₇ is substituted by a hydrogen atom, C₁₋₅ alkyl or 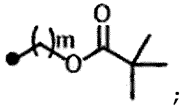
m is an integer of 1 to 3;
n is independently an integer of 0 or 1.

The present disclosure relates to a compound represented by Chemical Formula 1, a stereoisomer thereof, a solvate thereof, a prodrug thereof, or a pharmaceutically acceptable salt thereof, wherein the Chemical Formula 1 is specified by the following Chemical Formula 6: 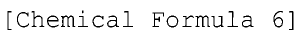 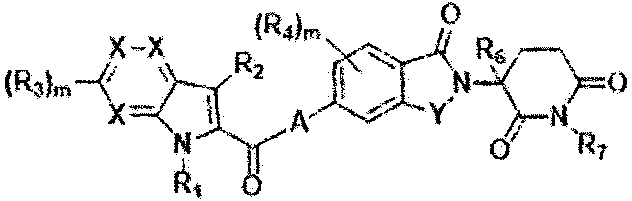 wherein,
X is independently CH, N or NR₃;
Y is -CH₂- or -C(O)-;
R₁ is substituted by a hydrogen atom or C₁₋₅ alkyl;
R₂ is substituted by halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 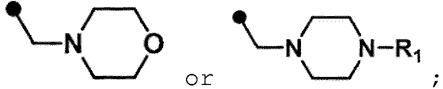
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 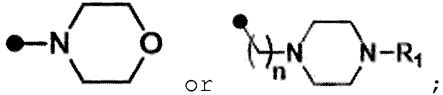
R₄ is independently substituted by a hydrogen atom, halogen, or hydroxy;
A is substituted by 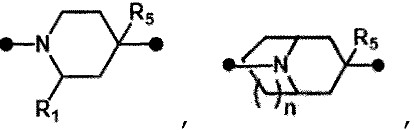 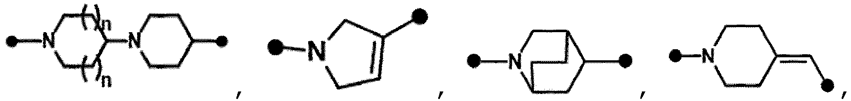 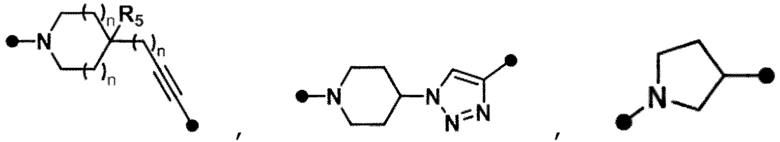 or 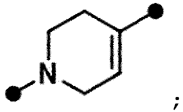
R₅ is substituted by a hydrogen atom, deuterium atom, halogen, or hydroxy;
R₆ is substituted by a hydrogen atom or deuterium atom;
R₇ is substituted by a hydrogen atom, C₁₋₅ alkyl or 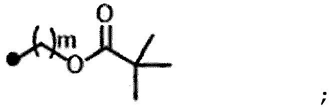
m is an integer of 1 to 3; and
n is independently an integer of 0 or 1.

The present disclosure relates to a compound represented by Chemical Formula 1, a stereoisomer thereof, a solvate thereof, a prodrug thereof, or a pharmaceutically acceptable salt thereof, wherein the Chemical Formula 1 is specified by the following Chemical Formula 6:

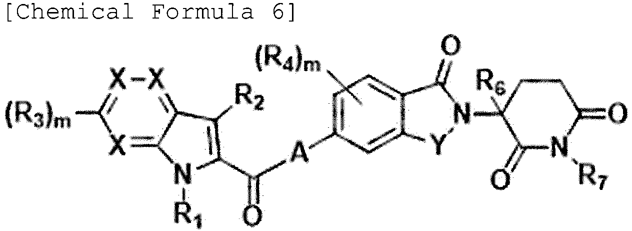

wherein,
X is independently CH, N or NR₃;
Y is -CH₂- or -C(O)-;
R₁ is substituted by a hydrogen atom or C₁₋₅ alkyl;
R₂ is substituted by halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 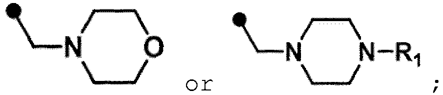
R₃ is independently substituted by halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, -NHCOC₁₋₆ alkyl, -NHCO aryl, -NHSO₂C₁₋₆ alkyl, -NHSO₂ aryl, - NHCONHC₁₋₆ alkyl, -NHCONH aryl, 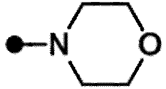 or 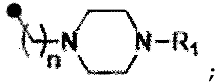
R₄ is independently substituted by a hydrogen atom, halogen, or hydroxy;
A is substituted by 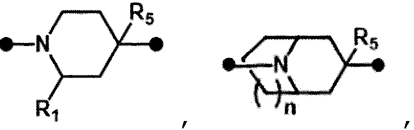 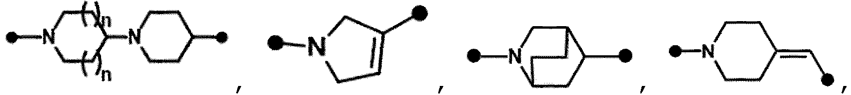 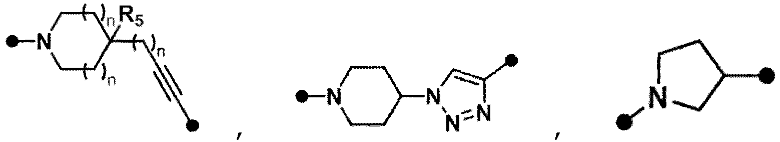 or 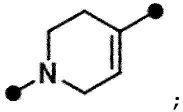
R₅ is substituted by a hydrogen atom, deuterium atom, halogen, or hydroxy;
R₆ is substituted by a hydrogen atom or deuterium atom;
R₇ is substituted by a hydrogen atom, C₁₋₅ alkyl or 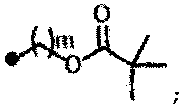
m is an integer of 1 to 3; and
n is independently an integer of 0 or 1.

Concrete examples of the compound of Chemical Formula1 include:
3-(5-(1-(6-chloro-1H-indole-2-carbonyl)piperidin-4-yl)-1-oxoinsoindolin-2-yl)piperidine-2,6-dione (Compound 1);
3-(5-(1-(6,7-dichloro-1H-indole-2-carbonyl)piperidin-4-yl)-1-oxoinsoindolin-2-yl)piperidine-2,6-dione (Compound 2);
3-(5-(1-(5-chloro-1H-indole-2-carbonyl)piperidin-4-yl)-1-oxoinsoindolin-2-yl)piperidine-2,6-dione (Compound 3);
3-(5-(1-(4-chloro-1H-indole-2-carbonyl)piperidin-4-yl)-1-oxoinsoindolin-2-yl)piperidine-2,6-dione (Compound 4);
3-(5-(1-(6-methoxy-1H-indole-2-carbonyl)piperidin-4-yl)-1-oxoinsoindolin-2-yl)piperidine-2,6-dione (Compound 5);
3-(5-(1-(4,6-dichloro-1H-indole-2-carbonyl)piperidin-4-yl)-1-oxoinsoindolin-2-yl)piperidine-2,6-dione (Compound 6);
3-(5-(1-(6-fluoro-1H-indole-2-carbonyl)piperidin-4-yl)-1-oxoinsoindolin-2-yl)piperidine-2,6-dione (Compound 7);
3-(5-(1-(7-chloro-1H-indole-2-carbonyl)piperidin-4-yl)-1-oxoinsoindolin-2-yl)piperidine-2,6-dione (Compound 8);
3-(5-(1-(1H-indole-2-carbonyl)piperidin-4-yl)-1-oxoinsoindolin-2-yl)piperidine-2,6-dione (Compound 9);
3-(5-(1-(5-methoxy-1H-indole-2-carbonyl)piperidin-4-yl)-1-oxoinsoindolin-2-yl)piperidine-2,6-dione (Compound 10);
3-(5-(1-(4-methoxy-1H-indole-2-carbonyl)piperidin-4-yl)-1-oxoinsoindolin-2-yl)piperidine-2,6-dione (Compound 11);
3-(5-(1-(7-methoxy-1H-indole-2-carbonyl)piperidin-4-yl)-1-oxoinsoindolin-2-yl)piperidine-2,6-dione (Compound 12);
3-(5-(1-(5-methyl-1H-indole-2-carbonyl)piperidin-4-yl)-1-oxoinsoindolin-2-yl)piperidine-2,6-dione (Compound 13);
3-(5-(1-(4-methyl-1H-indole-2-carbonyl)piperidin-4-yl)-1-oxoinsoindolin-2-yl)piperidine-2,6-dione (Compound 14);
3-(5-(1-(6-methyl-1H-indole-2-carbonyl)piperidin-4-yl)-1-oxoinsoindolin-2-yl)piperidine-2,6-dione (Compound 15);
3-(5-(1-(7-methyl-1H-indole-2-carbonyl)piperidin-4-yl)-1-oxoinsoindolin-2-yl)piperidine-2,6-dione (Compound 16);
3-(5-(1-(5-fluoro-1H-indole-2-carbonyl)piperidin-4-yl)-1-oxoinsoindolin-2-yl)piperidine-2,6-dione (Compound 17);
3-(5-(1-(6-hydroxy-1H-indole-2-carbonyl)piperidin-4-yl)-1-oxoinsoindolin-2-yl)piperidine-2,6-dione (Compound 18);
3-(5-(1-(7-fluoro-1H-indole-2-carbonyl)piperidin-4-yl)-1-oxoinsoindolin-2-yl)piperidine-2,6-dione (Compound 19);
3-(5-(1-(4-fluoro-1H-indole-2-carbonyl)piperidin-4-yl)-1-oxoinsoindolin-2-yl)piperidine-2,6-dione (Compound 20);
3-(5-(1-(3-methyl-1H-indole-2-carbonyl)piperidin-4-yl)-1-oxoinsoindolin-2-yl)piperidine-2,6-dione (Compound 21);
3-(5-(1-(3-chloro-1H-indole-2-carbonyl)piperidin-4-yl)-1-oxoinsoindolin-2-yl)piperidine-2,6-dione (Compound 22);
3-(5-(3-(1-(1H-indole-3-carbonyl)piperidin-4-yl)prop-1-yn-1-yl)-1-oxoinsoindolin-2-yl)piperidine-2,6-dione (Compound 23);
3-(5-((1-(1H-indole-2-carbonyl)piperidin-4-ylidene)methyl)-1-oxoinsoindolin-2-yl)piperidine-2,6-dione (Compound 24);
3-(5-((1-(1H-indole-3-carbonyl)piperidin-4-ylidene)methyl)-1-oxoinsoindolin-2-yl)piperidine-2,6-dione (Compound 25);
3-(5-((1-(6-chloro-1H-indole-2-carbonyl)piperidin-4-ylidene)methyl)-1-oxoinsoindolin-2-yl)piperidine-2,6-dione (Compound 26);
3-(5-((1-(3-methyl-1H-indole-2-carbonyl)piperidin-4-ylidene)methyl)-1-oxoinsoindolin-2-yl)piperidine-2,6-dione (Compound 27);
3-(5-(1-(1,3-dimethyl-1H-indole-2-carbonyl)piperidin-4-yl)-1-oxoinsoindolin-2-yl)piperidine-2,6-dione (Compound 28);
3-(5-(1-(3-ethyl-1H-indole-2-carbonyl)piperidin-4-yl)-1-oxoinsoindolin-2-yl)piperidine-2,6-dione (Compound 29);
3-(5-(1-(3-methoxy-1H-indole-2-carbonyl)piperidin-4-yl)-1-oxoinsoindolin-2-yl)piperidine-2,6-dione (Compound 30);
3-(5-(1-(3-methyl-1H-picolo[2,3-b]pyridine-2-carbonyl)piperidin-4-yl)-1-oxoinsoindolin-2-yl)piperidine-2,6-dione (Compound 31);
3-(5-(1-(6-amino-1H-indole-2-carbonyl)piperidin-4-yl)-1-oxoinsoindolin-2-yl)piperidine-2,6-dione (Compound 32);
3-(5-(1-(5-bromo-3-methyl-1H-indole-2-carbonyl)piperidin-4-yl)-1-oxoinsoindolin-2-yl)piperidine-2,6-dione (Compound 33);
3-(5-(1-(3-(morpholinomethyl)-1H-indole-2-carbonyl)piperidin-4-yl)-1-oxoinsoindolin-2-yl)piperidine-2,6-dione (Compound 34);
2-(4-(2-(2,6-dioxopiperidin-3-yl)-1-oxoindolin-5-yl)piperidine-1-carbonyl)-1H-indole-3-carbaldehyde (Compound 35);
3-(5-(1-(3-bromo-1H-indole-2-carbonyl)piperidin-4-yl)-1-oxoinsoindolin-2-yl)piperidine-2,6-dione (Compound 36);
3-(5-(1-(3-fluoro-1H-indole-2-carbonyl)piperidin-4-yl)-1-oxoinsoindolin-2-yl)piperidine-2,6-dione (Compound 37);
3-(5-(1-(5-chloro-3-methyl-1H-indole-2-carbonyl)piperidin-4-yl)-1-oxoinsoindolin-2-yl)piperidine-2,6-dione (Compound 38);
3-(5-(1-(6-chloro-3-methyl-1H-indole-2-carbonyl)piperidin-4-yl)-1-oxoinsoindolin-2-yl)piperidine-2,6-dione (Compound 39);
3-(5-(1-(5,6-methoxy-1H-indole-2-carbonyl)piperidin-4-yl)-1-oxoinsoindolin-2-yl)piperidine-2,6-dione (Compound 40);
3-(1-oxo-5-(1-(6-(trifluoromethyl)-1H-indole-2-carbonyl)piperidin-4-yl)isoindolin-2-yl)piperidine-2,6-dione (Compound 41);
3-(5-(1-(6-methoxy-3-methyl-1H-indole-2-carbonyl)piperidin-4-yl)-1-oxoinsoindolin-2-yl)piperidine-2,6-dione (Compound 42);
3-(5-((1-(6-chloro-3-methyl-1H-indole-2-carbonyl)piperidin-4-yl)ethynyl)-1-oxoinsoindolin-2-yl)piperidine-2,6-dione (Compound 43);
3-(5-((1-(6-chloro-3-methyl-1H-indole-2-carbonyl)piperidin-4-ylidene)methyl)-1-oxoinsoindolin-2-yl)piperidine-2,6-dione (Compound 44);
3-(5-(1-(6-chloro-1H-picolo[3,2-b]pyridine-2-carbonyl)piperidin-4-yl)-1-oxoinsoindolin-2-yl)piperidine-2,6-dione (Compound 45);
3-(5-(1-(6-chloro-1H-picolo[3,2-c]pyridine-2-carbonyl)piperidin-4-yl)-1-oxoinsoindolin-2-yl)piperidine-2,6-dione (Compound 46);
3-(5-(1-(6-hydroxy-3-methyl-1H-indole-2-carbonyl)piperidin-4-yl)-1-oxoinsoindolin-2-yl)piperidine-2,6-dione (Compound 47);
3-(5-(1-(3-methyl-6-(trifluoromethyl)-1H-indole-2-carbonyl)piperidin-4-yl)-1-oxoinsoindolin-2-yl)piperidine-2,6-dione (Compound 48);
3-(5-(1-(6-bromo-3-methyl-1H-indole-2-carbonyl)piperidin-4-yl)-1-oxoinsoindolin-2-yl)piperidine-2,6-dione (Compound 49);
3-(5-(1-(6-chloro-1H-picolo[2,3-b]pyridine-2-carbonyl)piperidin-4-yl)-1-oxoinsoindolin-2-yl)piperidine-2,6-dione (Compound 50);
2-(4-(2-(2,6-dioxopiperidin-3-yl)-1-oxoindolin-5-yl)piperidine-1-carbonyl)-1H-indole-3-carbonitrile (Compound 51);
2-(4-(2-(2,6-dioxopiperidin-3-yl)-1-oxoindolin-5-yl)piperidine-1-carbonyl)-3-methyl-1H-indole-6-carbonitrile (Compound 52);
3-(5-(1-(3-methyl-6-(trifluoromethoxy)-1H-indole-2-carbonyl)piperidin-4-yl)-1-oxoinsoindolin-2-yl)piperidine-2,6-dione (Compound 53);
3-(5-(1-(6-fluoro-3-methyl-1H-indole-2-carbonyl)piperidin-4-yl)-1-oxoinsoindolin-2-yl)piperidine-2,6-dione (Compound 54);
3-(5-(1-(3-methyl-6-nitro-1H-indole-2-carbonyl)piperidin-4-yl)-1-oxoinsoindolin-2-yl)piperidine-2,6-dione (Compound 55);
3-(5-(1-(6-(tert-butyl)-3-methyl-1H-indole-2-carbonyl)piperidin-4-yl)-1-oxoinsoindolin-2-yl)piperidine-2,6-dione (Compound 56);
3-(5-(8-(6-chloro-1H-indole-2-carbonyl)-8-azabicyclo[3.2.1]octan-3-yl)-1-oxoinsoindolin-2-yl)piperidine-2,6-dione (Compound 57);
3-(5-(8-(6-chloro-3-methyl-1H-indole-2-carbonyl)-8-azabicyclo[3.2.1]octan-3-yl)-1-oxoinsoindolin-2-yl)piperidine-2,6-dione (Compound 58);
3-(5-(1-(6-chloro-1H-indole-2-carbonyl)-2-methylpiperidin-4-yl)-1-oxoinsoindolin-2-yl)piperidine-2,6-dione (Compound 59);
3-(5-(1-(6-chloro-3-methyl-1H-indole-2-carbonyl)-2-methylpiperidin-4-yl)-1-oxoinsoindolin-2-yl)piperidine-2,6-dione (Compound 60);
3-(5-(1-(6-amino-3-methyl-1H-indole-2-carbonyl)piperidin-4-yl)-1-oxoinsoindolin-2-yl)piperidine-2,6-dione (Compound 61);
3-(5-(1-(6-chloro-3-methyl-1H-indole-2-carbonyl)-[1,4'-bipiperidin]-4-yl)-1-oxoinsoindolin-2-yl)piperidine-2,6-dione (Compound 62);
3-(5-(1-(1-(6-chloro-3-methyl-1H-indole-2-carbonyl)azetidin-3-yl)piperidin-4-yl)-1-oxoinsoindolin-2-yl)piperidine-2,6-dione (Compound 63);
3-(5-(1-(6-chloro-3-methyl-1H-indole-2-carbonyl)-4-hydroxypiperidin-4-yl)-1-oxoinsoindolin-2-yl)piperidine-2,6-dione (Compound 64);
3-(5-(1-(6-chloro-3-methyl-1H-indole-2-carbonyl)-2,5-dihydro-1H-pyrrol-3-yl)-1-oxoinsoindolin-2-yl)piperidine-2,6-dione (Compound 65);
3-(5-(1-(1-(6-chloro-3-methyl-1H-indole-2-carbonyl)piperidin-4-yl)-1H-1,2,3-triazol-4-yl)-1-oxoinsoindolin-2-yl)piperidine-2,6-dione (Compound 66);
3-(5-(1-(6-chloro-3-methyl-1H-indole-2-carbonyl)pyrrolidin-3-yl)-1-oxoinsoindolin-2-yl)piperidine-2,6-dione (Compound 67);
3-(5-((1-(3-methyl-6-nitro-1H-indole-2-carbonyl)piperidin-4-yl)ethynyl)-1-oxoinsoindolin-2-yl)piperidine-2,6-dione (Compound 68);
3-(5-(1-(3,6-dimethyl-1H-indole-2-carbonyl)piperidin-4-yl)-1-oxoinsoindolin-2-yl)piperidine-2,6-dione (Compound 69);
3-(5-(1-(6-chloro-3-methyl-1H-indole-2-carbonyl)-1,2,3,6-tetrahydropyridin-4-yl)-1-oxoinsoindolin-2-yl)piperidine-2,6-dione (Compound 70);
3-(5-(1-(6-chloro-5-methoxy-3-methyl-1H-indole-2-carbonyl)piperidin-4-yl)-1-oxoinsoindolin-2-yl)piperidine-2,6-dione (Compound 71);
3-(5-(1-(5,6-dichloro-3-methyl-1H-indole-2-carbonyl)piperidin-4-yl)-1-oxoinsoindolin-2-yl)piperidine-2,6-dione (Compound 72);
N-(2-(4-(2-(2,6-dioxopiperidin-3-yl)-1-oxoindolin-5-yl)piperidine-1-carbonyl)-3-methyl-1H-indol-6-yl) acetamide (Compound 73);
N-(2-(4-(2-(2,6-dioxopiperidin-3-yl)-1-oxoindolin-5-yl)piperidine-1-carbonyl)-3-methyl-1H-indol-6-yl) methanesulfonamide (Compound 74);
1-(2-(4-(2-(2,6-dioxopiperidin-3-yl)-1-oxoindolin-5-yl)piperidine-1-carbonyl)-3-methyl-1H-indol-6-yl)-3-ethylurea (Compound 75);
3-(5-((1-(6-chloro-3-methyl-1H-indole-2-carbonyl)azetidin-3-yl)ethynyl)-1-oxoinsoindolin-2-yl)piperidine-2,6-dione (Compound 76);
3-(5-((1-(6-chloro-3-methyl-1H-indole-2-carbonyl)-3-hydroxyazetidin-3-yl)ethynyl)-1-oxoinsoindolin-2-yl)piperidine-2,6-dione (Compound 77);
3-(5-((1-(6-chloro-3-methyl-1H-indole-2-carbonyl)pyrrolidin-3-yl)ethynyl)-1-oxoinsoindolin-2-yl)piperidine-2,6-dione (Compound 78);
3-(5-((1-(6-chloro-3-methyl-1H-indole-2-carbonyl)-3-hydroxypyrrolidin-3-yl)ethynyl)-1-oxoinsoindolin-2-yl)piperidine-2,6-dione (Compound 79);
3-(5-((1-(6-chloro-3-methyl-1H-indole-2-carbonyl)-4-hydroxypiperidin-4-yl)ethynyl)-1-oxoinsoindolin-2-yl)piperidine-2,6-dione (Compound 80);
6-chloro-2-(4-(2-(2,6-dioxopiperidin-3-yl)-1-oxoindolin-5-yl)piperidine-1-carbonyl)-1H-indole-3-carbaldehyde (Compound 81);
3-(5-(1-(6-chloro-3-(morpholinomethyl)-1H-indole-2-carbonyl)piperidin-4-yl)-1-oxoinsoindolin-2-yl)piperidine-2,6-dione (Compound 82);
3-(5-(1-(5-bromo-6-chloro-3-methyl-1H-indole-2-carbonyl)piperidin-4-yl)-1-oxoinsoindolin-2-yl)piperidine-2,6-dione (Compound 83);
3-(5-(1-(5-chloro-3-methyl-6-nitro-1H-indole-2-carbonyl)piperidin-4-yl)-1-oxoinsoindolin-2-yl)piperidine-2,6-dione (Compound 84);
3-(5-(1-(3,5-dimethyl-6-nitro-1H-indole-2-carbonyl)piperidin-4-yl)-1-oxoinsoindolin-2-yl)piperidine-2,6-dione (Compound 85);
3-(5-(1-(5-hydroxy-3-methyl-6-nitro-1H-indole-2-carbonyl)piperidin-4-yl)-1-oxoinsoindolin-2-yl)piperidine-2,6-dione (Compound 86);
3-(5-(1-(6-chloro-3-((dimethylamino)methyl)-1H-indole-2-carbonyl)piperidin-4-yl)-1-oxoinsoindolin-2-yl)piperidine-2,6-dione (Compound 87);
3-(5-((1-(6-chloro-3-methyl-1H-indole-2-carbonyl)piperidin-4-yl)ethynyl)-6-fluoro-1-oxoinsoindolin-2-yl)piperidine-2,6-dione (Compound 88);
3-(5-((1-(6-chloro-3-methyl-1H-indole-2-carbonyl)piperidin-4-yl)ethynyl)-4-fluoro-1-oxoinsoindolin-2-yl)piperidine-2,6-dione (Compound 89);
3-(5-(1-(6-chloro-3-methyl-1H-indole-2-carbonyl)piperidin-4-yl)-6-fluoro-1-oxoinsoindolin-2-yl)piperidine-2,6-dione (Compound 90);
3-(5-(1-(6-chloro-3-methyl-1H-indole-2-carbonyl)piperidin-4-yl)-4-fluoro-1-oxoinsoindolin-2-yl)piperidine-2,6-dione (Compound 91);
5-(1-(6-chloro-3-methyl-1H-indole-2-carbonyl)piperidin-4-yl)-2-(2,6-dioxopiperidin-3-yl)isoindoline-1,3-dione (Compound 92);
3-(6-fluoro-5-(1-(3-methyl-6-nitro-1H-indole-2-carbonyl)piperidin-4-yl)-1-oxoinsoindolin-2-yl)piperidine-2,6-dione (Compound 93);
3-(4-fluoro-5-(1-(3-methyl-6-nitro-1H-indole-2-carbonyl)piperidin-4-yl)-1-oxoinsoindolin-2-yl)piperidine-2,6-dione (Compound 94);
2-(2,6-dioxopiperidin-3-yl)-5-(1-(3-methyl-6-nitro-1H-indole-2-carbonyl)piperidin-4-yl)isoindoline-1,3-dione (Compound 95);
5-((1-(6-chloro-3-methyl-1H-indole-2-carbonyl)piperidin-4-yl)ethynyl)-2-(2,6-dioxopiperidin-3-yl)isoindoline-1,3-dione (Compound 96);
3-(5-(1-(5-methoxy-3-methyl-6-nitro-1H-indole-2-carbonyl)piperidin-4-yl)-1-oxoinsoindolin-2-yl)piperidine-2,6-dione (Compound 97);
3-(5-(1-(6-chloro-7-fluoro-3-methyl-1H-indole-2-carbonyl)piperidin-4-yl)-1-oxoinsoindolin-2-yl)piperidine-2,6-dione (Compound 98);
3-(5-(1-(6-chloro-7-fluoro-3-methyl-1H-indole-2-carbonyl)piperidin-4-yl)-1-oxoinsoindolin-2-yl)piperidine-2,6-dione (Compound 99);
3-(5-(1-(6-chloro-3,7-dimethyl-1H-indole-2-carbonyl)piperidin-4-yl)-1-oxoinsoindolin-2-yl)piperidine-2,6-dione (Compound 100);
3-(5-(1-(6-chloro-5-hydroxy-3-methyl-1H-indole-2-carbonyl)piperidin-4-yl)-1-oxoinsoindolin-2-yl)piperidine-2,6-dione (Compound 101);
3-(5-(1-(6-chloro-5-fluoro-3-methyl-1H-indole-2-carbonyl)piperidin-4-yl)-1-oxoinsoindolin-2-yl)piperidine-2,6-dione (Compound 102);
3-(5-(1-(6-chloro-3,5-dimethyl-1H-indole-2-carbonyl)piperidin-4-yl)-1-oxoinsoindolin-2-yl)piperidine-2,6-dione (Compound 103);
3-(5-(1-(3-methyl-6-morpholino-1H-indole-2-carbonyl)piperidin-4-yl)-1-oxoinsoindolin-2-yl)piperidine-2,6-dione (Compound 104);
3-(5-(1-(6-chloro-3-(hydroxymethyl)-1H-indole-2-carbonyl)piperidin-4-yl)-1-oxoinsoindolin-2-yl)piperidine-2,6-dione (Compound 105);
3-(5-(1-(3-methyl-6-(piperazin-1-ylmethyl)-1H-indole-2-carbonyl)piperidin-4-yl)-1-oxoinsoindolin-2-yl)piperidine-2,6-dione chloride (Compound 106);
3-(5-(1-(4,6-dichloro-3-methyl-1H-indole-2-carbonyl)piperidin-4-yl)-1-oxoinsoindolin-2-yl)piperidine-2,6-dione (Compound 107);
3-(5-(1-(6-chloro-5-methoxy-3-methyl-1H-indole-2-carbonyl)piperidin-4-yl)-4-fluoro-1-oxoinsoindolin-2-yl)piperidine-2,6-dione (Compound 108);
3-(5-(1-(6-chloro-3-methyl-5-(piperazin-1-ylmethyl)-1H-indole-2-carbonyl)piperidin-4-yl)-1-oxoinsoindolin-2-yl)piperidine-2,6-dione dichloride (Compound 109);
3-(5-(1-(6-methoxy-3-(morpholinomethyl)-1H-indole-2-carbonyl)piperidin-4-yl)-1-oxoinsoindolin-2-yl)piperidine-2,6-dione (Compound 110);
3-(5-(1-(6-methoxy-3-(morpholinomethyl)-1H-indole-2-carbonyl)piperidin-4-yl)-1-oxoinsoindolin-2-yl)piperidine-2,6-dione (Compound 111);
3-(5-(1-(6-chloro-5-fluoro-3-methyl-1H-indole-2-carbonyl)piperidin-4-yl)-6-fluoro-1-oxoinsoindolin-2-yl)piperidine-2,6-dione (Compound 112);
3-(5-(1-(6-chloro-5-fluoro-3-methyl-1H-indole-2-carbonyl)piperidin-4-yl)-4-fluoro-1-oxoinsoindolin-2-yl)piperidine-2,6-dione (Compound 113);
3-(5-(1-(6-chloro-3-((4-methylpiperazin-1-yl)methyl)-1H-indole-2-carbonyl)piperidin-4-yl)-1-oxoinsoindolin-2-yl )piperidine-2,6-dione (Compound 114);
3-(5-(1-(6-chloro-5-hydroxy-3-methyl-1H-indole-2-carbonyl)piperidin-4-yl)-4-fluoro-1-oxoinsoindolin-2-yl)piperidine-2,6-dione (Compound 115);
3-(4-fluoro-5-(1-(3-methyl-6-morpholino-1H-indole-2-carbonyl)piperidin-4-yl)-1-oxoinsoindolin-2-yl)piperidine-2,6-dione (Compound 116);
3-(5-(1-(6-chloro-7-fluoro-3-methyl-1H-indole-2-carbonyl)piperidin-4-yl)-4-fluoro-1-oxoinsoindolin-2-yl)piperidine-2,6-dione (Compound 117);
2-(4-(2-(2,6-dioxopiperidin-3-yl)-4-fluoro-1-oxoindolin-5-yl)piperidine-1-carbonyl)-3-methyl-1H-indole-6-carbonitrile (Compound 118);
6-chloro-2-(4-(2-(2,6-dioxopiperidin-3-yl)-4-fluoro-1-oxoindolin-5-yl)piperidine-1-carbonyl)-3-methyl-1H-indole-5-carbonitrile (Compound 119);
2-(4-(2-(2,6-dioxopiperidin-3-yl)-4-fluoro-1-oxoindolin-5-yl)piperidine-1-carbonyl)-5-fluoro-3-methyl-1H-indole-6-carbonitrile (Compound 120);
5-(1-(6-chloro-3-methyl-1H-indole-2-carbonyl)piperidin-4-yl)-2-(2,6-dioxopiperidin-3-yl)-4-fluoroisoindoline-1,3-dione (Compound 121);
2-(4-(2-(2,6-dioxopiperidin-3-yl)-4-fluoro-1-oxoindolin-5-yl)piperidine-1-carbonyl)-7-fluoro-3-methyl-1H-indole-6-carbonitrile (Compound 122);
(3-(5-(1-(6-chloro-3-methyl-1H-indole-2-carbonyl)piperidin-4-yl)-4-fluoro-1-oxoinsoindolin-2-yl)-2,6-dioxopiperidin-1-yl)methyl pivalate (Compound 123);
3-(4-fluoro-5-(1-(7-fluoro-3-methyl-6-morpholino-1H-indole-2-carbonyl)piperidin-4-yl)-1-oxoinsoindolin-2-yl)piperidine-2,6-dione (Compound 124);
3-(4-fluoro-5-(1-(5-fluoro-3-methyl-6-morpholino-1H-indole-2-carbonyl)piperidin-4-yl)-1-oxoinsoindolin-2-yl)piperidine-2,6-dione (Compound 125);
3-(4-fluoro-5-(1-(7-fluoro-3-methyl-6-(4-methylpiperazin-1-yl)-1H-indole-2-carbonyl)piperidin-4-yl)-1-oxoinsoindolin-2-yl)piperidine-2,6-dione (Compound 126); and
3-(4-fluoro-5-(1-(5-fluoro-3-methyl-6-(4-methylpiperazin-1-yl)-1H-indole-2-carbonyl)piperidin-4-yl)-1-oxoinsoindolin-2-yl)piperidine-2,6-dione (Compound 127); stereoisomers thereof, solvates thereof, prodrugs thereof, or pharmaceutically acceptable salts thereof.

The present disclosure relates to a pharmaceutical composition for the treatment, prevention, suppression, or elimination of an IKZF2-dependent disease or disorder, including as an active ingredient a compound represented by Chemical Formula 1, a stereoisomer thereof, a solvate thereof, a prodrug thereof, or a pharmaceutically acceptable salt thereof.

The present disclosure also relates to a pharmaceutical composition for the prevention or treatment of a solid tumor or hematological cancer, which is an IKZF2-dependent disease, including as an active ingredient a compound represented by Chemical Formula 1, a stereoisomer thereof, a solvate thereof, a prodrug thereof, or a pharmaceutically acceptable salt thereof.

The solid tumor may be selected from the group consisting of non-small cell lung cancer (NSCLC), melanoma, triple-negative breast cancer (TNBC), nasopharyngeal carcinoma (NPC), microsatellite stable colorectal cancer (MSS CRC), thymoma, carcinoid, gastrointestinal stromal tumor (GIST), prostate cancer, breast carcinoma, lymphoma, leukemia, melanoma, bladder carcinoma, colon cancer, cutaneous melanoma, hepatocellular carcinoma, endometrial cancer, ovarian cancer, cervical cancer, lung cancer, renal cancer, glioblastoma multiforme, glioma, thyroid cancer, parathyroid cancer, nasopharyngeal cancer, tongue cancer, pancreatic cancer, esophageal cancer, cholangiocarcinoma, gastric cancer, soft tissue sarcoma, rhabdomyosarcoma (RMS), synovial sarcoma, osteosarcoma, sarcomatoid carcinoma, and Ewing's sarcoma, but is not limited thereto.

The hematological cancer may be selected from the group consisting of acute myeloid leukemia (AML), chronic myeloid leukemia (CML), Bcr-ABL translocation-related CML, myelodysplastic syndrome (MDS), acute B-lymphoblastic leukemia (B-ALL), acute T-lymphoblastic leukemia (T-ALL), chronic lymphocytic leukemia (CLL), multiple myeloma (MM), myeloproliferative neoplasms (MPN), Richter's syndrome, hairy cell leukemia (HCL), blastic plasmacytoid dendritic cell neoplasm (BPDCN), non-Hodgkin's lymphoma (NHL), mantle cell lymphoma (MCL), small lymphocytic lymphoma (SLL), Hodgkin's lymphoma, systemic mastocytosis, and Burkitt's lymphoma, but is not limited thereto.

The compound represented by Chemical Formula 1 of the present disclosure, a stereoisomer thereof, a solvate thereof, a prodrug thereof, or a pharmaceutically acceptable salt thereof may enhance the therapeutic effect of cancer treatment when used in combination with an anticancer agent. The anticancer agent for use in the combination therapy may be selected from the group consisting of taxane-based anticancer agents, antitumor alkylating agents, antitumor antimetabolites, antitumor antibiotics, plant-derived antitumor agents, antitumor platinum complexes, antitumor camptothecin derivatives, antitumor kinase inhibitors, antitumor antibodies, hormone-based antitumor agents, antitumor virotherapeutics, and angiogenesis inhibitors.

The taxane-based anticancer agents may be selected from paclitaxel, docetaxel, cabazitaxel, larotaxel, BMS-184476, BMS-188797, BMS275183, milataxel, ortataxel, TL-310, docosahexaenoic acid-paclitaxel (DHA-paclitaxel), nab-paclitaxel, EndoTAG+paclitaxel, XRP9881, polymer-micelle paclitaxel, or RPR-109881A. The antitumor alkylating agents may be selected from nitrogen mustard N-oxide, cyclophosphamide, ifosfamide, melphalan, busulfan, mitobronitol, carbazilquinone, thiotepa, ranimustine, nimustine, temozolomide, or carmustine. The antitumor antimetabolites may be selected from methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, or pemetrexed disodium. The antitumor antibiotics may be selected from actinomycin D, doxorubicin, daunorubicin, neocarcinostatin, bleomycin, peplomycin, mitomycin C, aclarubicin, pirarubicin, epirubicin, zinostatin stimalamer, idarubicin, sirolimus, or valrubicin. The plant-derived antitumor agents may be selected from vincristine, vinblastine, vindesine, etoposide, sobuzoxane, docetaxel, paclitaxel, or vinorelbine. The antitumor platinum complexes may be selected from cisplatin, carboplatin, nedaplatin, or oxaliplatin. The antitumor camptothecin derivatives may be selected from irinotecan, topotecan, or camptothecin. The antitumor kinase inhibitors may be selected from gefitinib, imatinib, or erlotinib. The antitumor antibodies may be selected from cetuximab, bevacizumab, rituximab, bevacizumab, alemtuzumab, or trastuzumab. The hormone-based antitumor agents may be selected from goserelin, leuprolide, or tamoxifen. The antitumor virotherapeutic may be Imlygic, and the angiogenesis inhibitors may be selected from Avastin, bevacizumab, ramucirumab, aflibercept, cetuximab, panitumumab, regorafenib, sunitinib, sorafenib, pazopanib, vandetanib, axitinib, cediranib, vatalanib, motesanib, tucatinib, nintedanib, semaxanib, apatinib, lenvatinib, cabozantinib, or combinations thereof, but are not limited thereto.

The anticancer agent usable in the combination therapy may be one or more selected from the group consisting of paclitaxel, docetaxel, cabazitaxel, larotaxel, BMS-184476, BMS-188797, BMS275183, milataxel, ortataxel, TL-310, DHA-paclitaxel, nab-paclitaxel, EndoTAG+paclitaxel, XRP9881, polymer-micelle paclitaxel, RPR-109881A, nitrogen mustard N-oxide, cyclophosphamide, ifosfamide, melphalan, busulfan, mitobronitol, carbazilquinone, thiotepa, ranimustine, nimustine, temozolomide, carmustine, methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, pemetrexed disodium, actinomycin D, doxorubicin, daunorubicin, neocarcinostatin, bleomycin, peplomycin, mitomycin C, aclarubicin, pirarubicin, epirubicin, zinostatin stimalamer, idarubicin, sirolimus, valrubicin, vincristine, vinblastine, vindesine, etoposide, sobuzoxane, docetaxel, paclitaxel, vinorelbine, cisplatin, carboplatin, nedaplatin, oxaliplatin, irinotecan, topotecan, camptothecin, gefitinib, imatinib, erlotinib, cetuximab, bevacizumab, rituximab, bevacizumab, alemtuzumab, trastuzumab, goserelin, leuprolide, tamoxifen, Imlygic, Avastin, bevacizumab, ramucirumab, aflibercept, cetuximab, panitumumab, regorafenib, sunitinib, sorafenib, pazopanib, vandetanib, axitinib, cediranib, vatalanib, motesanib, tucatinib, nintedanib, semaxanib, apatinib, lenvatinib, and cabozantinib.

The compound represented by Chemical Formula 1 of the present disclosure, a stereoisomer thereof, a solvate thereof, a prodrug thereof, or a pharmaceutically acceptable salt thereof may also enhance the therapeutic effect of cancer treatment when used in combination therapy with one or more immune cells or immunomodulators selected from PD-1 inhibitors, PD-L1 inhibitors, LAG-3 inhibitors, CTLA-4 antagonists, A2A antagonists, GITR agonists, TIM-3 inhibitors, STING agonists, and TLR7 agonists.

The PD-1 inhibitors may be selected from the group consisting of nivolumab (BMS), pembrolizumab (Merck), BCD-100 (BIOCAD), cemiplimab (Regeneron Pharmaceuticals Inc.), sintilimab (Eli Lilly/InnoCare Biologics Inc.'s IBI-308), spartalizumab (Novartis AG's PDR-001), camrelizumab (Incyte Corporation/Jiangsu Hengrui's SHR-1210), tislelizumab (Beigene Ltd.), AGEN-2034 (Agenus Inc.), MEDI-0680 (AMP-514; Amplimmune/MedImmune LLC), toripalimab (Shanghai Junshi Biosciences Co., Ltd.'s JS-001), dostarlimab (Tesaro Inc.'s TSR-042), ABBV-181 (AbbVie Inc.), AK-104 (Akeso Biopharma Inc.), AK-105 (Akeso Biopharma Inc.), BAT-1306 (Bio-Thera Solutions Ltd.), BI754091 (Boehringer Ingelheim GmbH), CBT-501, xenolimab (CBT Pharmaceuticals Inc./Xeno), GLS-010 (Harbin Gloria/WuXi/Arcus), LZM-009 (Livzon Pharmaceutical Group Inc.), MGA-012 (Incyte Corporation/MacroGenics), MGD-013 (MacroGenics Inc.), PF-06801591 (Pfizer Inc.), Sym-021 (Symphogen A/S), CS-1003 (CStone Pharmaceuticals Co., Ltd.), HLX-10 (Henlius Biotech/Shanghai Henlius Biotech Co., Ltd.), AK-103 (Akeso Biopharma Inc.), AM-0001 (Armo Biosciences Inc.), TILT-123 (TILT Biotherapeutics Ltd.), BH-2922 (Beijing Hanmi Pharmaceutical), BH-2941 (Beijing Hanmi Pharmaceutical), BH-2950 (Beijing Hanmi Pharmaceutical), CX-188 (CytomX Therapeutics Inc.), ENUM244C8 (Enumeral Biomedical Holdings), ENUM-388D4 (Enumeral Biomedical Holdings), HAB-21 (Suzhou Steinway Biotech Inc.), HEISCOIII-003 (Sichuan Haisco Pharmaceutical), IKT-202 (Icelle K.Alex Therapeutics), JS-003 (Shanghai Junshi Biosciences), JTX-4014 (Jounce Therapeutics Inc.), MCLA-134 (Merus NV), MGD-019 (MacroGenics Inc.), MT-17000 (Molecular Templates Inc.), PEGMP-7 (D5 Pharma Inc.), PRS-332 (Pieris Pharmaceuticals Inc.), RXI-762 (RXi Pharmaceuticals Corporation), STI-1110 (Les Laboratoires Servier/Sorrento), VXM-10 (Vaximm AG), XmAb-20717 (Xencor Inc.), XmAb-23104 (Xencor Inc.), AK-112 (Akeso Biopharma Inc.), HLX-20 (Henlius Biotech/Shanghai Henlius Biotech Co., Ltd.), SSI-361 (Livzon Biopharma Ltd.), AT-16201 (AIMM Therapeutics BV), and SNA-01 (Fountain Biopharma Inc.).

The PD-L1 inhibitors may be selected from the group consisting of atezolizumab (Genentech Inc.), avelumab (Merck KGaA/Pfizer), durvalumab (AstraZeneca Pharmaceuticals LP/MedImmune), BGB-A333 (Beigene Ltd.), CX-072 (CytomX Therapeutics Inc.), GNS-1480 (Yuhan Corporation/Genosco), AMP-224 (MedImmune LLC), CA-170 (Curis Inc./Origin), CK-301 (Checkpoint Therapeutics/TG Therapeutics), CS-1001 (CStone Pharmaceuticals Co., Ltd.), FAZ-053 (Novartis AG), envafolimab (ASC22 or KN-035; Suzhou Alphamab/3DMed), LY-3300054 (Eli Lilly and Company), M-7824 (Merck KGaA), HTI-1088 (HTI-131, SHR-1316; Atria and Jiangsu Hengrui Medicine Co.), MSB-2311 (Mapspace Biosciences (Suzhou) Co., Ltd.), STI-A1014 (Lee's Pharmaceutical/Sorrento), AK106 (Akeso Biopharma Inc.), AVA-004 (Avacta Life Sciences Ltd.), BBI-801 (Boston Biomedical Inc.), CA-327 (Origin/Curis), CBA-0710 (Sorrento Therapeutics Inc.), CBT-502 (CBT Pharmaceuticals/Chiatai Tianqing Pharmaceutical), FPT-155 (Five Prime Therapeutics Inc.), FS-118 (F-star Biotechnology Ltd.), IKT-201 (Icelle K.Alex Therapeutics), IKT-703 (Icelle K.Alex Therapeutics), IO-103 (IO Biotech ApS), JS-003 (Shanghai Junshi Biosciences), KD-033 (Cardamon Corp./Zhenghua Pharmaceutical), KY-1003 (Kymab Ltd.), MCLA-145 (Merus NV/Incyte), MT-5050 (Molecular Templates Inc.), and SNA-02 (Fountain Biopharma Inc.).

The CTLA-4 antagonists may be selected from the group consisting of ipilimumab (BMS), tremelimumab (AstraZeneca), and MEDI5752 (a bispecific antibody simultaneously targeting PD-1 and CTLA-4).

The compound represented by Chemical Formula 1 of the present disclosure, a stereoisomer thereof, a solvate thereof, a prodrug thereof, or a pharmaceutically acceptable salt thereof may be administered simultaneously (as a single formulation or as separate formulations), sequentially, individually, or over a defined period, in combination with other therapeutic agents or treatment modalities. In general, combination therapy anticipates the administration of two or more agents within a single cycle or course of treatment. The therapeutic agents may be, for example, chemical compounds, peptides, antibodies, antibody fragments, or nucleic acids that exhibit therapeutic activity or enhance therapeutic activity when administered to a subject in combination with the compound of Chemical Formula 1 of the present disclosure.

The compound represented by Chemical Formula 1 of the present disclosure, a stereoisomer thereof, a solvate thereof, a prodrug thereof, or a pharmaceutically acceptable salt thereof may be used in combination with one or more anti-HER₂ antibodies, such as trastuzumab, pertuzumab, margetuximab, or the aforementioned HT-19, or with other anti-HER₂ conjugates, such as ado-trastuzumab emtansine (also known as Kadcyla^{®} or T-DM1). In another embodiment, the compound represented by Chemical Formula 1 of the present disclosure, a stereoisomer thereof, a solvate thereof, a prodrug thereof, or a pharmaceutically acceptable salt thereof may be used in combination with one or more tyrosine kinase inhibitors, including, but not to limited to, EFGR inhibitors, Her3 inhibitors, IGFR inhibitors, and Met inhibitors, for treating a disease such as cancer. The tyrosine kinase inhibitors may include, but are not limited to, erlotinib hydrochloride (Tarceva^{®}); linifanib (N-[4-(3-amino-1H-indazol-4-yl)phenyl]-N'-(2-fluoro-5-methylphenyl)urea, also known as ABT-869); sunitinib malate (Sutent^{®}); bosutinib (4-[(2,4-dichloro-5-methoxyphenyl)amino]-6-methoxy-7-[3-(4-methylpiperazin-1-yl)propoxy]quinoline-3-carbonitrile, also known as SKI-606); dasatinib (Sprycel^{®}); pazopanib (Votrient^{®}); sorafenib (Nexavar^{®}); Zactima (ZD6474); and imatinib or imatinib mesylate (Gilvec^{®} or Gleevec^{®}). Epidermal growth factor receptor (EGFR) inhibitors may include, but are not limited to, erlotinib hydrochloride (Tarceva^{®}), gefitinib (Iressa^{®}); N-[4-[ (3-chloro-4-fluorophenyl)amino]-7-[[ (3"s")-tetrahydro-3-furanyl]oxy]-6-quinazolinyl]-4-(dimethylamino)-2-butenamide (Tovok^{®}); vandetanib (Caprelsa^{®}); lapatinib (Tykerb^{®}); (3R,4R)-4-amino-1-((4-((3-methoxyphenyl)amino)pyrazolo[2,1-f][1,2,4]triazine-5-yl)methyl)piperidin-3-ol (BMS690514); canertinib dihydrochloride (CI-1033); 6-[4-[(4-ethyl-1-piperazinyl)methyl]phenyl]-N-[ (1R)-1-phenylethyl]-7H-pyrrolo[2,3-d]pyrimidin-4-amine (AEE788, CAS 497839-62-0); mubritinib (TAK165); pelitinib (EKB569); afatinib (Gilotrif^{®}); neratinib (HKI272); N-[4-[[1-[ (3-fluorophenyl)methyl]-1H-indazol-5-yl]amino]-5-methylpyrazolo[2,1-f][1,2,4]triazine-6-yl]carbamic acid, (3S)-3-morpholinylmethyl ester (BMS599626); N-(3,4-dichloro-2-fluorophenyl)-6-methoxy-7-[[ (3aα,5β,6aα)-octahydro-2-methylcyclopenta[c]pyrrol-5-yl]methoxy]-4-quinazolinamine (XL647, CAS 781613-23-8); and 4-[4-[[(1R)-1-phenylethyl]amino]-7H-pyrrolo[2,3-d]pyrimidin-6-yl]phenol (PKI166, CAS 187724-61-4). EGFR antibodies may include, but are not limited to, cetuximab (Erbitux^{®}); panitumumab (Vectibix^{®}); matuzumab (EMD-72000); nimotuzumab (hR3); zalutumumab; TheraCIM h-R3; MDX0447 (CAS 339151-96-1); and ch806 (mAb-806, CAS 946414-09-1).

Unless otherwise indicated, the following terms used in the present disclosure have the meanings set forth below. Any undefined terms shall be interpreted as having the meanings commonly understood by those skilled in the art.

The term "halo" or "halogen" as used in the present disclosure refers to fluorine (F), chlorine (Cl), bromine (Br), and iodine (I).

The term "alkyl" as used in the present disclosure refers to a straight or branched saturated hydrocarbon group having a single bond. Examples include, but are not limited to, methyl, ethyl, and propyl.

The term "alkoxy" as used in the present disclosure refers to an oxygen atom bonded to a straight or branched saturated hydrocarbon group having a single bond. Examples include, but are not limited to, methoxy, ethoxy, and propoxy.

The term "halo alkyl" as used in the present disclosure refers to a substituted alkyl group as described above, in which one or more hydrogen atoms on the alkyl group are replaced with halo groups. Examples include, but are not limited to, trifluoromethyl, difluoromethyl, and trifluoroethyl.

The term "halo alkoxy" as used in the present disclosure refers to a group of the structure alkyl-O-, in which one or more hydrogen atoms on the alkyl group are replaced with halo groups. Examples include, but are not limited to, trifluoromethoxy.

The term "aryl" as used in the present disclosure refers to an aromatic ring compound, and includes, but is not limited to, phenyl, naphthalene, and anthracene.

The compound represented by Chemical Formula 1 of the present disclosure may include not only pharmaceutically acceptable salts, but also any salts, solvates, stereoisomers, and prodrugs that can be prepared by conventional methods.

The term "pharmaceutically acceptable" means that a substance or composition must be chemically and/or toxicologically suitable for use with other components of the formulation and/or with a mammal to be treated.

The term "hydrate" refers to a solvate in which the solvent molecule (s) is/are water.

The term "prodrug" refers to a compound that is converted in vivo into the disclosed compound via metabolic means (e.g., hydrolysis). Generally, such prodrugs have metabolically cleavable moieties and are rapidly converted in vivo, for example by hydrolysis in the bloodstream, to yield the parent compound. Prodrugs typically include ester or amide analogs of the parent compound. Prodrugs may be formulated for improved chemical stability, enhanced patient acceptability and compliance, increased bioavailability, prolonged duration of action, improved organ selectivity, improved formulation properties (e.g., increased solubility), and/or reduced side effects (e.g., toxicity). Generally, the prodrug itself has little or no biological activity and is stable under standard conditions.

The term "solvate" refers to a complex of various stoichiometries formed by a solute (e.g., a compound of Chemical Formula I' or Chemical Formula I) and a solvent (e.g., water, ethanol, or acetic acid). This physical association may involve ionic and covalent bonding to varying degrees, including hydrogen bonding. In specific instances, such as when one or more solvent molecules are incorporated into the crystal lattice of a crystalline solid, the solvate can be isolated. In general, for the purposes of the present disclosure, the selected solvent does not interfere with the biological activity of the solute. The term "solvate" includes both solution-phase and isolable solvates. Representative solvates include hydrates, ethanolate, methanolate, and the like.

The term "stereoisomer" includes mirror image isomers, partial stereoisomers, and cis/trans isomers generated from the above-described processes. Depending on their separation properties, they can be separated into individual components using methods such as chiral salt formation or chromatography employing normal-phase, reverse-phase, or chiral columns.

Any racemates of the compounds or intermediates of the present disclosure may be resolved into their optical enantiomers by known methods, for example, by separating the salts of the partial stereoisomers obtained using optically active acids or bases, and by liberating the optically active acid or base compound. Accordingly, in particular, a basic moiety of the present disclosure may be resolved into optical enantiomers by fractional crystallization of salts formed with optically active acids such as tartaric acid, dibenzoyltartaric acid, diacetyl tartaric acid, di-O,O'-p-toluoyltartaric acid, mandelic acid, malic acid, or camphor-10-sulfonic acid. In addition, racemic compounds or racemic intermediates of the present disclosure may be resolved by chiral chromatography, for example, by high-performance liquid chromatography (HPLC) using a chiral adsorbent. Any mixtures of stereoisomers thus generated may be separated into pure or substantially pure geometric or optical isomers, partial stereoisomers, or racemates based on physicochemical differences in the components, for example, by chromatography and/or fractional crystallization. All such compounds and stereoisomers are within the scope of the present disclosure.

The pharmaceutically acceptable salt in the present disclosure refers to a salt or complex of the compound of Chemical Formula 1 exhibiting desirable biological activity. Examples of such salts include, but are not limited to, acid addition salts formed with inorganic acids [for example, hydrochloric acid, hydrobromic acid, sulfuric acid, phosphoric acid, nitric acid, etc.] and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and polygalacturonic acid. The compound may also be administered as pharmaceutically acceptable quaternary salts known to those skilled in the art, including, in particular, chloride, bromide, iodide, -O- alkyl, toluenesulfonate, methanesulfonate, sulfonate, phosphate, or carboxylate (e.g., benzoate, succinate, acetate, glycolate, maleate, malate, fumarate, citrate, tartrate, ascorbate, cinnamoate, mandeloate, and diphenylacetate).

The acid addition salts according to the present disclosure may be prepared by conventional methods, for example, by dissolving a derivative of Chemical Formula 1 in an organic solvent such as methanol, ethanol, acetone, dichloromethane, or acetonitrile, adding an organic or inorganic acid thereto, filtering the resulting precipitate, and drying the product; or by distilling off the solvent and excess acid under reduced pressure and subsequently crystallizing the product from an organic solvent.

In addition, pharmaceutically acceptable metal salts may be prepared using a base. Alkali metal or alkaline earth metal salts may be prepared, for example, by dissolving the compound in an excess of alkali metal hydroxide or alkaline earth metal hydroxide solution, filtering the resulting insoluble salt, and evaporating and drying the filtrate. In this case, sodium, potassium, or calcium salts are pharmaceutically preferred. The corresponding salts may also be obtained by reacting alkali metal or alkaline earth metal salts with appropriate silver salts (e.g., silver nitrate).

The pharmaceutical composition according to the present disclosure may be formulated into a suitable form together with pharmaceutically acceptable carriers commonly used in the art. The term "pharmaceutically acceptable" refers to a composition that is physiologically acceptable and does not typically cause allergic reactions or similar responses, such as gastrointestinal disorders or dizziness, when administered to humans. The composition may be formulated, according to conventional methods, into oral dosage forms such as powders, granules, tablets, capsules, suspensions, emulsions, syrups, or aerosols, as well as external preparations, suppositories, or sterile injectable solutions.

The carriers, excipients, and diluents that may be included in the composition may include, but are not limited to, lactose, dextrose, sucrose, sorbitol, mannitol, xylitol, erythritol, maltitol, starch, gum arabic, alginate, gelatin, calcium phosphate, calcium silicate, cellulose, methylcellulose, microcrystalline cellulose, polyvinylpyrrolidone, water, methylparaben, propylparaben, talc, magnesium stearate, and mineral oil. For formulation, commonly used diluents or excipients such as fillers, stabilizers, binders, disintegrants, and surfactants may be used. Solid dosage forms for oral administration include tablets, pills, powders, granules, and capsules, which may be prepared by mixing the compound of the present disclosure with at least one excipient such as starch, microcrystalline cellulose, sucrose or lactose, low-substituted hydroxypropylcellulose, or hypromellose. In addition to simple excipients, lubricants such as magnesium stearate and talc may also be used. Liquid formulations for oral administration include suspensions, internal solutions, emulsions, and syrups, and may include, in addition to commonly used simple diluents such as water and liquid paraffin, various excipients such as wetting agents, sweeteners, flavoring agents, and preservatives. Parenteral formulations may include sterile aqueous solutions, non-aqueous solvents, suspensions, emulsions, lyophilized preparations, and suppositories. Non-aqueous solvents or suspending agents may include propylene glycol, polyethylene glycol, vegetable oils such as olive oil, and injectable esters such as ethyl oleate. Suppository bases may include witepsol, macrogol, Tween 61, cacao butter, laurin butter, glycerol, and gelatin. To formulate for parenteral administration, the compound of Chemical Formula 1 or a pharmaceutically acceptable salt thereof may be mixed with water together with sterilizing agents and/or preservatives, stabilizers, hydration agents, emulsifying agents, salts for osmotic control and/or buffering agents, and other therapeutically useful substances to prepare a solution or suspension, which can be provided in unit dosage forms such as ampoules or vials.

The pharmaceutical composition provides a pharmaceutical composition comprising the compound of Chemical Formula 1 and an excipient. Preferably, the compound may be included in an amount of 0.001 wt% to 50 wt%, more preferably 0.001 wt% to 40 wt%, and most preferably 0.001 wt% to 30 wt% based on the total weight of the composition.

The pharmaceutical composition comprising the compound of Chemical Formula 1 as an active ingredient may be administered via various routes to mammals such as mice, livestock, or humans. All routes of administration may be anticipated, for example, oral, rectal, or intravenous, intramuscular, subcutaneous, intraperitoneal, or intracerebroventricular injection. The dosage may vary depending on the age, sex, weight of the subject to be treated, specific disease or pathological condition to be treated, severity of the disease or condition, timing and route of administration, absorption, distribution, and excretion rate of the drug, type of other drugs used, and the discretion of the prescriber. Determination of such dosage based on these factors is within the scope of those skilled in the art. In general, the dosage may range from 0.01 mg/kg/day to about 2000 mg/kg/day. A more preferable dosage range is from 1 mg/kg/day to 500 mg/kg/day. Administration may be performed once a day or divided into multiple doses per day. The dosage mentioned above is not intended to limit the scope of the present disclosure in any way.

### Advantageous Effects of Invention

The compound of Chemical Formula 1 according to the present disclosure provides an agent having a novel mechanism of action against the IKZF2 protein and is useful for the treatment of diseases and disorders associated with the IKZF2 protein, particularly for the prevention or treatment of cancer.

### Brief Description of Drawings

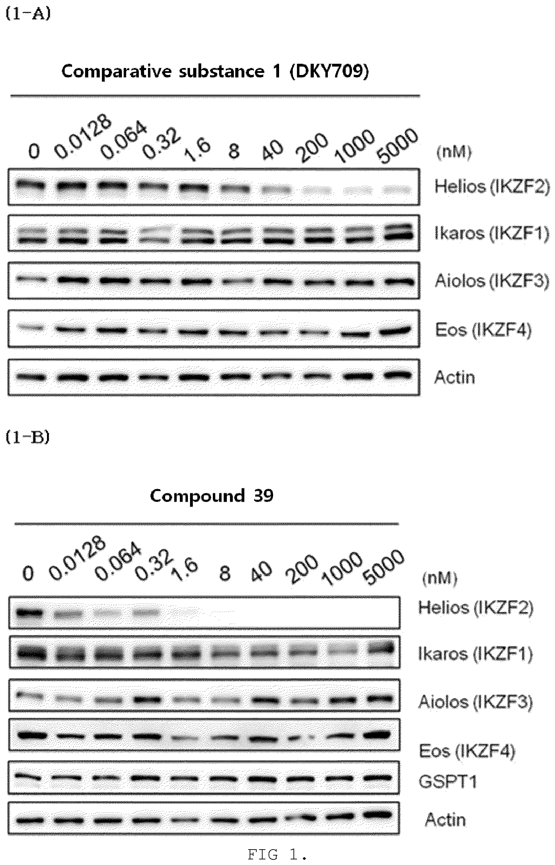

FIG. 1 shows the results of a Western blot experiment measuring Helios protein degradation using compound 39 of the present disclosure (FIG. 1-B) and comparative compound 1 (DKY709) (FIG. 1-A).

### Best Mode for Carrying out the Invention

Hereinafter, preferred embodiments of the present disclosure will be described in detail. However, the present disclosure is not limited to the embodiments described herein and may be embodied in other forms. Rather, the following description is provided to thoroughly and completely explain the present disclosure and to fully convey the concept of the invention to those skilled in the art.

### <PREPARATION EXAMPLE 1. Synthesis and Physicochemical Characterization of Indole Derivative Compounds for Degrading IKZF2>

Compounds represented by the following Chemical Formula 1 of the present disclosure are prepared in the following Reaction Schemes 1 to 15. The preparation is performed as follows:

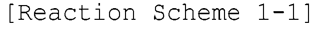

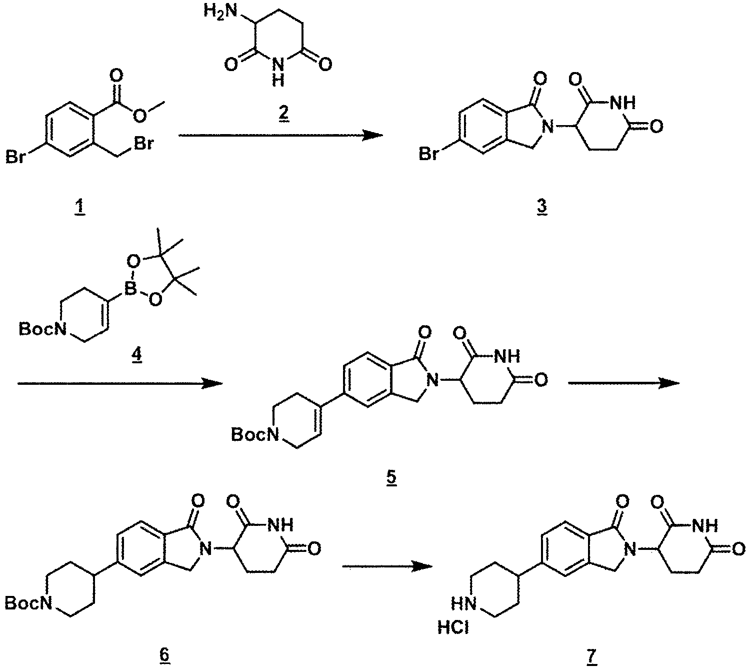

### Synthesis of 3-(5-bromo-1-oxoinsoindolin-2-yl)piperidine-2,6-dione (3)

A solution of 1 (5.0 g, 16.2 mmol) in DMF (15 mL) was added with 2 (3.1 g, 24.4 mmol) and potassium carbonate (6.7 g, 48.7 mmol) before stirring at 110°C for 3 hours. The reaction mixture was cooled and then added with water. The solid precipitate was washed with water, filtered, and dried to obtain 3 (4.5 g, 86%).

¹H NMR (300 MHz, DMSO-*d*₆) δ 11.01 (s, 1H), 7.90 (s, 1H), 7.73 (d, J = 7.7 Hz, 1H), 7.67 (d, J = 8.1 Hz, 1H), 5.12 (dd, J = 13.3, 5.1 Hz, 1H), 4.48 (d, J = 17.6 Hz, 1H), 4.34 (d, J = 17.7 Hz, 1H), 2.97-2.85 (m, 1H), 2.63-2.57 (m, 1H), 2.44-2.29 (m, 1H), 2.07-1.95 (m, 1H).

### Synthesis of tert-butyl 4-(2-(2,6-dioxopiperidin-3-yl)-1-oxoindolin-5-yl)-3,6-dihydropyridine-1 (2H)-carboxylate (5)

A solution of 3 (4.0 g, 12.4 mmol), 4 (5.0 g, 16.1 mmol), potassium carbonate (4.3 g, 30.9 mmol), and Pd (dppf)Cl₂ · DCM (1.0 g, 1.2 mmol) in DMF (15 mL) was stirred at 110°C for 12 hours in a nitrogen atmosphere. After completion of the reaction, the reaction mixture was filtered through Celite. The filtrate was diluted with water, followed by extraction with ethylacetate. The organic layers were pooled, dried over anhydrous magnesium sulfate, filtered, and concentrated. The concentrated mixture was subjected to column chromatography to afford 5 (1.6 g, 30%).
¹H NMR (300 MHz, DMSO-*d*₆) δ 11.00 (s, 1H), 7.70 (d, J = 7.9 Hz, 1H), 7.66 (s, 1H), 7.60 (d, J = 8.2 Hz, 1H), 6.31 (s, 1H), 5.12 (dd, J = 13.3, 5.1 Hz, 1H), 4.46 (d, J = 17.3 Hz, 1H), 4.32 (d, J = 17.3 Hz, 1H), 4.04 (s, 2H), 3.57 (t, J = 5.6 Hz, 2H), 3.00-2.84 (m, 1H), 2.66-2.55 (m, 1H), 2.47-2.26 (m, 2H), 2.07-1.96 (m, 1H), 1.44 (s, 9H), 1.30-1.17 (m, 1H)

### Synthesis of tert-butyl 4-(2-(2,6-dioxopiperidin-3-yl)-1-oxoindolin-5-yl)piperidine-1-carboxylate (6)

A mixture of 5 (1.6 g, 3.8 mmol) and 10% Pd/C (0.4 g) in DMF (15 mL) was stirred for 12 hours under a hydrogen pressure. After completion of the reaction, the reaction mixture was filtered through Celite. The filtrate was diluted with water, followed by extraction with ethylacetate. The organic layers were pooled, dried over anhydrous magnesium sulfate, filtered, and concentrated to afford 6 (1.4 g, 87%).
¹H NMR (300 MHz, DMSO-*d*₆) δ 11.00 (s, 1H), 7.66 (d, J = 7.9 Hz, 1H), 7.51 (s, 1H), 7.41 (d, J = 7.9 Hz, 1H), 5.11 (dd, J = 13.3, 5.0 Hz, 1H), 4.43 (d, J = 17.2 Hz, 1H), 4.29 (d, J = 17.2 Hz, 1H), 4.10 (s, 2H), 2.97-2.74 (m, 4H), 2.63 (d, J = 13.2 Hz, 1H), 2.44-2.38 (m, 1H), 2.03-1.96 (m, 1H), 1.79 (d, J = 12.8 Hz, 2H), 1.56 (t, J = 12.3 Hz, 2H), 1.43 (d, J = 2.6 Hz, 9H)

### Synthesis of 3-(1-oxo-5-(piperidin-4-yl)isoindolin-2-yl)piperidine-2,6-dione chloride (7)

A solution of 6 (2.5 g, 5.8 mmol) in DCM (20 mL) was added with a solution of 4 N HCl in 1,4-dioxane (15 mL) before stirring at room temperature for 3 hours. After completion of the reaction, the reaction mixture was concentrated in a vacuum to afford 7 (1.2 g, 88%).

¹H NMR (300 MHz, DMSO-*d*₆) δ 11.00 (s, 1H), 8.81 (s, 2H), 7.71 (d, J = 7.8 Hz, 1H), 7.47 (s, 1H), 7.39 (d, J = 7.9 Hz, 1H), 5.12 (dd, J = 13.2, 5.0 Hz, 1H), 4.46 (d, J = 17.3 Hz, 1H), 4.32 (d, J = 17.3 Hz, 1H), 3.43-3.37 (m, 2H), 3.08-2.96 (m, 3H), 2.66-2.55 (m, 1H), 2.44-2.35 (m, 1H), 2.03-1.81 (m, 5H).

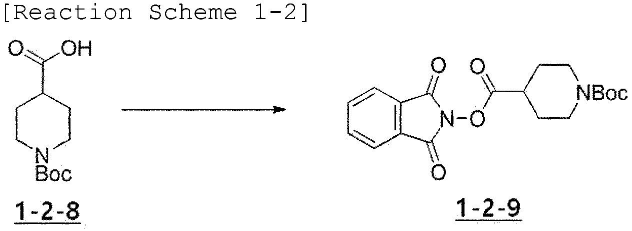

### Synthesis of 1-(tert-butyl) 4-(1,3-dioxoinsoindolin-2-yl)piperidine-1,4-dicarboxylate (1-2-9)

1-2-8 (4.22 g, 18.39 mmol), 2-hydroxyisoindoline-1,3-dione (3 g, 18.39 mmol), EDCI (3.88 g, 20.23 mmol), and DMAP (0.225 g, 1.839 mmol) were added to DCM (15 mL) and stirred at room temperature for 12 hours. After completion of the reaction, the reaction mixture was diluted in water and subjected to extraction with EA. The organic layer thus obtained was washed with NaHCO₃, pooled, concentrated in a vacuum, and subjected to column chromatography to afford 1-2-9.

¹H NMR (400 MHz, DMSO-d ₆) δ 8.01-7.93 (m, 4H), 3.91-3.84 (m, 2H), 3.19-3.10 (m, 1H), 3.06-2.93 (m, 2H), 2.03-1.95 (m, 2H), 1.64-1.53 (m, 2H), 1.41 (s, 9H).

### Synthesis of tert-butyl 4-(2-(2,6-dioxopiperidin-3-yl)-1-oxoindolin-5-yl)piperidine-1-carboxylate (6)

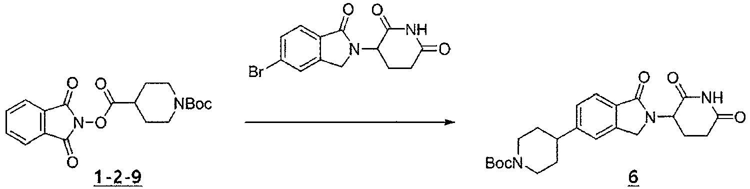

A mixture of 1-2-9 (52.1 mg, 0.139 mmol), 3-(5-bromo-1-oxoisoindolin-2-yl)piperidine-2,6-dione (30 mg, 0.093 mmol), NiBR₂ (dtbbpy) (22.47 mg, 0.046 mmol), Hantzsch ester (47.0 mg, 0.186 mmol), and NaHCO₃ (31.2 mg, 0.371 mmol) in DMA (1 mL) was purged with nitrogen for 15 min and then stirred at room temperature for 12 hours under purple LEDs (160~390nm). After completion of the reaction, the reaction mixture was diluted with water and subjected to extraction with EA. The organic layer was washed with brine, pooled, and concentrated in a vacuum, followed by purification through column chromatography to afford 6.

¹H NMR (400 MHz, DMSO-d ₆) δ 10.98 (s, 1H), 7.65 (d, J = 7.8 Hz, 1H), 7.50 (s, 1H), 7.40 (d, 1H), 5.11 (dd, J = 13.3, 5.1 Hz, 1H), 4.43 (d, J = 17.2 Hz, 1H), 4.29 (d, J = 17.2 Hz, 1H), 4.14-4.04 (m, 2H), 2.96-2.79 (m, 4H), 2.64-2.57 (m, 1H), 2.45-2.34 (m, 1H), 2.03-1.96 (m, 1H), 1.83-1.76 (m, 2H), 1.60-1.48 (m, 2H), 1.43 (s, 9H) .

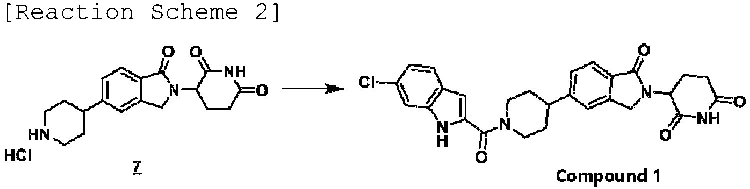

### Synthesis of 3-(5-(1-(6-chloro-1H-indole-2-carbonyl)piperidin-4-yl)-1-oxoinsoindolin-2-yl)piperidine-2,6-dione (Compound 1)

A solution of 7 (20 mg, 0.055 mmol), 6-chloro-1H-indole-2-carboxylic acid (10.8 mg, 0.055 mmol), EDCI-HCl (11.6 mg, 0.06 mmol), HOBt (8.2 mg, 0.06 mmol), and DIPEA (29 uL, 0.165 mmol) in DMF (1 mL) was stirred at room temperature for 12 hours. After completion of the reaction, the reaction mixture was diluted with water and subjected to extraction with ethylacetate. The organic layers thus obtained were pooled, dried over anhydrous magnesium sulfate, filtered, and concentrated. The concentrate mixture was subjected to column chromatography to afford Compound 1 (14 mg, 52%).

¹H NMR (300 MHz, DMSO-*d*₆) δ 13.62 (s, 1H), 10.98 (s, 1H), 8.00 (d, J = 8.7 Hz, 1H), 7.71 (d, J = 1.8 Hz, 1H), 7.66 (d, J = 7.9 Hz, 1H), 7.54 (s, 1H), 7.44 (dd, J = 7.9, 1.4 Hz, 1H), 7.25 (dd, J = 8.7, 1.8 Hz, 1H), 5.10 (dd, J = 13.2, 5.1 Hz, 1H), 4.90 (d, J = 13.1 Hz, 1H), 4.78 (d, J = 12.8 Hz, 1H), 4.42 (d, J = 17.3 Hz, 1H), 4.28 (d, J = 17.3 Hz, 1H), 3.09-2.83 (m, 4H), 2.65-2.53 (m, 1H), 2.39 (dd, J = 13.2, 4.6 Hz, 1H), 2.05-1.83 (m, 3H), 1.77-1.65 (m, 2H).

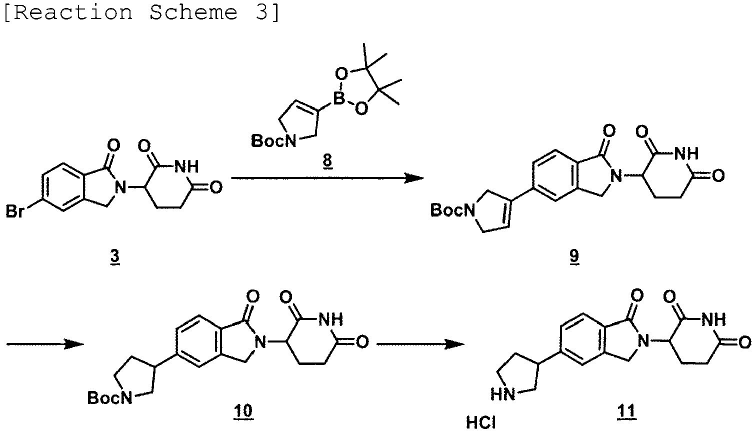

### Synthesis of tert-butyl 3-(2-(2,6-dioxopiperidin-3-yl)-1-oxoindolin-5-yl)-2,5-dihydro-1H-pyrrol-1-carboxylate (9)

A solution of 3 (200 mg, 0.62 mmol), 8 (201 mg, 0.68 mmol), potassium carbonate (214 mg, 1.55 mmol), and Pd (dppf) Cl₂ · DCM (51 mg, 0.06 mmol) in DMF (2 mL) was stirred at 110°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 9 (45 mg, 18%).

¹H NMR (500MHz, CDCl ₃) δ 8.14 (s, 1H), 7.86 (t, J = 7.5, 7.5Hz, 1H), 7.53 (s, 1H), 7.43 (s, 1H), 6.29 (s, 1H), 5.31-5.14 (m, 1H), 4.51 (d, J = 16.7Hz, 3H), 4.34 (s, 3H), 2.95-2.81 (m, 1H), 2.44-2.17 (m, 3H), 1.50 (s, 9H).

### Synthesis of tert-butyl 3-(2-(2,6-dioxopiperidin-3-yl)-1-oxoindolin-5-yl)pyrrolidine-1-carboxylate (10)

A solution of *9* (20 mg, 0.05 mmol) and 10% Pd/C (10 mg) in DMF (1 mL) was stirred for 12 hours under a hydrogen pressure. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford *10* (17 mg, 85%).

¹H NMR (500MHz, CDCl ₃) δ 8.07 (s, 1H), 7.87 (d, J = 7.7Hz, 1H), 7.40 (d, J = 8.1Hz, 1H), 7.36 (s, 1H), 5.26 (d, J = 12.7Hz, 1H), 4.51 (d, J = 15.7Hz, 1H), 4.36 (d, J = 15.7Hz, 1H), 3.90-3.83 (m, 1H), 3.70-3.62 (m, 1H), 3.53-3.44 (m, 2H), 3.39 (d, J = 9.9Hz, 1H), 2.91-2.83 (m, 1H), 2.41-2.30 (m, 2H), 2.29-2.22 (m, 1H), 2.09-2.01 (m, 2H), 1.51 (s, 9H).

### Synthesis of 3-(1-oxo-5-(pyrrolidin-3-yl)isoindolin-2-yl)piperidine-2,6-dione chloride (11)

A solution of 10 (20 mg, 5.8 mmol) in DCM (1 mL) was added with a solution of 4 N HCl in 1,4-dioxane (0.1 mL) before being stirred at room temperature for 3 hours. After completion of the reaction, the reaction mixture was concentrated in a vacuum to afford 11 (14 mg, 100%).

¹H NMR (300MHz, DMSO-*d*₆) δ 11.02 (s, 1H), 9.53 (s, 2H), 7.79-7.66 (m, 3H), 6.63 (s, 1H), 5.12 (dd, J = 13.4, 5.1Hz, 1H), 4.49 (d, J = 17.4Hz, 1H), 4.44-4.32 (m, 3H), 4.19 (s, 2H), 2.97-2.85 (m, 1H), 2.68-2.55 (m, 1H), 2.46-2.37 (m, 1H), 2.08-1.97 (m, 1H).

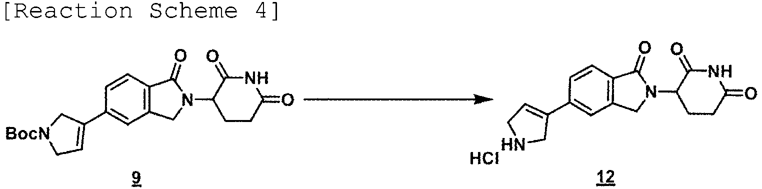

### Synthesis of 3-(5-(2,5-dihydro-1H-pyrrol-3-yl)-1-oxoinsoindolin-2-yl)piperidine-2,6-dione chloride (12)

A solution of 9 (20 mg, 0.06 mmol) in DCM (1 mL) was added with a solution of 4 N HCl in 1,4-dioxane (0.1 mL) before being stirred at room temperature for 3 hours. After completion of the reaction, the reaction mixture was concentrated in a vacuum to afford 12 (16 mg, 95%).

¹H NMR (300 MHz, DMSO-*d*₆) δ 11.02 (s, 1H), 9.53 (s, 2H), 7.79-7.66 (m, 3H), 6.63 (s, 1H), 5.12 (dd, J = 13.4, 5.1 Hz, 1H), 4.49 (d, J = 17.4 Hz, 1H), 4.44 - 4.32 (m, 3H), 4.19 (s, 2H), 2.97-2.85 (m, 1H), 2.68-2.55 (m, 1H), 2.46-2.37 (m, 1H), 2.08-1.97 (m, 1H).

### Synthesis of 3-(1-oxo-5-(1,2,3,6-tetrahydropyridin-4-yl)isoindolin-2-yl)piperidine-2,6-dione (5-2)

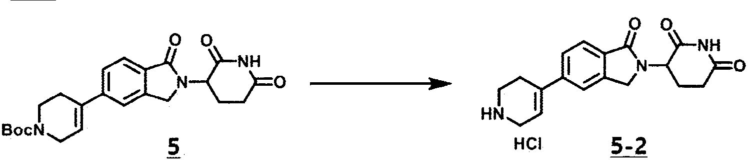

A solution of 5 (20 mg) in DCM (1 mL) was added with a solution of 4 N HCl in 1,4-dioxane (0.1 mL) before being stirred at room temperature for 3 hours. After completion of the reaction, the reaction mixture was concentrated in a vacuum to afford 5-2 (15 mg).

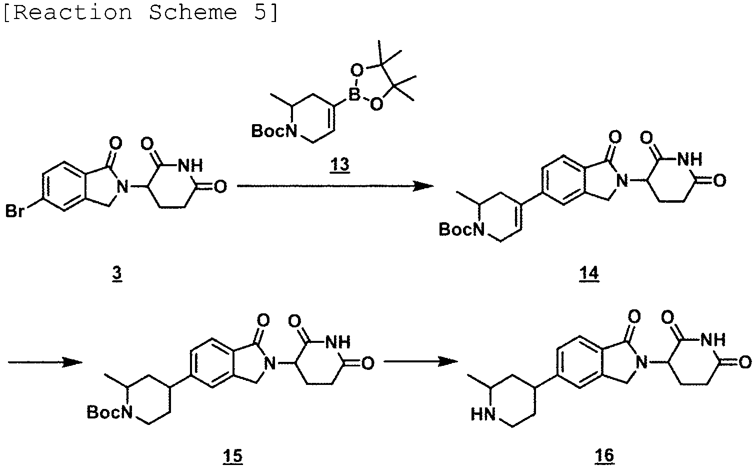

### Synthesis of tert-butyl 4-(2-(2,6-dioxopiperidin-3-yl)-1-oxoindolin-5-yl)-2-methyl-3,6-dihydropyridine-1 (2H)-carboxylate (14)

A solution of 3 (100 mg, 0.31 mmol), 13 (130 mg, 0.40 mmol), potassium carbonate (107 mg, 0.77 mmol), and Pd (dppf) Cl₂ · DCM (25 mg, 0.03 mmol) in DMF (2 mL) was stirred at 110°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 14 (46 mg, 34%).

¹H NMR (300MHz, DMSO-*d*₆) δ 11.00 (s, 1H), 7.70 (d, J = 8.1Hz, 1H), 7.66 (s, 1H), 7.59 (d, J = 8.1Hz, 1H), 6.27 (s, 1H), 5.12 (dd, J = 13.2, 5.1Hz, 1H), 4.55 (s, 1H), 4.46 (d, J = 17.3Hz, 1H), 4.32 (d, J = 17.3Hz, 1H), 4.11 (d, J = 13.1Hz, 1H), 3.07-2.86 (m, 2H), 2.64-2.56 (m, 1H), 2.48-2.31 (m, 2H), 2.08-1.93 (m, 1H), 1.44 (s, 9H), 1.23 (d, J = 6.6Hz, 3H).

### Synthesis of tert-butyl 4-(2-(2,6-dioxopiperidin-3-yl)-1-oxoindolin-5-yl)-2-methylpiperidine-1-carboxylate (15)

A solution of 14 (40 mg, 0.09 mmol) and 10% Pd/C (13 mg) in DMF (1 mL) was stirred for 12 hours under a hydrogen pressure. After completion of the reaction, the reaction mixture was filtered through Celite. The filtrate was diluted with water, followed by extraction with ethylacetate. The organic layers were pooled, dried over anhydrous magnesium sulfate, filtered, and concentrated to afford 15 (35 mg, 86%).

¹H NMR (300MHz, DMSO-*d*₆) δ 10.99 (s, 1H), 7.65 (dd, J = 8.0, 1.7Hz, 1H), 7.51 (s, 1H), 7.42 (d, J = 7.8Hz, 1H), 5.11 (dd, J = 13.1, 5.0Hz, 1H), 4.43 (d, J = 17.2Hz, 1H), 4.29 (d, J = 17.3Hz, 1H), 3.94-3.79 (m, 1H), 3.64 (s, 1H), 2.89-2.82 (m, 1H), 2.65-2.55 (m, 1H), 2.41 (d, J = 13.2Hz, 1H), 2.20 (dd, J = 5.9, 1.8Hz, 1H), 2.10-1.94 (m, 2H), 1.86-1.68 (m, 2H), 1.56 (s, 1H), 1.43 (s, 9H), 1.25-1.20 (m, 1H), 1.16 (d, J = 6.4Hz, 3H).

### Synthesis of 3-(5-(2-methylpiperidin-4-yl)-1-oxoisoindolin-2-yl)piperidine-2,6-dione hydrochloride (16)

A solution of 15 (30 mg, 0.07 mmol) in DCM (1 mL) was added with a solution of 4 N HCl in 1,4-dioxane (0.1 mL) before being stirred at room temperature for 3 hours. After completion of the reaction, the reaction mixture was concentrated in a vacuum to afford 16 (26 mg, 100%).

¹H NMR (300MHz, DMSO-*d*₆) δ 11.00 (s, 1H), 8.92 (s, 1H), 8.62 (s, 1H), 7.71 (d, J = 7.9Hz, 1H), 7.49 (d, J = 12.5Hz, 1H), 7.41 (t, J = 9.2Hz, 1H), 5.11 (dd, J = 13.2, 5.0Hz, 1H), 4.46 (d, J = 17.3Hz, 1H), 4.32 (d, J = 17.3Hz, 1H), 3.68 (s, 1H), 3.30-3.13 (m, 2H), 3.10-2.96 (m, 2H), 2.95-2.85 (m, 1H), 2.69-2.56 (m, 1H), 2.43-2.34 (m, 1H), 2.05- 1.92 (m, 3H), 1.88-1.79 (m, 1H), 1.33 (dd, J = 32.6, 6.6Hz, 3H).

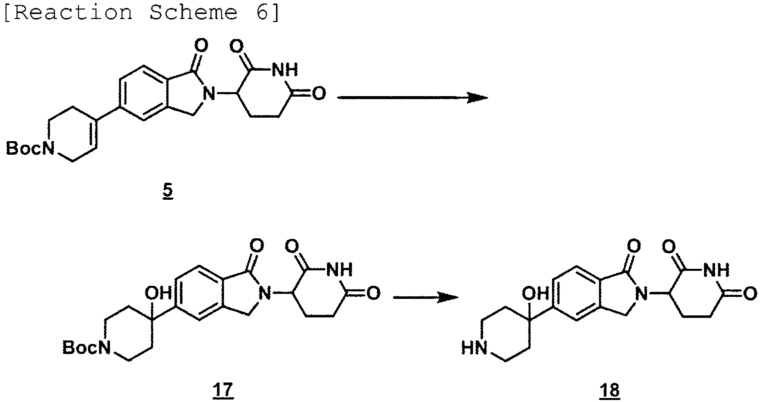

### Synthesis of tert-butyl 4-(2-(2,6-dioxopiperidin-3-yl)-1-oxoindolin-5-yl)-4-hydroxypiperidine-1-carboxylate (17)

A solution of 5 (50 mg, 0.12 mmol), Phenylsilane (25 mg, 0.24 mmol), and Mn (dpm)₃ (36 mg, 0.06 mmol) in a mixture of DCM (1 mL), i-PrOH (1 mL), and DMF (0.2 mL) was stirred for 16 hours in an oxygen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 17 (30 mg, 58%).

¹H NMR (300MHz, DMSO-*d*₆) δ 10.99 (s, 1H), 7.72 (s, 1H), 7.70-7.62 (m, 2H), 5.31 (s, 1H), 5.11 (dd, J = 13.3, 5.1Hz, 1H), 4.44 (d, J = 17.4Hz, 1H), 4.30 (d, J = 17.3Hz, 1H), 3.95-3.75 (m, 2H), 3.61-3.47 (m, 1H), 3.27 -3.08 (m, 2H), 3.01-2.82 (m, 2H), 2.65-2.59 (m, 1H), 2.43-2.36 (m, 1H), 2.13-1.79 (m, 4H), 1.61 (d, J = 13.2Hz, 2H), 1.43 (s, 9H).

### Synthesis of 3-(5-(4-hydroxypiperidin-4-yl)-1-oxoinsoindolin-2-yl)piperidine-2,6-dione chloride (18)

A solution of 17 (30 mg, 0.07 mmol, 1 eq) in DCM (1 mL) was added with 4 N HCl in 1,4-dioxane (0.1 mL) and then stirred at room temperature for 3 hours. After completion of the reaction, the reaction mixture was concentrated in a vacuum to afford 18 (26 mg, 100%).

¹H NMR (300MHz, DMSO-*d*₆) δ 11.00 (s, 1H), 8.93 (d, J = 28.5Hz, 2H), 7.74 (d, J = 7.9Hz, 1H), 7.69 (s, 1H), 7.60 (d, J = 8.0Hz, 1H), 7.43 (s, 1H), 5.12 (dd, J = 13.3, 4.8Hz, 1H), 4.47 (d, J = 17.3Hz, 1H), 4.33 (d, J = 17.2Hz, 1H), 3.71-3.57 (m, 3H), 3.10-2.84 (m, 3H), 2.65-2.56 (m, 2H), 2.34-2.19 (m, 3H), 2.08-1.93 (m, 2H).

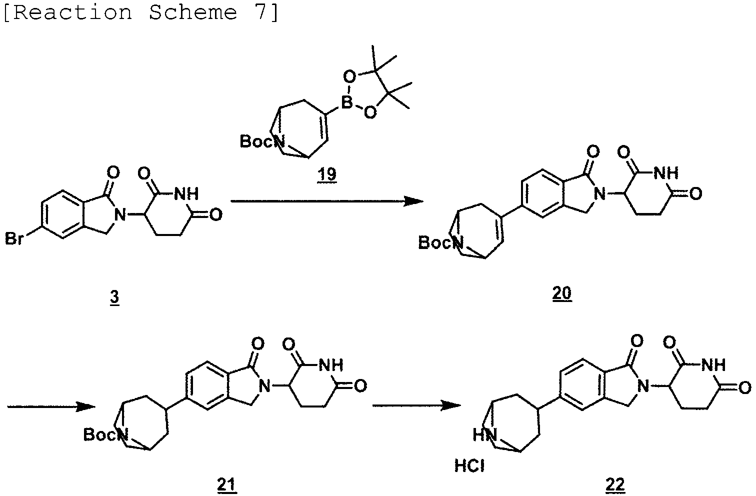

### Synthesis of tert-butyl 3-(2-(2,6-dioxopiperidin-3-yl)-1-oxoindolin-5-yl)-8-azabicyclo[3.2.1]oct-2-ene-8-carboxylate (20)

A solution of 3 (50 mg, 0.16 mmol), 19 (67 mg, 0.20 mmol), potassium carbonate (54 mg, 0.39 mmol), Pd (dppf)Cl₂ · DCM (13 mg, 0.02 mmol) in DMF (2 mL) was stirred at 110°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 20 (13 mg, 19%).

¹H NMR (400 MHz, DMSO-d ₆) δ 10.98 (d, J = 8.5 Hz, 1H), 7.72-7.54 (m, 2H), 7.25 (s, 1H), 6.69 (d, J = 5.3 Hz, 1H), 5.11 (dd, J = 13.3, 5.0 Hz, 1H), 4.49-4.22 (m, 4H), 3.08-2.83 (m, 2H), 2.65-2.55 (m, 1H), 2.47-2.28 (m, 2H), 2.22 - 2.06 (m, 1H), 2.04-1.80 (m, 3H), 1.80-1.61 (m, 1H), 1.38 (s, 6H), 1.08 (s, 3H).

### Synthesis of tert-butyl 3-(2-(2,6-dioxopiperidin-3-yl)-1-oxoindolin-5-yl)-8-azabicyclo[3.2.1]octane-8-carboxylate (21)

A solution of 20 (10 mg, 0.02 mmol) and 10% Pd/C (5 mg) in DMF (1 mL) was stirred for 12 hours under a hydrogen pressure. After completion of the reaction, the reaction mixture was filtered through Celite. The filtrate was diluted with water, followed by extraction with ethylacetate. The organic layers were pooled, dried over anhydrous magnesium sulfate, filtered, and concentrated to afford 21 (8 mg, 80%).

¹H NMR (500 MHz, DMSO-d ₆) δ 10.99 (s, 1H), 7.66 (dd, J = 7.9, 4.6 Hz, 1H), 7.55 (s, 1H), 7.49-7.42 (m, 1H), 5.11 (dd, J = 13.3, 5.1 Hz, 1H), 4.43 (d, J = 17.3 Hz, 1H), 4.30 (d, J = 17.1 Hz, 1H), 4.26-4.11 (m, 2H), 2.97-2.87 (m, 1H), 2.68-2.56 (m, 2H), 2.45-2.32 (m, 2H), 2.02-1.81 (m, 4H), 1.79-1.56 (m, 3H), 1.46 (d, J = 4.3 Hz, 6H), 1.28-1.26 (m, 1H), 1.24 (s, 3H).

### Synthesis of tert-butyl 3-(2-(2,6-dioxopiperidin-3-yl)-1-oxoisoindolin-5-yl)-8- azabicyclo[3.2.1]oct-2-ene-8-carboxylate (22)

A solution of 21 (8 mg, 0.18 mmol) in DCM (1 mL) was added with a solution of 4 N HCl/1,4-dioxane (0.1 mL) before being stirred at room temperature for 3 hours. After completion of the reaction, the reaction mixture was concentrated in a vacuum to afford 22 (8 mg, 100%).

¹H NMR (500 MHz, DMSO-d ₆) δ 11.01 (s, 1H), 9.10-8.93 (m, 1H), 8.91-8.73 (m, 1H), 7.76-7.67 (m, 2H), 7.64-7.46 (m, 1H), 5.12 (dd, J = 13.4, 5.4 Hz, 1H), 4.45 (d, J = 17.1 Hz, 1H), 4.36-4.30 (m, 1H), 4.11-4.00 (m, 2H), 3.55-3.45 (m, 1H), 3.31-3.22 (m, 1H), 3.00-2.85 (m, 1H), 2.70-2.58 (m, 1H), 2.48-2.34 (m, 2H), 2.28-2.16 (m, 1H), 2.16-1.98 (m, 4H), 1.93-1.82 (m, 2H).

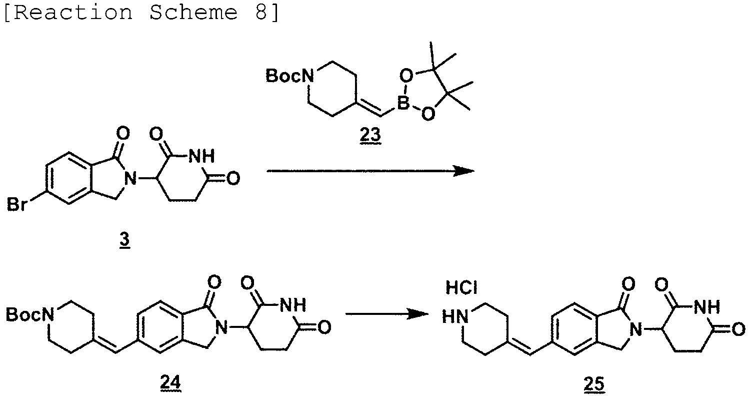

### Synthesis of tert-butyl 4-( (2-(2,6-dioxopiperidin-3-yl)-1-oxoisoindolin-5- yl)methylene) piperidine-1-carboxylate (24)

A solution of 3 (200 mg, 0.62 mmol), 23 (260 mg, 0.81 mmol), potassium carbonate (257 mg, 1.86 mmol), and Pd (dppf)Cl₂ · DCM (51 mg, 0.06 mmol) in DMF (3 mL) was stirred at 110°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 24 (71 mg, 26%).

¹H NMR (500MHz, CDCl ₃) δ 8.48 (s, 1H), 7.82 (d, J = 7.8Hz, 1H), 7.29 (d, J = 7.8Hz, 1H), 7.26 (s, 1H), 6.42 (s, 1H), 5.23 (dd, J = 13.3, 5.1Hz, 1H), 4.47 (d, J = 15.9Hz, 1H), 4.32 (d, J = 15.9Hz, 1H), 3.52 (t, J = 5.8Hz, 2H), 3.41 (t, J = 5.8Hz, 2H), 2.93-2.80 (m, 2H), 2.45 (t, J = 5.9Hz, 2H), 2.38-2.30 (m, 3H), 2.22- 2.16 (m, 1H), 1.47 (s, 9H).

### Synthesis of 3-(1-oxo-5-(piperidin-4-ylidenemethyl)isoindolin-2-yl)piperidine-2,6-dione chloride (25)

A solution of 24 (65 mg, 0.15 mmol, 1 eq) in DCM (1 mL) was added with a solution of 4 N HCl in 1,4-dioxane (0.4 mL) before being stirred at room temperature for 3 hours. After completion of the reaction, the reaction mixture was concentrated in a vacuum to afford 25 (55 mg, 100%).

¹H NMR (300MHz, DMSO-*d*₆) δ 10.99 (s, 1H), 9.00 (s, 2H), 7.71 (t, J = 6.8Hz, 1H), 7.48 (d, J = 5.4Hz, 1H), 7.38 (t, J = 6.6Hz, 1H), 6.58 (s, 1H), 5.11 (dd, J = 13.1, 6.4Hz, 1H), 4.46 (dd, J = 17.3, 5.4Hz, 1H), 4.32 (dd, J = 17.6, 5.4Hz, 1H), 3.57 (d, J = 5.5Hz, 3H), 3.13 (d, J = 26.6Hz, 4H), 2.90 (d, J = 15.2Hz, 1H), 2.66-2.57 (m, 4H).

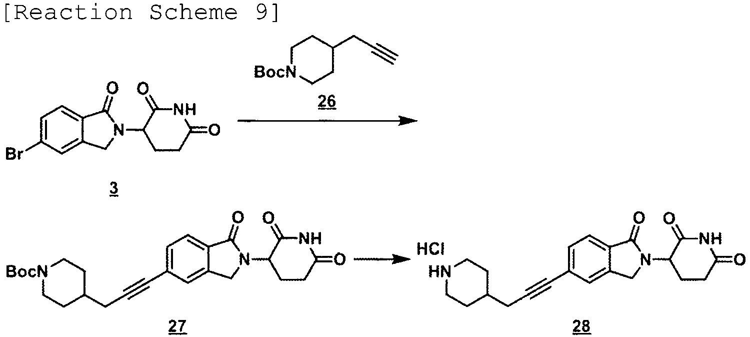

### Synthesis of tert-butyl 4-(3-(2-(2,6-dioxopiperidin-3-yl)-1-oxoindolin-5-yl)prop-2-yn-1-yl)piperidine-1-carboxylate (27)

A solution of 3 (600 mg, 1.86 mmol), 26 (456 mg, 2.04 mmol), Pd (dppf)Cl₂ (136 mg, 0.19 mmol), CuI (35 mg, 0.19 mmol), and DIPEA (0.65 mL,) in DMSO (10 mL) was stirred at 100°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 27 (375 mg, 43%).

¹H NMR (500MHz, CDCl ₃) δ 8.27 (s, 1H), 7.82 (d, J = 7.9Hz, 1H), 7.52 (d, J = 7.9Hz, 1H), 7.50 (s, 1H), 5.24 (dd, J = 13.3, 5.1Hz, 1H), 4.49 (d, J = 16.0Hz, 1H), 4.33 (d, J = 16.0Hz, 1H), 4.16 (d, J = 13.3Hz, 2H), 2.98-2.80 (m, 2H), 2.78-2.70 (m, 2H), 2.42 (d, J = 6.6Hz, 2H), 2.40-2.33 (m, 1H), 2.26-2.21 (m, 1H), 1.84 (d, J = 12.4Hz, 2H), 1.78-1.72 (m, 1H), 1.48 (s, 9H), 1.35-1.26 (m, 2H).

### Synthesis of 3-(1-oxo-5-(3-(piperidin-4-yl)prop-1-yn-1-yl)isoindolin-2-yl)piperidine-2,6-dione chloride (28)

A solution of 27 (370 mg, 0.80 mmol, 1 eq) in DCM (3 mL) was added with a solution of 4N HCl in 1,4-dioxane (2.0 mL) and then stirred at room temperature for 3 hours. After completion of the reaction, the reaction mixture was concentrated in a vacuum to afford 28 (300 mg, 94%).

¹H NMR (300MHz, DMSO-*d*₆) δ 11.01 (s, 1H), 8.92 (s, 1H), 8.60 (s, 1H), 7.71 (d, J = 7.8Hz, 1H), 7.67 (s, 1H), 7.54 (d, J = 7.9Hz, 1H), 5.12 (dd, J = 13.3, 5.1Hz, 1H), 4.45 (d, J = 17.5Hz, 1H), 4.32 (d, J = 17.5Hz, 1H), 3.76-3.63 (m, 1H), 3.57 (s, 1H), 3.27 (s, 2H), 2.98-2.86 (m, 3H), 2.60 (d, J = 18.1Hz, 2H), 2.45-2.37 (m, 1H), 2.06-1.80 (m, 4H), 1.60-1.51 (m, 2H).

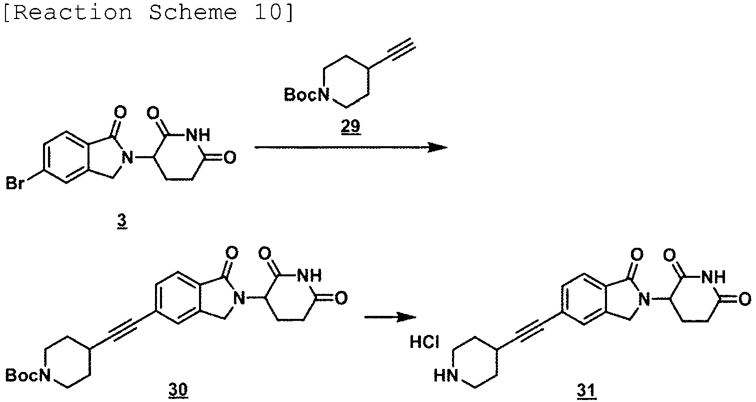

### Synthesis of tert-butyl 4-( (2-(2,6-dioxopiperidin-3-yl)-1-oxoindolin-5-yl)ethynyl)piperidine-1-carboxylate (30)

A solution of 3 (2.5 g, 7.74 mmol), 29 (1.8 g, 8.51 mmol), Pd (dppf)Cl₂ (566 mg, 0.77 mmol), CuI (147 mg, 0.77 mmol), and DIPEA (2.7 mL) in DMSO (30 mL) was stirred at 100°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 30 (2.0 g, 58%).

¹H NMR (300MHz, DMSO-*d*₆) δ 11.01 (s, 1H), 7.72-7.63 (m, 2H), 7.53 (d, J = 8.1Hz, 1H), 5.10 (dd, J = 13.3, 5.1 Hz, 1H), 4.45 (d, J = 17.6Hz, 1H), 4.32 (d, J = 17.5Hz, 1H), 3.70-3.60 (m, 2H), 3.15 (t, J = 10.9Hz, 2H), 2.96-2.84 (m, 2H), 2.60 (d, J = 16.7Hz, 1H), 2.43-2.27 (m, 1H), 2.08-1.95 (m, 1H), 1.84 (4.6Hz, 2H), 1.58-1.47 (m, 2H), 1.40 (s, 9H).

### Synthesis of 3-(1-oxo-5-(piperidin-4-ylethynyl)isoindolin-2-yl)piperidine-2,6-dione chloride (31)

A solution of 30 (2 g, 4.4 mmol, 1 eq) in DCM (10 mL) was added with a solution of 4 N HCl/1,4-dioxane (11 mL) and then stirred at room temperature for 3 hours. After completion of the reaction, the reaction mixture was concentrated in a vacuum to afford 31 (1.7 g, 99%).

¹H NMR (500MHz, DMSO-*d*₆) δ 11.01 (s, 1H), 8.78 (d, J = 26.0Hz, 2H), 7.71 (d, J = 7.9Hz, 1H), 7.67 (s, 1H), 7.55 (dd, J = 7.8, 1.4Hz, 1H), 5.10 (dd, J = 13.3, 5.1Hz, 1H), 4.43 (s, 1H), 4.32 (d, J = 17.5Hz, 1H), 3.26- 3.18 (m, 2H), 3.07-2.98 (m, 3H), 2.94-2.86 (m, 1H), 2.64-2.56 (m, 1H), 2.44-2.34 (m, 1H), 2.09-1.97 (m, 3H), 1.87-1.78 (m, 2H).

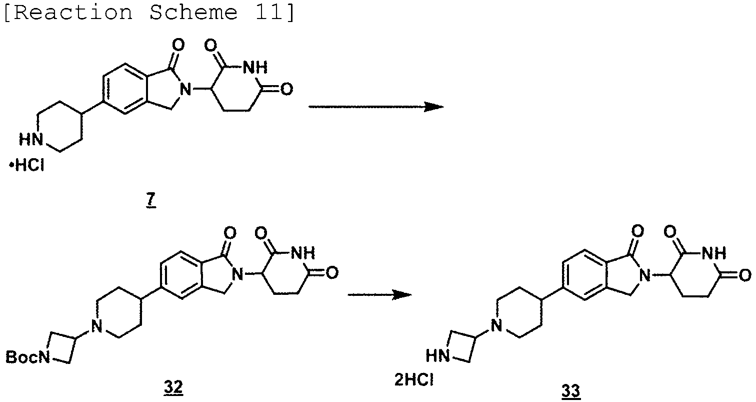

### Synthesis of tert-butyl 3-(4-(2-(2,6-dioxopiperidin-3-yl)-1-oxoindolin-5-yl)piperidin-1-yl)azetidine-1-carboxylate (32)

A solution of 7 (50 mg, 0.14 mmol), tert-butyl 3-oxoazetidine-1-carboxylate (26 mg, 0.15 mmol), NaBH (OAc)₃ (41 mg, 0.19 mmol), and DIPEA (71 uL) in DMF (1 mL) was stirred at room temperature for 12 hours. After completion of the reaction, the reaction mixture was diluted with water and subjected to extraction with ethylacetate. The organic layers thus obtained were pooled, dried over anhydrous magnesium sulfate, filtered, and concentrated. The concentrate mixture was subjected to column chromatography to afford 32 (33 mg, 50%).

¹H NMR (300MHz, DMSO-*d*₆) δ 11.20 (s, 1H), 11.00 (s, 1H), 7.72 (d, J = 7.9Hz, 1H), 7.48 (s, 1H), 7.41 (d, J = 7.6Hz, 1H), 5.12 (dd, J = 13.2, 5.0Hz, 1H), 4.46 (d, J = 17.3Hz, 1H), 4.32 (d, J = 17.4Hz, 1H), 4.21 (s, 1H), 4.14-4.00 (m, 3H), 3.58-3.49 (m, 2H), 3.10-2.82 (m, 5H), 2.63-2.55 (m, 2H), 2.10-1.95 (m, 5H), 1.40 (s, 9H).

### Synthesis of 3-(5-(1-(azetidin-3-yl)piperidin-4-yl)-1-oxoinsoindolin-2-yl)piperidine-2,6-dione dihydrochloride (33)

A solution of Compound 32 (33 mg, 0.07 mmol, 1 eq) in DCM (1 mL) was added with a solution of 4 N HCl in 1,4-dioxane (170 uL) before being stirred at room temperature for 3 hours. After completion of the reaction, the reaction mixture was concentrated in a vacuum to afford 33 (31 mg, 100%).

¹H NMR (300MHz, DMSO-*d*₆) δ 12.43 (s, 1H), 11.00 (s, 1H), 9.66 (s, 1H), 9.19 (s, 1H), 7.72 (d, J = 7.8Hz, 1H), 7.48 (s, 1H), 7.41 (d, J = 7.9Hz, 1H), 5.12 (dd, J = 13.2, 5.1Hz, 1H), 4.62-4.42 (m, 3H), 4.32 (d, J = 17.4Hz, 1H), 4.12 (s, 2H), 3.78-3.65 (m, 1H), 3.10-2.83 (m, 4H), 2.65-2.60 (m, 1H), 2.43-2.25 (m, 1H), 2.20-1.93 (m, 5H).

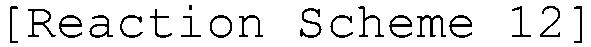

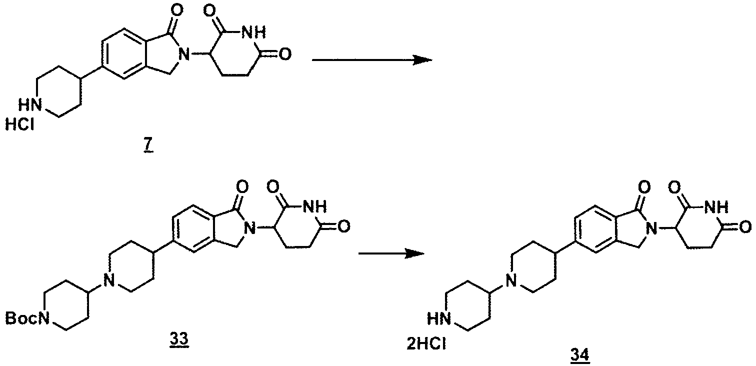

**Synthesis of tert-butyl 4-(2-(2,6-dioxopiperidin-3-yl)-1-oxoindolin-5-yl)-[1,4'-** 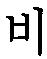 **piperidine]-1'-carboxylate** (33)

A solution of 7 (50 mg, 0.14 mmol), tert-butyl 4-oxopiperidine-1-carboxylate (30 mg, 0.15 mmol), NaBH (OAc)₃ (41 mg, 0.19 mmol), and DIPEA (71 uL) in DMF (1 mL) was stirred at room temperature for 12 hours. After completion of the reaction, the reaction mixture was diluted with water and subjected to extraction with ethylacetate. The organic layers thus obtained were pooled, dried over anhydrous magnesium sulfate, filtered, and concentrated. The concentrate mixture was subjected to column chromatography to afford 33 (20 mg, 29%).

¹H NMR (300MHz, DMSO-*d*₆) δ 11.00 (s, 1H), 10.30 (s, 1H), 7.72 (d, J = 7.9Hz, 1H), 7.47 (s, 1H), 7.40 (d, J = 7.9Hz, 1H), 5.12 (dd, J = 13.2, 5.1Hz, 1H), 4.46 (d, J = 17.4Hz, 1H), 4.32 (d, J = 17.4Hz, 1H), 4.09 (d, J = 13.1Hz, 2H), 3.56 (d, J = 11.9Hz, 2H), 3.17-3.07 (m, 2H), 3.02-2.90 (m, 2H), 2.81-2.70 (m, 2H), 2.65-2.56 (m, 1H), 2.15-1.96 (m, 8H), 1.66-1.51 (m, 3H), 1.41 (s, 9H).

### Synthesis of 3-(5-([1,4'-bipiperidin]-4-yl)-1-oxoinsoindolin-2-yl)piperidine-2,6-dione dihydrochloride (34)

A solution of 33 (20 mg, 0.04 mmol) in DCM (1 mL) was added with a solution of 4 N HCl in 1,4-dioxane (0.1 mL) and then stirred at room temperature for 3 hours. After completion of the reaction, the reaction mixture was concentrated in a vacuum to afford 34 (14 mg, 74%).

¹H NMR (300MHz, DMSO-*d*₆) δ 11.26 (s, 1H), 11.00 (s, 1H), 9.14 (s, 1H), 8.99 (d, J = 11.8Hz, 1H), 7.72 (d, J = 7.8Hz, 1H), 7.47 (s, 1H), 7.41 (d, J = 7.9Hz, 1H), 5.12 (dd, J = 13.2, 5.1Hz, 1H), 4.47 (d, J = 17.4Hz, 1H), 4.32 (d, J = 17.4Hz, 1H), 3.55-3.47 (m, 3H), 3.21-2.82 (m, 7H), 2.64-2.57 (m, 1H), 2.43-2.17 (m, 6H), 2.09 -1.93 (m, 5H).

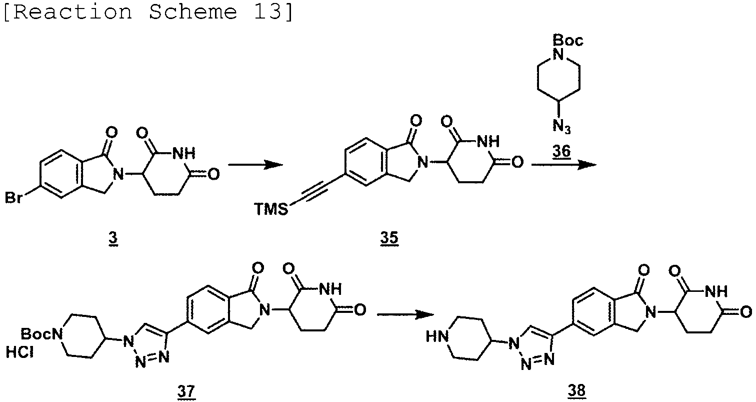

### Synthesis of 3-(1-oxo-5-((trimethylsilyl)ethynyl)isoindolin-2-yl)piperidine-2,6-dione (35)

A solution of 3 (680 mg, 2.1 mmol), ethynyltrimethylsilane (1 g, 10.5 mmol), Pd (PPh₃)₂ ·Cl₂ (148 mg, 0.21 mmol), CuI (40 mg, 0.21 mmol), and DIPEA (1.1 mL, 6.31 mmol) in DMF (10 mL) was stirred at 65°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford 35 (400 mg, 56%).

¹H NMR (500MHz, DMSO-*d*₆) δ 11.02 (s, 1H), 7.75-7.70 (m, 2H), 7.58 (d, J = 8.0Hz, 1H), 5.12 (dd, J = 13.3, 5.1 Hz, 1H), 4.46 (d, J = 17.5Hz, 1H), 4.34 (d, J = 17.5Hz, 1H), 2.96-2.87 (m, 1H), 2.65-2.56 (m, 1H), 2.46-2.35 (m, 1H), 2.05-1.98 (m, 1H), 0.26 (s, 9H).

### Synthesis of tert-butyl 4-(4-(2-(2,6-dioxopiperidin-3-yl)-1-oxoindolin-5-yl)-1H-1,2,3-triazol-1-yl)piperidine-1- carboxylate (37)

A solution of 35 (20 mg, 0.06 mmol), 36 (15 mg, 0.068 mmol), sodium ascorbate (2.3 mg, 0.01 mmol), and CuSO ₄ (1.9 mg, 0.01 mmol) in a mixture of THF (1 mL)/H ₂O (1 mL) was stirred at room temperature for 10 hours. After completion of the reaction, the reaction mixture was filtered through Celite. The filtrate was diluted with water and then subjected to extraction with ethylacetate. The organic layers thus obtained were pooled, dried over anhydrous magnesium sulfate, filtered, and concentrated. The concentrate mixture was subjected to column chromatography to afford 37 (8 mg, 28%) .

¹H NMR (500MHz, CDCl₃) δ 8.09 (s, 1H), 8.02 (s, 1H), 7.94 (s, 1H), 7.88 (s, 1H), 7.84 (d, J = 8.0Hz, 1H), 5.24 (dd, J = 13.4, 5.0Hz, 1H), 4.74-4.65 (m, 1H), 4.55 (d, J = 16.0Hz, 1H), 4.40 (d, J = 15.9Hz, 1H), 4.31 (s, 2H), 3.03-2.79 (m, 4H), 2.44-2.34 (m, 1H), 2.30-2.21 (m, 3H), 2.07-1.95 (m, 2H), 1.49 (s, 9H).

### Synthesis of 3-(1-oxo-5-(1-(piperidin-4-yl)-1H-1,2,3-triazol-4-yl)isoindolin-2-yl)piperidine-2,6-dione chloride (38)

A solution of 37 (8 mg, 0.02 mmol, 1 eq) in DCM (1 mL) was added with a solution of 4 N HCl in 1,4-dioxane (0.04 mL) before being stirred at room temperature for 3 hours. After completion of the reaction, the reaction mixture was concentrated in a vacuum to afford 38 (7 mg, 100%).
LC/MS (ESI) m/z 395 [M+H]⁺

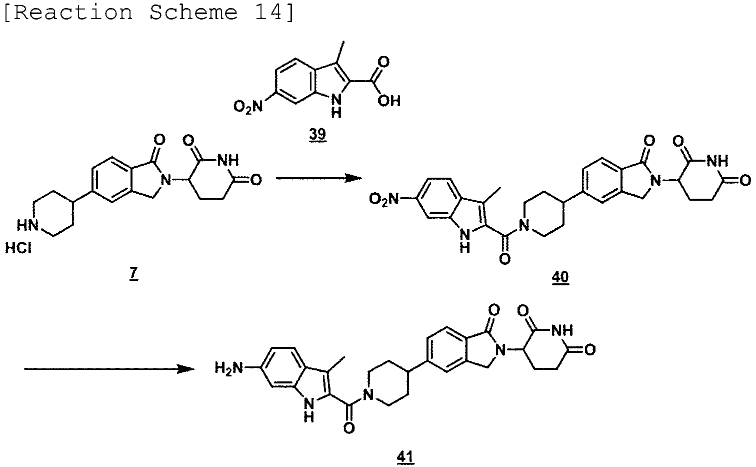

### Synthesis of 3-(5-(1-(3-methyl-6-nitro-1H-indole-2-carbonyl)piperidin-4-yl)-1-oxoinsoindolin-2-yl)piperidine-2,6-dione (40)

A solution of 7 (50 mg, 0.14 mmol), 39 (33 mg, 0.15 mmol), EDCI-HCl (29 mg, 0.15 mmol), HOBt (20 mg, 0.15 mmol), and DIPEA (72 uL) in DMF (1 mL) was stirred at room temperature for 12 hours. After completion of the reaction, the reaction mixture was diluted with water and subjected to extraction with ethylacetate. The organic layers thus obtained were pooled, dried over anhydrous magnesium sulfate, filtered, and concentrated. Purification of the concentrate through column chromatography afforded 40 (35 mg, 48%).

¹H NMR (300MHz, DMSO-*d*₆) δ 12.10 (s, 1H), 11.01 (s, 1H), 8.28 (d, J = 2.1Hz, 1H), 7.93 (dd, J = 8.9, 2.1Hz, 1H), 7.77 (d, J = 8.8Hz, 1H), 7.69 (d, J = 7.8Hz, 1H), 7.52 (s, 1H), 7.44 (dd, J = 8.0, 1.2Hz, 1H), 5.11 (dd, J = 13.2, 5.1Hz, 1H), 4.55 (s, 1H), 4.45 (d, J = 17.3Hz, 1H), 4.31 (d, J = 17.3Hz, 1H), 3.85 (s, 1H), 3.13-2.82 (m, 4H), 2.65-2.56 (m, 1H), 2.44-2.36 (m, 1H), 2.34 (s, 3H), 2.02-1.82 (m, 3H), 1.75-1.57 (m, 2H)).

### Synthesis of 3-(5-(1-(6-amino-3-methyl-1H-indole-2-carbonyl)piperidin-4-yl)-1-oxoinsoindolin-2-yl)piperidine-2,6-dione (41)

A solution of 40 (5 mg, 0.01 mmol, 1 eq) and 10% Pd/C (5 mg) in DMF (1 mL) was stirred for 12 hours under a hydrogen pressure. After completion of the reaction, the reaction mixture was filtered through Celite. The filtrate was diluted with water and then subjected to extraction with ethylacetate. The organic layer thus formed was pooled and dried over magnesium sulfate, followed by filtration and concentration to afford 41 (13 mg, 92%).

¹H NMR (500MHz, DMSO-*d*₆) δ 11.00 (s, 1H), 10.56 (s, 1H), 7.68 (d, J = 7.8Hz, 1H), 7.52 (s, 1H), 7.43 (d, J = 7.9Hz, 1H), 7.19 (d, J = 8.3Hz, 1H), 6.49 (s, 1H), 6.42 (d, J = 8.5Hz, 1H), 5.11 (dd, J = 13.4, 5.1Hz, 1H), 4.91 (s, 2H), 4.44 (d, J = 17.2Hz, 1H), 4.36-4.21 (m, 3H), 3.11-2.90 (m, 4H), 2.67-2.60 (m, 1H), 2.42 -2.36 (m, 1H), 2.22 (s, 3H), 2.04-1.94 (m, 1H), 1.92-1.83 (m, 2H), 1.74-1.60 (m, 2H).

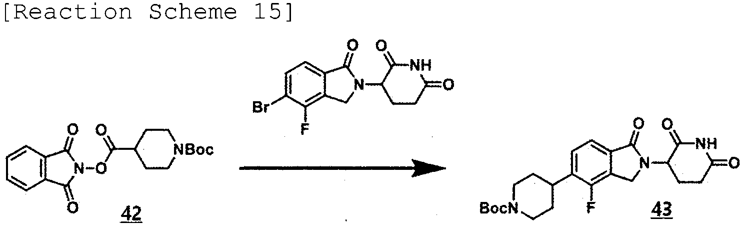

### Synthesis of tert-butyl-4 (2-(2,6-dioxopiperidin-3-yl)-4-fluoro-1-oxoindolin-5-yl)piperidine-1-carboxylate (43)

A solution of 42 (52.1 mg, 0.139 mmol), 3-(5-bromo-4-fluoro-1-oxoisoindolin-2-yl)piperidine-2,6-dione (30 mg, 0.088 mmol), NiBR₂ (dtbbpy) (22.47 mg, 0.046 mmol), Hantzsch ester (47.0 mg, 0.186 mmol), and NaHCO₃ (31.2 mg, 0.371 mmol) in DMA (1 mL) was purged with nitrogen for 15 min and then stirred at room temperature for 12 hours under purple LEDs (160~390nm). After completion of the reaction, the reaction mixture was diluted with water and subjected to extraction with EA. The organic layer thus formed was washed with brine. The organic was pooled and concentrated in a vacuum. Thereafter, purification by column chromatography afforded 43.

¹H NMR (400 MHz, DMSO-*d*₆) δ 11.00 (s, 1H), 7.57-7.50 (m, 2H), 5.11 (dd, J = 13.3, 5.1 Hz, 1H), 4.55 (d, J = 17.4 Hz, 1H), 4.38 (d, J = 17.3 Hz, 1H), 4.15-4.04 (m, 2H), 3.16-3.07 (m, 1H), 2.98-2.80 (m, 3H), 2.64-2.57 (m, 1H), 2.47-2.39 (m, 1H), 2.04-1.96 (m, 1H), 1.80-1.72 (m, 2H), 1.67-1.55 (m, 2H), 1.43 (s, 9H).

### <EXAMPLE 1. Synthesis and Physicochemical Characterization of Indole Derivative Compounds for Degrading IKZF2>

Preparation processes and physicochemical properties of Compounds 1 to 127 of the present disclosure are as follows:

### Compound 1. Preparation of 3-(5-(1-(6-chloro-1H-indole-2-carbonyl)piperidin-4-yl)-1-oxoinsoindolin-2-yl)piperidine-2,6-dione)

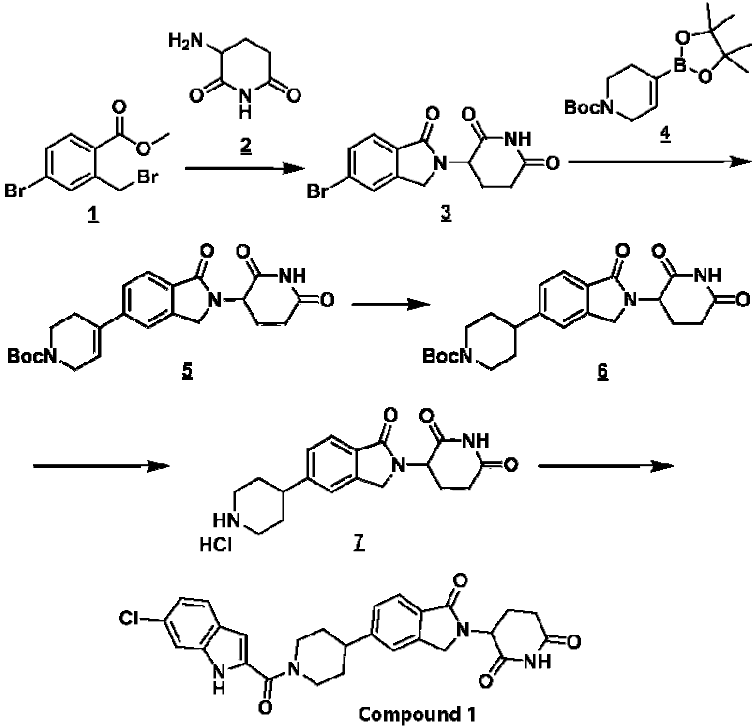

### Compound 1. 3-(5-(1-(6-chloro-1H-indole-2-carbonyl)piperidin-4-yl)-1-oxoinsoindolin-2-yl)piperidine-2,6-dione

A solution of Intermediate 7 (20 mg, 0.055 mmol), 6-chloro-1H-indole-2-carboxylic acid (10.8 mg, 0.055 mmol), EDCI-HCl (11.6 mg, 0.06 mmol), HOBt (8.2 mg, 0.06 mmol), and DIPEA (29 uL, 0.165 mmol) in DMF (1 mL) was stirred at room temperature for 12 hours. After completion of the reaction, the reaction mixture was diluted with water and subjected to extraction with ethylacetate. The organic layers thus obtained were pooled, dried over anhydrous magnesium sulfate, filtered, and concentrated. The concentrate mixture was subjected to column chromatography to afford Compound 1 (14 mg, 52%).

¹H NMR (300 MHz, DMSO-*d*₆) δ 13.62 (s, 1H), 10.98 (s, 1H), 8.00 (d, J = 8.7 Hz, 1H), 7.71 (d, J = 1.8 Hz, 1H), 7.66 (d, J = 7.9 Hz, 1H), 7.54 (s, 1H), 7.44 (dd, J = 7.9, 1.4 Hz, 1H), 7.25 (dd, J = 8.7, 1.8 Hz, 1H), 5.10 (dd, J = 13.2, 5.1 Hz, 1H), 4.90 (d, J = 13.1 Hz, 1H), 4.78 (d, J = 12.8 Hz, 1H), 4.42 (d, J = 17.3 Hz, 1H), 4.28 (d, J = 17.3 Hz, 1H), 3.09-2.83 (m, 4H), 2.65-2.53 (m, 1H), 2.39 (dd, J = 13.2, 4.6 Hz, 1H), 2.05-1.83 (m, 3H), 1.77-1.65 (m, 2H).

### Compound 2. 3-(5-(1-(6,7-dichloro-1H-indole-2-carbonyl)piperidin-4-yl)-1-oxoinsoindolin-2-yl)piperidine-2,6-dione

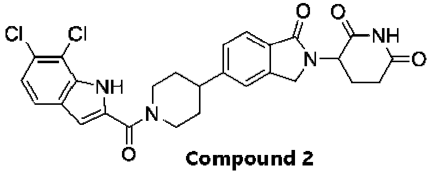

Compound 2 was synthesized in the same manner as for Compound 1, but for using Intermediate 7 and 6,7-dichloro-1H-indole-2-carboxylic acid instead of 6-chloro-1H-indole-2-carboxylic acid.

¹H NMR (500 MHz, DMSO-d6) δ 12.07 (s, 1H), 11.00 (s, 1H), 7.69 (d, J = 7.9 Hz, 1H), 7.62 (d, J = 8.5 Hz, 1H), 7.54 (s, 1H), 7.46 (d, J = 8.0 Hz, 1H), 7.27 (d, J = 8.5 Hz, 1H), 6.87 (s, 1H), 5.12 (dd, J = 13.9, 4.9 Hz, 1H), 4.45 (d, J = 17.2 Hz, 1H), 4.31 (d, J = 17.2 Hz, 1H), 3.26 (s, 1H), 3.10-2.90 (m, 3H), 2.61 (d, J = 17.2 Hz, 2H), 2.55 (s, 1H), 2.01 (d, J = 8.8 Hz, 2H), 1.90 (s, 2H), 1.74 (s, 2H).

### Compound 3. 3-(5-(1-(5-chloro-1H-indole-2-carbonyl)piperidin-4-yl)-1-oxoinsoindolin-2-yl)piperidine-2,6-dione

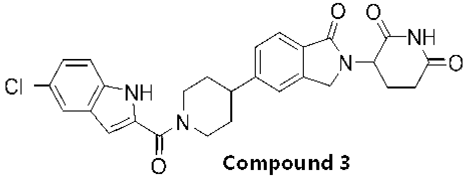

Compound 3 was synthesized in the same manner as for Compound 1, using Intermediate 7 and 5-chloro-1H-indole-2-carboxylic acid instead of 6-chloro-1H-indole-2-carboxylic acid.

¹H NMR (500 MHz, DMSO-d6) δ 11.82 (s, 1H), 11.00 (s, 1H), 7.70-7.66 (m, 2H), 7.56 (s, 1H), 7.45 (t, J = 9.2 Hz, 2H), 7.20 (dd, J = 8.7, 2.2 Hz, 1H), 6.82 (s, 1H), 5.12 (d, J = 12.0 Hz, 1H), 4.60 (s, 2H), 4.44 (d, J = 17.2 Hz, 1H), 4.31 (d, J = 17.2 Hz, 1H), 3.05 (s, 2H), 2.92 (s, 1H), 2.61 (d, J = 16.5 Hz, 2H), 2.38 (d, J = 12.7 Hz, 1H), 2.00 (s, 1H), 1.93 (d, J = 12.8 Hz, 2H), 1.74 (s, 2H).

### Compound 4. 3-(5-(1-(4-chloro-1H-indole-2-carbonyl)piperidin-4-yl)-1-oxoinsoindolin-2-yl)piperidine-2,6-dione

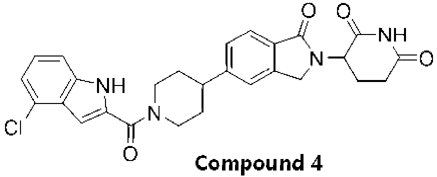

Compound 4 was synthesized in the same manner as for Compound 1, using Intermediate 7 and 4-chloro-1H-indole-2-carboxylic acid instead of 6-chloro-1H-indole-2-carboxylic acid.

¹H NMR (500 MHz, DMSO-d6) δ 12.00 (s, 1H), 11.00 (s, 1H), 7.68 (d, J = 8.0 Hz, 1H), 7.57 (s, 1H), 7.47 (d, J = 8.1 Hz, 1H), 7.42 (d, J = 8.4 Hz, 1H), 7.20 (t, J = 7.8 Hz, 1H), 7.15 (d, J = 7.4 Hz, 1H), 6.81 (s, 1H), 5.12 (dd, J = 13.4, 4.9 Hz, 1H), 4.60 (s, 2H), 4.44 (d, J = 17.2 Hz, 1H), 4.31 (d, J = 17.2 Hz, 1H),3.10-2.89 (m, 3 H), 2.61 (d, J = 17.4 Hz, 2H), 2.44-2.37 (m, 1H), 2.00 (s, 1H), 1.93 (d, J = 11.8 Hz, 2H), 1.76 (s, 2H).

### Compound 5. 3-(5-(1-(6-methoxy-1H-indole-2-carbonyl)piperidin-4-yl)-1-oxoinsoindolin-2-yl)piperidine-2,6-dione

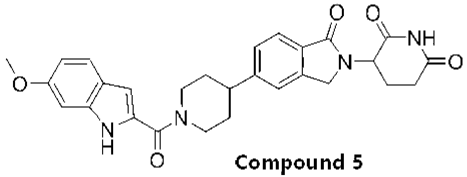

Compound 5 was synthesized in the same manner as for Compound 1, using Intermediate 7 and 6-methoxy-1H-indole-2-carboxylic acid instead of 6-chloro-1H-indole-2-carboxylic acid.

¹H NMR (500 MHz, DMSO-d6) δ 11.40 (s, 1H), 11.00 (s, 1H), 7.68 (d, J = 7.9 Hz, 1H), 7.55 (s, 1H), 7.48 (dd, J = 14.7, 8.5 Hz, 2H), 6.90 (d, J = 2.7 Hz, 1H), 6.78 (d, J = 2.4 Hz, 1H), 6.71 (dd, J = 8.9, 2.3 Hz, 1H), 5.12 (dd, J = 13.4, 4.9 Hz, 1H), 4.64 (d, J = 13.0 Hz, 2H), 4.44 (d, J = 17.2 Hz, 1H), 4.31 (d, J = 17.2 Hz, 1H), 3.78 (s, 3H), 3.21-3.00 (m, 3H), 2.97-2.88 (m, 1H), 2.65-2.59 (m, 1H), 2.45-2.36 (m, 1H), 2.00 (s, 1H), 1.92 (d, J = 12.8 Hz, 2H), 1.72 (d, J = 13.0 Hz, 2H)

### Compound 6. 3-(5-(1-(4,6-dichloro-1H-indole-2-carbonyl)piperidin-4-yl)-1-oxoinsoindolin-2-yl)piperidine-2,6-dione

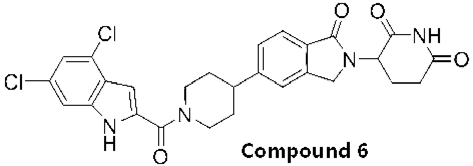

Compound 6 was synthesized in the same manner as for Compound 6, using Intermediate 7 and 4,6-dichloro-1H-indole-2-carboxylic acid instead of 6-chloro-1H-indole-2-carboxylic acid.

¹H NMR (500 MHz, DMSO-d6) δ 12.15 (s, 1H), 11.00 (s, 1H), 7.68 (d, J = 7.9 Hz, 1H), 7.56 (s, 1H), 7.47 (d, J = 10.6 Hz, 2H), 7.26 (d, J = 2.2 Hz, 1H), 6.84 (d, J = 2.9 Hz, 1H), 5.12 (dd, J = 13.3, 5.0 Hz, 1H), 4.58 (s, 2H), 4.44 (d, J = 17.2 Hz, 1H), 4.31 (d, J = 17.2 Hz, 1H), 3.09-3.00 (m, 1H), 2.92 (t, J = 15.3 Hz, 1H), 2.62-2.59 (m, 1H), 2.44-2.37 (m, 1H), 1.99 (d, J = 10.4 Hz, 1H), 1.93 (d, J = 12.6 Hz, 2H), 1.75 (s, 2H).

### Compound 7. 3-(5-(1-(6-fluoro-1H-indole-2-carbonyl)piperidin-4-yl)-1-oxoinsoindolin-2-yl)piperidine-2,6-dione

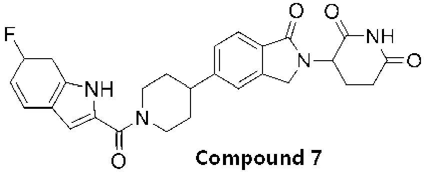

Compound 7 was synthesized in the same manner as for Compound 1, using Intermediate 7 and 6-fluoro-1H-indole-2-carboxylic acid instead of 6-chloro-1H-indole-2-carboxylic acid.

¹H NMR (500 MHz, DMSO-d6) δ 11.67 (s, 1H), 10.99 (s, 1H), 7.67 (d, J = 8.0 Hz, 1H), 7.63 (dd, J = 8.7, 5z.8 Hz, 1H), 7.54 (s, 1H), 7.45 (d, J = 8.0 Hz, 1H), 7.15 (d, J = 10.1 Hz, 1H), 6.95-6.89 (m, 1H), 6.86 (s, 1H), 5.11 (dd, J = 13.3, 4.9 Hz, 1H), 4.61 (d, J = 12.9 Hz, 2H), 4.43 (d, J = 17.1 Hz, 1H), 4.30 (d, J = 17.2 Hz, 1H), 3.12-2.87 (m, 3H), 2.59 (d, J = 17.1 Hz, 1H), 2.44-2.36 (m, 1H), 2.01-1.96 (m, 1H), 1.91 (d, J = 12.8 Hz, 2H), 1.79-1.66 (m, 2H).

### Compound 8. 3-(5-(1-(7-chloro-1H-indole-2-carbonyl)piperidin-4-yl)-1-oxoinsoindolin-2-yl)piperidine-2,6-dione

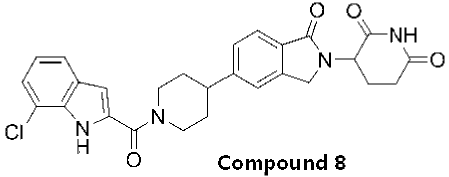

Compound 8 was synthesized in the same manner as for Compound 1, using Intermediate 7 and 7-chloro-1H-indole-2-carboxylic acid instead of 6-chloro-1H-indole-2-carboxylic acid.

¹H NMR (500 MHz, DMSO-d6) δ 11.85 (s, 1H), 11.01 (s, 1H), 7.69 (d, J = 7.9 Hz, 1H), 7.60 (d, J = 8.0 Hz, 1H), 7.55 (s, 1H), 7.46 (d, J = 8.0 Hz, 1H), 7.27 (d, J = 7.6 Hz, 1H), 7.07 (t, J = 7.7 Hz, 1H), 6.85 (s, 1H), 5.12 (dd, J = 13.4, 4.9 Hz, 1H), 4.66 (s, 1H), 4.45 (d, J = 17.1 Hz, 1H), 4.31 (d, J = 17.2 Hz, 1H), 4.25 (s, 1H), 3.09-2.89 (m, 3H), 2.61 (k, J = 17.1 Hz, 1H), 2.45-2.36 (m, 1H), 2.06-1.95 (m, 1H), 1.90 (s, 2H), 1.74 (s, 2H).

### Compound 9. 3-(5-(1-(1H-indole-2-carbonyl)piperidin-4-yl)-1-oxoinsoindolin-2-yl)piperidine-2,6-dione

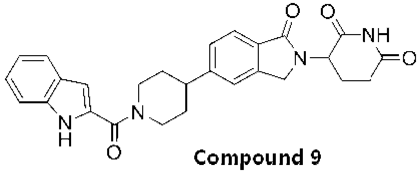

Compound 9 was synthesized in the same manner as for Compound 1, using Intermediate 7 and 1H-indole-2-carboxylic acid instead of 6-chloro-1H-indole-2-carboxylic acid.

¹H NMR (300 MHz, DMSO-d6) δ 11.59 (m, 1H), 11.00 (s, 1H), 7.68 (d, J = 7.9 Hz, 1H), 7.62 (d, J = 8.0 Hz, 1H), 7.55 (s, 1H), 7.45 (t, J = 8.1 Hz, 2H), 7.19 (t, J = 7.6 Hz, 1H), 7.05 (t, J = 7.5 Hz, 1H), 6.83 (d, J = 2.1 Hz, 1H), 4.63 (d, J = 12.7 Hz, 1H), 4.44 (d, J = 17.2 Hz, 1H), 4.30 (d, J = 17.3 Hz, 1H), 3.28-2.85 (m, 4H), 2.64-2.56 (m, 1H), 2.44-2.32 (m, 1H), 2.04-1.88 (m, 3H), 1.80-1.65 (m, 2H).

### Compound 10. 3-(5-(1-(5-methoxy-1H-indole-2-carbonyl)piperidin-4-yl)-1-oxoinsoindolin-2-yl)piperidine-2,6-dione

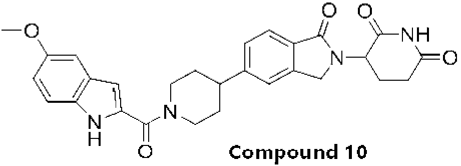

Compound 10 was synthesized in the same manner as for Compound 1, using Intermediate 7 and 5-methoxy-1H-indole-2-carboxylic acid instead of 6-chloro-1H-indole-2-carboxylic acid.

¹H NMR (300 MHz, DMSO-d6) δ 11.44 (s, 1H), 11.00 (s, 1H), 7.68 (d, J = 7.8 Hz, 1H), 7.55 (s, 1H), 7.46 (d, J = 7.9 Hz, 1H), 7.32 (d, J = 8.9 Hz, 1H), 7.08 (d, J = 2.4 Hz, 1H), 6.84 (dd, J = 8.9, 2.5 Hz, 1H), 6.74 (d, J = 2.1 Hz, 1H), 5.11 (dd, J = 13.3, 5.1 Hz, 1H), 4.62 (d, J = 13.0 Hz, 2H), 4.44 (d, J = 17.3 Hz, 1H), 4.30 (d, J = 17.3 Hz, 1H), 3.76 (s, 3H), 3.23-2.85 (m, 4H), 2.64-2.56 (m, 1H), 2.45-2.26 (m, 1H), 2.04-1.87 (m, 3H), 1.80-1.65 (m, 2H).

### Compound 11. 3-(5-(1-(4-methoxy-1H-indole-2-carbonyl)piperidin-4-yl)-1-oxoinsoindolin-2-yl)piperidine-2,6-dione

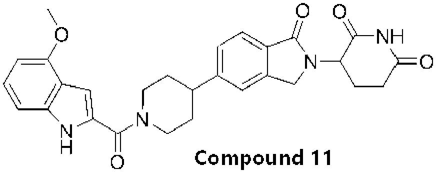

Compound 11 was synthesized in the same manner as for Compound 1, using Intermediate 7 and 4-methoxy-1H-indole-2-carboxylic acid instead of 6-chloro-1H-indole-2-carboxylic acid.

¹H NMR (300 MHz, DMSO-d6) δ 11.60 (d, J = 2.3 Hz, 1H), 10.99 (s, 1H), 7.67 (d, J = 7.8 Hz, 1H), 7.56 (s, 1H), 7.46 (d, J = 8.0 Hz, 1H), 7.10 (d, J = 7.8 Hz, 1H), 7.02 (d, J = 8.2 Hz, 1H), 6.78 (d, J = 2.2 Hz, 1H), 6.53 (d, J = 7.5 Hz, 1H), 5.11 (dd, J = 13.3, 5.0 Hz, 1H), 4.63 (d, J = 12.9 Hz, 2H), 4.44 (d, J = 17.2 Hz, 1H), 4.30 (d, J = 17.3 Hz, 1H), 3.88 (s, 3H), 3.24-2.91 (m, 4H), 2.62 (s, 1H), 2.57 (s, 1H), 2.05-1.90 (m, 3H), 1.73 (d, J = 12.8 Hz, 2H).

### Compound 12. 3-(5-(1-(7-methoxy-1H-indole-2-carbonyl)piperidin-4-yl)-1-oxoinsoindolin-2-yl)piperidine-2,6-dione

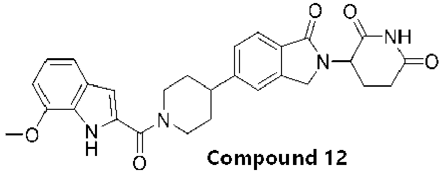

Compound 12 was synthesized in the same manner as for Compound 1, using Intermediate 7 and 7-methoxy-1H-indole-2-carboxylic acid instead of 6-chloro-1H-indole-2-carboxylic acid.

¹H NMR (300 MHz, DMSO-d6) δ 11.48 (s, 1H), 10.99 (s, 1H), 7.68 (d, J = 7.9 Hz, 1H), 7.54 (s, 1H), 7.45 (d, J = 7.9 Hz, 1H), 7.18 (d, J = 8.0 Hz, 1H), 6.97 (t, J = 7.8 Hz, 1H), 6.76-6.69 (m, 2H), 5.11 (dd, J = 13.3, 5.1 Hz, 1H), 4.44 (d, J = 17.3 Hz, 1H), 4.30 (d, J = 17.3 Hz, 1H), 3.92 (s, 3H), 3.18-2.82 (m, 4H), 2.63 (s, 1H), 2.57 (s, 1H), 2.08-1.65 (m, 5H).

### Compound 13. 3-(5-(1-(5-methyl-1H-indole-2-carbonyl)piperidin-4-yl)-1-oxoinsoindolin-2-yl)piperidine-2,6-dione

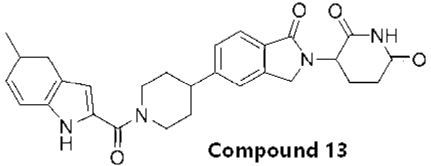

Compound 13 was synthesized in the same manner as for Compound 1, using Intermediate 7 and 5-methyl-1H-indole-2-carboxylic acid instead of 6-chloro-1H-indole-2-carboxylic acid.

¹H NMR (300 MHz, DMSO-d6) δ 11.44 (s, 1H), 10.99 (s, 1H), 7.68 (d, J = 7.9 Hz, 1H), 7.55 (s, 1H), 7.46 (d, J = 7.9 Hz, 1H), 7.38 (s, 1H), 7.32 (d, J = 8.4 Hz, 1H), 7.02 (dd, J = 8.4, 1.6 Hz, 1H), 6.72 (d, J = 2.1 Hz, 1H), 5.11 (dd, J = 13.2, 5.1 Hz, 1H), 4.62 (d, J = 13.0 Hz, 2H), 4.44 (d, J = 17.3 Hz, 1H), 4.30 (d, J = 17.2 Hz, 1H), 3.23-2.98 (m, 3H), 2.93-2.84 (m, 1H), 2.63 (s, 1H), 2.57 (s, 1H), 2.37 (s, 3H), 2.04-1.88 (m, 3H), 1.80-1.65 (m, 2H).

### Compound 14. 3-(5-(1-(4-methyl-1H-indole-2-carbonyl)piperidin-4-yl)-1-oxoinsoindolin-2-yl)piperidine-2,6-dione

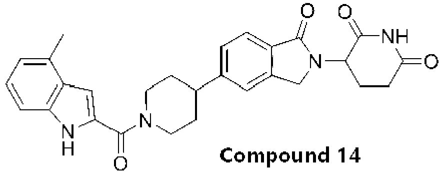

Compound 14 was synthesized in the same manner as for Compound 1, using Intermediate 7 and 4-methyl-1H-indole-2-carboxylic acid instead of 6-chloro-1H-indole-2-carboxylic acid.

¹H NMR (300 MHz, DMSO-d6) δ 11.54 (s, 1H), 10.98 (s, 1H), 7.67 (d, J = 7.8 Hz, 1H), 7.55 (s, 1H), 7.46 (d, J = 8.2 Hz, 1H), 7.24 (d, J = 8.2 Hz, 1H), 7.07 (dd, J = 8.2, 7.0 Hz, 1H), 6.84 (s, 1H), 6.83 (d, J = 2.5 Hz, 1H), 5.11 (dd, J = 13.3, 5.0 Hz, 1H), 4.64 (d, J = 12.9 Hz, 2H), 4.44 (d, J = 17.2 Hz, 1H), 4.30 (d, J = 17.2 Hz, 1H), 3.21-2.96 (m, 3H), 2.94-2.84 (m, 1H), 2.65-2.55 (m, 1H), 2.49 (s, 3H), 2.44-2.35 (m, 1H), 2.03-1.89 (m, 3H), 1.80-1.60 (m, 2H).

### Compound 15. 3-(5-(1-(6-methyl-1H-indole-2-carbonyl)piperidin-4-yl)-1-oxoinsoindolin-2-yl)piperidine-2,6-dione

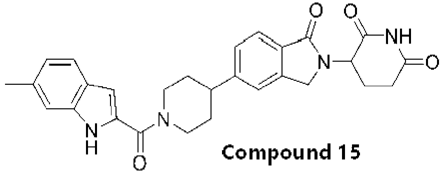

Compound 15 was synthesized in the same manner as for Compound 1, using Intermediate 7 and 6-methyl-1H-indole-2-carboxylic acid instead of 6-chloro-1H-indole-2-carboxylic acid.

¹H NMR (300 MHz, DMSO-d6) δ 11.42 (s, 1H), 10.99 (s, 1H), 7.68 (d, J = 7.8 Hz, 1H), 7.55 (s, 1H), 7.47 (t, J = 8.8 Hz, 2H), 7.22 (s, 1H), 6.89 (dd, J = 8.2, 1.5 Hz, 1H), 6.77 (d, J = 2.1 Hz, 1H), 5.11 (dd, J = 13.2, 5.1 Hz, 1H), 4.63 (d, J = 12.9 Hz, 2H), 4.44 (d, J = 17.3 Hz, 1H), 4.30 (d, J = 17.2 Hz, 1H), 3.20-3.00 (m, 3H), 2.94-2.84 (m, 1H), 2.63 (s, 1H), 2.57 (s, 1H), 2.40 (s, 3H), 2.06-1.86 (m, 3H), 1.78-1.63 (m, 2H).

### Compound 16. 3-(5-(1-(7-methyl-1H-indole-2-carbonyl)piperidin-4-yl)-1-oxoinsoindolin-2-yl)piperidine-2,6-dione

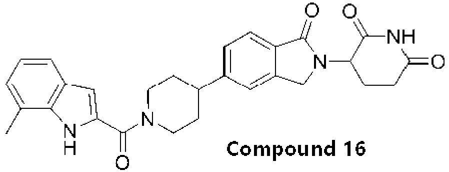

Compound 16 was synthesized in the same manner as for Compound 1, using Intermediate 7 and 7-methyl-1H-indole-2-carboxylic acid instead of 6-chloro-1H-indole-2-carboxylic acid.

¹H NMR (300 MHz, DMSO-d6) δ 11.44 (s, 1H), 10.99 (s, 1H), 7.68 (d, J = 7.9 Hz, 1H), 7.55 (s, 1H), 7.48-7.40 (m, 2H), 6.96 (d, J = 6.1 Hz, 2H), 6.78 (d, J = 2.0 Hz, 1H), 5.11 (dd, J = 13.2, 5.1 Hz, 1H), 4.59 (d, J = 12.5 Hz, 2H), 4.44 (d, J = 17.3 Hz, 1H), 4.31 (d, J = 17.3 Hz, 1H), 3.20-2.96 (m, 3H), 2.92-2.86 (m, 1H), 2.60 (d, J = 16.9 Hz, 1H), 2.43-2.37 (m, 1H), 2.04-1.88 (m, 3H), 1.81-1.63 (m, 2H).

### Compound 17. 3-(5-(1-(5-fluoro-1H-indole-2-carbonyl)piperidin-4-yl)-1-oxoinsoindolin-2-yl)piperidine-2,6-dione

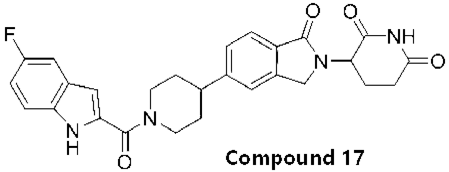

Compound 17 was synthesized in the same manner as for Compound 1, using Intermediate 7 and 5-fluoro-1H-indole-2-carboxylic acid instead of 6-chloro-1H-indole-2-carboxylic acid.

¹H NMR (300 MHz, DMSO-d6) δ 11.67 (s, 1H), 10.96 (s, 1H), 7.67 (d, J = 7.9 Hz, 1H), 7.54 (s, 1H), 7.47-7.33 (m, 3H), 7.07-7.00 (m, 1H), 6.80 (d, J = 2.1 Hz, 1H), 5.10 (dd, J = 13.3, 5.1 Hz, 1H), 4.59 (d, J = 13.0 Hz, 2H), 4.43 (d, J = 17.2 Hz, 1H), 4.29 (d, J = 17.3 Hz, 1H), 3.20-2.83 (m, 4H), 2.59 (d, J = 18.0 Hz, 1H), 2.42-4.36 (mz, 1H), 2.03-1.88 (m, 3H), 1.78-1.70 (m, 2H) .

### Compound 18. 3-(5-(1-(6-hydroxy-1H-indole-2-carbonyl)piperidin-4-yl)-1-oxoinsoindolin-2-yl)piperidine-2,6-dione

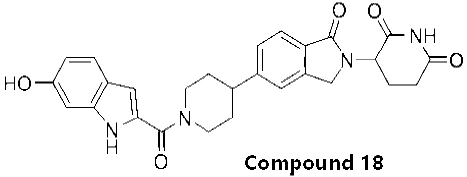

Compound 18 was synthesized in the same manner as for Compound 1, using Intermediate 7 and 6-hydroxy-1H-indole-2-carboxylic acid instead of 6-chloro-1H-indole-2-carboxylic acid.

¹H NMR (300 MHz, DMSO-d6) δ 11.14 (s, 1H), 10.97 (s, 1H), 9.15 (s, 1H), 7.67 (d, J = 7.8 Hz, 1H), 7.54 (s, 1H), 7.45 (d, J = 7.9 Hz, 1H), 7.38 (d, J = 8.6 Hz, 1H), 6.79 (d, J = 2.1 Hz, 1H), 6.72 (d, J = 2.1 Hz, 1H), 6.58 (dd, J = 8.6, 2.1 Hz, 1H), 5.11 (dd, J = 13.2, 5.1 Hz, 1H), 4.64 (d, J = 13.0 Hz, 2H), 4.44 (d, J = 17.2 Hz, 1H), 4.30 (d, J = 17.3 Hz, 1H), 3.19-2.84 (m, 4H), 2.60 (d, J = 17.3 Hz, 1H), 2.45-2.26 (m, 1H), 2.02-1.90 (m, 3H), 1.78-1.65 (m, 2H).

### Compound 19. 3-(5-(1-(7-fluoro-1H-indole-2-carbonyl)piperidin-4-yl)-1-oxoinsoindolin-2-yl)piperidine-2,6-dione

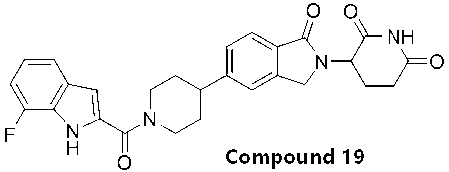

Compound 19 was synthesized in the same manner as for Compound 1, using Intermediate 7 and 7-fluoro-1H-indole-2-carboxylic acid instead of 6-chloro-1H-indole-2-carboxylic acid.

¹H NMR (300 MHz, DMSO-d6) δ 12.04 (s, 1H), 10.97 (s, 1H), 7.68 (d, J = 7.8 Hz, 1H), 7.55 (s, 1H), 7.45 (dd, J = 8.6, 5.8 Hz, 2H), 7.07-6.98 (m, 2H), 6.86 (d, J = 3.1 Hz, 1H), 5.11 (dd, J = 13.2, 5.1 Hz, 1H), 4.45 (d, J = 17.2 Hz, 3H), 4.31 (d, J = 17.2 Hz, 1H), 3.22-2.83 (m, 4H), 2.64-2.56 (m, 1H), 2.43-2.35 (m, 1H), 2.07-1.84 (m, 4H), 1.80-1.65 (m 2H).

### Compound 20. 3-(5-(1-(4-fluoro-1H-indole-2-carbonyl)piperidin-4-yl)-1-oxoinsoindolin-2-yl)piperidine-2,6-dione

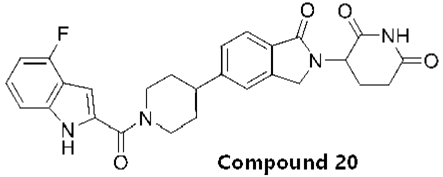

Compound 20 was synthesized in the same manner as for Compound 1, using Intermediate 7 and 4-fluoro-1H-indole-2-carboxylic acid instead of 6-chloro-1H-indole-2-carboxylic acid.

¹H NMR (300 MHz, DMSO-d6) δ 11.92 (s, 1H), 10.96 (s, 1H), 7.68 (d, J = 7.8 Hz, 1H), 7.56 (s, 1H), 7.46 (d, J = 8.0 Hz, 1H), 7.28 (d, J = 8.2 Hz, 1H), 7.121-7.13 (m, 1H), 6.89-6.80 (m, 2H), 5.09 (d, J = 5.1 Hz, 1H), 4.60 (d, J = 13.1 Hz, 2H), 4.44 (d, J = 17.2 Hz, 1H), 4.31 (d, J = 17.3 Hz, 1H), 3.24-2.86 (m, 4H), 2.63-2.57 (m, 1H), 2.44-2.38 (m, 1H), 2.09-1.89 (m, 3H), 1.85-1.70 (m, 2H).

### Compound 21. 3-(5-(1-(3-methyl-1H-indole-2-carbonyl)piperidin-4-yl)-1-oxoinsoindolin-2-yl)piperidine-2,6-dione

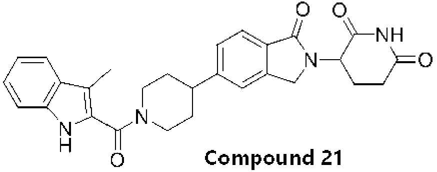

Compound 21 was synthesized in the same manner as for Compound 1, using Intermediate 7 and 3-methyl-1H-indole-2-carboxylic acid instead of 6-chloro-1H-indole-2-carboxylic acid.

¹H NMR (300 MHz, DMSO-d6) δ 11.20 (s, 1H), 10.97 (s, 1H), 7.68 (d, J = 7.9 Hz, 1H), 7.58-7.50 (m, 2H), 7.43 (d, J = 7.9 Hz, 1H), 7.35 (d, J = 8.1 Hz, 1H), 7.21-7.12 (m, 1H), 7.04 (t, J = 7.4 Hz, 1H), 5.11 (dd, J = 13.2, 5.1 Hz, 1H), 4.45 (d, J = 17.2 Hz, 1H), 4.31 (d, J = 17.2 Hz, 3H), 3.17-2.84 (m, 4H), 2.65-2.55 (m 1H), 2.45-2.35 (m, 1H), 2.31 (s, 3H), 2.04-1.84 (m, 3H), 1.77-1.59 (m, 2H).

### Compound 22. 3-(5-(1-(3-chloro-1H-indole-2-carbonyl)piperidin-4-yl)-1-oxoinsoindolin-2-yl)piperidine-2,6-dione

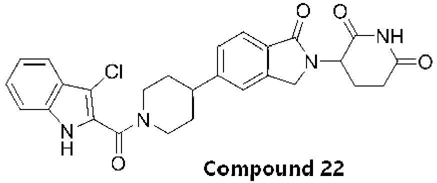

Compound 22 was synthesized in the same manner as for Compound 1, using Intermediate 7 and 3-chloro-1H-indole-2-carboxylic acid instead of 6-chloro-1H-indole-2-carboxylic acid.

¹H NMR (300 MHz, DMSO-d6) δ 11.91 (s, 1H), 10.97 (s, 1H), 7.69 (d, J = 7.9 Hz, 1H), 7.58-7.50 (m, 2H), 7.44 (t, J = 6.8 Hz, 1H), 7.31-7.24 (m, 1H), 7.18 (t, J = 7.5 Hz, 1H), 5.11 (dd, J = 13.2, 5.1 Hz, 1H), 4.60 (s, 1H), 4.45 (d, J = 17.3 Hz, 1H), 4.31 (d, J = 17.2 Hz, 1H), 3.89 (s, 1H), 3.24-2.81 (m, 3H), 2.61 (d, J = 17.6 Hz, 2H), 2.09-1.84 (m, 3H), 1.77-1.68 (m, 2H).

### Compound 23. 3-(5-(3-(1-(1H-indole-3-carbonyl)piperidin-4-yl)prop-1-yn-1-yl)-1-oxoinsoindolin-2-yl)piperidine-2,6-dione

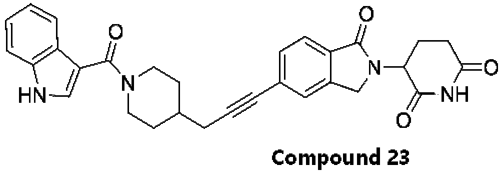

Compound 28 was synthesized in the same manner as for Compound 1, with the exception of using Intermediate 28 and 1H-indole-2-carboxylic acid.

¹H NMR (300 MHz, DMSO-d6) δ 11.55 (s, 1H), 11.00 (s, 1H), 7.70-7.64 (m, 4H), 7.52 (d, J = 7.9 Hz, 1H), 7.44 (d, J = 7.9 Hz, 1H), 7.19-7.05 (m, 2H), 5.11 (dd, J = 13.3, 5.1 Hz, 1H), 4.45 (d, J = 17.5 Hz, 1H), 4.32 (d, J = 12.6 Hz, 3H), 3.17 (d, J = 5.3 Hz, 1H), 3.03-2.83 (m, 3H), 2.60 (d, J = 18.0 Hz, 2H), 2.42 -2.25 (m, 1H), 2.03-1.72 (m, 4H), 1.39-1.19 (m, 2H).

### Compound 24. 3-(5-((1-(1H-indole-2-carbonyl)piperidin-4-ylidene)methyl)-1-oxoinsoindolin-2-yl)piperidine-2,6-dione

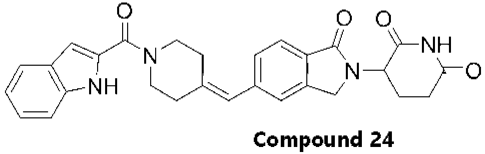

Compound 24 was synthesized in the same manner as for Compound 1, with the exception of using Intermediate 25 and 1H-indole-2-carboxylic acid.

¹H NMR (300 MHz, DMSO-d6 )δ 11.61 (s, 1H), 11.00 (s, 1H), 7.71 (d, J = 7.9 Hz, 1H), 7.62 (d, J = 8.0 Hz, 1H), 7.50 (s, 1H), 7.42 (t, J = 8.6 Hz, 2H),7.19 (t, J = 7.6 Hz, 1H), 7.05 (t, J = 7.6 Hz, 1H), 6.84 (d, J = 2.1 Hz, 1H), 6.57 (s, 1H), 5.12 (dd, J = 13.3, 5.0 Hz, 1H), 4.47 (d, J = 17.3 Hz, 1H), 4.33 (d, J = 17.3 Hz, 1H), 3.87 (s, 2H), 3.78 (s, 2H), 2.99-2.83 (m, 1H), 2.65-2.60 (m, 4H), 2.53-2.41 (m, 1H), 2.05-1.94 (m, 1H).

### Compound 25. 3-(5-((1-(1H-indole-3-carbonyl)piperidin-4-ylidene)methyl)-1-oxoinsoindolin-2-yl)piperidine-2,6-dione

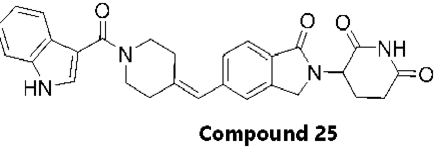

Compound 25 was synthesized in the same manner as for Compound 1, with the exception of using Intermediate 25 and 1H-indole-2-carboxylic acid.

¹H NMR (300 MHz, DMSO-d6) δ 11.60 (s, 1H), 11.00 (s, 1H), 7.72 (t, J = 3.4 Hz, 2H), 7.69 (d, J = 3.8 Hz, 1H), 7.48 (s, 1H), 7.45 (d, J = 8.0 Hz, 1H), 7.38 (d, J = 7.8 Hz, 1H), 7.20-7.13 (m, 1H), 7.13-7.07 (m, 1H), 6.54 (s, 1H), 5.12 (dd, J = 13.2, 5.0 Hz, 1H), 4.45 (d, J = 17.4 Hz, 1H), 4.32 (d, J = 17.3 Hz, 1H), 3.75 (t, J = 5.8 Hz, 2H), 3.65 (d, J = 6.3 Hz, 2H), 2.99-2.85 (m, 1H), 2.65-2.53 (m, 4H), 2.47-2.40 (m, 2H), 2.03-1.95 (m, 1H).

### Compound 26. 3-(5-((1-(6-chloro-1H-indole-2-carbonyl)piperidin-4-ylidene)methyl)-1-oxoinsoindolin-2-yl)piperidine-2,6-dione

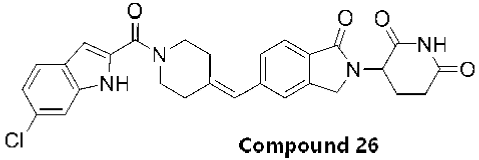

Compound 26 was synthesized in the same manner as for Compound 1, with the exception of using Intermediate 25 and 6-chloro-1H-indole-2-carboxylic acid.

¹H NMR (300 MHz, DMSO-d6) δ 11.77 (s, 1H), 11.00 (s, 1H), 7.71 (d, J = 7.9 Hz, 1H), 7.65 (d, J = 8.6 Hz, 1H), 7.50 (s, 1H), 7.44 (d, J = 1.9 Hz, 1H), 7.42-7.36 (m, 1H), 7.08 (dd, J = 8.5, 1.9 Hz, 1H), 6.89 (d, J = 2.5 Hz, 1H), 6.57 (s, 1H), 5.13 (dd, J = 13.2, 5.0 Hz, 1H), 4.46 (d, J = 17.4 Hz, 1H), 4.33 (d, J = 17.3 Hz, 1H), 3.86 (s, 2H), 3.77 (s, 2H), 3.03-2.81 (m, 1H), 2.62 (s, 4H), 2.43-2.33 (m, 1H), 2.03-1.99 (m, 1H).

### Compound 27. 3-(5-((1-(3-methyl-1H-indole-2-carbonyl)piperidin-4-ylidene)methyl)-1-oxoinsoindolin-2-yl)piperidine-2,6-dione

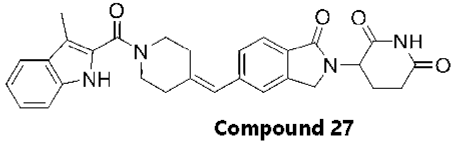

Compound 27 was synthesized in the same manner as for Compound 1, with the exception of using Intermediate 25 and 3-methyl-1H-indole-2-carboxylic acid.

¹H NMR (300 MHz, DMSO-d6) δ 11.25 (s, 1H), 11.00 (s, 1H), 7.69 (d, J = 7.8 Hz, 1H), 7.56 (d, J = 7.9 Hz, 1H), 7.48 (s, 1H), 7.36 (t, J = 9.0 Hz 2H),7.17 (t, J = 7.0 Hz, 1H), 7.04 (t, J = 7.5 Hz, 1H), 6.55 (s, 1H), 5.11 (dd, J = 13.2, 5.0 Hz, 1H), 4.45 (d, J = 17.3 Hz, 1H), 4.31 (d, J = 17.3 Hz, 1H), 3.68 (s, 2H), 3.58 (s, 2H), 2.98-2.86z (m, 1H), 2.66-2.52 (m, 3H), 2.43 (s, 2H), 2.29 (s, 3H), 2.10-1.93 (m, 1H).

### Compound 28. 3-(5-(1-(1,3-dimethyl-1H-indole-2-carbonyl)piperidin-4-yl)-1-oxoinsoindolin-2-yl)piperidine-2,6-dione

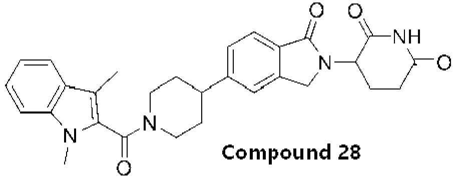

Compound 28 was synthesized in the same manner as for Compound 1, with the exception of using 1,3-dimethyl-1H-indole-2-carboxylic acid instead of 6-chloro-1H-indole-2-carboxylic acid.

¹H NMR (300 MHz, DMSO-d6) δ 10.99 (s, 1H), 7.67 (dd, J = 7.8, 4.0 Hz, 1H), 7.59-7.52 (m, 2H), 7.49-7.41 (m, 2H), 7.23 (t, J = 7.6 Hz, 1H), 7.13-6.98 (m, 1H), 5.11 (dd, J = 13.1, 5.1 Hz, 1H), 4.76 (d, J = 13.0 Hz, 1H), 4.44 (dd, J = 17.4, 4.1 Hz, 1H), 4.30 (dd, J = 17.3, 3.7 Hz, 1H), 3.81 (d, J = 13.3 Hz, 1H), 3.73 (s, 2H), 3.65 (s, 1H), 3.10-2.80 (m, 2H), 2.60 (d, J = 19.4 Hz, 2H), 2.31 (s, 1H), 2.23 (s, 2H), 2.05-1.90 (s, 2H), 1.80-1.45 (m, 2H).

### Compound 29. 3-(5-(1-(3-ethyl-1H-indole-2-carbonyl)piperidin-4-yl)-1-oxoinsoindolin-2-yl)piperidine-2,6-dione

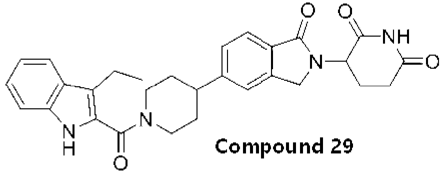

Compound 29 was synthesized in the same manner as for Compound 1, with the exception of using 3-ethyl-1H-indole-2-carboxylic acid instead of 6-chloro-1H-indole-2-carboxylic acid.

¹H NMR (300 MHz, DMSO-d6) δ 11.21 (s, 1H), 11.00 (s, 1H), 7.69 (d, J = 7.8 Hz, 1H), 7.60 (d, J = 7.9 Hz, 1H), 7.51 (s, 1H), 7.43 (d, J = 7.9 Hz, 1H), 7.36 (d, J = 8.1 Hz, 1H), 7.18-7.11 (m, 1H), 7.06-7.00 (m, 1H), 5.11 (dd, J = 13.2, 5.1 Hz, 1H), 4.45 (d, J = 17.3 Hz, 1H), 4.31 (d, J = 17.3 Hz, 1H), 3.15-2.84 (m, 4H), 2.79-2.72 (m, 2H), 2.60 (d, J = 17.0 Hz, 1H), 2.43-2.37 (m, 1H), 2.05-1.92 (m, 1H), 1.88 (d, J = 12.6 Hz, 2H), 1.72-1.60 (m, 2H), 1.23 (t, J = 7.5 Hz, 3H).

### Compound 30. 3-(5-(1-(3-methoxy-1H-indole-2-carbonyl)piperidin-4-yl)-1-oxoinsoindolin-2-yl)piperidine-2,6-dione

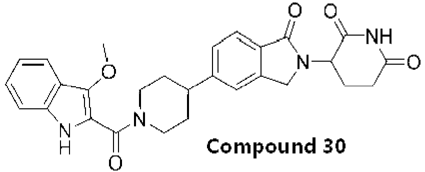

Compound 30 was synthesized in the same manner as for Compound 1, with the exception of using 3-methoxy-1H-indole-2-carboxylic acid instead of 6-chloro-1H-indole-2-carboxylic acid.

¹H NMR (500 MHz, DMSO-d6) δ δ 11.07 (s, 1H), 10.99 (s, 1H), 7.67 (t, J = 7.6 Hz, 2H), 7.53 (s, 1H), 7.44 (d, J = 7.9 Hz, 1H), 7.34 (d, J = 8.2 Hz, 1H), 7.20-7.15 (m, 1H), 7.07-6.98 (m, 1H), 5.11 (dd, J = 13.2, 5.0 Hz, 1H), 4.45 (d, J = 17.3 Hz, 1H), 4.31 (d, J = 17.3 Hz, 1H), 3.97 (s, 3H), 3.25-2.85 (m, 4H), 2.58 (s, 1H), 2.44-2.28 (m, 1H), 2.05-1.91 (m, 3H), 1.77-1.59 (m, 2H).

### Compound 31. 3-(5-(1-(3-methyl-1H-picolo[2,3-b]pyridine-2-carbonyl)piperidin-4-yl)-1-oxoinsoindolin-2-yl)piperidine-2,6-dione

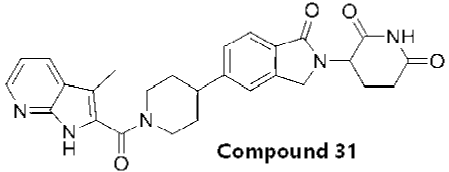

Compound 31 was synthesized in the same manner as for Compound 1, with the exception of using 3-methyl-1H-picolo[2,3-b]pyridine-2-carboxylic acid instead of 6-chloro-1H-indole-2-carboxylic acid.

¹H NMR (300 MHz, DMSO-d6) δ 11.75 (s, 1H), 10.99 (s, 1H), 8.27 (dd, J = 4.7, 1.6 Hz, 1H), 7.99 (dd, J = 8.0, 1.6 Hz, 1H), 7.69 (d, J = 7.8 Hz, 1H), 7.52 (s, 1H), 7.44 (d, J = 7.9 Hz, 1H), 7.10 (dd, J = 7.9, 4.6 Hz, 1H), 5.11 (dd, J = 13.2, 5.1 Hz, 1H), 4,70 (s,1 H), 4.45 (d, J = 17.3 Hz, 1H), 4.31 (d, J = 17.2 Hz, 1H), 3.90 (s,1 H), 3.18-2.84 (m, 4H), 2.60 (d, J = 16.8 Hz, 1H), 2.46-2.37 (m, 1H), 2.29 (s, 3H), 2.05-1.95 (m, 1H), 1.86 (s, 2H), 1.76-1.63 (m, 2H).

### Compound 32. 3-(5-(1-(6-amino-1H-indole-2-carbonyl)piperidin-4-yl)-1-oxoinsoindolin-2-yl)piperidine-2,6-dione

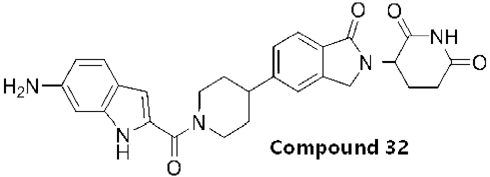

Compound 32 was synthesized in the same manner as for Compound 1, with the exception of using 6-amino-1H-indole-2-carboxylic acid instead of 6-chloro-1H-indole-2-carboxylic acid.

¹H NMR (300 MHz, DMSO-d6) δ 10.99 (s, 1H), 10.92 (s, 1H), 7.67 (d, J = 7.9 Hz, 1H), 7.54 (s, 1H), 7.45 (d, J = 8.5 Hz, 1H), 7.25 (d, J = 8.4 Hz, 1H), 6.64 (s, 1H), 6.55 (s, 1H), 6.44 (dd, J = 8.5, 2.0 Hz, 1H), 5.11 (dd, J = 13.2, 5.1 Hz, 1H), 4.99 (s, 1H), 4.64 (d, J = 12.8 Hz, 2H), 4.44 (d, J = 17.3 Hz, 1H), 4.30 (d, J = 17.3 Hz, 1H), 3.81 (s, 1H), 3.20-2.82 (m, 3H), 2.65-2.54 (m, 2H), 2.38 (d, J = 4.2 Hz, 1H), 2.06-1.84 (m, 3H), 1.76-1.67 (m, 2H).

### Compound 33. 3-(5-(1-(5-bromo-3-methyl-1H-indole-2-carbonyl)piperidin-4-yl)-1-oxoinsoindolin-2-yl)piperidine-2,6-dione

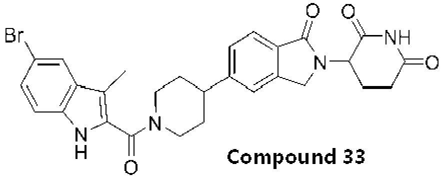

Compound 33 was synthesized in the same manner as for Compound 1, with the exception of using 5-bromo-3-methyl-1H-indole-2-carboxylic acid instead of 6-chloro-1H-indole-2-carboxylic acid.

¹H NMR (300 MHz, DMSO-d6) δ 11.46 (s, 1H), 10.99 (s, 1H), 7.75 (d, J = 1.8 Hz, 1H), 7.68 (d, J = 7.8 Hz, 1H), 7.51 (s, 1H), 7.43 (d, J = 8.0 Hz, 1H), 7.32 (d, J = 8.6 Hz, 1H), 7.27 (dd, J = 8.6, 1.8 Hz, 1H), 5.11 (dd, J = 13.2, 5.1 Hz, 1H), 4.44 (d, J = 17.3 Hz, 1H), 4.30 (d, J = 17.3 Hz, 1H), 3.26-2.81 (m, 4H), 2.60 (d, J = 19.4 Hz, 1H), 2.47-2.37 (m, 1H), 2.01-1.97 (m, 3H), 1.73-1.64 (m, 2H).

### Compound 34. 3-(5-(1-(3-(morpholinomethyl) -1H-indole-2-carbonyl)piperidin-4-yl)-1-oxoinsoindolin-2-yl)piperidine-2,6-dione

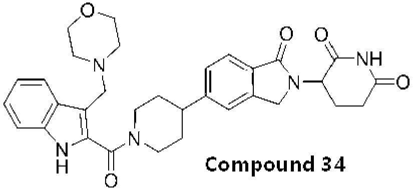

Compound 34 was synthesized in the same manner as for Compound 1, with the exception of using 3-(morpholinomethyl)-1H-indole-2-carboxylic acid instead of 6-chloro-1H-indole-2-carboxylic acid.

¹H NMR (300 MHz, DMSO) δ 11.41 (s, 1H), 10.99 (s, 1H), 7.73 (d, J = 7.9 Hz, 1H), 7.69 (d, J = 7.9 Hz, 1H), 7.50 (s, 1H), 7.42 (d, J = 7.9 Hz, 1H), 7.36 (d, J = 8.1 Hz, 1H), 7.16 (t, J = 7.5 Hz, 1H), 7.04 (t, J = 7.4 Hz, 1H), 5.11 (dd, J = 13.2, 5.1 Hz, 1H), 4.45 (d, J = 17.3 Hz, 1H), 4.31 (d, J = 17.2 Hz, 1H), 3.66 (s, 2H), 3.53 (t, J = 4.6 Hz, 4H), 3.19-2.83 (m, 4H), 2.63-2.57 (m, 1H), 2.42 (d, J = 4.5 Hz, 1H), 2.36 (s, 4H), 2.03-1.81 (m, 3H), 1.78-1.59 (m, 2H).

### Compound 35. 2-(4-(2-(2,6-dioxopiperidin-3-yl)-1-oxoindolin-5-yl)piperidine-1-carbonyl)-1H-indole-3-carbaldehyde

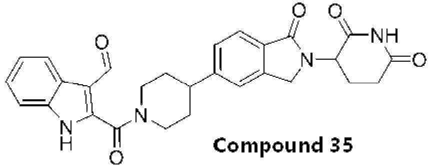

Compound 35 was synthesized in the same manner as for Compound 1, with the exception of using 3-formyl-1H-indole-2-carboxylic acid instead of 6-chloro-1H-indole-2-carboxylic acid.

¹H NMR (300 MHz, DMSO-d6) δ 12.68 (s, 1H), 11.00 (s, 1H), 10. 03 (s, 1H), 8.15 (dd, J = 6.9, 1.7 Hz, 1H), 7.69 (d, J = 7.8 Hz, 1H), 7.54 (d, J = 6.2 Hz, 2H), 7.45 (d, J = 7.6 Hz, 1H), 7.36-7.25 (m, 2H), 5.11 (dd, J = 13.3, 5.1 Hz, 1H), 4.72 (s, 1H), 4.45 (d, J = 17.3 Hz, 1H), 4.31 (d, J = 17.3 Hz, 1H), 3.63 (s, 1H), 3.30 (s, 1H), 3.12-2.82 (m, 4H), 2.60 (d, J = 16.8 Hz, 1H), 2.44-2.27 (m, 1H), 2.07-1.87 (m, 2H), 1.80-1.60 (m, 3H).

### Compound 36. 3-(5-(1-(3-bromo-1H-indole-2-carbonyl)piperidin-4-yl)-1-oxoinsoindolin-2-yl)piperidine-2,6-dione

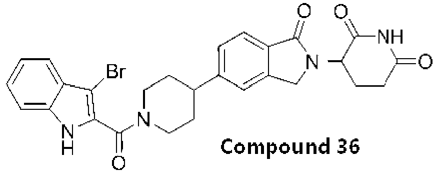

Compound 36 was synthesized in the same manner as for Compound 1, with the exception of using 3-bromo-1H-indole-2-carboxylic acid instead of 6-chloro-1H-indole-2-carboxylic acid.

¹H NMR (300 MHz, DMSO) δ 12.03 (s, 1H), 11.00 (s, 1H), 7.69 (d, J = 7.8 Hz, 1H), 7.52-7.40 (m, 4H), 7.27 (td, J = 7.6, 1.3 Hz, 1H), 7.18 (td, J = 7.4, 1.1 Hz, 1H), 5.11 (dd, J = 13.2, 5.0 Hz, 1H), 4.66 (s, 1H), 4.45 (d, J = 17.3 Hz, 1H), 4.31 (d, J = 17.3 Hz, 1H), 3.78 (s, 1H), 3.21-2.83 (m, 4H), 2.66-2.56 (m, 1H), 2.44-2.26 (m, 1H), 2.04-1.82 (m, 3H), 1.80-1.60 (m, 2H).

### Compound 37. 3-(5-(1-(3-fluoro-1H-indole-2-carbonyl)piperidin-4-yl)-1-oxoinsoindolin-2-yl)piperidine-2,6-dione

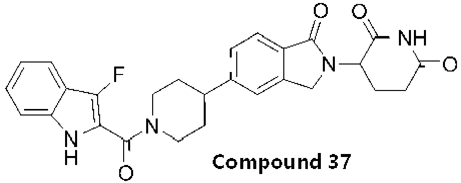

Compound 37 was synthesized in the same manner as for Compound 1, with the exception of using 3-fluoro-1H-indole-2-carboxylic acid instead of 6-chloro-1H-indole-2-carboxylic acid.

¹H NMR (500 MHz, DMSO-d6) δ 11.46 (s, 1H), 11.00 (s, 1H), 7.69 (d, J = 7.8 Hz, 1H), 7.61 (d, J = 8.2 Hz, 1H), 7.54 (s, 1H), 7.45 (d, J = 7.9 Hz, 1H), 7.40 (d, J = 8.5 Hz, 1H), 7.28 (d, J = 8.0 Hz, 1H), 7.13 (d, J = 8.5 Hz, 1H), 5.12 (d, J = 13.5 Hz, 1H), 4.61 (s, 1H), 4.45 (d, J = 17.1 Hz, 1H), 4.31 (d, J = 17.1 Hz, 1H), 4.17 (s, 1H), 3.13-2.84 (m, 3H), 2.70-2.55 (m, 2H), 2.46-2.33 (m, 1H), 2.06-1.85 (m, 3H), 1.75-1.65 (m, 2H).

### Compound 38. 3-(5-(1-(5-chloro-3-methyl-1H-indole-2-carbonyl)piperidin-4-yl)-1-oxoinsoindolin-2-yl)piperidine-2,6-dione

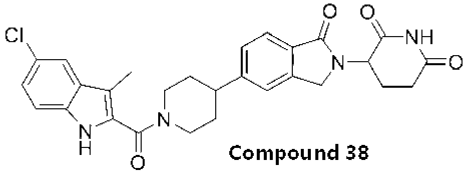

Compound 38 was synthesized in the same manner as for Compound 1, with the exception of using 5-chloro-3-methyl-1H-indole-2-carboxylic acid instead of 6-chloro-1H-indole-2-carboxylic acid.

¹H NMR (500 MHz, DMSO-d6) δ 11.46 (s, 1H), 11.00 (s, 1H), 7.69 (d, J = 7.9 Hz, 1H), 7.62 (d, J = 2.3 Hz, 1H), 7.52 (s, 1H), 7.44 (d, J = 8.0 Hz, 1H), 7.37 (d, J = 8.7 Hz, 1H), 7.17 (dd, J = 8.9, 2.1 Hz, 1H), 5.12 (dd, J = 13.3, 5.0 Hz, 1H), 4.44 (d, J = 17.2 Hz, 1H), 4.31 (d, J = 17.1 Hz, 1H), 3.24-2.86 (m, 4H), 2.66-2.57 (m, 1H), 2.48-2.34 (m, 1H), 2.28 (s, 3H), 2.04-1.80 (m, 3H), 1.75-1.65 (m, 2H).

### Compound 39. 3-(5-(1-(6-chloro-3-methyl-1H-indole-2-carbonyl)piperidin-4-yl)-1-oxoinsoindolin-2-yl)piperidine-2,6-dione

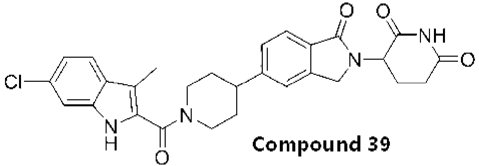

Compound 39 was synthesized in the same manner as for Compound 1, with the exception of using 6-chloro-1H-indole-2-carboxylic acid instead of 6-chloro-3-methyl-1H-indole-2-carboxylic acid.

¹H NMR (500 MHz, DMSO-d6) δ 11.43 (s, 1H), 11.00 (s, 1H), 7.69 (d, J = 8.0 Hz, 1H), 7.58 (d, J = 8.8 Hz, 1H), 7.52 (s, 1H), 7.44 (d, J = 8.0 Hz, 1H), 7.38 (s, 1H), 7.06 (d, J = 8.6 Hz, 1H), 5.12 (d, J = 13.5 Hz, 1H), 4.44 (d, J = 17.2 Hz, 1H), 4.31 (d, J = 17.1 Hz, 1H), 3.19-2.88 (m, 4H), 2.66-2.56 (m, 1H), 2.45-2.34 (m, 1H), 2.30 (s, 3H), 2.05-1.80 (m, 3H), 1.75-1.60. (m, 2H) .

### Compound 40. 3-(5-(1-(5,6-dimethoxy-1H-indole-2-carbonyl)piperidin-4-yl)-1-oxoinsoindolin-2-yl)piperidine-2,6-dione

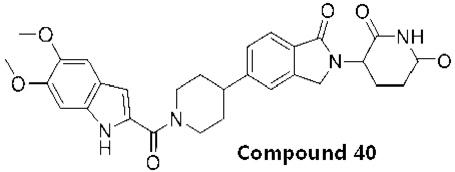

Compound 40 was synthesized in the same manner as for Compound 1, with the exception of using 5,6-dimethoxy-1H-indole-2-carboxylic acid instead of 6-chloro-1H-indole-2-carboxylic acid.

¹H NMR (500 MHz, DMSO-d6) δ 11.29 (s, 1H), 11.00 (s, 1H), 7.68 (d, J = 7.9 Hz, 1H), 7.55 (s, 1H), 7.46 (d, J = 7.9 Hz, 1H), 7.08 (s, 1H), 6.91 (s, 1H), 6.72 (d, J = 2.2 Hz, 1H), 5.12 (dd, J = 13.3, 5.1 Hz, 1H), 4.65 (d, J = 12.9 Hz, 2H), 4.44 (d, J = 17.2 Hz, 1H), 4.31 (d, J = 17.2 Hz, 1H), 3.79 (s, 3H), 3.76 (s, 3H), 3.23-2.98 (m, 3H), 2.95-2.85 (m, 1H), 2.67-2.57 (m, 1H), 2.47-2.34 (m, 1H), 2.05-1.86 (m, 3H), 1.72 (q, J = 12.3 Hz, 2H).

### Compound 41. 3-(1-oxo-5-(1-(6-(trifluoromethyl)-1H-indole-2-carbonyl)piperidin-4-yl)isoindolin-2-yl)piperidine-2,6-dione

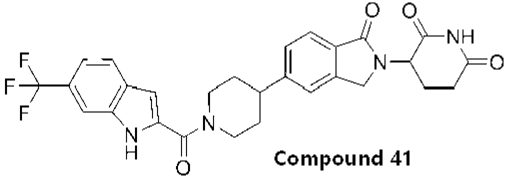

Compound 41 was synthesized in the same manner as for Compound 1, with the exception of using 6-(trifluoromethyl)-1H-indole-2-carboxylic acid instead of 6-chloro-1H-indole-2-carboxylic acid.

¹H NMR (500 MHz, DMSO-d6) δ 12.08 (s, 1H), 10.99 (s, 1H), 7.84 (d, J = 8.4 Hz, 1H), 7.75 (s, 1H), 7.67 (d, J = 7.8 Hz, 1H), 7.55 (s, 1H), 7.46 (d, J = 7.9 Hz, 1H), 7.34 (dd, J = 8.4, 1.7 Hz, 1H), 6.97 (d, J = 2.1 Hz, 1H), 5.11 (dd, J = 13.3, 5.1 Hz, 1H), 4.59 (s, 2H), 4.43 (d, J = 17.2 Hz, 1H), 4.30 (d, J = 17.2 Hz, 1H), 3.28-2.99 (m, 3H), 2.95-2.84 (m, 1H), 2.66-2.56 (m, 1H), 2.44-2.34 (m, 1H), 2.03-1.88 (m, 3H), 1.75 (s, 2H).

### Compound 42. 3-(5-(1-(6-methoxy-3-methyl-1H-indole-2-carbonyl)piperidin-4-yl)-1-oxoinsoindolin-2-yl)piperidine-2,6-dione

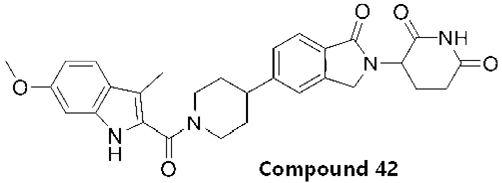

Compound 42 was synthesized in the same manner as for Compound 1, with the exception of using 6-methoxy-3-methyl-1H-indole-2-carboxylic acid instead of 6-chloro-1H-indole-2-carboxylic acid.

¹H NMR (300 MHz, DMSO-d6) δ 11.02 (s, 1H), 10.99 (s, 1H), 7.68 (d, J = 7.8 Hz, 1H), 7.52 (s, 1H), 7.44 (d, J = 2.j4 Hz, 1H), 7.41 (d, J = 2.4 Hz, 1H), 6.82 (d, J = 2.2 Hz, 1H), 6.69 (dd, J = 8.7, 2.3 Hz, 1H), 5.11 (dd, J = 13.2, 5.1 Hz, 1H), 4.44 (d, J = 17.3 Hz, 1H), 4.30 (d, J = 17.3 Hz, 3H), 3.77 (s, 3H), 3.19-2.83 (m, 4H), 2.66-2.55 (m, 1H), 2.45-2.35 (m, 1H), 2.27 (s, 3H), 2.06-1.84 (m, 3H), 1.76-1.56 (m, 2H).

### Compound 43. 3-(5-((1-(6-chloro-3-methyl-1H-indole-2-carbonyl)piperidin-4-yl)ethynyl)-1-oxoinsoindolin-2-yl)piperidine-2,6-dione

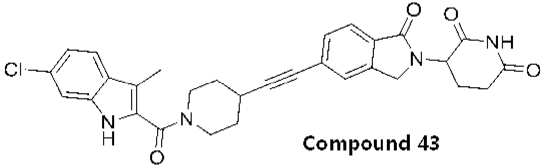

Compound 43 was synthesized in the same manner as for Compound 1, with the exception of using Intermediate 31 and 6-chloro-3-methyl-1H-indole-2-carboxylic acid.

¹H NMR (300 MHz, DMSO-d6) δ 11.39 (s, 1H), 11.01 (s, 1H), 7.70 (d, J = 7.9 Hz, 1H), 7.68 (s, 1H), 7.56 (t, J = 8.3 Hz, 2H), 7.36 (d, J = 1.9 Hz, 1H), 7.06 (dd, J = 8.5, 1.9 Hz, 1H), 5.12 (dd, J = 13.2, 5.1 Hz, 1H), 4.45 (d, J = 17.6 Hz, 1H), 4.32 (d, J = 17.6 Hz, 1H), 3.86 (s, 2H), 3.50-3.41 (m, 2H), 3.11-3.00 (m, 1H), 2.98-2.84 (m, 1H), 2.64-2.56 (m, 1H), 2.45 - 2.35 (m, 1H), 2.27 (s, 3H), 2.07-1.91 (m, 3H), 1.75-1.61 (m, 2H).

### Compound 44. 3-(5-((1-(6-chloro-3-methyl-1H-indole-2-carbonyl)piperidin-4-ylidene)methyl)-1-oxoinsoindolin-2-yl)piperidine-2,6-dione

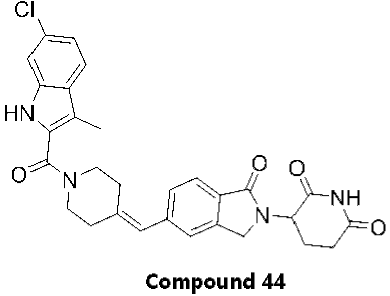

Compound 44 was synthesized in the same manner as for Compound 1, with the exception of using Intermediate 25 and 6-chloro-3-methyl-1H-indole-2-carboxylic acid.

¹H NMR (300 MHz, DMSO-d6) δ 11.43 (s, 1H), 10.99 (s, 1H), 7.69 (d, J = 7.8 Hz, 1H), 7.58 (d, J = 8.5 Hz, 1H), 7.48 (s, 1H), 7.40-7.34 (m, 2H), 7.06 (dd, J = 8.5, 1.9 Hz, 1H), 6.55 (s, 1H), 5.11 (dd, J = 13.2, 5.1 Hz, 1H), 4.45 (d, J = 17.4 Hz, 1H), 4.31 (d, J = 17.3 Hz, 1H), 3.62 (d, J = 27.6 Hz, 4H), 3.00-2.82 (m, 1H), 2.63 (s, 1H), 2.47-2.40 (m, 2H), 2.28 (s, 3H), 2.08-1.94 (m, 1H).

### Compound 45. 3-(5-(1-(6-chloro-1H-picolo[3,2-b]pyridine-2-carbonyl)piperidin-4-yl)-1-oxoinsoindolin-2-yl)piperidine-2,6-dione

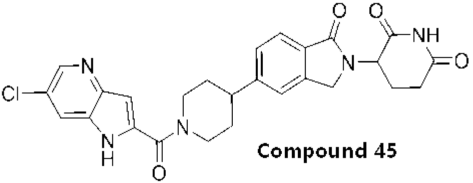

Compound 45 was synthesized in the same manner as for Compound 1, with the exception of using 6-chloro-1H-picolo[3,2-b]pyridine-2-carboxylic acid instead of 6-chloro-1H-indole-2-carboxylic acid.

¹H NMR (500 MHz, DMSO) δ 12.30 (s, 1H), 11.01 (s, 1H), 8.40 (d, J = 2.2 Hz, 1H), 7.88 (d, J = 2.2 Hz, 1H), 7.68 (d, J = 7.8 Hz, 1H), 7.56 (s, 1H), 7.47 (d, J = 7.9 Hz, 1H), 6.98 (s, 1H), 5.11 (dd, J = 13.3, 5.1 Hz, 1H), 4.70-4.55 (m, 2H), 4.44 (d, J = 17.2 Hz, 1H), 4.31 (d, J = 17.2 Hz, 1H), 3.15-2.88 (m, 4H), 2.63-2.58 (m, 1H), 2.46-2.36 (m, 1H), 2.03-1.90 (m, 3H), 1.86-1.68 (m, 2H).

### Compound 46. 3-(5-(1-(6-chloro-1H-picolo[3,2-c]pyridine-2-carbonyl)piperidin-4-yl)-1-oxoinsoindolin-2-yl)piperidine-2,6-dione

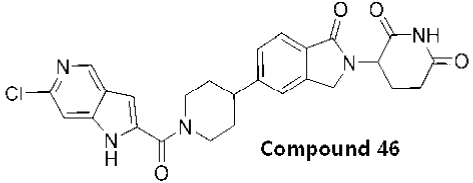

Compound 46 was synthesized in the same manner as for Compound 1, with the exception of using 6-chloro-1H-picolo[3,2-c]pyridine-2-carboxylic acid instead of 6-chloro-1H-indole-2-carboxylic acid.

¹H NMR (300 MHz, DMSO-d6) δ 12.30 (s, 1H), 11.01 (s, 1H), 8.73 (s, 1H), 7.68 (d, J = 7.8 Hz, 1H), 7.55 (s, 1H), 7.46 (d, J = 8.3 Hz, 1H), 7.41 (s, 1H), 7.02 (s, 1H), 5.11 (dd, J = 13.2, 5.1 Hz, 1H), 4.56 (s, 2H), 4.44 (d, J = 17.3 Hz, 1H), 4.30 (d, J = 17.3 Hz, 1H), 3.2202.86 (m, 4H), 2.66-2.56 (m, 1H), 2.45-2.27 (m, 1H), 2.04-j1.86 (m, 3H), 1.84-1.58 (m, 2H).

### Compound 47. 3-(5-(1-(6-hydroxy-3-methyl-1H-indole-2-carbonyl)piperidin-4-yl)-1-oxoinsoindolin-2-yl)piperidine-2,6-dione

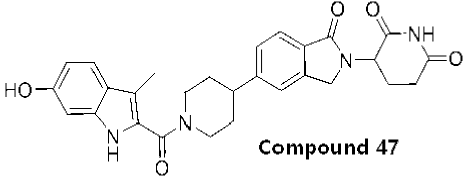

Compound 47 was synthesized in the same manner as for Compound 1, with the exception of using 6-hydroxy-3-methyl-1H-indole-2-carboxylic acid instead of 6-chloro-1H-indole-2-carboxylic acid.

¹H NMR (300 MHz, DMSO-d6) δ 10.99 (s, 1H), 10.78 (s, 1H), 9.11 (s, 1H), 7.68 (d, J = 7.9 Hz, 1H), 7.51 (s, 1H), 7.43 (d, J = 7.9 Hz, 1H), 7.32 (d, J = 8.5 Hz, 1H), 6.69 (d, J = 2.0 Hz, 1H), 6.56 (dd, J = 8.5, 2.1 Hz, 1H), 5.11 (dd, J = 13.2, 5.1 Hz, 1H), 4.44 (d, J = 17.2 Hz, 1H), 4.37-4.21 (m, 3H), 3.12-2.91 (m, 3H), 2.67-2.55 (m, 1H), 2.44-2.30 (m, 1H), 2.25 (s, 3H), 2.03-1.97 (m, 1H), 1.92-1.84 (m, 2H), 1.75-1.60 (m, 2H).

### Compound 48. 3-(5-(1-(3-methyl-6-(trifluoromethyl)-1H-indole-2-carbonyl)piperidin-4-yl)-1-oxoinsoindolin-2-yl)piperidine-2,6-dione

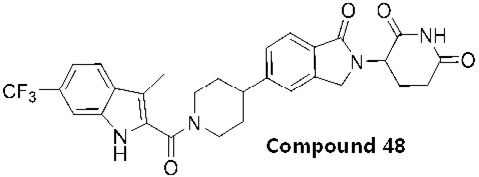

Compound 48 was synthesized in the same manner as for Compound 1, with the exception of using 3-methyl-6-(trifluoromethyl)-1H-indole-2-carboxylic acid instead of 6-chloro-1H-indole-2-carboxylic acid.

¹H NMR (300 MHz, DMSO-d6) δ 11.76 (s, 1H), 11.00 (s, 1H), 7.78 (d, J = 8.4 Hz, 1H), 7.71-7.65 (m, 2H), 7.52 (s, 1H), 7.44 (d, J = 7.6 Hz, 1H), 7.33 (dd, J = 8.4, 1.6 Hz, 1H), 5.11 (dd, J = 13.3, 5.0 Hz, 1H), 4.45 (d, J = 17.3 Hz, 1H), 4.31 (d, J = 17.3 Hz, 1H), 3.24-2.84 (m, 4H), 2.66-2.56 (m, 1H), 2.40 (dd, J = 13.1, 4.5 Hz, 1H), 2.33 (s, 1H). 2.09-1.80 (m, 3H), 1.77-1.56 (m, 2H).

### Compound 49. 3-(5-(1-(6-bromo-3-methyl-1H-indole-2-carbonyl)piperidin-4-yl)-1-oxoinsoindolin-2-yl)piperidine-2,6-dione

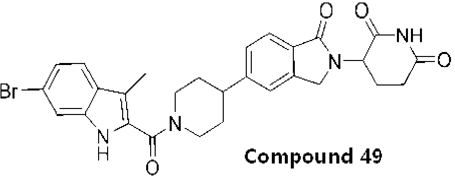

Compound 49 was synthesized in the same manner as for Compound 1, with the exception of using 6-bromo-3-methyl-1H-indole-2-carboxylic acid instead of 6-chloro-1H-indole-2-carboxylic acid.

¹H NMR (300 MHz, DMSO-d6) δ 11.42 (s, 1H), 10.99 (s, 1H), 7.68 (d, J = 7.8 Hz, 1H), 7.55-7.50 (m, 2H), 7.43 (d, J = 7.9 Hz, 1H), 7.17 (dd, J = 8.4, 1.9 Hz, 1H), 5.11 (dd, J = 13.2, 5.0 Hz, 1H), 4.44 (d, J = 17.3 Hz, 1H), 4.30 (d, J = 17.3 Hz, 1H), 3.22-2.83 (m, 4H), 2.64-2.56 (m, 1H), 2.46-2.30 (m, 1H), 2.29 (s, 3H), 2.04-1.80 (m, 3H), 1.75-1.58 (m, 2H).

### Compound 50. 3-(5-(1-(6-chloro-1H-picolo[2,3-b]pyridine-2-carbonyl)piperidin-4-yl)-1-oxoinsoindolin-2-yl)piperidine-2,6-dione

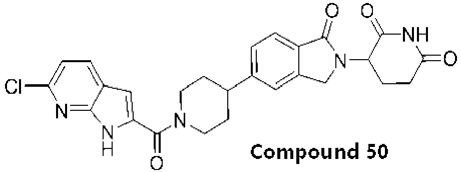

Compound 50 was synthesized in the same manner as for Compound 1, with the exception of using 6-chloro-1H-picolo[2,3-b]pyridine-2-carboxylic acid instead of 6-chloro-1H-indole-2-carboxylic acid.

¹H NMR (300 MHz, DMSO-d6) δ 12.40 (s, 1H), 11.02 (s, 1H), 8.09 (d, J = 8.3 Hz, 1H), 7.68 (d, J = 7.8 Hz, 1H), 7.55 (s, 1H), 7.46 (d, J = 8.0 Hz, 1H), 7.18 (d, J = 8.2 Hz, 1H), 6.84 (s, 1H), 5.11 (dd, J = 13.2, 5.1 Hz, 1H), 4.66-4.39 (m, 3H), 4.30 (d, J = 17.3 Hz, 1H), 3.23-2.83 (m, 4H), 2.66-2.56 (m, 1H), 2.44-2.24 (m, 1H), 2.07-1.85 (m, 3H), 1.83-1.63 (m, 2H).

### Compound 51. 2-(4-(2-(2,6-dioxopiperidin-3-yl)-1-oxoindolin-5-yl)piperidine-1-carbonyl)-1H-indole-3-carbonitrile

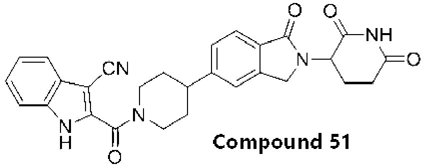

Compound 51 was synthesized in the same manner as for Compound 1, with the exception of using 3-cyano-1H-indole-2-carboxylic acid instead of 6-chloro-1H-indole-2-carboxylic acid.

¹H NMR (300 MHz, DMSO-d6) δ 12.80 (s, 1H), 10.99 (s, 1H), 7.69 (dd, J = 7.8, 3.9 Hz, 2H), 7.56 (d, J = 1.3 Hz, 1H), 7.51 (s, 1H), 7.43 (d, J = 7.9 Hz, 1H), 7.40-7.24 (m, 2H), 5.11 (dd, J = 13.3, 5.1 Hz, 1 H) , 4.70 (s,1 H) 4.45 (d, J = 17.3 Hz, 1H), 4.31 (d, J = 17.3 Hz, 1H), 4.05 (s, 1H), 3.23-2.83 (m, 4H), 2.67-2.55 (m, 1H), 2.45-2.33 (m, 1H), 2.04-1.90 (m, 3H), 1.82-1.60 (m, 2H).

### Compound 52. 2-(4-(2-(2,6-dioxopiperidin-3-yl)-1-oxoindolin-5-yl)piperidine-1-carbonyl)-3-methyl-1H-indole-6-carbonitrile

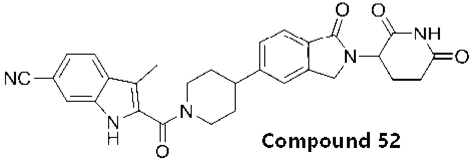

Compound 52 was synthesized in the same manner as for Compound 1, with the exception of using 6-cyano-3-methyl-1H-indole-2-carboxylic acid instead of 6-chloro-1H-indole-2-carboxylic acid.

¹H NMR (500 MHz, DMSO-d6) δ 11.91 (s, 1H), 11.00 (s, 1H), 7.84 (s, 1H), 7.76 (d, J = 8.3 Hz, 1H), 7.69 (d, J = 7.9 Hz, 1H), 7.52 (s, 1H), 7.44 (d, J = 7.9 Hz, 1H), 7.39 (dd, J = 8.3, 1.4 Hz, 1H), 5.11 (dd, J = 13.3, 5.1 Hz, 1H), 4.66 (s, 1H), 4.44 (d, J = 17.2 Hz, 1H), 4.31 (d, J = 17.2 Hz, 1H), 3.77 (s, 1H), 3.25-2.82 (m, 4H), 2.64-2.58 (m, 1H), 2.45-2.35 (m, 1H), 2.04-1.82 (m, 3H), 1.76-1.60 (m, 2H).

### Compound 53. 3-(5-(1-(3-methyl-6-(trifluoromethoxy)-1H-indole-2-carbonyl)piperidin-4-yl)-1-oxoinsoindolin-2-yl)piperidine-2,6-dione

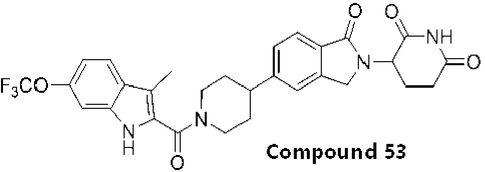

Compound 53 was synthesized in the same manner as for Compound 1, with the exception of using 3-methyl-6-(trifluoromethoxy)-1H-indole-2-carboxylic acid instead of 6-chloro-1H-indole-2-carboxylic acid.

¹H NMR (300 MHz, DMSO-d6) 511.54 (s, 1H), 10.99 (s, 1H), 7.67 (dd, J = 8.3, 6.7 Hz, 2H), 7.52 (s, 1H), 7.43 (d, J = 7.9 Hz, 1H), 7.30 (s, 1H), 7.03 (ddd, J = 8.7, 2.2, 1.1 Hz, 1H), 5.11 (dd, J = 13.3, 5.0 Hz, 1H), 4.44 (d, J = 17.3 Hz, 1H), 4.35-3.88 (m, 3H), 3.23-2.84 (m, 4H), 2.66-2.56 (m, 1H), 2.45-2.37 (m, 1H), 2.31 (s, 3H), 2.05-1.83 (m, 3H), 1.77-1.60 (m, 2H)

### Compound 54. 3-(5-(1-(6-fluoro-3-methyl-1H-indole-2-carbonyl)piperidin-4-yl)-1-oxoinsoindolin-2-yl)piperidine-2,6-dione

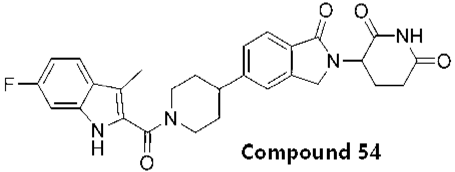

Compound 54 was synthesized in the same manner as for Compound 1, with the exception of using 6-fluoro-3-methyl-1H-indole-2-carboxylic acid instead of 6-chloro-1H-indole-2-carboxylic acid.

¹H NMR (500 MHz, DMSO-d6) δ 11.34 (s, 1H), 11.00 (s, 1H), 7.69 (d, J = 7.8 Hz, 1H), 7.60-7.54 (m, 1H), 7.52 (s, 1H), 7.44 (d, J = 7.9 Hz, 1H), 7.14-7.06 (m, 1H), 6.94-6.88 (m, 1H), 5.12 (dd, J = 13.3, 5.1 Hz, 1H), 4.44 (d, J = 17.2 Hz, 1H), 4.31 (d, J = 17.1 Hz, 1H), 3.21-2.86 (m, 4H), 2.66-2.56 (m, 1H), 2.46-2.34 (m, 2H), 2.30 (s, 2H), 2.03-1.97 (m, 1H), 1.93-1.85 (m, 2H), 1.72-1.62 (m, 2H).

### Compound 55. 3-(5-(1-(3-methyl-6-nitro-1H-indole-2-carbonyl)piperidin-4-yl)-1-oxoinsoindolin-2-yl)piperidine-2,6-dione

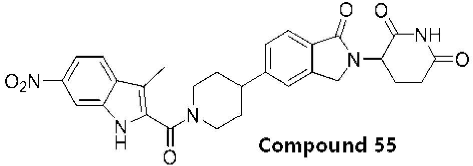

Compound 55 was synthesized in the same manner as for Compound 1, with the exception of using 3-methyl-6-nitro-1H-indole-2-carboxylic acid instead of 6-chloro-1H-indole-2-carboxylic acid.

¹H NMR (300 MHz, DMSO-d6 δ 12.10 (s, 1H), 11.01 (s, 1H), 8.28 (d, J = 2.1 Hz, 1H), 7.93 (dd, J = 8.9, 2.1 Hz, 1H), 7.77 (d, J = 8.8 Hz, 1H), 7.69 (d, J = 7.8 Hz, 1H), 7.52 (s, 1H), 7.44 (dd, J = 8.0, 1.2 Hz, 1H), 5.11 (dd, J = 13.2, 5.1 Hz, 1H), 4.55 (s, 1H), 4.45 (d, J = 17.3 Hz, 1H), 4.31 (d, J = 17.3 Hz, 1H), 3.85 (s, 1H), 3.13-2.82 (m, 4H), 2.65-2.56 (m, 1H), 2.44-2.36 (m, 1H), 2.02-1.82 (m, 3H), 1.75-1.57 (m, 2H).

### Compound 56. 3-(5-(1-(6-(tert-butyl)-3-methyl-1H-indole-2-carbonyl)piperidin-4-yl)-1-oxoinsoindolin-2-yl)piperidine-2,6-dione

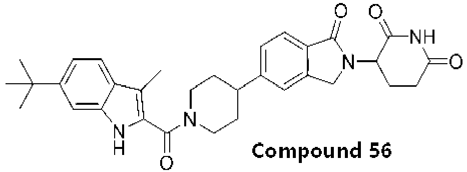

Compound 56 was synthesized in the same manner as for Compound 1, with the exception of using 6-*(tert -* butyl)-3-methyl-1H-indole-2-carboxylic acid instead of 6-chloro-1H-indole-2-carboxylic acid.

¹H NMR (300 MHz, DMSO-d6) δ 11.05 (s, 1H), 10.99 (s, 1H), 7.68 (d, J = 7.8 Hz, 1H), 7.51 (s, 1H), 7.47 (d, J = 8.5 Hz, 1H), 7.43 (d, J = 7.8 Hz, 1H), 7.29 (d, J = 1.6 Hz, 1H), 7.14 (dd, J = 8.5, 1.7 Hz, 1H), 5.11 (dd, J = 13.2, 5.1 Hz, 1H), 4.50 - 4.21 (m, 3H), 3.21 - 2.83 (m, 4H), 2.66 - 2.54 (m, 1H), 2.43 - 2.36 (m, 1H), 2.28 (s, 3H), 2.04 - 1.82 (m, 3H), 1.76 - 1.57 (m, 2H), 1.33 (s, 9H).

### Compound 57. 3-(5-(8-(6-chloro-1H-indole-2-carbonyl)-8-azabicyclo[3.2.1]octan-3-yl)-1-oxoinsoindolin-2-yl)piperidine-2,6-dione

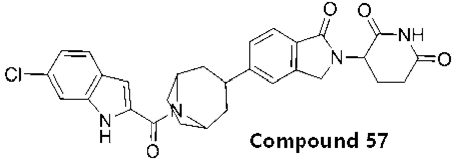

Compound 57 was synthesized in the same manner as for Compound 1, with the exception of using Intermediate 22 and 6-chloro-1H-indole-2-carboxylic acid.

¹H NMR (300 MHz, DMSO-d6) δ 11.77 (s, 1H), 10.99 (s, 1H), 7.69-7.59 (m, 2H), 7.56-7.42 (m, 3H), 7.08 (dt, J = 8.5, 2.4 Hz, 1H), 7.03 (d, J = 4.7 Hz, 1H), 5.10 (dd, J = 13.2, 4.8 Hz, 1H), 4.93-4.78 (m, 2H), 4.42 (dd, J = 17.4, 4.6 Hz, 1H), 4.28 (dd, J = 17.2, 4.6 Hz, 1H), 3.02-2.75 (m, 2H), 2.64-2.56 (m, 1H), 2.43-2.32 (m, 1H), 2.06-1.76 (m, 9H).

### Compound 58. 3-(5-(8-(6-chloro-3-methyl-1H-indole-2-carbonyl)-8-azabicyclo[3.2.1]octan-3-yl)-1-oxoinsoindolin-2-yl)piperidine-2,6-dione

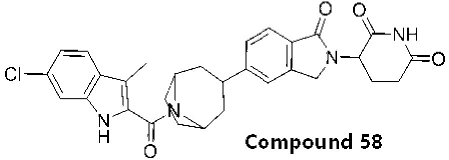

Compound 58 was synthesized in the same manner as for Compound 1, with the exception of using Intermediate 22 and 6-chloro-3-methyl-1H-indole-2-carboxylic acid.

H NMR (300 MHz, DMSO-d6) δ 11.51 (d, J = 3.6 Hz, 1H), 10.99 (s, 1H), 7.67 (dd, J = 7.9, 2.9 Hz, 1H), 7.62-7.56 (m, 1H), 7.49 (d, J = 8.1 Hz, 1H), 7.43 (d, J = 8.0 Hz, 1H), 7.38 (t, J = 2.0 Hz, 1H), 7.06 (dt, J = 8.5, 2.3 Hz, 1H), 5.11 (dd, J = 13.3, 5.1 Hz, 1H), 4.73-4.51 (m, 1H), 4.44 (d, J = 17.3 Hz, 1H), 4.30 (d, J = 17.3 Hz, 1H), 4.25-4.10 (m, 1 H), 3.00-2.84 (m, 2H), 2.64-2.57 (m, 1H), 2.44-2.36 (m, 1H), 2.33 (d, J = 8.0 Hz, 3H), 2.08-1.69 (m, 9H).

### Compound 59. 3-(5-(1-(6-chloro-1H-indole-2-carbonyl)-2-methylpiperidin-4-yl)-1-oxoinsoindolin-2-yl)piperidine-2,6-dione

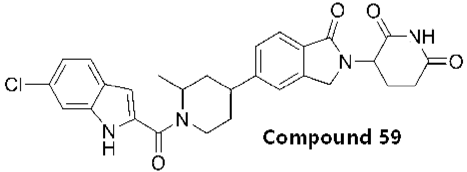

Compound 59 was synthesized in the same manner as for Compound 1, with the exception of using Intermediate 16 and 6-chloro-1H-indole-2-carboxylic acid.

¹H NMR (300 MHz, DMSO-d6) δ 11.76 (s, 1H), 10.99 (s, 1H), 7.72-7.61 (m, 2H), 7.56 (s, 1H), 7.48 (d, J = 7.8 Hz, 1H), 7.44 (d, J = 1.9 Hz, 1H), 7.07 (dd, J = 8.5, 1.9 Hz, 1H), 6.91-6.83 (m, 1H), 5.11 (dd, J = 13.2, 5.0 Hz, 1H), 4.44 (d, J = 17.0 Hz, 1H), 4.30 (d, J = 17.2 Hz, 1H), 4.25-4.14 (m, 1H), 3.01-2.84 (m, 2H), 2.65-2.56 (m, 1H), 2.41-2.35 (m, 1H), 2.05-1.84 (m, 4H), 1.81-1.69 (m, 1H), 1.50-1.37 (m, 1H), 1.31 (d, J = 6.3 Hz, 3H).

### Compound 60. 3-(5-(1-(6-chloro-3-methyl-1H-indole-2-carbonyl)-2-methylpiperidin-4-yl)-1-oxoinsoindolin-2-yl)piperidine-2,6-dione

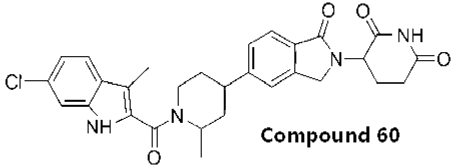

Compound 60 was synthesized in the same manner as for Compound 1, with the exception of using Intermediate 16 and 6-chloro-3-methyl-1H-indole-2-carboxylic acid.

¹H NMR (300 MHz, DMSO-d6) δ 11.42 (s, 1H), 10.99 (s, 1H), 7.68 (dd, J = 7.9, 1.8 Hz, 1H), 7.57 (d, J = 8.5 Hz, 1H), 7.54 (s, 1H), 7.45 (d, J = 7.7 Hz, 1H), 7.37 (d, J = 1.9 Hz, 1H), 7.06 (dd, J = 8.5, 1.9 Hz, 1H), 5.11 (dd, J = 13.2, 5.1 Hz, 1H), 4.44 (d, J = 17.2 Hz, 1H), 4.30 (d, J = 17.3 Hz, 1H), 4.24-4.17 (m, 1H), 3.65-3.57 (m, 1H), 3.02-2.86 (m, 2H), 2.65-2.59 (m, 1H), 2.44-2.36 (m, 1H), 2.28 (d, J = 1.8 Hz, 3H), 2.07-1.85 (m, 4H), 1.73-1.62 (m, 1H), 1.40-1.33 (m, 1H), 1.26 (d, J = 6.5 Hz, 3H).

### Compound 61. 3-(5-(1-(6-amino-3-methyl-1H-indole-2-carbonyl)piperidin-4-yl)-1-oxoinsoindolin-2-yl)piperidine-2,6-dione

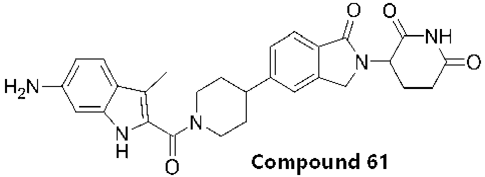

Compound 61 was synthesized in the same manner as for Compound 1, using Intermediate 7 and 6-amino-3-methyl-1H-indole-2-carboxylic acid instead of 6-chloro-1H-indole-2-carboxylic acid.

¹H NMR (500 MHz, DMSO-d6) δ 11.00 (s, 1H), 10.56 (s, 1H), 7.68 (d, J = 7.8 Hz, 1H), 7.52 (s, 1H), 7.43 (d, J = 7.9 Hz, 1H), 7.19 (d, J = 8.3 Hz, 1H), 6.49 (s, 1H), 6.42 (d, J = 8.5 Hz, 1H), 5.11 (dd, J = 13.4, 5.1 Hz, 1H), 4.91 (s, 2H), 4.44 (d, J = 17.2 Hz, 1H), 4.36-4.21 (m, 3H), 3.11-2.90 (m, 4H), 2.67-2.60 (m,, 1H), 2.42-2.36 (m, 1H), 2.22 (s, 3H), 2.04-1.94 (m, 1H), 1.92-1.83 (m, 2H), 1.74-1.60 (m, 2H).

### Compound 62. 3-(5-(1-(6-chloro-3-methyl-1H-indole-2-carbonyl)-[1,4'-bipiperidin]-4-yl)-1-oxoinsoindolin-2-yl)piperidine-2,6-dione

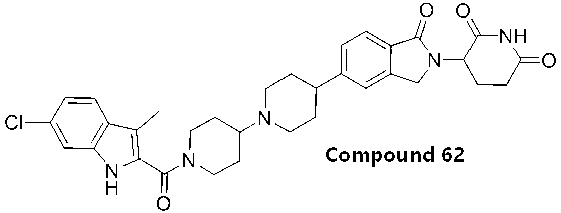

Compound 62 was synthesized in the same manner as for Compound 1, with the exception of using Intermediate 34 and 6-chloro-3-methyl-1H-indole-2-carboxylic acid.

¹H NMR (300 MHz, DMSO-d6) δ 11.39 (s, 1H), 10.99 (s, 1H), 7.64 (d, J = 7.9 Hz, 1H), 7.57 (d, J = 8.4 Hz, 1H), 7.49 (s, 1H), 7.40 (d, J = 8.0 Hz, 1H), 7.35 (d, J = 1.9 Hz, 1H), 7.06 (dd, J = 8.5, 1.9 Hz, 1H), 5.11 (dd, J = 13.2, 5.1 Hz, 1H), 4.42 (d, J = 17.3 Hz, 1H), 4.28 (d, J = 17.3 Hz, 1H), 3.07-2.82 (m, 5H), 2.66-2.55 (m, 3H), 2.45-2.28 (m, 3H), 2.25 (s, 3H), 2.06-1.93 (m, 2H), 1.87-1.75 (m, 4H), 1.74-1.58 (m, 2H), 1.54-1.37 (m, 3H).

### Compound 63. 3-(5-(1-(1-(6-chloro-3-methyl-1H-indole-2-carbonyl)azetidin-3-yl)piperidin-4-yl)-1-oxoinsoindolin-2-yl)piperidine-2,6-dione

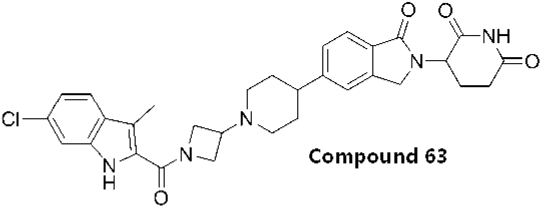

Compound 63 was synthesized in the same manner as for Compound 1, with the exception of using Intermediate 33 and 6-chloro-3-methyl-1H-indole-2-carboxylic acid.

¹H NMR (300 MHz, DMSO-d6) δ 11.23 (s, 1H), 10.99 (s, 1H), 7.65 (d, J = 7.9 Hz, 1H), 7.61 (d, J = 8.5 Hz, 1H), 7.50 (s, 1H), 7.41 (d, J = 8.0 Hz, 1H), 7.37 (d, J = 1.9 Hz, 1H), 7.07 (dd, J = 8.5, 1.9 Hz, 1H), 5.09 (d, J = 5.0 Hz, 1H), 4.43 (d, J = 17.3 Hz, 1H), 4.33-3.89 (m, 5H), 3.27-3.16 (m, 1H), 3.03-2.85 (m, 3H), 2.75-2.58 (m, 3H), 2.39 (s, 3H), 2.04-1.89 (m, 3H), 1.87-1.64 (m, 4H).

### Compound 64. 3-(5-(1-(6-chloro-3-methyl-1H-indole-2-carbonyl)-4-hydroxypiperidin-4-yl)-1-oxoinsoindolin-2-yl)piperidine-2,6-dione

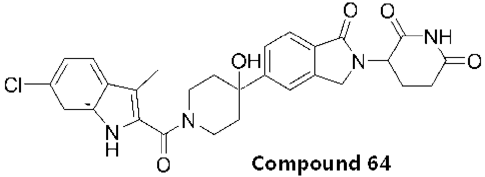

Compound 64 was synthesized in the same manner as for Compound 1, with the exception of using Intermediate 18 and 6-chloro-3-methyl-1H-indole-2-carboxylic acid.

¹H NMR (300 MHz, DMSO-d6) δ 11.40 (s, 1H), 11.00 (s, 1H), 7.76-7.65 (m, 3H), 7.58 (d, J = 8.5 Hz, 1H), 7.38 (d, J = 1.9 Hz, 1H), 7.06 (dd, J = 1.9, 8.5 Hz, 1H), 5.48 (s, 1H), 5.12 (dd, J = 5.1, 13.2 Hz, 1H), 4.46 (d, J = 17.3 Hz, 1H), 4.32 (d, J = 17.3 Hz, 1H), 3.56-3.42 (m,1 H), 3.01-2.82 (m, 1H), 2.66-2.58 (m, 1H), 2.43-2.37 (m, 2H), 2.29 (s, 3H), 2.14-1.91 (m, 4H), 1.77-1.61 (m, 2H).

### Compound 65. 3-(5-(1-(6-chloro-3-methyl-1H-indole-2-carbonyl)-2,5-dihydro-1H-pyrrol-3-yl)-1-oxoinsoindolin-2-yl)piperidine-2,6-dione

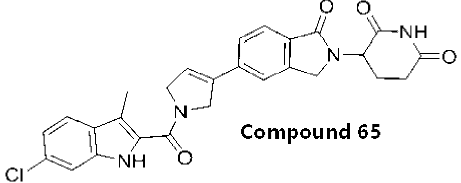

Compound 65 was synthesized in the same manner as for Compound 1, with the exception of using Intermediate 12 and 6-chloro-3-methyl-1H-indole-2-carboxylic acid.

¹H NMR (300 MHz, DMSO-d6) δ 11.35 (s, 1H), 10.99 (s, 1H), 7.69 (s, 1H), 7.63-7.43 (m, 3H), 7.37 (s, 1H), 7.05 (d, J = 8.6 Hz, 1H), 5.11 (d, J = 10.5 Hz, 1H), 4.41 (s, 1H), 4.38-4.24 (m, 1H), 3.69-3.57 (m, 5H), 2.99-2.84 (m, 1H), 2.65-2.60 (m, 1H), 2.45-2.36 (m, 2H), 2.33 (s, 3H), 2.14-1.96 (m, 2H).

### Compound 66. 3-(5-(1-(1-(6-chloro-3-methyl-1H-indole-2-carbonyl)piperidin-4-yl)-1H-1,2,3-triazol-4-yl)-1-oxoinsoindolin-2-yl)piperidine-2,6-dione

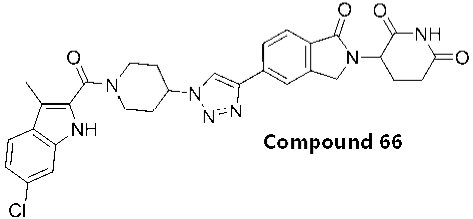

Compound 66 was synthesized in the same manner as for Compound 1, with the exception of using Intermediate 38 and 6-chloro-3-methyl-1H-indole-2-carboxylic acid.

¹H NMR (300 MHz, DMSO-d6) δ 11.43 (s, 1H), 11.02 (s, 1H), 8.87 (d, J = 3.4 Hz, 1H), 8.11 (s, 1H), 8.01 (d, J = 8.1 Hz, 1H), 7.81 (d, J = 8.1 Hz, 1H), 7.59 (d, J = 7.8 Hz, 1H), 7.38 (s, 1H), 7.07 (d, J = 8.6 Hz, 1H), 5.13 (d, J = 13.1 Hz, 1H), 4.94 (s, 1H), 4.54 (d, J = 17.4 Hz, 1H), 4.43 (s, 1H), 4.23 (s, 2H), 3.07-2.80 (m, 2H), 2.65-2.58 (m, 1H), 2.31 (s, 3H), 2.28-2.20 (m, 2H), 2.12-1.90 (m, 4H).

### Compound 67. 3-(5-(1-(6-chloro-3-methyl-1H-indole-2-carbonyl)pyrrolidin-3-yl)-1-oxoinsoindolin-2-yl)piperidine-2,6-dione

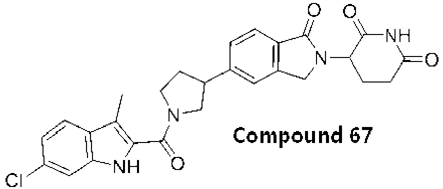

Compound 67 was synthesized in the same manner as for Compound 1, with the exception of using Intermediate 11 and 6-chloro-3-methyl-1H-indole-2-carboxylic acid.

¹H NMR (500 MHz, DMSO-d6) δ 11.36 (s, 1H), 11.01 (s, 1H), 7.78-7.37 (m, 5H), 7.06 (s, 1H), 5.12 (s, 1H), 4.44 (s, 1H), 4.32 (s, 1H), 4.04-3.86 (m, 3H), 2.97-2.86 (m, 1H), 2.67-2.59 (m, 3H), 2.44-2.36 (m, 2H), 2.33 (s, 3H), 2.22-1.95 (m, 2H).

### Compound 68. 3-(5-((1-(3-methyl-6-nitro-1H-indole-2-carbonyl)piperidin-4-yl)ethynyl)-1-oxoinsoindolin-2-yl)piperidine-2,6-dione

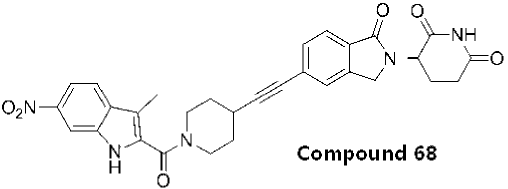

Compound 68 was synthesized in the same manner as for Compound 1, with the exception of using Intermediate 31 and 3-methyl-6-nitro-1H-indole-2-carboxylic acid.

¹H NMR (300 MHz, DMSO-d6) δ 12.06 (s, 1H), 11.02 (s, 1H), 8.27 (d, J = 2.1 Hz, 1H), 7.93 (dd, J = 8.9, 2.1 Hz, 1H), 7.77 (d, J = 8.8 Hz, 1H), 7.71 (d, J = 7.9 Hz, 1H), 7.68 (s, 1H), 7.55 (dd, J = 7.8, 1.4 Hz, 1H), 5.12 (dd, J = 13.2, 5.0 Hz, 1H), 4.46 (d, J = 17.7 Hz, 1H), 4.32 (d, J = 17.6 Hz, 1H), 3.13-2.86 (m, 4H), 2.65-2.56 (m, 1H), 2.46-2.35 (m, 2H), 2.31 (s, 3H), 2.06-1.88 (s, 4H), 1.74-1.60 (d, J = 10.5 Hz, 2H).

### Compound 69. 3-(5-(1-(3,6-dimethyl-1H-indole-2-carbonyl)piperidin-4-yl)-1-oxoinsoindolin-2-yl)piperidine-2,6-dione

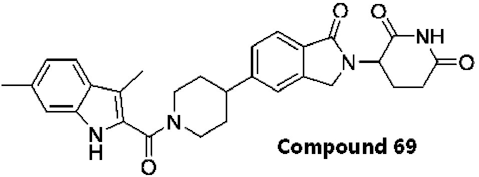

Compound 69 was synthesized in the same manner as for Compound 1, with the exception of using Intermediate 7 and 3,6-dimethyl-1H-indole-2-carboxylic acid.

¹H NMR (300 MHz, DMSO-d6) δ 11.05 (s, 1H), 10.99 (s, 1H), 7.68 (d, J = 7.8 Hz, 1H), 7.52 (s, 1H), 7.43 (d, J = 8.0 Hz, 2H), 7.13 (s, 1H), 6.87 (d, J = 8.2 Hz, 1H), 5.11 (dd, J = 13.1, 5.0 Hz, 1H), 4.45 (d, J = 17.3 Hz, 1H), 4.34-4.15 (m, 3H), 3.14-2.86 (m, 4H), 2.67-2.61 (m, 1H), 2.39 (s, 3H), 2.28 (s, 3H), 1.95 (d, J = 21.5 Hz, 3H), 1.76-1.60 (m, 2H).

### Compound 70. 3-(5-(1-(6-chloro-3-methyl-1H-indole-2-carbonyl)-1,2,3,6-tetrahydropyridin-4-yl)-1-oxoinsoindolin-2-yl)piperidine-2,6-dione

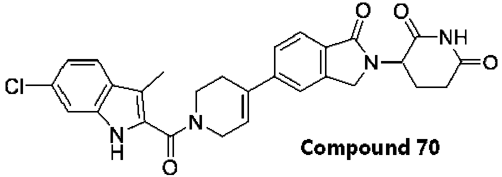

Compound 70 was synthesized in the same manner as for Compound 1, with the exception of using the compound of tert-Boc-removed Intermediate 5 and 6-chloro-3-methyl-1H-indole-2-carboxylic acid.

¹H NMR (300 MHz, DMSO-d6) δ 11.43 (s, 1H), 10.99 (s, 1H), 7.73-7.66 (m, 2H), 7.65-7.57 (m, 2H), 7.37 (d, J = 1.9 Hz, 1H), 7.07 (dd, J = 8.5, 1.9 Hz, 1H), 6.37 (s, 1H), 5.11 (dd, J = 13.3, 5.1 Hz, 1H), 4.46 (d, J = 17.3 Hz, 1H), 4.37-4.27 (m, 3H), 3.82-3.77 (m, 2H), 2.98-2.84 (m, 2H), 2.67-2.62 (m, 3H), 2.30 (s, 3H), 2.05-1.97 (m, 1H).

### Compound 71. 3-(5-(1-(6-chloro-5-methoxy-3-methyl-1H-indole-2-carbonyl)piperidin-4-yl)-1-oxoinsoindolin-2-yl)piperidine-2,6-dione

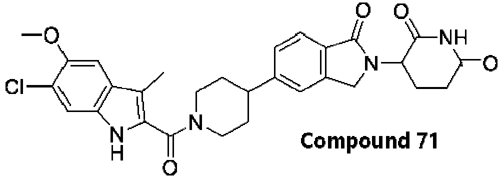

Compound 71 was synthesized in the same manner as for Compound 1, with the exception of using Intermediate 1 and 6-chloro-5-methoxy-3-methyl-1H-indole-2-carboxylic acid.

¹H NMR (400 MHz, DMSO-d6) δ 11.20 (s, 1H), 11.00 (s, 1H), 7.68 (d, J = 7.7 Hz, 1H), 7.52 (s, 1H), 7.43 (d, J = 7.9 Hz, 1H), 7.39 (s, 1H), 7.20 (s, 1H), 5.11 (dd, J = 13.3, 5.1 Hz, 1H), 4.44 (d, J = 17.3 Hz, 1H), 4.31 (d, J = 17.2 Hz, 1H), 3.87 (s, 3H), 3.19-2.87 (m, 4H), 2.68-2.56 (m, 1H), 2.46-2.32 (m, 1H), 2.29 (s, 3H), 2.02-1.85 (m, 3H), 1.74-1.61 (m, 2H).

### Compound 72. 3-(5-(1-(5,6-dichloro-3-methyl-1H-indole-2-carbonyl)piperidin-4-yl)-1-oxoinsoindolin-2-yl)piperidine-2,6-dione

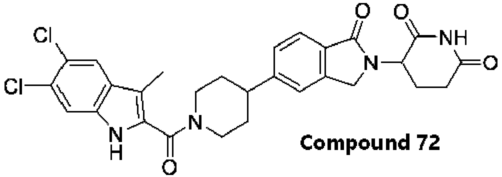

Compound 72 was synthesized in the same manner as for Compound 1, with the exception of using Intermediate 1 and 5,6-dichloro-3-methyl-1H-indole-2-carboxylic acid.

¹H NMR (400 MHz, DMSO-d6) δ 11.59 (s, 1H), 11.00 (s, 1H), 7.85 (s, 1H), 7.68 (d, J = 7.8 Hz, 1H), 7.57 (s, 1H), 7.52 (s, 1H), 7.43 (d, J = 7.9 Hz, 1H), 5.11 (dd, J = 13.3, 5.1 Hz, 1H), 4.70-4.50 (m, 1H), 4.44 (d, J = 17.3 Hz, 1H), 4.30 (d, J = 17.3 Hz, 1H), 4.16-3.66 (m, 1H), 3.16-2.84 (m, 4H), 2.64-2.57 (m, 1H), 2.44-2.33 (m, 1H), 2.03-1.95 (m, 1H), 1.93-1.81 (m, 2H), 1.77-1.59 (m, 2H).

### Compound 73. N-(2-(4-(2-(2,6-dioxopiperidin-3-yl)-1-oxoindolin-5-yl)piperidine-1-carbonyl)-3-methyl-1H-indol-6-yl)acetamide

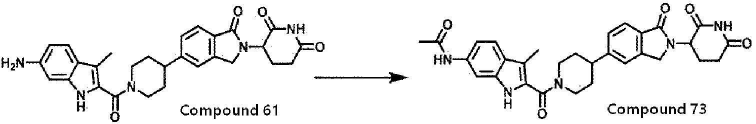

Compound 61 (15 mg, 0.03 mmol), acetic acid (1.89 uL, 0.033 mmol), EDCI-HCl (6.33 mg, 0.033 mmol), HOBt (4.46 mg, 0.033 mmol), and DIPEA (16 uL, 0.033 mmol) in DMF (1 mL) was stirred at room temperature for 12 hours. After completion of the reaction, the reaction mixture was diluted with water and then subjected to extraction with ethylacetate. The organic layer is dried over anhydrous magnesium sulfate, followed by filtration and concentration. Purification of the concentrate by column chromatography afforded the target Compound 73 (2 mg, 15%).

¹H NMR (300 MHz, DMSO-d6) δ 11.10 (s, 1H), 11.01 (s, 1H), 9.90 (s, 1H), 7.95 (d, J = 1.0 Hz, 1H), 7.95-7.92 (m, 1H), 7.70-7.63 (m, 2H), 7.52 (s, 1H), 7.51-7.47 (m, 1H), 7.45 (d, J = 1.7 Hz, 1H), 7.42 (d, J = 2.6 Hz, 1H), 7.40-7.34 (m, 1H), 7.06 (dd, J = 8.6, 1.8 Hz, 1H), 5.12 (dd, J = 13.2, 5.1 Hz, 1H), 4.44 (d, J = 17.3 Hz, 1H), 4.35-4.18 (m, 3H), 3.15-2.84 (m, 4H), 2.65-2.56 (m, 1H), 2.44-2.35 (m, 1H), 2.27 (s, 3H), 2.05 (s, 3H), 2.02-1.96 (m, 1H), 1.93-1.83 (m, 2H), 1.75-1.58 (m, 2H).

### Compound 74. N-(2-(4-(2-(2,6-dioxopiperidin-3-yl)-1-oxoindolin-5-yl)piperidine-1-carbonyl)-3-methyl-1H-indol-6-yl) methanesulfonamide

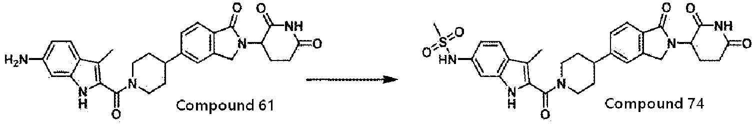

A solution of Compound 61 (15 mg, 0.03 mmol) and methanesulfonyl chloride (2.56 uL, 0.033 mmol in dioxane (1 mL) was stirred at 90°C for 12 hours. After completion of the reaction, the reaction mixture was diluted with water and then subjected to extraction with ethylacetate. The organic layer was dried over anhydrous magnesium sulfate, followed by filtration and concentration. Purification of the concentrate by column chromatography afforded the target Compound 74 (3 mg, 15%).

¹H NMR (300 MHz, DMSO-d6) δ 11.20 (s, 1H), 11.00 (s, 1H), 9.55 (s, 1H), 7.68 (d, J = 7.8 Hz, 1H), 7.50 (d, J = 9.6 Hz, 2H), 7.43 (d, J = 8.0 Hz, 1H), 7.27 (d, J = 1.8 Hz, 1H), 6.95 (dd, J = 8.5, 1.9 Hz, 1H), 5.11 (dd, J = 13.3, 5.1 Hz, 1H), 4.45 (d, J = 17.3 Hz, 1H), 4.36-4.17 (m, 3H), 3.18-2.95 (m, 4H), 2.90 (s, 3H), 2.65-2.60 (m, 1H), 2.43-2.36 (m, 1H), 2.28 (s, 3H), 2.04-1.84 (m, 3H), 1.74-1.62 (m, 2H).

### Compound 75. 1-(2-(4-(2-(2,6-dioxopiperidin-3-yl)-1-oxoindolin-5-yl)piperidine-1-carbonyl)-3-methyl-1H-indol-6-yl)-3-ethylurea

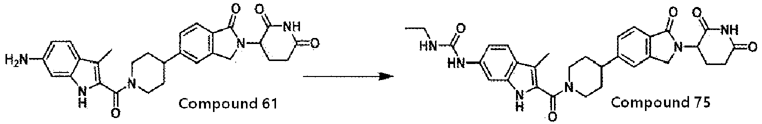

A solution of Compound 61 (15 mg, 0.03 mmol) and isocyanatoethane (2.1 mg, 0.03 mmol in THF (1 mL) was stirred at room temperature for 12 hours. After completion of the reaction, the reaction mixture was diluted with water and then subjected to extraction with ethylacetate. The organic layer was dried over anhydrous magnesium sulfate, followed by filtration and concentration. Purification of the concentrate by column chromatography afforded the target Compound 75 (3 mg, 15%).

¹H NMR (400 MHz, DMSO-d6) δ 11.00 (s, 1H), 10.96 (s, 1H), 8.41 (s, 1H), 7.71 (s, 1H), 7.68 (d, J = 7.8 Hz, 1H), 7.52 (s, 1H), 7.43 (d, J = 7.9 Hz, 1H), 7.37 (d, J = 8.4 Hz, 1H), 6.84 (d, J = 8.6 Hz, 1H), 6.06 (s, 1H), 5.11 (dd, J = 13.4, 5.1 Hz, 1H), 4.44 (d, J = 17.2 Hz, 1H), 4.36-4.18 (m, 3H), 3.11 (t, J = 6.8, 6.8 Hz, 2H), 3.02-2.84 (m, 4H), 2.64-2.56 (m, 1H), 2.42-2.38 (m, 1H), 2.26 (s, 3H), 2.06-1.95 (m, 2H), 1.94-1.80 (m, 3H), 1.06 (t, J = 6.9, 6.9 Hz, 3H).

### Compound 76. 3-(5-((1-(6-chloro-3-methyl-1H-indole-2-carbonyl)azetidin-3-yl)ethynyl)-1-oxoinsoindolin-2-yl)piperidine-2,6-dione

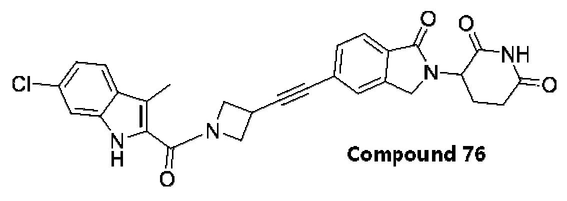

Compound 76 was synthesized in the same manner as for Compound 1, with the exception of using 6-chloro-3-methyl-1H-indole-2-carboxylic acid and 3-(5-(azetidin-3-ylethynyl)-1-oxoinsoindolin-2-yl)piperidine-2,6-dione chloride.

¹H NMR (400 MHz, DMSO-d6) δ 11.24 (s, 1H), 11.02 (s, 1H), 7.75-7.69 (m, 2H), 7.60 (dd, J = 12.1, 8.2 Hz, 2H), 7.38 (s, 1H), 7.07 (dd, J = 8.5, 1.8 Hz, 1H), 5.11 (d, J = 5.1 Hz, 1H), 4.58-4.43 (m, 3H), 4.33 (d, J = 17.5 Hz, 1H), 4.14 (s, 1H), 3.94-3.84 (m, 1H), 2.98-2.85 (m, 1H), 2.64-2.55 (m, 1H), 2.40 (s, 3H), 2.38-2.33 (m, 1H), 2.04-1.97 (m, 1H).

### Compound 77. 3-(5-((1-(6-chloro-3-methyl-1H-indole-2-carbonyl)-3-hydroxyazetidin-3-yl)ethynyl)-1-oxoinsoindolin-2-yl)piperidine-2,6-dione

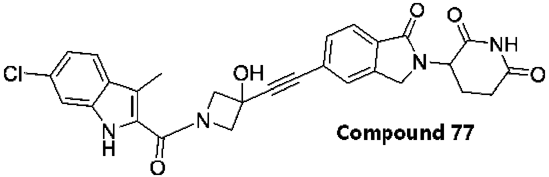

Compound 77 was synthesized in the same manner as for Compound 1, with the exception of using 6-chloro-3-methyl-1H-indole-2-carboxylic acid and 3-(5-((3-hydroxyazetidin-3-yl)ethynyl)-1-oxoinsoindolin-2-yl)piperidine-2,6-dione chloride.

¹H NMR (400 MHz, DMSO-d6) δ 11.27 (s, 1H), 11.02 (s, 1H), 7.75 (d, J = 6.8 Hz, 2H), 7.66-7.56 (m, 2H), 7.38 (s, 1H), 7.07 (d, J = 8.4 Hz, 1H), 6.88 (s, 1H), 5.12 (dd, J = 13.5, 5.0 Hz, 1H), 4.67-4.41 (m, 3H), 4.39-4.19 (m, 3H), 2.98-2.84 (m, 1H), 2.68-2.57 (m, 1H), 2.41 (s, 3H), 2.39-2.34 (m, 1H), 2.07-1.90 (m, 2H).

### Compound 78. 3-(5-((1-(6-chloro-3-methyl-1H-indole-2-carbonyl)pyrrolidin-3-yl)ethynyl)-1-oxoinsoindolin-2-yl)piperidine-2,6-dione

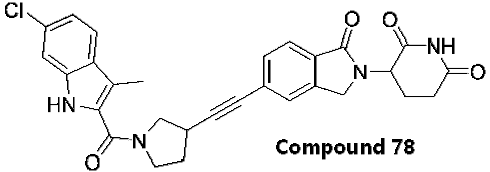

Compound 78 was synthesized in the same manner as for Compound 1, with the exception of using 6-chloro-3-methyl-1H-indole-2-carboxylic acid and 3-(1-oxo-5-(pyrrolidin-3-ylethynyl)isoindolin-2-yl)piperidine-2,6-dione chloride.

¹H NMR (400 MHz, DMSO-d6) δ 11.37 (s, 1H), 11.02 (s, 1H), 7.71 (d, J = 7.9 Hz, 1H), 7.65 (s, 1H), 7.59 (d, J = 8.5 Hz, 1H), 7.53 (s, 1H), 7.37 (d, J = 1.8 Hz, 1H), 7.06 (dd, J = 8. 5, 1.9 Hz, 1H), 5.12 (dd, J = 13.3, 5.1 Hz, 1H), 4.45 (d, J = 17.5 Hz, 1H), 4.33 (d, J = 17.5 Hz, 1H), 3.93-3.79 (m, 1H), 3.77-3.66 (m, 1H), 3.65-3.56 (m, 2H), 3.42 (s, 1H), 2.99-2.83 (m, 1H), 2.64-2.58 (m, 1H), 2.45-2.36 (m, 2H), 2.33 (s, 3H), 2.14-1.90 (m, 3H).

### Compound 79. 3-(5-((1-(6-chloro-3-methyl-1H-indole-2-carbonyl)-3-hydroxypyrrolidin-3-yl)ethynyl)-1-oxoinsoindolin-2-yl)piperidine-2,6-dione

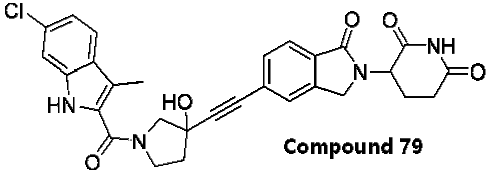

Compound 79 was synthesized in the same manner as for Compound 1, with the exception of using 6-chloro-3-methyl-1H-indole-2-carboxylic acid and 3-(5-((3-hydroxypyrrolidin-3-yl)ethynyl)-1-oxoinsoindolin-2-yl)piperidine-2,6-dione chloride.

¹H NMR (400 MHz, DMSO-d6) δ 11.38 (s, 1H), 11.02 (s, 1H), 7.76 - 7.65 (m, 2H), 7.62 - 7.52 (m, 2H), 7.37 (s, 1H), 7.07 (d, J = 8.5 Hz, 1H), 6.18 (d, J = 51.9 Hz, 1H), 5.13 (d, J = 13.3 Hz, 1H), 4.46 (d, J = 17.1 Hz, 1H), 4.34 (d, J = 17.6 Hz, 1H), 3.91 - 3.54 (m, 5H), 2.97 - 2.86 (m, 1H), 2.62 (s, 1H), 2.45 - 2.35 (m, 2H), 2.33 (s, 3H), 2.04 - 1.96 (m, 1H).

### Compound 80. 3-(5-((1-(6-chloro-3-methyl-1H-indole-2-carbonyl)-4-hydroxypiperidin-4-yl)ethynyl)-1-oxoinsoindolin-2-yl)piperidine-2,6-dione

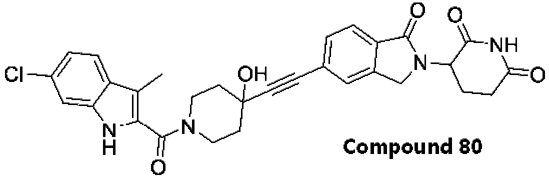

Compound 80 was synthesized in the same manner as for Compound 1, with the exception of using 6-chloro-3-methyl-1H-indole-2-carboxylic acid and 3-(5-((4-hydroxypiperidin-4-yl)ethynyl)-1-oxoinsoindolin-2-yl)piperidine-2,6-dione chloride.

¹H NMR (400 MHz, DMSO-d6) δ 11.39 (s, 1H), 11.02 (s, 1H), 7.79-7.68 (m, 2H), 7.64-7.56 (m, 2H), 7.36 (s, 1H), 7.06 (d, J = 8.5 Hz, 1H), 5.97 (s, 1H), 5.13 (dd, J = 13.4, 5.1 Hz, 1H), 4.47 (d, J = 17.5 Hz, 1H), 4.35 (d, J = 17.4 Hz, 1H), 3.85-3.75 (m, 2H), 3.65-3.47 (m, 2H), 2.98-2.84 (m, 1H), 2.65-2.57 (m, 1H), 2.41 (d, J = 11.7 Hz, 1H), 2.04-1.91 (m, 3H), 1.85-1.73 (m, 2H).

### Compound 81. 6-chloro-2-(4-(2-(2,6-dioxopiperidin-3-yl)-1-oxoindolin-5-yl)piperidine-1-carbonyl)-1H-indole-3-carbaldehyde

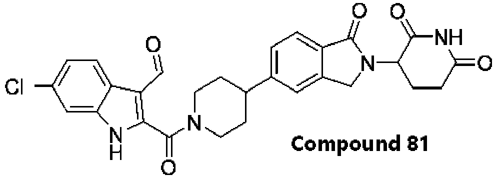

Compound 81 was synthesized in the same manner as for Compound 1, with the exception of using Intermediate 7 and 6-chloro-3-formyl-1H-indole-2-carboxylic acid.

¹H NMR (400 MHz, DMSO-d6) δ 12.82 (s, 1H), 11.00 (s, 1H), 10.02 (s, 1H), 8.13 (d, J = 8.5 Hz, 1H), 7.69 (d, J = 7.9 Hz, 1H), 7.59 (d, J = 1.8 Hz, 1H), 7.52 (s, 1H), 7.44 (d, J = 7.9 Hz, 1H), 7.32 (dd, J = 8.5, 1.9 Hz, 1H), 5.12 (dd, J = 13.3, 5.1 Hz, 1H), 4.78-4.64 (m, 1H), 4.45 (d, J = 17.3 Hz, 1H), 4.31 (d, J = 17.2 Hz, 1H), 3.71-3.56 (m, 1H), 3.32-3.27 (m, 1H), 3.10-2.85 (m, 3H), 2.63-2.58 (m, 1H), 2.45-2.32 (m, 1H), 2.05-1.89 (m, 2H), 1.83-1.65 (m, 3H).

### Compound 82. 3-(5-(1-(6-chloro-3-(morpholinomethyl)-1H-indole-2-carbonyl)piperidin-4-yl)-1-oxoinsoindolin-2-yl)piperidine-2,6-dione

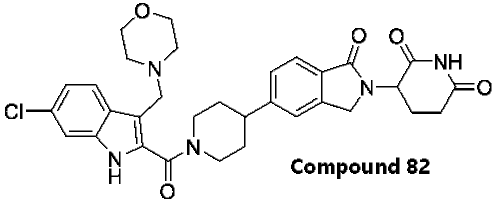

Compound 82 was synthesized in the same manner as for Compound 1, with the exception of using Intermediate 7 and 6-chloro-3-(morpholinomethyl)-1H-indole-2-carboxylic acid.

¹H NMR (400 MHz, DMSO-d6) δ 11.60 (s, 1H), 11.00 (s, 1H), 7.75 (d, J = 8.5 Hz, 1H), 7.68 (d, J = 7.7 Hz, 1H), 7.49 (s, 1H), 7.41 (d, J = 7.9 Hz, 1H), 7.39 (s, 1H), 7.06 (d, J = 8.5 Hz, 1H), 5.11 (dd, J = 13.2, 5.1 Hz, 1H), 4.44 (d, J = 17.2 Hz, 1H), 4.30 (d, J = 17.2 Hz, 1H), 3.63 (s, 2H), 3.52 (s, 4H), 3.05-2.86 (m, 4H), 2.63-2.58 (m, 1H), 2.45-2.39 (m, 1H), 2.34 (s, 4H), 2.01-1.91 (m, 2H), 1.89-1.83 (m, 1H), 1.70-1.60 (m, 2H).

### Compound 83. 3-(5-(1-(5-bromo-6-chloro-3-methyl-1H-indole-2-carbonyl)piperidin-4-yl)-1-oxoinsoindolin-2-yl)piperidine-2,6-dione

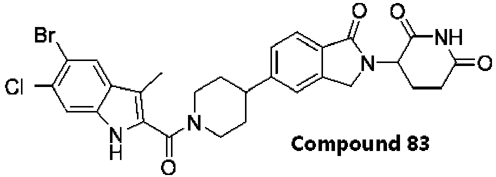

Compound 83 was synthesized in the same manner as for Compound 1, with the exception of using Intermediate 7 and 5-bromo-6-chloro-3-methyl-1H-indole-2-carboxylic acid.

¹H NMR (400 MHz, DMSO-d6) δ 11.59 (s, 1H), 10.99 (s, 1H), 7.98 (s, 1H), 7.67 (d, J = 7.8 Hz, 1H), 7.57 (s, 1H), 7.51 (s, 1H), 7.42 (d, J = 7.9 Hz, 1H), 5.11 (dd, J = 13.3, 5.1 Hz, 1H), 4.43 (d, J = 17.2 Hz, 1H), 4.30 (d, J = 17.3 Hz, 1H), 3.17 - 2.85 (m, 4H), 2.63-2.57 (m, 1H), 2.42-2.32 (m, 1H), 2.27 (s, 3H), 2.01-1.82 (m, 3H), 1.71-1.59 (m, 2H).

### Compound 84. 3-(5-(1-(5-chloro-3-methyl-6-nitro-1H-indole-2-carbonyl)piperidin-4-yl)-1-oxoinsoindolin-2-yl)piperidine-2,6-dione

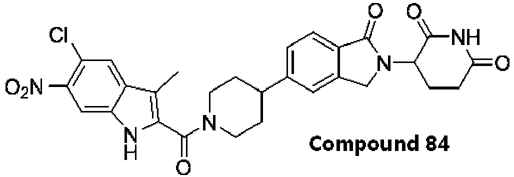

Compound 84 was synthesized in the same manner as for Compound 1, with the exception of using Intermediate 7 and 5-chloro-3-methyl-6-nitro-1H-indole-2-carboxylic acid.

¹H NMR (400 MHz, DMSO-d6) δ 12.16 (s, 1H), 11.00 (s, 1H), 8.12 (s, 1H), 7.95 (s, 1H), 7.69 (d, J = 7.8 Hz, 1H), 7.52 (s, 1H), 7.44 (d, J = 8.0 Hz, 1H), 5.11 (dd, J = 13.4, 5.1 Hz, 1H), 4. 64 (s, 1H), 4.44 (d, J = 17.2 Hz, 1H), 4.31 (d, J = 17.2 Hz, 1H), 3.73 (s, 1H), 3.13-2.82 (m, 4H), 2.66-2.58 (m, 1H), 2.47-2.38 (m, 1H), 2.31 (s, 3H), 2.03-1.84 (m, 3H), 1.76-1.60 (m, 2H).

### Compound 85. 3-(5-(1-(3,5-dimethyl-6-nitro-1H-indole-2-carbonyl)piperidin-4-yl)-1-oxoinsoindolin-2-yl)piperidine-2,6-dione

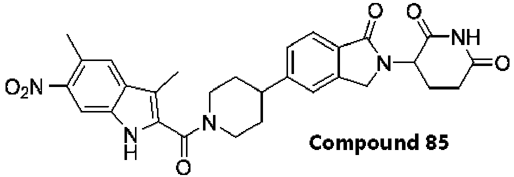

Compound 85 was synthesized in the same manner as for Compound 1, with the exception of using Intermediate 7 and 3,5-dimethyl-6-nitro-1H-indole-2-carboxylic acid.

¹H NMR (400 MHz, DMSO-d6) δ 11.86 (s, 1H), 11.00 (s, 1H), 8.10 (s, 1H), 7.68 (d, J = 7.8 Hz, 1H), 7.62 (s, 1H), 7.52 (s, 1H), 7.43 (d, J = 7.9 Hz, 1H), 5.11 (dd, J = 13.4, 5.1 Hz, 1H), 4.60 (s, 1H), 4.44 (d, J = 17.2 Hz, 1H), 4.31 (d, J = 17,2 Hz, 1H), 3.79 (s, 1H), 3.13-2.83 (m, 4H), 2.62 (s, 3H), 2.60-2.56 (m, 1H), 2.43-2.36 (m, 1H), 2.31 (s, 3H), 2.03-1.84 (m, 3H), 1.74-1.60 (m, 2H).

### Compound 86. 3-(5-(1-(5-hydroxy-3-methyl-6-nitro-1H-indole-2-carbonyl)piperidin-4-yl)-1-oxoinsoindolin-2-yl)piperidine-2,6-dione

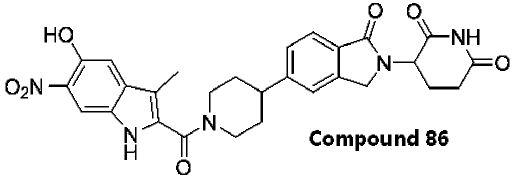

Compound 86 was synthesized in the same manner as for Compound 1, with the exception of using Intermediate 7 and 5-hydroxy-3-methyl-6-nitro-1H-indole-2-carboxylic acid.

¹H NMR (400 MHz, DMSO-d6) δ 11.64 (s, 1H), 11.00 (s, 1H), 10.07 (s, 1H), 8.00 (s, 1H), 7.68 (d, J = 7.8 Hz, 1H), 7.52 (s, 1H), 7.43 (d, J = 8.0 Hz, 1H), 7.16 (s, 1H), 5.11 (dd, J = 13.3, 5.1 Hz, 1H), 4.63 (s, 1H), 4.44 (d, J = 17.2 Hz, 1H), 4.33 (s, 1H), 3.76 (s, 1H), 3.12-2.86 (m, 4H), 2.62-2.57 (m, 1H), 2.45-2.34 (m, 1H), 2.23 (s, 3H), 2.03-1.84 (m, 3H), 1.76-1.60 (m, 2H).

### Compound 87. 3-(5-(1-(6-chloro-3-((dimethylamino)methyl)-1H-indole-2-carbonyl)piperidin-4-yl)-1-oxoinsoindolin-2-yl)piperidine-2,6-dione

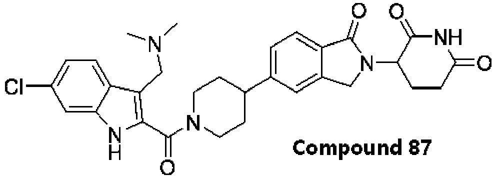

Compound 87 was synthesized in the same manner as for Compound 1, with the exception of using Intermediate 7 and 6-chloro-3-((dimethylamino)methyl)-1H-indole-2-carboxylic acid.

¹H NMR (400 MHz, DMSO-d6) δ 11.76 (s, 1H), 10.98 (s, 1H), 7.76 (d, J = 8.5 Hz, 1H), 7.68 (d, J = 7.7 Hz, 1H), 7.49 (s, 1H), 7.46-7.39 (m, 2H), 7.08 (d, J = 8.6 Hz, 1H), 5.10 (d, J = 13.5 Hz, 1H), 4.44 (d, J = 17.1 Hz, 1H), 4.30 (d, J = 17.2 Hz, 1H), 3.87-3.58 (m, 2H), 3.17-2.89 (m, 4H), 2.66-2.56 (m, 1H), 2.40-2.35 (m, 1H), 2.26 (s, 6H), 2.01-1.82 (m, 3H), 1.74-1.65 (m, 2H).

### Compound 88. 3-(5-((1-(6-chloro-3-methyl-1H-indole-2-carbonyl)piperidin-4-yl)ethynyl)-6-fluoro-1-oxoinsoindolin-2-yl)piperidine-2,6-dione

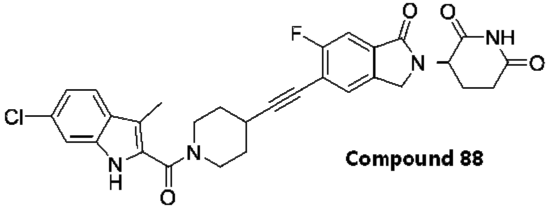

Compound 88 was synthesized in the same manner as for Compound 1, with the exception of using 3-(6-fluoro-1-oxo-5-(piperidin-4-ylethynyl)isoindolin-2-yl)piperidine-2,6-dione chloride and 6-chloro-3-methyl-1H-indole-2-carboxylic acid.

¹H NMR (400 MHz, DMSO-d6) δ 11.39 (s, 1H), 11.02 (s, 1H), 7.78 (d, J = 6.0 Hz, 1H), 7.61 (d, J = 7.9 Hz, 1H), 7.57 (d, J = 8.6 Hz, 1H), 7.36 (d, J = 2.0 Hz, 1H), 7.06 (dd, J = 8.4, 2.0 Hz, 1H), 5.12 (dd, J = 13.3, 5.1 Hz, 1H), 4.44 (d, J = 17.4 Hz, 1H), 4.32 (d, J = 17.4 Hz, 1H), 3.84 (s, 2H), 3.46-3.38 (m, 2H), 3.15-3.08 (m, 1H), 2.97-2.85 (m, 1H), 2.63-2.58 (m, 1H), 2.45-2.33 (m, 1H), 2.27 (s, 3H), 2.06-1.92 (m, 3H), 1.74-1.62 (m, 2H).

### Compound 89. 3-(5-((1-(6-chloro-3-methyl-1H-indole-2-carbonyl)piperidin-4-yl)ethynyl)-4-fluoro-1-oxoinsoindolin-2-yl)piperidine-2,6-dione

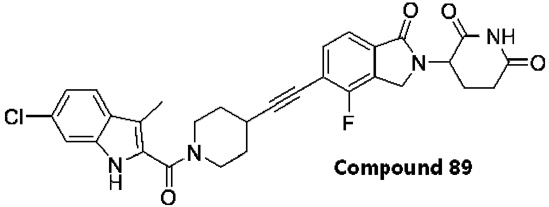

Compound 89 was synthesized in the same manner as for Compound 1, with the exception of using (3-(8-fluoro-1-oxo-5-(piperidin-4-ylethynyl)isoindolin-2-yl)piperidine-2,6-dione chloride and 6-chloro-3-methyl-1H-indole-2-carboxylic acid.

¹H NMR (400 MHz, DMSO-d6) δ 11.40 (s, 1H), 11.03 (s, 1H), 7.65 (d, J = 6.7 Hz, 1H), 7.59 (s, 1H), 7.56 (s, 1H), 7.37 (d, J = 2.1 Hz, 1H), 7.06 (dd, J = 8.5, 2.0 Hz, 1H), 5.13 (dd, J = 13.3, 5.0 Hz, 1H), 4.59 (d, J = 17.5 Hz, 1H), 4.42 (d, J = 17.5 Hz, 1H), 3.84 (s, 2H), 3.47-3.38 (m, 2H), 3.16-3.07 (m, 1H), 2.98-2.86 (m, 1H), 2.66-2.57 (m, 1H), 2.47-2.37 (m, 1H), 2.27 (s, 3H), 2.07-1.89 (m, 3H), 1.74-1.62 (m, 2H).

### Compound 90. 3-(5-(1-(6-chloro-3-methyl-1H-indole-2-carbonyl)piperidin-4-yl)-6-fluoro-1-oxoinsoindolin-2-yl)piperidine-2,6-dione

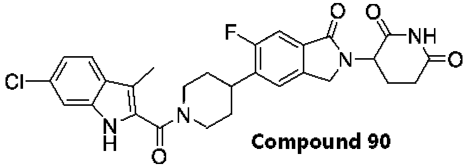

### (1) Synthesis of Intermediate 3-(6-fluoro-1-oxo-5-(piperidin-4-yl)isoindolin-2-yl)piperidine-2,6-dione chloride (E)

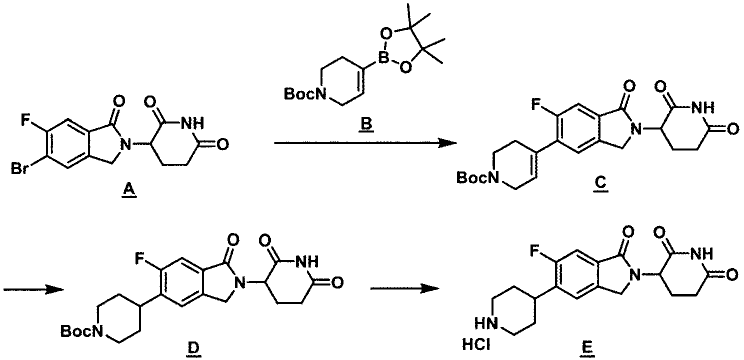

### Preparation of (1-1) tert-butyl 4-(2-(2,6-dioxopiperidin-3-yl)-6-fluoro-1-oxoisoindolin-5-yl)-3,6-dihydropyridine-1 (2H)-carboxylate (C)

Compound A (500 mg, 1.5 mmol), Compound B (589 mg, 1.91 mmol), potassium carbonate (506 mg, 3.7 mmol), and Pd (dppf)Cl₂·DCM (120 mg, 0.15 mmol) in DMF (10 mL) was stirred at 110°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford Compound C (692 mg, 48%).

¹H NMR (400 MHz, DMSO-d6) δ 11.01 (s, 1H), 7.61 (d, 0H), 7.52 (d, J = 7.7 Hz, 2H), 7.07-6.95 (m, 1H), 5.12 (dd, 1H), 4.97-4.81 (m, 1H), 4.44 (d, 1H), 4.30 (d, J = 5.4 Hz, 1H), 4.05-4.01 (m, 1H), 3.90-3.84 (m, 1H), 3.59-3.47 (m, 2H), 2.97-2.85 (m, 1H), 2.65-2.56 (m, 1H), 2.44-2.32 (m, 1H), 2.18-2.08 (m, 1H), 2.05-1.95 (m, 1H), 1.79-1.68 (m, 1H), 1.46 (s, 5H), 1.44 (s, 4H).

### Preparation of (1-2) tert-butyl 4-(2-(2,6-dioxopiperidin-3-yl)-6-fluoro- 1-oxoisoindolin-5-yl)piperidine-1-carboxylate (D)

A solution of Compound C (620 mg, 1.40 mmol) and 10% Pd/C (186 mg) in DMF (10 mL) was stirred for 12 hours under a hydrogen pressure. After completion of the reaction, the reaction mixture was filtered through Celite. The filtrate was diluted with water, followed by extraction with ethylacetate. The organic layers were pooled, dried over anhydrous magnesium sulfate, filtered, and concentrated to afford the target Compound D (528 mg, 85%).

¹H NMR (400 MHz, DMSO-d6) δ 11.00 (s, 1H), 7.63 (d, J = 5.9 Hz, 1H), 7.49 (d, J = 2.3 Hz, 1H), 5.12 (dd, J = 13.3, 2.4 Hz, 1H), 4.42 (d, J = 17.3 Hz, 1H), 4.29 (d, J = 17.2 Hz, 1H), 4.22-3.94 (m, 2H), 3.15-3.04 (m, 1H), 2.98-2.75 (m, 3H), 2.64-2.55 (m, 1H), 2.47-2.33 (m, 1H), 2.06-1.95 (m, 1H), 1.81-1.71 (m, 2H), 1.65-1.51 (m, 2H), 1.43 (s, 9H).

### (1-3) Preparation of 3-(6-fluoro-1-oxo-5-(piperidin-4-yl)isoindolin-2-yl)piperidine-2,6-dione chloride (E)

Compound D (250 mg, 0.56 mmol) in DCM (5 mL) was added with a solution of 4N HCl in 1,4-dioxane (1.4 mL) before being stirred at room temperature for 3 hours. After completion of the reaction, the reaction mixture was concentrated in a vacuum to afford the target Compound E (134 mg, 63%).

¹H NMR (300 MHz, DMSO-d6) δ 11.01 (s, 1H), 8.94 (s, 2H), 7.57-7.51 (m, 2H), 5.12 (dd, J = 13.3, 5.1 Hz, 1H), 4.46 (d, J = 17.3 Hz, 1H), 4.33 (d, J = 17.3 Hz, 1H), 3.32-3.19 (m, 1H), 3.15-2.85 (m, 3H), 2.65-2.55 (m, 1H), 2.47-2.31 (m, 1H), 2.06-1.87 (m, 5H).

(2) Compound 90 was synthesized in the same manner as for Compound 1 with the exception of using Intermediate E and 6-chloro-3-methyl-1H-indole-2-carboxylic acid.

¹H NMR (400 MHz, DMSO-d6) δ 11.43 (s, 1H), 11.01 (s, 1H), 7.63 (d, J = 6.2 Hz, 1H), 7.58 (d, J = 8.6 Hz, 1H), 7.51 (d, J = 9.1 Hz, 1H), 7.37 (d, J = 2.1 Hz, 1H), 7.06 (dd, J = 8.6, 2.0 Hz, 1H), 5.12 (dd, J = 13.6, 5.1 Hz, 1H), 4.41 (s, 1H), 4.31 (d, J = 17.2 Hz, 1H), 3.31-3.07 (m, 3H), 2.99-2.85 (m, 1H), 2.65-2.56 (m, 1H), 2.46-2.36 (m, 1H), 2.30 (s, 3H), 2.04-1.96 (m, 1H), 1.92-1.81 (m, 2H), 1.76-1.62 (m, 2H).

### Compound 91. 3-(5-(1-(6-chloro-3-methyl-1H-indole-2-carbonyl)piperidin-4-yl)-4- fluoro-1-oxoinsoindolin-2-yl)piperidine-2,6-dione

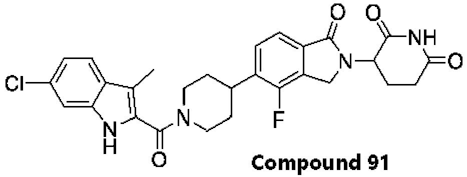

### (1) Synthesis of Intermediate 3-(4-fluoro-1-oxo-5-(piperidin-4-yl)isoindolin-2-yl)piperidine-2,6-dione chloride (K)

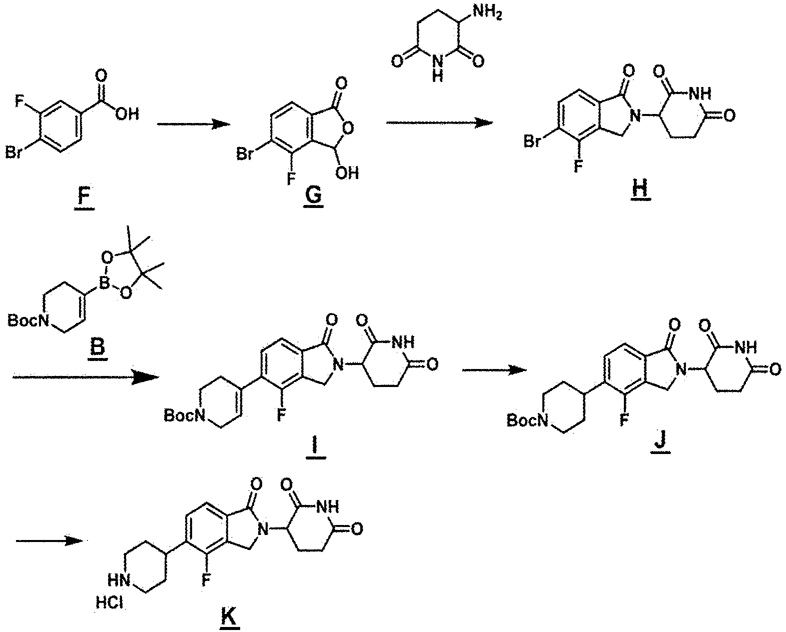

### (1-1) Preparation of bromo-4-fluoro-3-hydroxyisobenzofuran-1 (3H) (G)

In a round-bottom flask, 2 M n-BuLi/n-hexane (45.7 ml, 91 mmol) was chilled to -70°C. A solution of 2,2,6,6-tetramethylpiperidine (19.43 ml, 114 mmol) in tetrahydrofuran (50 ml) was added slowly. The reaction mixture was stirred at -70°C for 20 minutes and then at 4°C for 30 minutes. A solution of 4-bromo-3-fluorobenzoic acid (F) (5 g, 22.83 mmol) in tetrahydrofuran (12,5 mL) was added at -70°C little by little to the reaction mixture. The reaction mixture was stirred at 4°C for 1.5 hours. Dimethylformamide (3.54 ml, 45.7 mmol) was dissolved in tetrahydrofuran (7.5 mL) and added little by little at -70°C to the reaction mixture which was then stirred at 4°C for 2 hours. After completion of the reaction, the reaction mixture was added to 1 N HCl (300 mL) and then washed with dichloromethane.

¹H NMR (400 MHz, DMSO-d6) δ 8.48 (s, 1H), 8.03 (dd, J = 8.0, 5.9 Hz, 1H), 7.64 (d, J = 8.0 Hz, 1H), 6.85 (s, 1H) .

### Preparation of (1-2) 3-(5-bromo-4-fluoro-1-oxoinsoindolin-2-yl)piperidine-2,6-dione (H)

A solution of Compound G (5.64 g, 22.83 mmol), 3-aminopiperidine-2,6-dione (5.64 g, 34.2 mmol), and NaBH (OAc)₃ (9.68 g, 45.7 mmol) in dimethylformamide (50 ml) was stirred at room temperature for 12 hours. After completion of the reaction, the reaction mixture was diluted with water and then subjected to extraction with ethylacetate. The organic layer was dried over anhydrous magnesium sulfate, followed by filtration and concentration. Purification of the concentrate by column chromatography afforded the target Compound H (2.7 g, 35%).

¹H NMR (300 MHz, DMSO-d6) δ 11.03 (s, 1H), 7.89 (dd, J = 8.0, 6.0 Hz, 1H), 7.55 (d, J = 8.0 Hz, 1H), 5.13 (dd, J = 13.3, 5.1 Hz, 1H), 4.63 (d, J = 17.7 Hz, 1H), 4.46 (d, J = 17.6 Hz, 1H), 3.00-2.85 (m, 1H), 2.65-2.55 (m, 1H), 2.47-2.36 (m, 1H), 2.10-1.96 (m, 1H).

### (1-3) tert-butyl 4-(2-(2,6-dioxopiperidin-3-yl)-4-fluoro-1-oxoisoindolin-5-yl)-3,6-dihydropyridine-1 (2H)-carboxylate (I)

A solution of Compound H (3.0 g, 8.8 mmol), Compound B (3.5 g, 11.4 mmol), potassium carbonate (3.0 g, 22.0 mmol), and Pd (dppf)Cl₂·DCM (0.7 g, 0.88 mmol) in dimethylformamide (30 mL) was stirred at 110°C for 12 hours in a nitrogen atmosphere. After completion of the reaction, the reaction mixture was filtered through Celite. The filtrate was diluted with water, followed by extraction with ethylacetate. The organic layers thus obtained were pooled, dried over anhydrous magnesium sulfate, filtered, and concentrated. The concentrate mixture was subjected to column chromatography to afford the target Compound I (1.7 g, 44%).

### Preparation of (1-4) tert-butyl 4-(2-(2,6-dioxopiperidin-3-yl)-4-fluoro-1-oxoindolin-5-yl)piperidine-1-carboxylateoindolin-5-yl)piperidine-1-carboxylate (J)

A solution of Compound I (2.0 g, 4.5 mmol) and 10% Pd/C (0.8 g) in dimethylformamide (15 mL) was stirred for 12 hours under a hydrogen pressure. After completion of the reaction, the reaction mixture was filtered through Celite. The filtrate was diluted with water, followed by extraction with ethylacetate. The organic layers were pooled, dried over anhydrous magnesium sulfate, filtered, and concentrated to afford Compound J (2.0 g, 100%).

¹H NMR (500 MHz, DMSO-d6) δ 11.02 (s, 1H), 7.58-7.51 (m, 2H), 5.12 (dd, J = 13.3, 5.1 Hz, 1H), 4.56 (d, J = 17.3 Hz, 1H), 4.38 (d, J = 17.3 Hz, 1H), 4.17-4.02 (m, 2H), 3.14-3.07 (m, 1H), 2.96-2.84 (m, 3H), 2.63-2.58 (m, 1H), 2.45-2.41 (m, 1H), 2.03-1.97 (m, 1H), 1.80-1.71 (m, 2H), 1.66-1.55 (m, 2H), 1.43 (s, 9H).

### (1-5) Preparation of 3-(4-fluoro-1-oxo-5-(piperidin-4-yl)isoindolin-2-yl)piperidine-2,6-dione chloride (K)

A solution of Compound J (2.0 g, 4.49 mmol) in dichloromethane (20 mL) was added with a solution of 4N HCl in 1,4-dioxane (15 mL) and then stirred at room temperature for 3 hours. After completion of the reaction, the reaction mixture was concentrated in a vacuum to afford Compound K (1.7 g, 88%).

¹H NMR (300 MHz, DMSO-d6) δ 11.01 (s, 1H), 9.20-9.00 (m, 2H), 7.62 (d, J = 7.7 Hz, 1H), 7.51-7.42 (m, 1H), 5.12 (dd, J = 13.3, 5.1 Hz, 1H), 4.57 (d, J = 17.4 Hz, 1H), 4.39 (d, J = 17.4 Hz, 1H), 3.14-2.77 (m, 5H), 2.66-2.57 (m, 1H), 2.45-2.36 (m, 1H), 2.10-1.86 (m, 6H).

**(2)** Compound 91 was synthesized in the same manner as for Compound 1, with the exception of using Intermediate K and 6-chloro-3-methyl-1H-indole-2-carboxylic acid.

¹H NMR (400 MHz, DMSO-d6) δ 11.43 (s, 1H), 11.02 (s, 1H), 7.61-7.51 (m, 3H), 7.37 (s, 1H), 7.06 (d, J = 8.5 Hz, 1H), 5.12 (dd, J = 13.2, 5.0 Hz, 1H), 4.56 (d, J = 17.4 Hz, 1H), 4.38 (d, J = 17.3 Hz, 1H), 3.31-3.07 (m, 3H), 2.99-2.85 (m, 1H), 2.64-2.57 (m, 1H), 2.48-2.37 (m, 1H), 2.29 (s, 3H), 2.06-1.95 (m, 1H), 1.93-1.80 (m, 2H), 1.79-1.67 (m, 2H).

### Compound 92. 5-(1-(6-chloro-3-methyl-1H-indole-2-carbonyl)piperidin-4-yl)-2-(2,6- dioxopiperidin-3-yl)isoindoline-1,3-dione

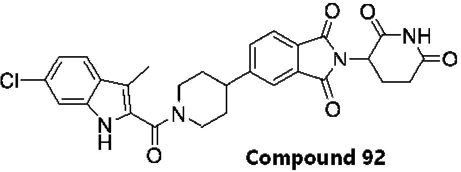

Compound 92 was synthesized in the same manner as for Compound 1, with the exception of using 2-(2,6-dioxopiperidin-3-yl)-5-(piperidin-4-yl)isoindoline-1,3-dione chloride and 6-chloro-3-methyl-1H-indole-2-carboxylic acid.

¹H NMR (400 MHz, DMSO-d6) δ 11.40 (s, 1H), 11.14 (s, 1H), 7.89 (d, J = 7.6 Hz, 1H), 7.86 (s, 1H), 7.80 (d, J = 7.8 Hz, 1H), 7.58 (d, J = 8.5 Hz, 1H), 7.38 (s, 1H), 7.06 (d, J = 8.5 Hz, 1H), 5.15 (dd, J = 12.8, 5.3 Hz, 1H), 4.60-4.08 (m, 2H), 3.19-3.04 (m, 3H), 2.93-2.84 (m, 1H), 2.64-2.57 (m, 2H), 2.29 (s, 3H), 2.08-2.01 (m, 1H), 1.95-1.84 (m, 2H), 1.78-1.65 (m, 3H).

### Compound 93. 3-(6-fluoro-5-(1-(3-methyl-6-nitro-1H-indole-2-carbonyl)piperidin-4-yl)-1-oxoinsoindolin-2-yl)piperidine-2,6-dione

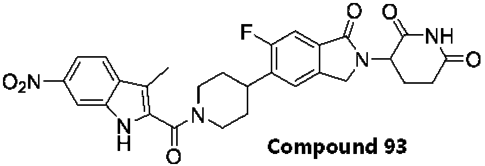

Compound 93 was synthesized in the same manner as for Compound 1, with the exception of using Intermediate E and 3-methyl-6-nitro-1H-indole-2-carboxylic acid.

¹H NMR (400 MHz, DMSO-d6) δ 12.11 (s, 1H), 11.01 (s, 1H), 8.28 (d, J = 2.1 Hz, 1H), 7.93 (dd, J = 8.9, 1.9 Hz, 1H), 7.78 (d, J = 8.9 Hz, 1H), 7.64 (d, J = 6.2 Hz, 1H), 7.51 (d, J = 9.1 Hz, 1H), 5.12 (dd, J = 13.4, 5.1 Hz, 1H), 4.65 (s, 1H), 4.44 (d, J = 17.2 Hz, 1H), 4.31 (d, J = 17.2 Hz, 1H), 3.81 (s, 1H), 3.30-3.02 (m, 3H), 2.98-2.86 (m, 1H), 2.64-2.57 (m, 1H), 2.42-2.37 (m, 1H), 2.34 (s, 3H), 2.04-1.98 (m, 1H), 1.95-1.79 (m, 2H), 1.78-1.62 (m, 2H).

### Compound 94. 3-(4-fluoro-5-(1-(3-methyl-6-nitro-1H-indole-2-carbonyl)piperidin-4-yl)-1-oxoinsoindolin-2-yl)piperidine-2,6-dione

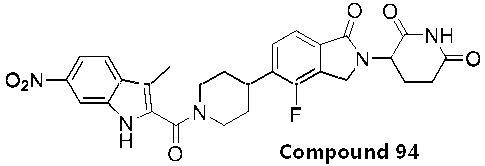

Compound 94 was synthesized in the same manner as for Compound 1, with the exception of using Intermediate K and 3-methyl-6-nitro-1H-indole-2-carboxylic acid.

¹H NMR (400 MHz, DMSO-d6) δ 12.12 (s, 1H), 11.02 (s, 1H), 8.28 (d, J = 1.9 Hz, 1H), 7.97-7.90 (m, 1H), 7.78 (d, J = 8.9 Hz, 1H), 7.63-7.51 (m, 2H), 5.12 (dd, J = 13.3, 5.0 Hz, 1H), 4.80-4.60 (m, 1H), 4.56 (d, J = 17.3 Hz, 1H), 4.39 (d, J = 17.3 Hz, 1H), 3.79 (s, 1H), 3.33-3.01 (m, 3H), 2.98-2.88 (m, 1H), 2.64-2.57 (m, 1H), 2.46-2.36 (m, 1H), 2.34 (s, 3H), 2.04-1.96 (m, 1H), 1.93-1.68 (m, 4H).

### Compound 95. 2-(2,6-dioxopiperidin-3-yl)-5-(1-(3-methyl-6-nitro-1H-indole-2-carbonyl)piperidin-4-yl)isoindoline-1,3-dione

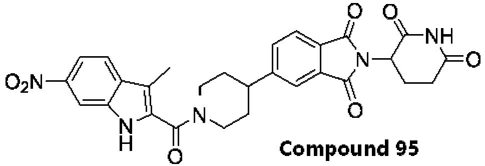

Compound 95 was synthesized in the same manner as for Compound 1, with the exception of using (2-(2,6-dioxopiperidin-3-yl)-5-(piperidin-4-yl)isoindoline-1,3-dione chloride and 3-methyl-6-nitro-1H-indole-2-carboxylic acid.

¹H NMR (400 MHz, DMSO-d6) δ 12.05 (s, 1H), 11.19 (s, 1H), 8.29 (d, J = 2.1 Hz, 1H), 7.95-7.85 (m, 3H), 7.83-7.74 (m, 2H), 5.15 (dd, J = 12.9, 5.4 Hz, 1H), 3.19-3.07 (m, 2H), 2.96-2.84 (m, 2H), 2.64-2.58 (m, 2H), 2.34 (s, 3H), 2.09-2.02 (m, 1H), 1.97-1.85 (m, 2H), 1.80-1.69 (m, 2H).

### Compound 96. 5-((1-(6-chloro-3-methyl-1H-indole-2-carbonyl)piperidin-4-yl)ethynyl)-2-(2,6-dioxopiperidin-3-yl)isoindoline-1,3-dione

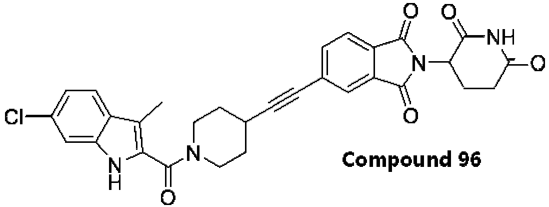

Compound 96 was synthesized in the same manner as for Compound 1, with the exception of using 2-(2,6-dioxopiperidin-3-yl)-5-(piperidin-4-ylethynyl)isoindoline-1,3-dione chloride and 6-chloro-3-methyl-1H-indole-2-carboxylic acid.

¹H NMR (400 MHz, DMSO-d6) δ 11.39 (s, 1H), 11.15 (s, 1H), 7.92 (d, J = 8.6 Hz, 3H), 7.58 (d, J = 8.4 Hz, 1H), 7.36 (d, J = 1.9 Hz, 1H), 7.06 (d, J = 8.9 Hz, 1H), 5.17 (dd, J = 12.8, 5.4 Hz, 1H), 3.86 (s, 2H), 3.48-3.38 (m, 2H), 3.10 (s, 1H), 2.95-2.82 (m, 1H), 2.65-2.57 (m, 1H), 2.56-2.53 (m, 1H), 2.27 (s, 3H), 2.12-2.03 (m, 1H), 1.99-1.89 (m, 2H), 1.74-1.63 (m, 2H).

### Compound 97. 3-(5-(1-(5-methoxy-3-methyl-6-nitro-1H-indole-2-carbonyl)piperidin-4-yl)-1-oxoinsoindolin-2-yl)piperidine-2,6-dione

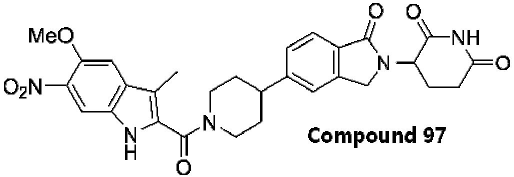

Compound 97 was synthesized in the same manner as for Compound 1, with the exception of using Intermediate 7 and 5-methoxy-3-methyl-6-nitro-1H-indole-2-carboxylic acid.

¹H NMR (500 MHz, DMSO-d6) δ 11.72 (s, 1H), 11.00 (s, 1H), 7.92 (s, 1H), 7.68 (d, J = 7.4 Hz, 1H), 7.51 (s, 1H), 7.43 (d, J = 8.0 Hz, 1H), 7.35 (s, 1H), 5.10 (dd, J = 13.7, 4.8 Hz, 1H), 4.76-4.52 (m, 1H), 4.43 (d, J = 17.0 Hz, 1H), 4.30 (d, J = 17.2 Hz, 1H), 3.92 (s, 3H), 3.90-3.61 (m, 1H), 3.26-2.86 (m, 4H), 2.64-2.56 (m, 1H), 2.45-2.33 (m, 1H), 2.30 (s, 3H), 2.02-1.80 (m, 3H), 1.74-1.59 (m, 2H).

### Compound 98. 3-(5-(1-(6-chloro-7-fluoro-3-methyl-1H-indole-2-carbonyl)piperidin-4-yl)-1-oxoinsoindolin-2-yl)piperidine-2,6-dione (KMG-2516)

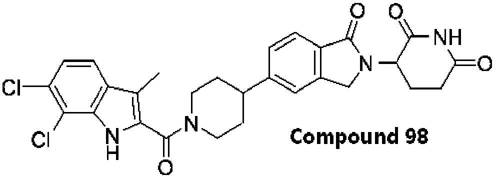

Compound 98 was synthesized in the same manner as for Compound 1, with the exception of using Intermediate 7 and 6,7-dichloro-3-methyl-1H-indole-2-carboxylic acid.

¹H NMR (300 MHz, DMSO-d6) δ 11.76 (s, 1H), 10.97 (s, 1H), 7.69 (d, J = 7.8 Hz, 1H), 7.56 (d, J = 8.5 Hz, 1H), 7.51 (s, 1H), 7.43 (d, J = 7.9 Hz, 1H), 7.25 (d, J = 8.5 Hz, 1H), 5.11 (dd, J = 13.3, 5.1 Hz, 1H), 4.81-4.53 (m, 1H), 4.45 (d, J = 17.3 Hz, 1H), 4.31 (d, J = 17.3 Hz, 1H), 3.74 (s, 1H), 3.13-2.78 (m, 4H), 2.67-2.54 (m, 1H), 2.44-2.39 (m, 1H), 2.27 (s, 3H), 1.94-1.59 (m, 5H).

### Compound 99. 3-(5-(1-(6-chloro-7-fluoro-3-methyl-1H-indole-2-carbonyl)piperidin-4-yl)-1-oxoinsoindolin-2-yl)piperidine-2,6-dione

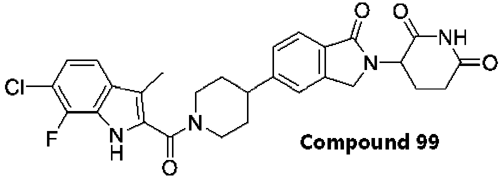

Compound 99 was synthesized in the same manner as for Compound 1, with the exception of using Intermediate 7 and 6-chloro-7-fluoro-3-methyl-1H-indole-2-carboxylic acid.

¹H NMR (300 MHz, DMSO-d6) δ 11.97 (s, 1H), 10.99 (s, 1H), 7.69 (d, J = 7.8 Hz, 1H), 7.51 (s, 1H), 7.46-7.39 (m, 2H), 7.17-7.09 (m, 1H), 5.11 (dd, J = 13.2, 5.1 Hz, 1H), 4.814.48-3.98 (m, 1H), 4.45 (d, J = 17.3 Hz, 1H), 4.31 (d, J = 17.3 Hz, 1H), 4.25-3.98 (m, 1H), 3.09-2.83 (m, 4H), 2.65-2.57 (m, 1H), 2.44-2.34 (m, 1H), 2.28 (s, 3H), 1.91-1.55 (m, 5H).

### Compound 100. 3-(5-(1-(6-chloro-3,7-dimethyl-1H-indole-2-carbonyl)piperidin-4-yl)-1-oxoinsoindolin-2-yl)piperidine-2,6-dione

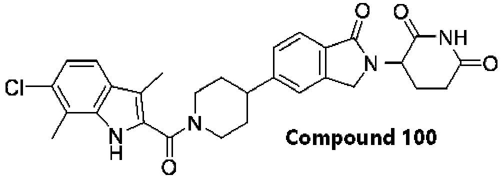

Compound 100 was synthesized in the same manner as for Compound 1, with the exception of using Intermediate 7 and 6-chloro-3,7-dimethyl-1H-indole-2-carboxylic acid.

¹H NMR (400 MHz, DMSO-d6) δ 11.37 (s, 1H), 10.99 (s, 1H), 7.69 (d, J = 7.8 Hz, 1H), 7.51 (s, 1H), 7.43 (d, J = 7.9 Hz, 1H), 7.40 (d, J = 8.4 Hz, 1H), 7.07 (d, J = 8.4 Hz, 1H), 5.11 (dd, J = 13.3, 5.1 Hz, 1H), 5.02-4.47 (m, 1H), 4.45 (d, J = 17.3 Hz, 1H), 4.31 (d, J = 17.3 Hz, 1H), 4.09-3.51 (m, 1H), 3.11-2.84 (m, 4H), 2.65-2.58 (m, 1H), 2.52 (s, 3H), 2.42-2.36 (m, 1H), 2.27 (s, 3H), 2.03-1.95 (m, 2H), 1.93-1.81 (m, 2H), 1.74-1.59 (m, 3H).

### Compound 101. 3-(5-(1-(6-chloro-5-hydroxy-3-methyl-1H-indole-2-carbonyl)piperidin-4-yl)-1-oxoinsoindolin-2-yl)piperidine-2,6-dione

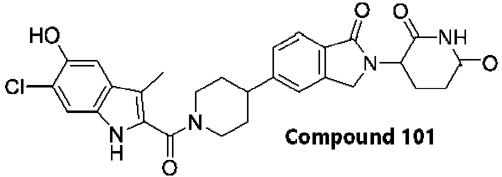

Compound 101 was synthesized in the same manner as for Compound 1, with the exception of using Intermediate 7 and 6-chloro-5-hydroxy-3-methyl-1H-indole-2-carboxylic acid.

¹H NMR (500 MHz, DMSO-d6) δ 11.03 (s, 1H), 11.00 (s, 1H), 9.49 (s, 1H), 7.68 (d, J = 7.9 Hz, 1H), 7.52 (s, 1H), 7.43 (d, J = 7.7 Hz, 1H), 7.30 (s, 1H), 7.03 (s, 1H), 5.12 (dd, J = 13.3, 5.1 Hz, 1H), 4.86-4.34 (m, 2H), 4.34-3.84 (m, 2H), 3.13-2.85 (m, 4H), 2.63-2.57 (m, 1H), 2.42-2.39 (m, 1H), 2.22 (s, 3H), 2.03-1.97 (m, 1H), 1.92-1.83 (m, 2H), 1.72-1.61 (m, 2H).

### Compound 102. 3-(5-(1-(6-chloro-5-fluoro-3-methyl-1H-indole-2-carbonyl)piperidin-4-yl)-1-oxoinsoindolin-2-yl)piperidine-2,6-dione

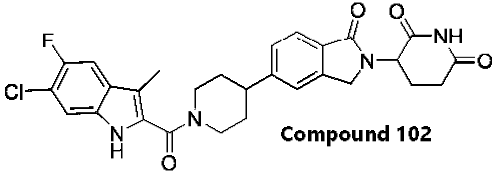

Compound 102 was synthesized in the same manner as for Compound 1, with the exception of using Intermediate 7 and 6-chloro-5-fluoro-3-methyl-1H-indole-2-carboxylic acid.

¹H NMR (400 MHz, DMSO-d6) δ 11.50 (s, 1H), 11.00 (s, 1H), 7.68 (d, J = 7.8 Hz, 1H), 7.58 (d, J = 9.9 Hz, 1H), 7.52 (s, 1H), 7.49 (d, J = 6.2 Hz, 1H), 7.43 (d, J = 8.0 Hz, 1H), 5.11 (dd, J = 13.4, 5.0 Hz, 1H), 4.44 (d, J = 17.2 Hz, 1H), 4.30 (d, J = 17.2 Hz, 1H), 3.23-2.85 (m, 4H), 2.63-2.56 (m, 1H), 2.42-2.36 (m, 1H), 2.27 (s, 3H), 2.04-1.95 (m, 1H), 1.94-1.83 (m, 2H), 1.74-1.60 (m, 2H).

### Compound 103. 3-(5-(1-(6-chloro-3,5-dimethyl-1H-indole-2-carbonyl)piperidin-4-yl)-1-oxoinsoindolin-2-yl)piperidine-2,6-dione

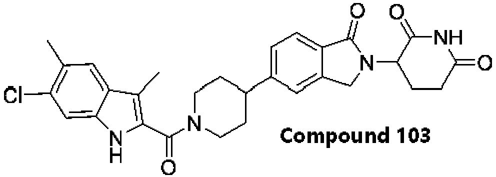

Compound 103 was synthesized in the same manner as for Compound 1, with the exception of using Intermediate 7 and 6-chloro-3,5-dimethyl-1H-indole-2-carboxylic acid.

¹H NMR (400 MHz, DMSO-d6) δ 11.26 (s, 1H), 11.00 (s, 1H), 7.68 (d, J = 7.8 Hz, 1H), 7.51 (s, 2H), 7.43 (d, J = 7.9 Hz, 1H), 7.38 (s, 1H), 5.11 (dd, J = 13.3, 5.1 Hz, 1H), 4.44 (d, J = 17.1 Hz, 1H), 4.30 (d, J = 17.2 Hz, 1H), 3.20-2.85 (m, 4H), 2.65-2.56 (m, 1H), 2.40 (s, 3H), 2.27 (s, 3H), 2.02-1.96 (m, 1H), 1.91-1.81 (m, 2H), 1.72-1.61 (m, 2H).

### Compound 104. 3-(5-(1-(3-methyl-6-morpholino-1H-indole-2-carbonyl)piperidin-4-yl)-1-oxoinsoindolin-2-yl)piperidine-2,6-dione

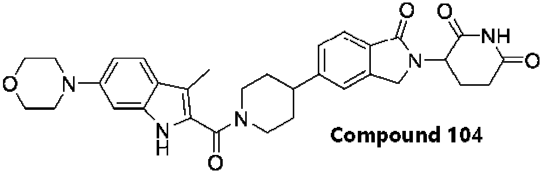

Compound 104 was synthesized in the same manner as for Compound 1, with the exception of using Intermediate 7 and 3-methyl-6-morpholino-1H-indole-2-carboxylic acid.

¹H NMR (400 MHz, DMSO-d6) δ 10.98 (s, 1H), 10.89 (s, 1H), 7.68 (d, J = 7.8 Hz, 1H), 7.51 (s, 1H), 7.42 (t, J = 8.6, 8.6 Hz, 2H), 6.85 (d, J = 8.8 Hz, 1H), 6.76 (s, 1H), 5.11 (dd, J = 13.4, 5.1 Hz, 1H), 4.44 (d, J = 17.2 Hz, 1H), 4.35-4.19 (m, 3H), 3.77 (t, J = 4.7, 4.7 Hz, 4H), 3.07 (t, J = 4.7, 4.7 Hz, 5H), 3.04-2.87 (m, 3H), 2.64-2.58 (m, 1H), 2.42-2.35 (m, 1H), 2.27 (s, 3H), 2.04-1.96 (m, 1H), 1.91-1.83 (m, 2H), 1.74-1.62 (m, 2H).

### Compound 105. 3-(5-(1-(6-chloro-3-(hydroxymethyl)-1H-indole-2-carbonyl)piperidin-4-yl)-1-oxoinsoindolin-2-yl)piperidine-2,6-dione

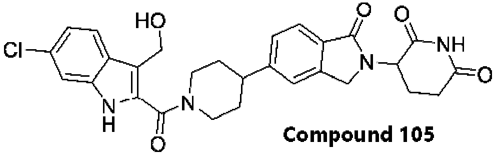

Compound 105 was synthesized in the same manner as for Compound 1, with the exception of using Intermediate 7 and 6-chloro-3-(hydroxymethyl)-1H-indole-2-carboxylic acid.

¹H NMR (400 MHz, DMSO-d6) δ 11.55 (s, 1H), 11.00 (s, 1H), 7.73 (d, J = 8.7 Hz, 1H), 7.69 (d, J = 7.8 Hz, 1H), 7.51 (s, 1H), 7.43 (d, J = 7.8 Hz, 1H), 7.39 (s, 1H), 7.08 (d, J = 8.5 Hz, 1H), 5.11 (dd, J = 13.5, 5.0 Hz, 1H), 4.98 (t, J = 5.3, 5.3 Hz, 1H), 4.66 (d, J = 5.1 Hz, 2H), 4.44 (d, J = 17.2 Hz, 1H), 4.31 (d, J = 17.2 Hz, 1H), 3.09-2.86 (m, 4H), 2.63-2.57 (m, 2H), 2.44-2.33 (m, 2H), 2.04-1.96 (m, 1H), 1.93-1.81 (m, 2H), 1.77-1.59 (m, 2H).

### Compound 106. 3-(5-(1-(3-methyl-6-(piperazin-1-ylmethyl)-1H-indole-2-carbonyl)piperidin-4-yl)-1-oxoinsoindolin-2-yl)piperidine-2,6-dione dichloride

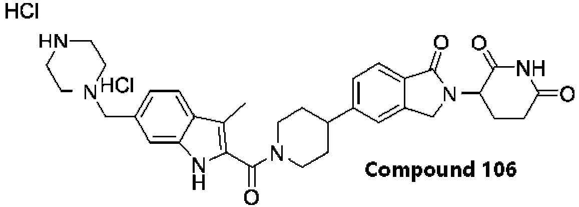

Compound 106 was synthesized in the same manner as for Compound 1, with the exception of using Intermediate 7.

¹H NMR (300 MHz, DMSO-d6) δ 11.78- 11.35 (m, 2H), 11.00 (s, 1H), 9.50-9.24 (m, 1H), 7.69 (d, J = 7.9 Hz, 1H), 7.62 (s, 2H), 7.52 (s, 1H), 7.43 (d, J = 8.1 Hz, 1H), 7.25 (s, 1H), 5.12 (dd, J = 13.1, 5.1 Hz, 1H), 4.55-4.39 (m, 3H), 4.31 (d, J = 17.4 Hz, 1H), 3.55 (s, 2H), 3.24-2.85 (m, 9H), 2.77-2.57 (m, 4H), 2.44-2.35 (m, 1H), 2.31 (s, 3H), 2.04-1.87 (m, 3H), 1.76-1.58 (m, 2H).

### Compound 107. 3-(5-(1-(4,6-dichloro-3-methyl-1H-indole-2-carbonyl)piperidin-4-yl)-1-oxoinsoindolin-2-yl)piperidine-2,6-dione

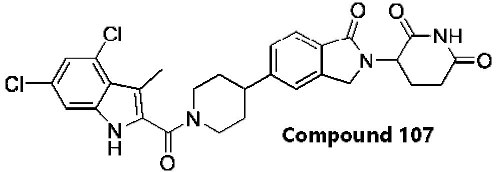

Compound 107 was synthesized in the same manner as for Compound 1, with the exception of using Intermediate 7 and 4,6-dichloro-1H-indole-2-carboxylic acid.

¹H NMR (300 MHz, DMSO-d6) δ 11.81 (s, 1H), 11.00 (s, 1H), 7.69 (d, J = 7.8 Hz, 1H), 7.51 (s, 1H), 7.43 (d, J = 7.9 Hz, 1H), 7.37 (d, J = 1.7 Hz, 1H), 7.13 (d, J = 1.7 Hz, 1H), 5.11 (dd, J = 13.2, 5.1 Hz, 1H), 4.44 (d, J = 17.3 Hz, 1H), 4.31 (d, J = 17.2 Hz, 1H), 3.21 - 2.82 (m, 4H), 2.64-2.54 (m, 1H), 2.44-2.36 (m, 1H), 2.05-1.85 (m, 3H), 1.74-1.51 (m, 2H).

### Compound 108. 3-(5-(1-(6-chloro-5-methoxy-3-methyl-1H-indole-2-carbonyl)piperidin-4-yl)-4-fluoro-1-oxoinsoindolin-2-yl)piperidine-2,6-dione

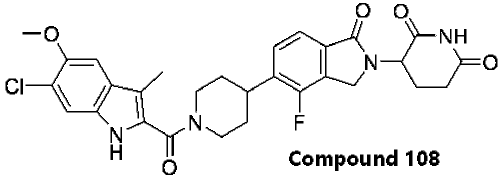

Compound 108 was synthesized in the same manner as for Compound 1, with the exception of using Intermediate K and 6-chloro-5-methoxy-1H-indole-2-carboxylic acid.

¹H NMR (300 MHz, DMSO-d6) δ 11.23 (s, 1H), 11.02 (s, 1H), 7.62-7.50 (m, 2H), 7.39 (s, 1H), 7.21 (s, 1H), 5.12 (dd, J = 13.2, 5.0 Hz, 1H), 4.57 (d, J = 17.4 Hz, 1H), 4.45-4.07 (m, 3H), 3.87 (s, 3H), 3.23-2.84 (m, 4H), 2.64-2.58 (m, 1H), 2.46-2.39 (m, 1H), 2.29 (s, 3H), 2.06-1.94 (m, 1H), 1.94-1.63 (m, 4H).

### Compound 109. 3-(5-(1-(6-chloro-3-methyl-5-(piperazin-1-ylmethyl)-1H-indole-2-carbonyl)piperidin-4-yl)-1-oxoinsoindolin-2-yl)piperidine-2,6-dione dichloride

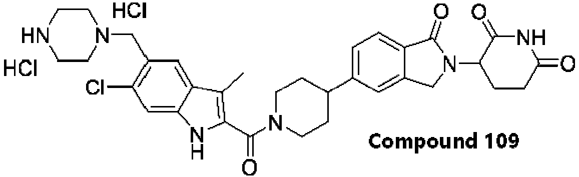

Compound 109 was synthesized in the same manner as for Compound 1, with the exception of using Intermediate 7.

¹H NMR (400 MHz, DMSO-d6) δ 12.01 (s, 1H), 11.70 (s, 1H), 11.00 (s, 1H), 9.89 (s, 2H), 8.15 (s, 1H), 7.68 (d, J = 7.9 Hz, 1H), 7.52 (d, J = 5.5 Hz, 2H), 7.44 (d, J = 8.2 Hz, 1H), 5.11 (dd, J = 13.3, 5.1 Hz, 1H), 4.55-4.40 (m, 3H), 4.31 (d, J = 17.4 Hz, 1H), 3.51 (s, 2H), 3.19-2.84 (m, 9H), 2.62-2.59 (m, 1H), 2.39 (s, 1H), 2.31 (s, 3H), 2.27-2.22 (m, 1H), 2.05-1.84 (m, 5H), 1.72-1.62 (m, 2H).

### Compound 110. 3-(5-(1-(6-methoxy-3-(morpholinomethyl)-1H-indole-2-carbonyl)piperidin-4-yl)-1-oxoinsoindolin-2-yl)piperidine-2,6-dione

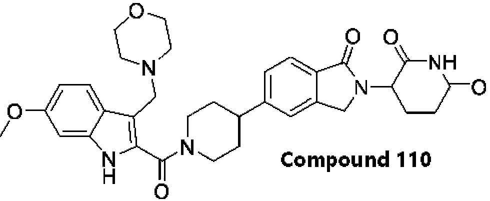

Compound 110 was synthesized in the same manner as for Compound 1, with the exception of using Intermediate 7.

¹H NMR (300 MHz, DMSO-d6) δ 11.22 (s, 1H), 11.00 (s, 1H), 7.69 (d, J = 7.8 Hz, 1H), 7.62 (d, J = 8.7 Hz, 1H), 7.50 (s, 1H), 7.42 (d, J = 8.0 Hz, 1H), 6.83 (d, J = 2.3 Hz, 1H), 6.70 (dd, J = 8.7, 2.3 Hz, 1H), 5.11 (dd, J = 13.2, 5.1 Hz, 1H), 4.45 (d, J = 17.3 Hz, 1H), 4.31 (d, J = 17.3 Hz, 3H), 3.77 (s, 3H), 3.63 (s, 2H), 3.53 (s, 4H), 3.1-2.83 (m, 4H), 2.66-2.58 (m, 1H), 2.46-2.39 (m, 1H), 2.35 (s, 4H), 2.05-1.94 (m, 1H), 1.94-1.83 (m, 2H), 1.76-1.59 (m, 2H).

### Compound 111. 3-(5-(1-(6-methoxy-3-(morpholinomethyl)-1H-indole-2-carbonyl)piperidin-4-yl)-1-oxoinsoindolin-2-yl)piperidine-2,6-dione

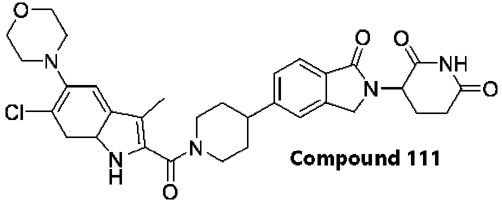

Compound 111 was synthesized in the same manner as for Compound 1, with the exception of using Intermediate 7 and 6-chloro-3-methyl-5-morpholino-1H-indole-2-carboxylic acid.

¹H NMR (300 MHz, DMSO-d6) δ 11.26 (s, 1H), 11.00 (s, 1H), 7.68 (d, J = 7.8 Hz, 1H), 7.52 (s, 1H), 7.43 (d, J = 7.6 Hz, 1H), 7.41 (s, 1H), 7.33 (s, 1H), 5.11 (dd, J = 13.2, 5.1 Hz, 1H), 4.45 (d, J = 17.3 Hz, 1H), 4.35-4.05 (m, 3H), 3.82-3.69 (m, 4H), 3.19-2.99 (m, 3H), 2.96 (t, J = 4.6, 4.6 Hz, 4H), 2.92-2.85 (m, 1H), 2.65-2.56 (m, 1H), 2.44-2.36 (m, 1H), 2.29 (s, 3H), 2.06-1.83 (m, 3H), 1.75-1.57 (m, 2H).

### Compound 112. 3-(5-(1-(6-chloro-5-fluoro-3-methyl-1H-indole-2-carbonyl)piperidin-4-yl)-6-fluoro-1-oxoinsoindolin-2-yl)piperidine-2,6-dione

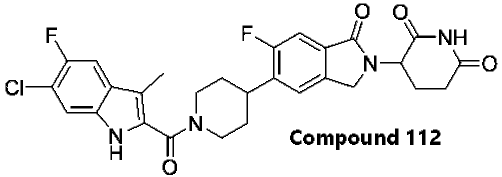

Compound 112 was synthesized in the same manner as for Compound 1, with the exception of using Intermediate E and 6-chloro-5-fluoro-3-methyl-1H-indole-2-carboxylic acid.

¹H NMR (400 MHz, DMSO-d6) δ 11.51 (s, 1H), 11.01 (s, 1H), 7.63 (d, J = 6.2 Hz, 1H), 7.58 (d, J = 10.0 Hz, 1H), 7.50 (dd, J = 9.4, 7.7 Hz, 2H), 5.12 (dd, J = 13.3, 5.1 Hz, 1H), 4.43 (d, J = 17.2 Hz, 1H), 4.33 (s, 1H), 3.31-3.10 (m, 3H), 2.97-2.86 (m, 1H), 2.64-2.58 (m, 1H), 2.46-2.34 (m, 1H), 2.27 (s, 3H), 2.05-1.96 (m, 1H), 1.93-1.81 (m, 2H), 1.78-1.63 (m, 2H).

### Compound 113. 3-(5-(1-(6-chloro-5-fluoro-3-methyl-1H-indole-2-carbonyl)piperidin-4-yl)-4-fluoro-1-oxoinsoindolin-2-yl)piperidine-2,6-dione

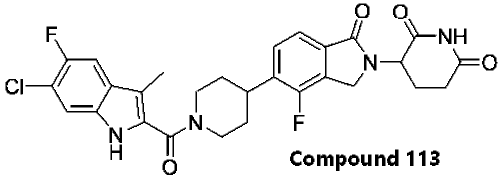

Compound 113 was synthesized in the same manner as for Compound 1, with the exception of using Intermediate K and 6-chloro-5-fluoro-3-methyl-1H-indole-2-carboxylic acid.

1¹H NMR (400 MHz, DMSO-d6) δ 11.51 (s, 1H), 11.01 (s, 1H), 7.64-7.51 (m, 3H), 7.49 (d, J = 6.3 Hz, 1H), 5.12 (dd, J = 13.3, 5.1 Hz, 1H), 4.56 (d, J = 17.3 Hz, 1H), 4.38 (d, J = 17.3 Hz, 1H), 3.31-3.04 (m, 3H), 2.97-2.87 (m, 1H), 2.64-2.58 (m, 1H), 2.47-2.37 (m, 1H), 2.27 (s, 3H), 2.03-1.94 (m, 1H), 1.91-1.81 (m, 2H), 1.78-1.66 (m, 2H).

### Compound 114. 3-(5-(1-(6-chloro-3-((4-methylpiperazin-1-yl)methyl)-1H-indole-2-carbonyl)piperidin-4-yl)-1-oxoinsoindolin-2-yl)piperidine-2,6-dione

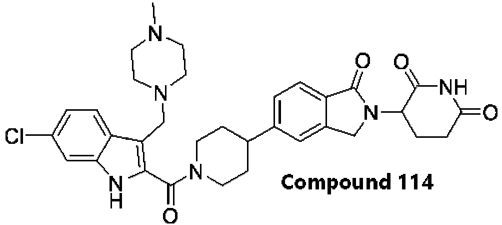

Compound 114 was synthesized in the same manner as for Compound 1, with the exception of using Intermediate 7.

¹H NMR (400 MHz, DMSO-d6) δ 11.63 (s, 1H), 10.99 (s, 1H), 7.74 (d, J = 8.5 Hz, 1H), 7.69 (d, J = 7.8 Hz, 1H), 7.50 (s, 1H), 7.43 (d, J = 8.4 Hz, 2H), 7.40 (d, J = 1.9 Hz, 1H), 7.08 (dd, J = 8.5, 1.9 Hz, 1H), 5.11 (dd, J = 13.2, 5.1 Hz, 1H), 4.44 (d, J = 17.3 Hz, 1H), 4.31 (d, J = 17.2 Hz, 1H), 3.75 (s, 2H), 3.03-2.84 (m, 7H), 2.68-2.65 (m, 1H), 2.63 (s, 3H), 2.61-2.52 (m, 4H), 2.43-2.33 (m, 2H), 2.03-1.85 (m, 3H), 1.71-1.63 (m, 2H).

### Compound 115. 3-(5-(1-(6-chloro-5-hydroxy-3-methyl-1H-indole-2-carbonyl)piperidin-4-yl)-4-fluoro-1-oxoinsoindolin-2-yl)piperidine-2,6-dione

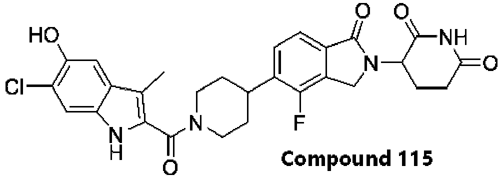

Compound 115 was synthesized in the same manner as for Compound 1, with the exception of using Intermediate K and 6-chloro-5-hydroxy-3-methyl-1H-indole-2-carboxylic acid.

¹H NMR (400 MHz, DMSO-d6) δ 11.03 (s, 1H), 11.01 (s, 1H), 9.47 (s, 1H), 7.60-7.50 (m, 2H), 7.30 (s, 1H), 7.02 (s, 1H), 5.12 (dd, J = 13.3, 5.1 Hz, 1H), 4.56 (d, J = 17.3 Hz, 1H), 4.45-4.08 (m, 3H), 3.25-3.03 (m, 3H), 2.97-2.87 (m, 1H), 2.63-2.59 (m, 1H), 2.46-2.38 (m, 1H), 2.21 (s, 3H), 2.07-1.92 (m, 2H), 1.91-1.79 (m, 2H), 1.76-1.71 (m, 1H).

### Compound 116. 3-(4-fluoro-5-(1-(3-methyl-6-morpholino-1H-indole-2-carbonyl)piperidin-4-yl)-1-oxoinsoindolin-2-yl)piperidine-2,6-dione

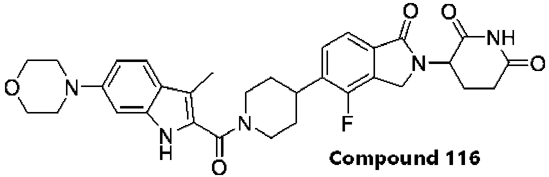

Compound 116 was synthesized in the same manner as for Compound 1, with the exception of using Intermediate K and 3-methyl-6-morpholino-1H-indole-2-carboxylic acid.

¹H NMR (400 MHz, DMSO-d6) δ 11.01 (s, 1H), 10.91 (s, 1H), 7.60-7.53 (m, 2H), 7.41 (d, J = 8.7 Hz, 1H), 6.85 (dd, J = 8.8, 2.2 Hz, 1H), 6.75 (d, J = 2.2 Hz, 1H), 5.12 (dd, J = 13.3, 5.1 Hz, 1H), 4.56 (d, J = 17.4 Hz, 1H), 4.39 (d, J = 17.3 Hz, 1H), 4.26 (s, 2H), 3.77 (t, J = 4.7, 4.7 Hz, 4H), 3.29-3.23 (m, 1H), 3.20-3.11 (m, 2H), 3.07 (t, J = 4.8, 4.8 Hz, 4H), 2.98-2.87 (m, 1H), 2.66-2.58 (m, 1H), 2.47-2.41 (m, 1H), 2.27 (s, 3H), 2.06-1.97 (m, 1H), 1.89-1.82 (m, 2H), 1.78-1.70 (m, 2H).

### Compound 117. 3-(5-(1-(6-chloro-7-fluoro-3-methyl-1H-indole-2-carbonyl)piperidin-4-yl)-4-fluoro-1-oxoinsoindolin-2-yl)piperidine-2,6-dione

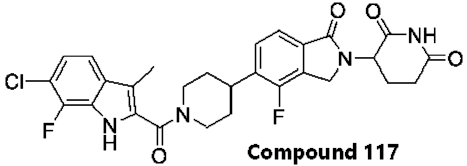

Compound 117 was synthesized in the same manner as for Compound 1, with the exception of using Intermediate K and 6-chloro-7-fluoro-3-methyl-1H-indole-2-carboxylic acid.

¹H NMR (400 MHz, DMSO-d6) δ 11.97 (s, 1H), 11.01 (s, 1H), 7.61-7.52 (m, 2H), 7.41 (d, J = 8.5 Hz, 1H), 7.13 (dd, J = 8.5, 6.4 Hz, 1H), 5.12 (dd, J = 13.3, 5.1 Hz, 1H), 4.56 (d, J = 17.3 Hz, 1H), 4.39 (d, J = 17.3 Hz, 1H), 3.3-3.20 (m, 3H), 2.97-2.87 (m, 1H), 2.64-2.58 (m, 1H), 2.48-2.35 (m, 1H), 2.28 (s, 3H), 2.07-1.95 (m, 1H), 1.92-1.63 (m, 4H).

### Compound 118. 2-(4-(2-(2,6-dioxopiperidin-3-yl)-4-fluoro-1-oxoindolin-5-yl)piperidine-1-carbonyl)-3-methyl-1H-indole-6-carbonitrile

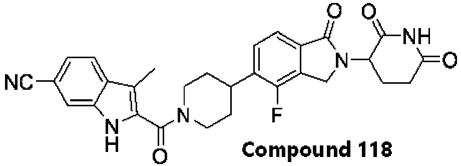

Compound 118 was synthesized in the same manner as for Compound 1, with the exception of using Intermediate K and 6-cyano-3-methyl-1H-indole-2-carboxylic acid.

¹H NMR (400 MHz, DMSO-d6) δ 11.89 (s, 1H), 11.01 (s, 1H), 7.83 (s, 1H), 7.76 (d, J = 8.2 Hz, 1H), 7.59 (d, J = 7.6 Hz, 1H), 7.57-7.52 (m, 1H), 7.39 (d, J = 8.3 Hz, 1H), 5.12 (dd, J = 13.4, 5.1 Hz, 1H), 4.56 (d, J = 17.3 Hz, 1H), 4.39 (d, J = 17.3 Hz, 1H), 3.27-2.87 (m, 4H), 2.65-2.58 (m, 1H), 2.46-2.38 (m, 2H), 2.32 (s, 3H), 2.05-1.97 (m, 1H), 1.94-1.82 (m, 2H), 1.79-1.70 (m, 2H).

### Compound 119. 6-chloro-2-(4-(2-(2,6-dioxopiperidin-3-yl)-4-fluoro-1-oxoindolin-5-yl)piperidine-1-carbonyl)-3-methyl-1H-indole-5-carbonitrile

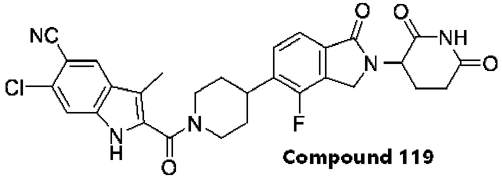

Compound 119 was synthesized in the same manner as for Compound 1, with the exception of using Intermediate K and 6-chloro-5-cyano-3-methyl-1H-indole-2-carboxylic acid.

¹H NMR (400 MHz, DMSO-d6) δ 12.04 (s, 1H), 11.01 (s, 1H), 8.32 (s, 1H), 7.63 (s, 1H), 7.60-7.51 (m, 3H), 5.12 (dd, J = 13.2, 5.1 Hz, 2H), 4.97-4.60 (m, 1H), 4.56 (d, J = 17.3 Hz, 1H), 4.39 (d, J = 17.3 Hz, 1H), 3.91-3.73 (m, 1H), 3.31-3.17 (m, 4H), 2.95-2.88 (m, 1H), 2.63-2.58 (m, 1H), 2.47-2.38 (m, 1H), 2.32 (s, 3H), 2.04-1.98 (m, 2H), 1.90-1.83 (m, 2H), 1.77-1.70 (m, 2H).

### Compound 120. 2-(4-(2-(2,6-dioxopiperidin-3-yl)-4-fluoro-1-oxoindolin-5-yl)piperidine-1-carbonyl)-5-fluoro-3-methyl-1H-indole-6-carbonitrile

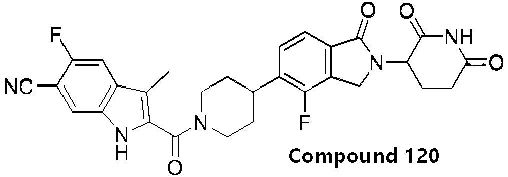

Compound 120 was synthesized in the same manner as for Compound 1, with the exception of using Intermediate K and 6-cyano-5-fluoro-3-methyl-1H-indole-2-carboxylic acid.

¹H NMR (300 MHz, DMSO-d6) δ 11.88 (s, 1H), 11.03 (s, 1H), 7.84 (s, 1H), 7.76 (d, J = 7.9 Hz, 1H), 7.62-7.52 (m, 2H), 7.41-7.36 (m, 1H), 5.12 (dd, J = 13.2, 5.1 Hz, 1H), 4.57 (d, J = 17.4 Hz, 1H), 4.38 (d, J = 17.4 Hz, 1H), 3.31-3.06 (m, 3H), 3.00-2.84 (m, 1H), 2.66-2.56 (m, 1H), 2.47-2.39 (m, 1H), 2.32 (s, 3H), 2.05-1.67 (m, 5H) .

### Compound 121. 5-(1-(6-chloro-3-methyl-1H-indole-2-carbonyl)piperidin-4-yl)-2-(2,6-dioxopiperidin-3-yl)-4-fluoroisoindoline-1,3-dione

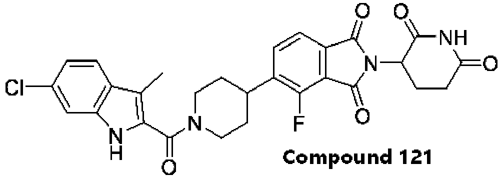

Compound 121 was synthesized in the same manner as for Compound 1, with the exception of using Intermediate K and 6-chloro-3-methyl-1H-indole-2-carboxylic acid.

¹H NMR (400 MHz, DMSO-d6) δ 11.42 (s, 1H), 11.15 (s, 1H), 7.92-7.87 (m, 1H), 7.76 (d, J = 7.7 Hz, 1H), 7.58 (d, J = 8.5 Hz, 1H), 7.39-7.35 (m, 1H), 7.08-7.04 (m, 1H), 5.15 (dd, J = 12.8, 5.4 Hz, 1H), 4.85-3.62 (m, 2H), 3.31-3.05 (m, 3H), 2.95-2.84 (m, 1H), 2.66-2.56 (m, 1H), 2.29 (s, 3H), 2.29-2.21 (m, 1H), 2.10-2.01 (m, 1H), 1.92-1.82 (m, 2H), 1.77-1.67 (m, 2H).

### Compound 122. 2-(4-(2-(2,6-dioxopiperidin-3-yl)-4-fluoro-1-oxoindolin-5-yl)piperidine-1-carbonyl)-7-fluoro-3-methyl-1H-indole-6-carbonitrile

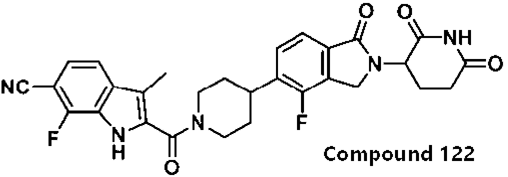

Compound 122 was synthesized in the same manner as for Compound 1, with the exception of using Intermediate K and 6-cyano-7-fluoro-3-methyl-1H-indole-2-carboxylic acid.

¹H NMR (400 MHz, DMSO-d6) δ 12.48 (s, 1H), 11.01 (s, 1H), 7.61-7.53 (m, 3H), 7.39-7.34 (m, 1H), 5.12 (dd, J = 13.3, 5.1 Hz, 1H), 4.83-4.60 (m, 1H), 4.56 (d, J = 17.3 Hz, 1H), 4.38 (d, J = 17.3 Hz, 1H), 3.81- 3.55 (m, 1H), 3.12-2.97 (m, 1H), 2.95-2.87 (m, 1H), 2.64-2.56 (m, 1H), 2.47-2.39 (m, 1H), 2.30 (s, 3H), 2.03-1.96 (m, 1H), 1.96-1.68 (m, 5H).

### Compound 123. (3-(5-(1-(6-chloro-3-methyl-1H-indole-2-carbonyl)piperidin-4-yl)-4-fluoro-1-oxoinsoindolin-2-yl)-2,6-dioxopiperidin-1-yl)methyl pivalate

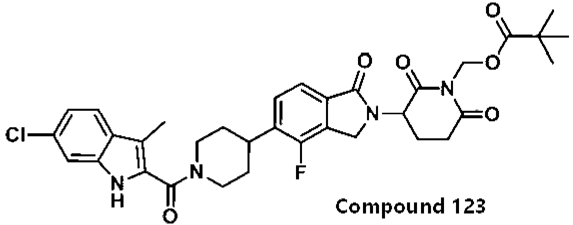

Compound 121 (20 mg, 0.037 mmol), chloromethyl pivalate (6.98 µl, 0.048 mmol), and K ₂CO₃ (7.72 mg, 0.056 mmol) were added to dimethylformamide (1 mL) and stirred at 50°C for 5 hours. After completion of the reaction, the reaction mixture was diluted with water and then subjected to extraction with ethylacetate. The organic layer thus formed was separated, washed with brine, and dried over anhydrous magnesium sulfate to remove residues. Following filtration and concentration in a vacuum, purification by column chromatography afforded Compound 123.

¹H NMR (400 MHz, DMSO-d6) δ 11.43 (s, 1H), 7.62-7.53 (m, 3H), 7.39-7.36 (m, 1H), 7.08-7.04 (m, 1H), 5.68-5.57 (m, 2H), 5.31 (dd, J = 13.2, 5.0 Hz, 1H), 4.93-4.68 (m, 1H), 4.60 (d, J = 17.1 Hz, 1H), 4.35 (d, J = 17.2 Hz, 1H), 4.21-3.75 (m, 1H), 3.25-3.06 (m, 3H), 2.88-2.79 (m, 1H), 2.69-2.54 (m, 1H), 2.46-2.41 (m, 1H), 2.29 (s, 3H), 2.01-1.94 (m, 1H), 1.91-1.81 (m, 2H), 1.77-1.70 (m, 2H), 1.11 (s, 9H).

### Compound 124. 3-(4-fluoro-5-(1-(7-fluoro-3-methyl-6-morpholino-1H-indole-2-carbonyl)piperidin-4-yl)-1-oxoinsoindolin-2-yl)piperidine-2,6-dione

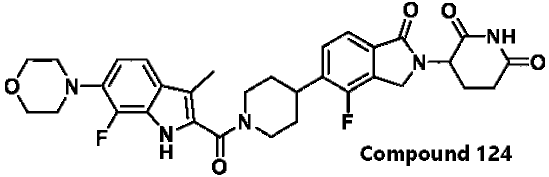

Compound 124 was synthesized in the same manner as for Compound 1, with the exception of using Intermediate K and 7-fluoro-3-methyl-6-morpholino-1H-indole-2-carboxylic acid.
LC/MS 606.2 [M+H]⁺

### Compound 125. 3-(4-fluoro-5-(1-(5-fluoro-3-methyl-6-morpholino-1H-indole-2-carbonyl)piperidin-4-yl)-1-oxoinsoindolin-2-yl)piperidine-2,6-dione

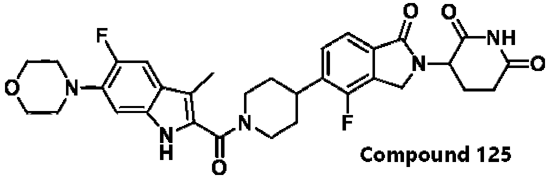

Compound 125 was synthesized in the same manner as for Compound 1, with the exception of using Intermediate K and 5-fluoro-3-methyl-6-morpholino-1H-indole-2-carboxylic acid.
LC/MS 606.4 [M+H]⁺

### Compound 126. 3-(4-fluoro-5-(1-(7-fluoro-3-methyl-6-(4-methylpiperazin-1-yl)-1H-indole-2-carbonyl)piperidin-4-yl)-1-oxoinsoindolin-2-yl)piperidine-2,6-dione

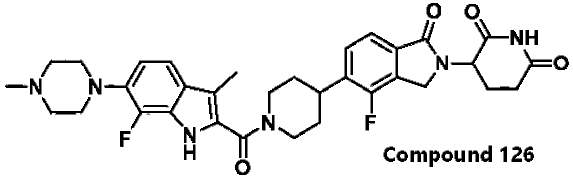

Compound 126 was synthesized in the same manner as for Compound 1, with the exception of using Intermediate K and 7-fluoro-3-methyl-6-(4-methylpiperazin-1-yl)-1H-indole-2-carboxylic acid.
LC/MS 609.3 [M+H]⁺

### Compound 127. 3-(4-fluoro-5-(1-(5-fluoro-3-methyl-6-(4-methylpiperazin-1-yl)-1H-indole-2-carbonyl)piperidin-4-yl)-1- oxoinsoindolin-2-yl)piperidine-2,6-dione

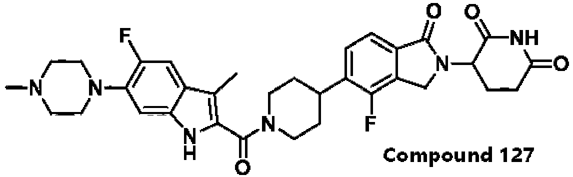

Compound 127 was synthesized in the same manner as for Compound 1, with the exception of using Intermediate K and 5-fluoro-3-methyl-6-(4-methylpiperazin-1-yl)-1H-indole-2-carboxylic acid.
LC/MS 609.1 [M+H]⁺

### <Preparation of Comparative Substances>

### Comparative Substance 2. 3-(5-(1-(6-chloro-1H-indole-3-carbonyl)piperidin-4-yl)-1-oxoinsoindolin-2-yl)piperidine-2,6-dione

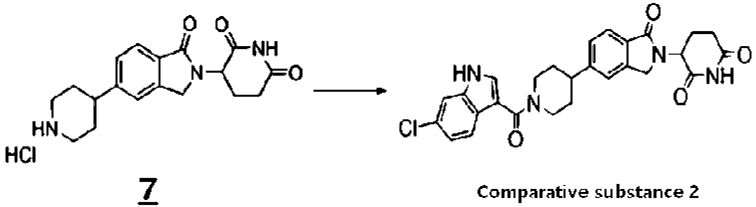

Comparative Substance 2 was synthesized in the same manner as for Compound 1, with the exception of using 6-chloro-1H-indole-3-carboxylic acid instead of 6-chloro-1H-indole-2-carboxylic acid.

¹H NMR (300 MHz, DMSO-d₆) δ 13.62 (s, 1H), 10.98 (s, 1H), 8.00 (d, J = 8.7 Hz, 1H), 7.71 (d, J = 1.8 Hz, 1H), 7.66 (d, J = 7.9 Hz, 1H), 7.54 (s, 1H), 7.44 (dd, J = 7.9, 1.4 Hz, 1H), 7.25 (dd, J = 8.7, 1.8 Hz, 1H), 5.10 (dd, J = 13.2, 5.1 Hz, 1H), 4.90 (d, J = 13.1 Hz, 1H), 4.78 (d, J = 12.8 Hz, 1H), 4.42 (d, J = 17.3 Hz, 1H), 4.28 (d, J = 17.3 Hz, 1H), 3.09-2.83 (m, 4H), 2.65-2.53 (m, 1H), 2.39 (dd, J = 13.2, 4.6 Hz, 1H), 2.05-1.83 (m, 3H), 1.77-1.65 (m, 2H).

### Comparative Substance 3. 3-(5-(1-(6-chloro-1H-benzo[d]imidazole-2-carbonyl)piperidin-4-yl)-1-oxoinsoindolin-2-yl)piperidine-2,6-dione

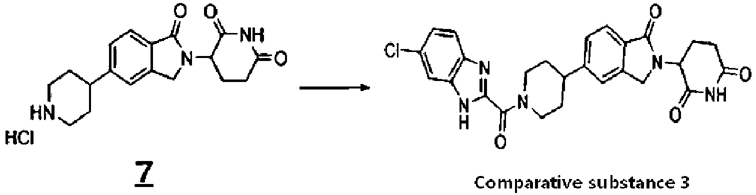

Comparative Substance 3 was synthesized in the same manner as for Compound 1, with the exception of using 6-chloro-1H-benzo[d]imidazole-2-carboxylic acid instead of 6-chloro-1H-indole-2-carboxylic acid.

¹H NMR (300 MHz, DMSO-d6) δ 13.31 (s, 1H), 10.99 (s, 1H), 7.67 (d, J = 7.8 Hz, 3H), 7.55 (s, 1H), 7.45 (d, J = 7.9 Hz, 1H), 7.32 (dd, J = 8.8, 1.9 Hz, 1H), 5.66 (d, J = 13.1 Hz, 1H), 5.11 (dd, J = 13.3, 5.1 Hz, 1H), 4.74 (d, J = 12.8 Hz, 1H), 4.43 (d, J = 17.2 Hz, 1H), 4.29 (d, J = 17.3 Hz, 1H), 3.18-2.84 (m, 4H), 2.59 (d, J = 17.9 Hz, 1H), 2.44-2.34 (m, 1H), 1.97 (s, 3H), 1.81-1.68 (m, 2H).

### Comparative Substance 4. N-(2-(2,6-dioxopiperidin-3-yl)-1-oxoindolin-5-yl)-6-methoxy-2-naphthamide

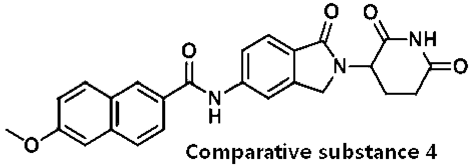

Synthesis was made with reference to the disclosure of the document (Sun-Mi Park et al. 2023).

¹H NMR (300 MHz, DMSO-d6) δ 11.01 (s, 1H), 10.68 (s, 1H), 8.56 (s, 1H), 8.20 (s, 1H), 8.05-7.96 (m, 3H), 7.89 (d, J = 8.2 Hz, 1H), 7.74 (d, J = 8.4 Hz, 1H), 7.44 (d, J = 2.5 Hz, 1H), 7.29 (dd, J = 9.0, 2.5 Hz, 1H), 5.13 (d, J = 13.3 Hz, 1H), 4.50 (d, J = 17.2 Hz, 1H), 4.35 (d, J = 17.3 Hz, 1H), 3.93 (s, 3H), 2.97-2.85 (m, 1H), 2.75-2.62 (m, 1H), 2.30-2.25 (m, 1H), 2.11-1.95 (m, 1H) .

### <EXPERIMENTAL EXAMPLE 1. Evaluation of IKZF2 for Degradation Activity>

### Experimental Example 1. Screening of Helios Protein Degraders Using Nano-Glo HiBiT Cell Line

To observe intracellular degradation of Helios, the Nano-Glo HiBiT detection system (Promega) was employed. A HiBiT-tagged gene sequence was knocked in at the N-terminus of the Helios gene in the Jurkat T lymphocyte cell line using the CRISPR system. The cells expressing Helios fused with HiBiT were validated using the Nano-Glo HiBiT detection system (Promega) according to a predetermined protocol, isolated as single cells, and cultured in complete medium to establish a monoclonal cell line.

For compound screening, including comparative compounds 1 to 4, the established cells were seeded at 50,000 cells per well in a 96-well plate with complete medium, and compounds (10 µM) were treated at 37°C in a CO₂ incubator for 6 hours. After 6 hours, the Nano-Glo HiBiT detection reagent was added to each well in an equal volume to the cell culture medium and incubated at room temperature for 10 minutes. Following incubation, the HiBiT luminescence signal was measured using a microplate reader (Perkin Elmer). Each compound was tested in triplicate wells, and the results are shown in Table 1.

### <Experimental Example 2. Screening of DC₅₀ (nM) and Dₘₐₓ (%) Using WB-IKZF2 Cell Line>

Candidate degraders were serially diluted and treated in cells cultured in complete medium, followed by incubation for 6 hours at 37°C in a CO₂ incubator. After 6 hours, the cells were harvested and lysed in RIPA buffer (150 mM NaCl, 1% Triton X-100, 1% sodium deoxycholate, 0.1% SDS, 50 mM Tris-HCl pH 7.5, and 2 mM EDTA) containing a protease inhibitor cocktail (Roche). The mixture was vortexed every 10 minutes and incubated on ice for 30 minutes, followed by centrifugation at 13,000 rpm for 10 minutes at 4°C to isolate proteins. The isolated proteins were quantified using the Bradford assay (Bio-Rad, 5000006). Proteins were separated on 8% SDS-polyacrylamide gel, transferred onto nitrocellulose membranes (Amersham, 10600002), and incubated with primary antibodies at 4°C for 18 hours. The following primary antibodies were used: anti-Helios rabbit monoclonal antibody (Cell Signaling, 42427, 1:3000) and anti-β-actin mouse monoclonal antibody (Santa Cruz, sc-47778, 1:5000). β-actin was used as a loading control. The next day, the membranes were washed with 1X TBST for 30 minutes and incubated with HRP-conjugated secondary antibodies corresponding to the primary antibodies at room temperature for 1 hour. The following secondary antibodies were used: anti-mouse IgG HRP-linked antibody (Sigma, A9044, 1:5000) and anti-rabbit IgG HRP-linked antibody (Cell Signaling, 7074, 1:5000). After washing with 1X TBST for 30 minutes, protein expression was visualized using the Pierce ECL Western blotting substrate (Thermo Fisher, 32106), and images were captured using a chemiluminescence image analysis system (GE Healthcare, AI680). The obtained images were quantified using the ImageJ software.

**TABLE 1**

| **Compound No.** | **HiBiT-IKZF2 Residual amount of Helios (%)** | | **WB-IKZF2** | | |
|---|---|---|---|---|---|
| | **1 µM** | **10 µM** | **DC ₅₀ (nM)** | **D** ₘₐₓ **(%)** | |
| Compound 1 | 48.1 | 49.1 | nt | | |
| Compound 2 | 78.3 | 92.1 | nt | | |
| Compound 3 | 102.4 | 105.9 | nt | | |
| Compound 4 | 104.0 | 106.0 | nt | | |
| Compound 5 | 86.7 | 88.2 | nt | | |
| Compound 6 | 106.6 | 107.1 | nt | | |
| Compound 7 | 93.3 | 98.6 | nt | | |
| Compound 8 | 99.5 | 108.6 | nt | | |
| Compound 9 | 94.5 | 99.5 | nt | | |
| Compound 10 | 94.6 | 97.3 | nt | | |
| Compound 11 | 99.5 | 98.0 | nt | | |
| Compound 12 | 89.9 | 94.1 | nt | | |
| Compound 14 | 89.0 | 98.4 | nt | | |
| Compound 15 | 66.9 | 73.0 | nt | | |
| Compound 16 | 78.5 | 80.4 | nt | | |
| Compound 17 | 95.0 | 96.6 | nt | | |
| Compound 18 | 53.6 | 54.4 | nt | | |
| Compound 19 | 80.5 | 82.7 | nt | | |
| Compound 21 | 37.0 | 37.3 | nt | | |
| Compound 22 | 64.5 | 67.0 | nt | | |
| Compound 23 | 100.7 | 99.6 | nt | | |
| Compound 24 | 93.7 | 88.2 | nt | | |
| Compound 25 | 93.6 | 87.5 | nt | | |
| Compound 26 | 96.0 | 97.6 | nt | | |
| Compound 27 | 96.4 | 91.7 | nt | | |
| Compound 29 | 73.6 | 72.4 | nt | | |
| Compound 30 | 86.7 | 78.7 | nt | | |
| Compound 31 | 75.0 | 94.3 | nt | | |
| Compound 32 | 62.1 | 57.3 | nt | | |
| Compound 33 | 71.3 | 64.4 | nt | | |
| Compound 34 | 66.5 | 43.5 | nt | | |
| Compound 35 | 76.6 | 52.3 | nt | | |
| Compound 36 | 50.9 | 40.6 | nt | | |
| Compound 37 | 99.6 | 77.5 | nt | | |
| Compound 38 | 80.0 | 65.1 | nt | | |
| Compound 39 | 6.6 | 5.5 | 0.19 | 100.0 | |
| Compound 40 | 92.2 | 74.5 | nt | | |
| Compound 41 | 41.7 | 38.5 | | | |
| Compound 42 | 13.9 | 13.0 | | | |
| Compound 43 | 22.7 | 15.7 | | | |
| Compound 44 | 69.7 | 61.1 | | | |
| Compound 45 | 70.3 | 58.2 | | | |
| Compound 46 | 58.1 | 47.4 | | | |
| Compound 47 | 27.9 | 24.0 | + | nt | |
| Compound 48 | nt | 8.21 | ++ | | |
| Compound 49 | | 8.67 | + | | |
| Compound 50 | | 97.67 | nt | | |
| Compound 51 | | 76.56 | | | |
| Compound 52 | | 10.79 | ++ | nt | |
| Compound 53 | | 7.7 | + | | |
| Compound 54 | | 26.8 | nt | | |
| Compound 55 | | 5.1 | +++ | 100.0 | |
| Compound 56 | | 31.3 | nt | | |
| Compound 57 | | 64.5 | | | |
| Compound 58 | | 5.2 | ++ | nt | |
| Compound 59 | | 25.4 | nt | | |
| Compound 60 | | 5.4 | ++ | nt | |
| Compound 61 | | 35.0 | nt | | |
| Compound 62 | | 23.8 | | | |
| Compound 63 | | 30.7 | | | |
| Compound 64 | | 8.1 | ++ | nt | |
| Compound 65 | | 108.8 | nt | | |
| Compound 66 | | 87.8 | | | |
| Compound 67 | | 78.7 | | | |
| Compound 69 | 22.0 | 41.8 | | | |
| Compound 70 | 94.8 | 101.4 | | | |
| Compound 71 | 14.2 | 25.3 | +++ | 100.0 | |
| Compound 72 | 43.3 | 34.3 | nt | | |
| Compound 73 | 59.2 | 27.8 | | | |
| Compound 74 | 73.2 | 75.8 | | | |
| Compound 75 | 29.6 | 19.0 | + | nt | |
| Compound 81 | nt | 28.7 | nt | | |
| Compound 82 | | 7.4 | ++ | nt | |
| Compound 83 | | 49.0 | nt | | |
| Compound 84 | | 9.7 | + | nt | |
| Compound 85 | | 11.0 | + | | |
| Compound 86 | | 9.9 | ++ | | |
| Compound 87 | | 8.7 | + | | |
| Compound 90 | | 6.2 | +++ | 100.0 | |
| Compound 91 | | 6.4 | +++ | 100.0 | |
| Compound 92 | | 36.1 | nt | | |
| Compound 93 | | 26.5 | +++ | 100.0 | |
| Compound 94 | | 8.5 | +++ | 100.0 | |
| Compound 95 | | 10.9 | ++ | nt | |
| Compound 97 | 14.6 | 21.4 | ++ | | |
| Compound 98 | 20.8 | 13.8 | nt | | |
| Compound 99 | 10.9 | 14.6 | ++ | nt | |
| Compound 100 | 16.8 | 11.4 | nt | | |
| Compound 101 | 15.7 | 20.7 | ++ | 100.0 | |
| Compound 102 | 21.8 | 26.2 | +++ | 100.0 | |
| Compound 103 | 26.8 | 20.6 | nt | | |
| Compound 104 | 18.1 | 21.2 | ++ | 98.0 | |
| Compound 105 | 15.2 | 8.5 | nt | | |
| Compound 106 | 42.8 | 33.6 | | | |
| Compound 107 | 56.6 | 51.5 | | | |
| Compound 108 | 8.3 | 7.5 | | +++ | 100.0 |
| Compound 109 | 38.4 | 24.6 | | nt | |
| Compound 110 | 14.2 | nt | | | |
| Compound 111 | 46.5 | | | | |
| Compound 112 | 23.0 | | | ++ | 98 |
| Compound 113 | 14.1 | | | +++ | 96 |
| Compound 114 | 31.8 | | | nt | |
| Compound 115 | 34.6 | | | +++ | 100 |
| Compound 116 | nt | | | ++ | 96 |
| Compound 117 | | | | +++ | 100 |
| Compound 118 | | | | +++ | 100 |
| Compound 119 | | | | ++ | 92 |
| Compound 120 | | | | ++ | nt |
| Compound 121 | | | | + | |
| Compound 122 | nt | 11.2 | | nt | |
| Compound 123 | | 14.3 | | | |
| Comparative Substance 1 (DKY709) | nt | 40 | | nt | |
| Comparative Substance 2 | | 100 | | | |
| Comparative Substance 3 | | 100 | | | |
| Comparative Substance 4 | 58.0 | 56.2 | | | |
| <Comparative Substance 1> | | | <Comparative Substance 2> | | |
| 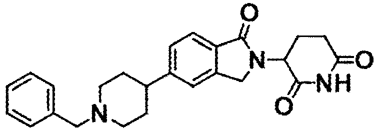 | | | 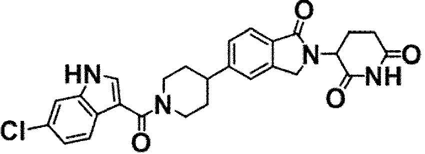 | | |

| <Comparative Substance 3> | <Comparative Substance 4> | | | | |
|---|---|---|---|---|---|
| 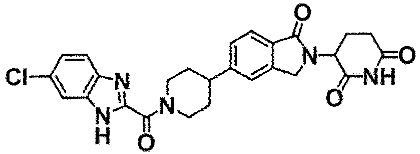 | 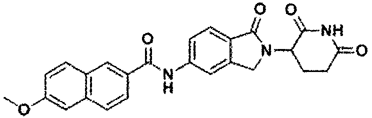 | | | | |
| → nt : not tested | | | | | |
| → WB-IKZF2 DC₅₀ (Degradation concentration 50%,,nM) : +++: <10 nM, ++: 10~100 nM, +: 100-1000 nM | | | | | |

As shown in Table 1, the compounds of the present disclosure in which a piperidine moiety is linked to an indole carbonyl structure exhibited superior IKZF2 degradation activity compared to the comparative compound 1, in which the linkage is through a benzyl structure.

In the case of compounds in which a piperidine moiety is linked to an indole carbonyl structure, compounds having a carbonyl group substituted at the 2-position of the indole exhibited superior IKZF2 degradation activity compared to comparative compound 2, in which substitution occurs at the 3-position. Furthermore, in the case of comparative compound 3, in which the indole structure is replaced with a benzimidazole structure, no IKZF2 degradation activity was observed.

Among the compounds of the present disclosure with substituted indole structures, the compound having a substituent at the 6-position of the indole ring (compound 1) showed superior IKZF2 degradation activity compared to compounds with substitutions at the 4-, 5-, or 7-positions (compounds 3 and 4).

The compounds of the present disclosure having a substituent at the 3-position of the indole ring (compounds 21, 34-36) exhibited superior IKZF2 degradation activity compared to unsubstituted compounds. In particular, compounds with simultaneous substitution at both the 3- and 6-positions of the indole ring (compounds 39, 42, 47-49, 52-56, 60-64, 84-87, 90-95, and 97-121) demonstrated even greater IKZF2 degradation activity.

In addition, compounds of the present disclosure in which the piperidine moiety was replaced with azabicyclo[3.2.1]octane or azetidine (compounds 58 and 63) also exhibited excellent IKZF2 degradation activity.

Furthermore, the compound of the present disclosure bearing an amino group such as methylmorpholine at the 3-position of the indole ring (compound 82) showed selective degradation activity for IKZF2 over IKZF1, IKZF3, and IKZF4.

### <Experimental Example 3. Measurement of Helios Protein Degradation Using Western Blot Analysis>

To confirm the degradation of Helios protein by the degrader, Western blot analysis was performed. Jurkat cells (5 × 10⁶) in complete medium were treated with serial dilutions of the compound at 37°C in a CO₂ incubator for 6 hours.

After harvesting the cells, they were lysed in RIPA buffer (LPS solution, CRB002) containing a protease inhibitor cocktail (Roche). The lysates were incubated on ice for 30 minutes with vortexing every 10 minutes, followed by centrifugation at 13,000 rpm for 10 minutes at 4°C to isolate the cell lysates. The lysates were quantified using the Bradford assay (Bio-Rad, 5000006).

30 µg of the lysates were separated by 8% SDS-polyacrylamide gel electrophoresis (SDS-PAGE), and the proteins were transferred to a nitrocellulose (NC) membrane (Amersham, 10600002), followed by incubation with primary antibodies at 4°C for 18 hours. The following primary antibodies were used: anti-Helios rabbit monoclonal antibody (Cell Signaling, 42427, 1:3000) and anti-β-actin mouse monoclonal antibody (Santa Cruz, sc-47778, 1:5000). β-actin was used as a loading control.

The NC membrane was washed with 1X TBST for 30 minutes, followed by incubation at room temperature for 1 hour with secondary antibodies conjugated to horseradish peroxidase (HRP) corresponding to the primary antibodies. The following secondary antibodies were used: anti-mouse IgG HRP-linked antibody (Sigma, A9044, 1:5000) and anti-rabbit IgG HRP-linked antibody (Cell Signaling, 7074, 1:5000).

After washing with 1X TBST for 30 minutes, protein expression was visualized using Pierce ECL Western blotting substrate (Thermo Fisher, 32106), and the images were acquired using a chemiluminescence imaging system (GE Healthcare, AI680). The resulting images were quantified using ImageJ software. The results of Helios protein degradation measured with compound 39 of the present disclosure and comparative compound DKY709 are shown in FIG. 1.

As shown in FIG. 1, compound 39 of the present disclosure exhibited more than 1,000-fold greater Helios protein degradation activity in Western blot analysis compared to comparative compound 1 (DKY709).

### <Formulation Example 1. Preparation of Powder>

2 g of compound 39 of the present disclosure and 1 g of lactose were mixed and loaded into airtight pouches to prepare a powder formulation.

### <Formulation Example 2. Preparation of Tablet>

100 mg of compound 39 of the present disclosure, 100 mg of microcrystalline cellulose, 60 mg of lactose hydrate, 20 mg of low-substituted hydroxypropyl cellulose, and 2 mg of magnesium stearate were mixed and compressed into tablets according to a conventional tablet manufacturing method.

### [Formulation Example 3. Preparation of Capsule]

100 mg of compound 39 of the present disclosure, 100 mg of microcrystalline cellulose, 60 mg of lactose hydrate, 20 mg of low-substituted hydroxypropyl cellulose, and 2 mg of magnesium stearate were mixed, and the mixture was filled into gelatin capsules according to a conventional capsule preparation method to prepare a capsule formulation.

### [Formulation Example 4. Preparation of Pill]

90 mg of compound 39 of the present disclosure, 5 mg of glutinous rice starch, 5 mg of purified water, and small amounts of additives to inhibit hygroscopicity such as dextrin, maltodextrin, corn starch, and microcrystalline cellulose (MCC) were mixed, and pills of 100 mg were prepared by a conventional method.

### [Formulation Example 5. Preparation of Injection]

10 mg of compound 39 of the present disclosure, an appropriate amount of sterile distilled water for injection, and an appropriate amount of pH-adjusting agent were mixed, and the solution was filled in ampoules (2 mL per ampoule) according to a conventional injection preparation method.

## Claims

1. A compound represented by the following Chemical Formula 1, a stereoisomer thereof, a solvate thereof, a prodrug thereof, or a pharmaceutically acceptable salt thereof. 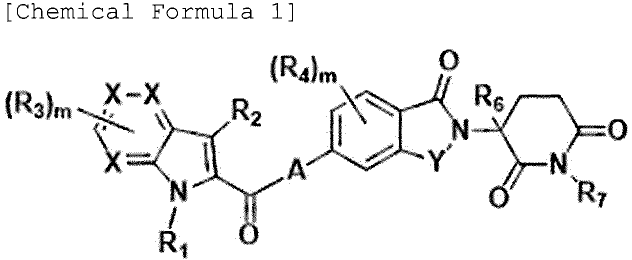 wherein,
X is independently C, CH, N, or NR₃:
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 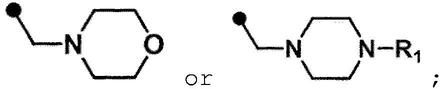
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 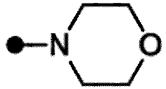 or 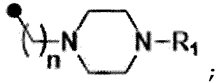
R₄ is independently substituted by a hydrogen atom, halogen or hydroxy;
A is substituted by 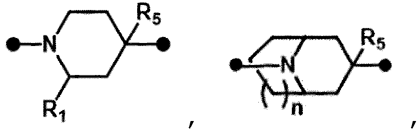 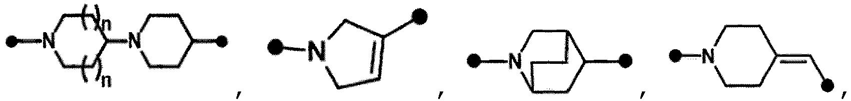 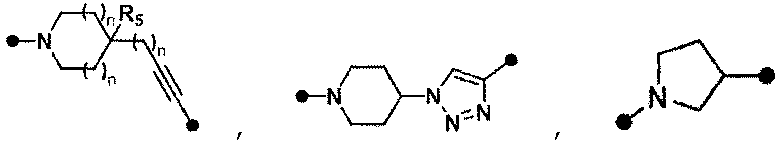 or 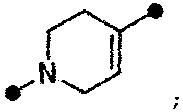
R₅ is substituted by a hydrogen atom, deuterium atom, halogen or hydroxy;
R₆ is substituted by a hydrogen atom or deuterium atom;
R₇ is substituted by a hydrogen atom, C₁₋₅ alkyl or 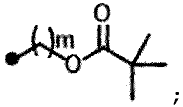
m is an integer of 1 to 3; and
n is independently an integer of 0 or 1.

2. The compound of Claim 1, wherein the Chemical Formula 1 is specified by the following Chemical Formula 2: 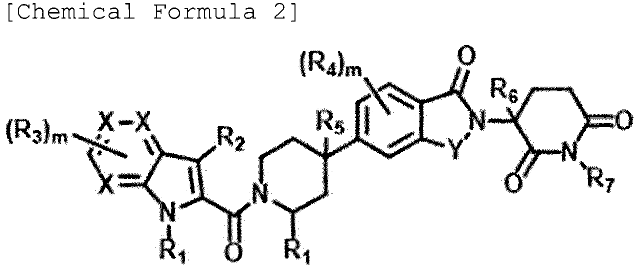 wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 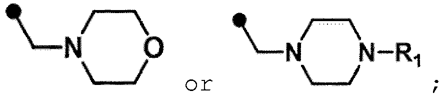
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 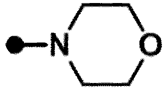 or 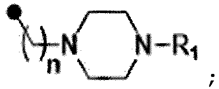
R₄ is independently substituted by a hydrogen atom, halogen or hydroxy;
R₅ is substituted by a hydrogen atom, deuterium atom, halogen or hydroxy;
R₆ is substituted by a hydrogen atom or deuterium atom;
R₇ is substituted by a hydrogen atom, C₁₋₅ alkyl or 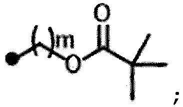
m is an integer of 1 to 3; and
n is independently an integer of 0 or 1,
a stereoisomer thereof, a solvate thereof, a prodrug thereof, or a pharmaceutically acceptable salt thereof.

3. The compound of Claim 1, wherein the Chemical Formula 1 is specified by the following Chemical Formula 3: 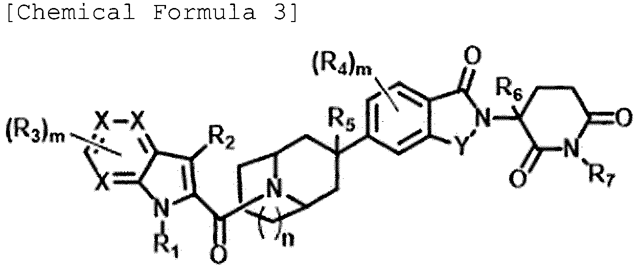 wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 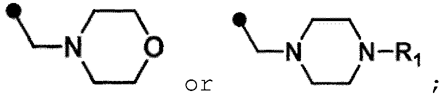
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 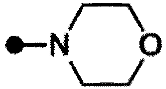 or 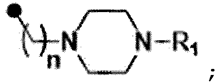
R₄ is independently substituted by a hydrogen atom, halogen or hydroxy;
R₅ is substituted by a hydrogen atom, deuterium atom, halogen or hydroxy;
R₆ is substituted by a hydrogen atom or deuterium atom;
R₇ is substituted by a hydrogen atom, C₁₋₅ alkyl or 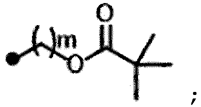
m is an integer of 1 to 3; and
n is independently an integer of 0 or 1,
a stereoisomer thereof, a solvate thereof, a prodrug thereof, or a pharmaceutically acceptable salt thereof.

4. The compound of Claim1, wherein the Chemical Formula 1 is specified by the following Chemical Formula 4: 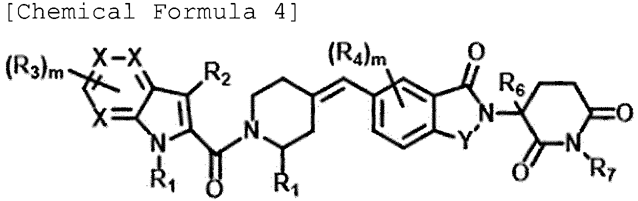 wherein,
X is independently C, CH, N or NR₃;
Y is -CH₂- or -C(O)-;
R₁ is substituted by a hydrogen atom or C₁₋₅ alkyl;
R₂ is substituted by a hydrogen atom, halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 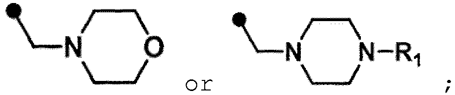
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 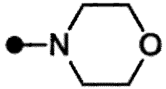 or 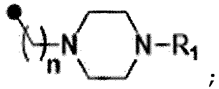
R₄ is independently substituted by a hydrogen atom, halogen or hydroxy;
R₆ is substituted by a hydrogen atom or deuterium atom;
R₇ is substituted by a hydrogen atom, C₁₋₅ alkyl or 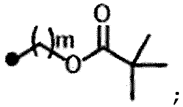
m is an integer of 1 to 3;
n is independently an integer of 0 or 1,
a stereoisomer thereof, a solvate thereof, a prodrug thereof, or a pharmaceutically acceptable salt thereof.

5. The compound of Claim 1, wherein the Chemical Formula 1 is specified by the following Chemical Formula 5: 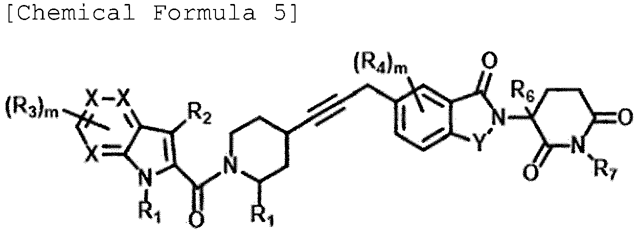 wherein,
X is independently C, CH, N or NR3;
Y is -CH₂- or -C(O)-;
R₁ is substituted by a hydrogen atom or C1-5 alkyl;
R₂ is substituted by a hydrogen atom, halogen, hydroxy, C1-6 alkyl, C1-6 alkoxy, haloC1-6 alkyl, haloC1-6 alkoxy, aldehyde, cyano, diC1-6 alkylaminoC1-6 alkyl, hydroxyC1-6 alkyl, 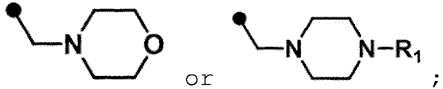
R₃ is independently substituted by a hydrogen atom, halogen, hydroxy, C1-6 alkyl, C1-6 alkoxy, haloC1-6 alkyl, haloC1-6 alkoxy, amino, nitro, cyano, diC1-6 alkylamino, -NHCOC1-6 alkyl, -NHCO aryl, -NHSO2C1-6 alkyl, -NHSO2 aryl, -NHCONHC1-6 alkyl, -NHCONH aryl, 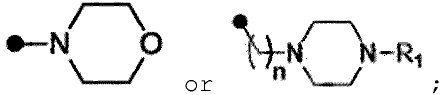
R₄ is independently substituted by a hydrogen atom, halogen or hydroxy;
R₆ is substituted by a hydrogen atom or deuterium atom;
R₇ is substituted by a hydrogen atom, C1-5 alkyl or 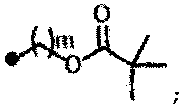
m is an integer of 1 to 3;
n is independently an integer of 0 or 1,
a stereoisomer thereof, a solvate thereof, a prodrug thereof, or a pharmaceutically acceptable salt thereof.

6. The compound of Claim 1, wherein the Chemical Formula 1 is specified by the following Chemical Formula 6: 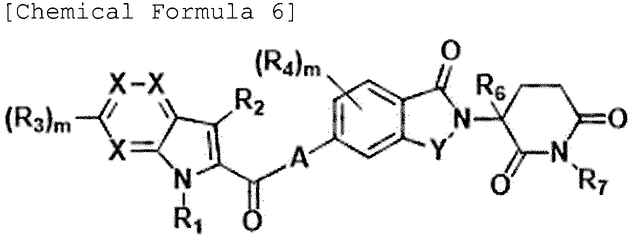 wherein,
X is independently CH, N or NR₃;
Y is -CH₂- or -C(O)-;
R₁ is substituted by a hydrogen atom or C₁₋₅ alkyl;
R₂ is substituted by halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 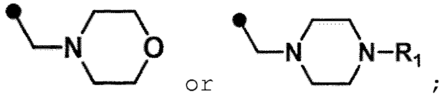
R₃ is independently substituted by a hydrogen atom, halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, - NHCOC₁₋₆ alkyl, -NHCO aryl, -NHSO₂C₁₋₆ alkyl, -NHSO₂ aryl, -NHCONHC₁₋₆ alkyl, -NHCONH aryl, 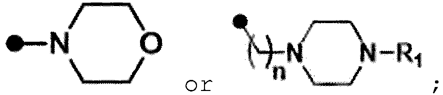
R₄ is independently substituted by a hydrogen atom, halogen or hydroxy;
A is substituted by 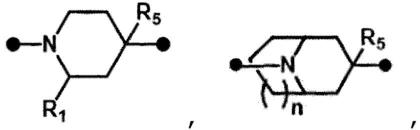 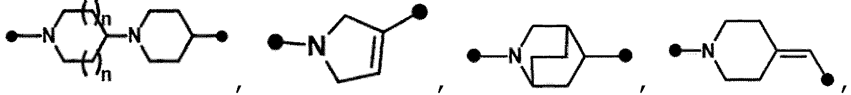 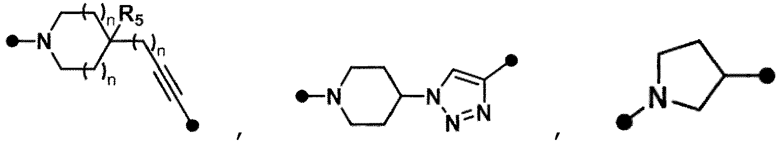 or 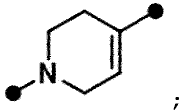
R₅ is substituted by a hydrogen atom, deuterium atom, halogen or hydroxy;
R₆ is substituted by a hydrogen atom or deuterium atom,
R₇ is substituted by a hydrogen atom, C₁₋₅ alkyl or 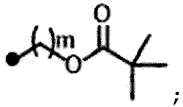
m is an integer of 1 to 3; and
n is independently an integer of 0 or 1,
a stereoisomer thereof, a solvate thereof, a prodrug thereof, or a pharmaceutically acceptable salt thereof,

7. The compound of Claim 6, wherein the compound is represented by the following Chemical Formula 6: 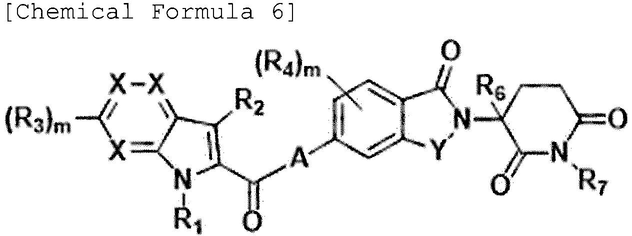 wherein,
X is independently CH, N or NR₃;
Y is -CH₂- or -C(O)-;
R₁ is substituted by a hydrogen atom or C₁₋₅ alkyl;
R₂ is substituted by halogen, hydroxy, C₁₋₆ alkyl, C₁₋₆ alkoxy, haloC₁₋₆ alkyl, haloC₁₋₆ alkoxy, aldehyde, cyano, diC₁₋₆ alkylaminoC₁₋₆ alkyl, hydroxyC₁₋₆ alkyl, 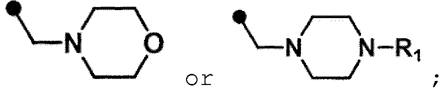
R₃ is independently substituted by halogen, hydroxy, C₁₋₆ alkyl, C₁₋₆ alkoxy, haloC₁₋₆ alkyl, haloC₁₋₆ alkoxy, amino, nitro, cyano, diC₁₋₆ alkylamino, -NHCOC₁₋₆ alkyl, - NHCO aryl, -NHSO₂C₁₋₆ alkyl, -NHSO₂ aryl, -NHCONHC₁₋₆ alkyl, -NHCONH aryl, 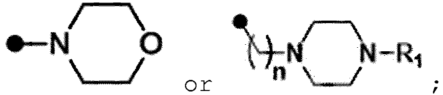
R₄ is independently substituted by a hydrogen atom, halogen or hydroxy;
A is substituted by 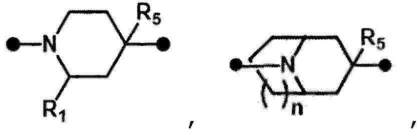 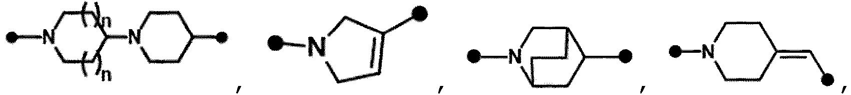 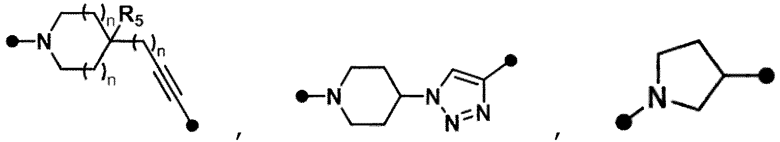 or 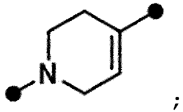
R₅ is substituted by a hydrogen atom, deuterium atom, halogen or hydroxy;
R₆ is substituted by a hydrogen atom or deuterium atom;
R₇ is substituted by a hydrogen atom, C₁₋₅ alkyl or 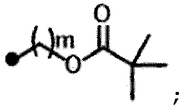
m is an integer of 1 to 3; and
n is independently an integer of 0 or 1,
a stereoisomer thereof, a solvate thereof, a prodrug thereof, or a pharmaceutically acceptable salt thereof.

8. The compound of Claim 1, wherein the compound of Chemical Formula 1 is selected from the group consisting of:
3-(5-(1-(6-chloro-1H-indole-2-carbonyl)piperidin-4-yl)-1-oxoinsoindolin-2-yl)piperidine-2,6-dione (Compound 1);
3-(5-(1-(6,7-dichloro-1H-indole-2-carbonyl)piperidin-4-yl)-1-oxoinsoindolin-2-yl)piperidine-2,6-dione (Compound 2);
3-(5-(1-(5-chloro-1H-indole-2-carbonyl)piperidin-4-yl)-1-oxoinsoindolin-2-yl)piperidine-2,6-dione (Compound 3);
3-(5-(1-(4-chloro-1H-indole-2-carbonyl)piperidin-4-yl)-1-oxoinsoindolin-2-yl)piperidine-2,6-dione (Compound 4);
3-(5-(1-(6-methoxy-1H-indole-2-carbonyl)piperidin-4-yl)-1-oxoinsoindolin-2-yl)piperidine-2,6-dione (Compound 5);
3-(5-(1-(4,6-dichloro-1H-indole-2-carbonyl)piperidin-4-yl)-1-oxoinsoindolin-2-yl)piperidine-2,6-dione (Compound 6);
3-(5-(1-(6-fluoro-1H-indole-2-carbonyl)piperidin-4-yl)-1-oxoinsoindolin-2-yl)piperidine-2,6-dione (Compound 7);
3-(5-(1-(7-chloro-1H-indole-2-carbonyl)piperidin-4-yl)-1-oxoinsoindolin-2-yl)piperidine-2,6-dione (Compound 8);
3-(5-(1-(1H-indole-2-carbonyl)piperidin-4-yl)-1-oxoinsoindolin-2-yl)piperidine-2,6-dione (Compound 9);
3-(5-(1-(5-methoxy-1H-indole-2-carbonyl)piperidin-4-yl)-1-oxoinsoindolin-2-yl)piperidine-2,6-dione (Compound 10);
3-(5-(1-(4-methoxy-1H-indole-2-carbonyl)piperidin-4-yl)-1-oxoinsoindolin-2-yl)piperidine-2,6-dione (Compound 11);
3-(5-(1-(7-methoxy-1H-indole-2-carbonyl)piperidin-4-yl)-1-oxoinsoindolin-2-yl)piperidine-2,6-dione (Compound 12);
3-(5-(1-(5-methyl-1H-indole-2-carbonyl)piperidin-4-yl)-1-oxoinsoindolin-2-yl)piperidine-2,6-dione (Compound 13);
3-(5-(1-(4-methyl-1H-indole-2-carbonyl)piperidin-4-yl)-1-oxoinsoindolin-2-yl)piperidine-2,6-dione (Compound 14);
3-(5-(1-(6-methyl-1H-indole-2-carbonyl)piperidin-4-yl)-1-oxoinsoindolin-2-yl)piperidine-2,6-dione (Compound 15);
3-(5-(1-(7-methyl-1H-indole-2-carbonyl)piperidin-4-yl)-1-oxoinsoindolin-2-yl)piperidine-2,6-dione (Compound 16);
3-(5-(1-(5-fluoro-1H-indole-2-carbonyl)piperidin-4-yl)-1-oxoinsoindolin-2-yl)piperidine-2,6-dione (Compound 17);
3-(5-(1-(6-hydroxy-1H-indole-2-carbonyl)piperidin-4-yl)-1-oxoinsoindolin-2-yl)piperidine-2,6-dione (Compound 18);
3-(5-(1-(7-fluoro-1H-indole-2-carbonyl)piperidin-4-yl)-1-oxoinsoindolin-2-yl)piperidine-2,6-dione (Compound 19);
3-(5-(1-(4-fluoro-1H-indole-2-carbonyl)piperidin-4-yl)-1-oxoinsoindolin-2-yl)piperidine-2,6-dione (Compound 20);
3-(5-(1-(3-methyl-1H-indole-2-carbonyl)piperidin-4-yl)-1-oxoinsoindolin-2-yl)piperidine-2,6-dione (Compound 21);
3-(5-(1-(3-chloro-1H-indole-2-carbonyl)piperidin-4-yl)-1-oxoinsoindolin-2-yl)piperidine-2,6-dione (Compound 22);
3-(5-(3-(1-(1H-indole-3-carbonyl)piperidin-4-yl)prop-1-yn-1-yl)-1-oxoinsoindolin-2-yl)piperidine-2,6-dione (Compound 23);
3-(5-((1-(1H-indole-2-carbonyl)piperidin-4-ylidene)methyl)-1-oxoinsoindolin-2-yl)piperidine-2,6-dione (Compound 24);
3-(5-((1-(1H-indole-3-carbonyl)piperidin-4-ylidene)methyl)-1-oxoinsoindolin-2-yl)piperidine-2,6-dione (Compound 25);
3-(5-((1-(6-chloro-1H-indole-2-carbonyl)piperidin-4-ylidene)methyl)-1-oxoinsoindolin-2-yl)piperidine-2,6-dione (Compound 26);
3-(5-((1-(3-methyl-1H-indole-2-carbonyl)piperidin-4-ylidene)methyl)-1-oxoinsoindolin-2-yl)piperidine-2,6-dione (Compound 27);
3-(5-(1-(1,3-dimethyl-1H-indole-2-carbonyl)piperidin-4-yl)-1-oxoinsoindolin-2-yl)piperidine-2,6-dione (Compound 28);
3-(5-(1-(3-ethyl-1H-indole-2-carbonyl)piperidin-4-yl)-1-oxoinsoindolin-2-yl)piperidine-2,6-dione (Compound 29);
3-(5-(1-(3-methoxy-1H-indole-2-carbonyl)piperidin-4-yl)-1-oxoinsoindolin-2-yl)piperidine-2,6-dione (Compound 30);
3-(5-(1-(3-methyl-1H-picolo[2,3-b]pyridine-2-carbonyl)piperidin-4-yl)-1-oxoinsoindolin-2-yl)piperidine-2,6-dione (Compound 31);
3-(5-(1-(6-amino-1H-indole-2-carbonyl)piperidin-4-yl)-1-oxoinsoindolin-2-yl)piperidine-2,6-dione (Compound 32);
3-(5-(1-(5-bromo-3-methyl-1H-indole-2-carbonyl)piperidin-4-yl)-1-oxoinsoindolin-2-yl)piperidine-2,6-dione (Compound 33);
3-(5-(1-(3-(morpholinomethyl)-1H-indole-2-carbonyl)piperidin-4-yl)-1-oxoinsoindolin-2-yl)piperidine-2,6-dione (Compound 34);
2-(4-(2-(2,6-dioxopiperidin-3-yl)-1-oxoindolin-5-yl)piperidine-1-carbonyl)-1H-indole-3-carbaldehyde (Compound 35);
3-(5-(1-(3-bromo-1H-indole-2-carbonyl)piperidin-4-yl)-1-oxoinsoindolin-2-yl)piperidine-2,6-dione (Compound 36) ;
3-(5-(1-(3-fluoro-1H-indole-2-carbonyl)piperidin-4-yl)-1-oxoinsoindolin-2-yl)piperidine-2,6-dione (Compound 37);
3-(5-(1-(5-chloro-3-methyl-1H-indole-2-carbonyl)piperidin-4-yl)-1-oxoinsoindolin-2-yl)piperidine-2,6-dione (Compound 38);
3-(5-(1-(6-chloro-3-methyl-1H-indole-2-carbonyl)piperidin-4-yl)-1-oxoinsoindolin-2-yl)piperidine-2,6-dione (Compound 39);
3-(5-(1-(5,6-dimethoxy-1H-indole-2-carbonyl)piperidin-4-yl)-1-oxoinsoindolin-2-yl)piperidine-2,6-dione (Compound 40);
3-(1-oxo-5-(1-(6-(trifluoromethyl)-1H-indole-2-carbonyl)piperidin-4-yl)isoindolin-2-yl)piperidine-2,6-dione (Compound 41);
3-(5-(1-(6-methoxy-3-methyl-1H-indole-2-carbonyl)piperidin-4-yl)-1-oxoinsoindolin-2-yl)piperidine-2,6-dione (Compound 42);
3-(5-((1-(6-chloro-3-methyl-1H-indole-2-carbonyl)piperidin-4-yl)ethynyl)-1-oxoinsoindolin-2-yl)piperidine-2,6-dione (Compound 43);
3-(5-((1-(6-chloro-3-methyl-1H-indole-2-carbonyl)piperidin-4-ylidene)methyl)-1-oxoinsoindolin-2-yl)piperidine-2,6-dione (Compound 44);
3-(5-(1-(6-chloro-1H-picolo[3,2-b]pyridine-2-carbonyl)piperidin-4-yl)-1-oxoinsoindolin-2-yl)piperidine-2,6-dione (Compound 45);
3-(5-(1-(6-chloro-1H-picolo[3,2-c]pyridine-2-carbonyl)piperidin-4-yl)-1-oxoinsoindolin-2-yl)piperidine-2,6-dione (Compound 46);
3-(5-(1-(6-hydroxy-3-methyl-1H-indole-2-carbonyl)piperidin-4-yl)-1-oxoinsoindolin-2-yl)piperidine-2,6-dione (Compound 47);
3-(5-(1-(3-methyl-6-(trifluoromethyl)-1H-indole-2-carbonyl)piperidin-4-yl)-1-oxoinsoindolin-2-yl)piperidine-2,6-dione (Compound 48);
3-(5-(1-(6-bromo-3-methyl-1H-indole-2-carbonyl)piperidin-4-yl)-1-oxoinsoindolin-2-yl)piperidine-2,6-dione (Compound 49);
3-(5-(1-(6-chloro-1H-picolo[2,3-b]pyridine-2-carbonyl)piperidin-4-yl)-1-oxoinsoindolin-2-yl)piperidine-2,6-dione (Compound 50);
2-(4-(2-(2,6-dioxopiperidin-3-yl)-1-oxoindolin-5-yl)piperidine-1-carbonyl)-1H-indole-3-carbonitrile (Compound 51);
2-(4-(2-(2,6-dioxopiperidin-3-yl)-1-oxoindolin-5-yl)piperidine-1-carbonyl)-3-methyl-1H-indole-6-carbonitrile (Compound 52);
3-(5-(1-(3-methyl-6-(trifluoromethoxy)-1H-indole-2-carbonyl)piperidin-4-yl)-1-oxoinsoindolin-2-yl)piperidine-2,6-dione (Compound 53);
3-(5-(1-(6-fluoro-3-methyl-1H-indole-2-carbonyl)piperidin-4-yl)-1-oxoinsoindolin-2-yl)piperidine-2,6-dione (Compound 54);
3-(5-(1-(3-methyl-6-nitro-1H-indole-2-carbonyl)piperidin-4-yl)-1-oxoinsoindolin-2-yl)piperidine-2,6-dione (Compound 55);
3-(5-(1-(6-(tert-butyl)-3-methyl-1H-indole-2-carbonyl)piperidin-4-yl)-1-oxoinsoindolin-2-yl)piperidine-2,6-dione (Compound 56);
3-(5-(8-(6-chloro-1H-indole-2-carbonyl)-8-azabicyclo[3.2.1]octan-3-yl)-1-oxoinsoindolin-2-yl)piperidine-2,6-dione (Compound 57);
3-(5-(8-(6-chloro-3-methyl-1H-indole-2-carbonyl)-8-azabicyclo[3.2.1]octan-3-yl)-1-oxoinsoindolin-2-yl)piperidine-2,6-dione (Compound 58);
3-(5-(1-(6-chloro-1H-indole-2-carbonyl)-2-methylpiperidin-4-yl)-1-oxoinsoindolin-2-yl)piperidine-2,6-dione (Compound 59);
3-(5-(1-(6-chloro-3-methyl-1H-indole-2-carbonyl)-2-methylpiperidin-4-yl)-1-oxoinsoindolin-2-yl)piperidine-2,6-dione (Compound 60);
3-(5-(1-(6-amino-3-methyl-1H-indole-2-carbonyl)piperidin-4-yl)-1-oxoinsoindolin-2-yl)piperidine-2,6-dione (Compound 61);
3-(5-(1-(6-chloro-3-methyl-1H-indole-2-carbonyl)-[1,4'-bipiperidin]-4-yl)-1-oxoinsoindolin-2-yl)piperidine-2,6-dione (Compound 62);
3-(5-(1-(1-(6-chloro-3-methyl-1H-indole-2-carbonyl)azetidin-3-yl)piperidin-4-yl)-1-oxoinsoindolin-2-yl)piperidine-2,6-dione (Compound 63);
3-(5-(1-(6-chloro-3-methyl-1H-indole-2-carbonyl)-4-hydroxypiperidin-4-yl)-1-oxoinsoindolin-2-yl)piperidine-2,6-dione (Compound 64);
3-(5-(1-(6-chloro-3-methyl-1H-indole-2-carbonyl)-2,5-dihydro-1H-pyrrol-3-yl)-1-oxoinsoindolin-2-yl)piperidine-2,6-dione (Compound 65);
3-(5-(1-(1-(6-chloro-3-methyl-1H-indole-2-carbonyl)piperidin-4-yl)-1H-1,2,3-triazol-4-yl)-1-oxoinsoindolin-2-yl)piperidine-2,6-dione (Compound 66);
3-(5-(1-(6-chloro-3-methyl-1H-indole-2-carbonyl)pyrrolidin-3-yl)-1-oxoinsoindolin-2-yl)piperidine-2,6-dione (Compound 67);
3-(5-((1-(3-methyl-6-nitro-1H-indole-2-carbonyl)piperidin-4-yl)ethynyl)-1-oxoinsoindolin-2-yl)piperidine-2,6-dione (Compound 68);
3-(5-(1-(3,6-dimethyl-1H-indole-2-carbonyl)piperidin-4-yl)-1-oxoinsoindolin-2-yl)piperidine-2,6-dione (Compound 69);
3-(5-(1-(6-chloro-3-methyl-1H-indole-2-carbonyl)-1,2,3,6-tetrahydropyridin-4-yl)-1-oxoinsoindolin-2-yl)piperidine-2,6-dione (Compound 70);
3-(5-(1-(6-chloro-5-methoxy-3-methyl-1H-indole-2-carbonyl)piperidin-4-yl)-1-oxoinsoindolin-2-yl)piperidine-2,6-dione (Compound 71);
3-(5-(1-(5,6-dichloro-3-methyl-1H-indole-2-carbonyl)piperidin-4-yl)-1-oxoinsoindolin-2-yl)piperidine-2,6-dione (Compound 72);
N-(2-(4-(2-(2,6-dioxopiperidin-3-yl)-1-oxoindolin-5-yl)piperidine-1-carbonyl)-3-methyl-1H-indol-6-yl) acetamide (Compound 73);
N-(2-(4-(2-(2,6-dioxopiperidin-3-yl)-1-oxoindolin-5-yl)piperidine-1-carbonyl)-3-methyl-1H-indol-6-yl) methanesulfonamide (Compound 74);
1-(2-(4-(2-(2,6-dioxopiperidin-3-yl)-1-oxoindolin-5-yl)piperidine-1-carbonyl)-3-methyl-1H-indol-6-yl)-3-ethylurea (Compound 75);
3-(5-((1-(6-chloro-3-methyl-1H-indole-2-carbonyl)azetidin-3-yl)ethynyl)-1-oxoinsoindolin-2-yl)piperidine-2,6-dione (Compound 76);
3-(5-((1-(6-chloro-3-methyl-1H-indole-2-carbonyl)-3-hydroxyazetidin-3-yl)ethynyl)-1-oxoinsoindolin-2-yl)piperidine-2,6-dione (Compound 77);
3-(5-((1-(6-chloro-3-methyl-1H-indole-2-carbonyl)pyrrolidin-3-yl)ethynyl)-1-oxoinsoindolin-2-yl)piperidine-2,6-dione (Compound 78);
3-(5-((1-(6-chloro-3-methyl-1H-indole-2-carbonyl)-3-hydroxypyrrolidin-3-yl)ethynyl)-1-oxoinsoindolin-2-yl)piperidine-2,6-dione (Compound 79);
3-(5-((1-(6-chloro-3-methyl-1H-indole-2-carbonyl)-4-hydroxypiperidin-4-yl)ethynyl)-1-oxoinsoindolin-2-yl)piperidine-2,6-dione (Compound 80);
6-chloro-2-(4-(2-(2,6-dioxopiperidin-3-yl)-1-oxoindolin-5-yl)piperidine-1-carbonyl)-1H-indole-3-carbaldehyde (Compound 81);
3-(5-(1-(6-chloro-3-(morpholinomethyl)-1H-indole-2-carbonyl)piperidin-4-yl)-1-oxoinsoindolin-2-yl)piperidine-2,6-dione (Compound 82);
3-(5-(1-(5-bromo-6-chloro-3-methyl-1H-indole-2-carbonyl)piperidin-4-yl)-1-oxoinsoindolin-2-yl)piperidine-2,6-dione (Compound 83);
3-(5-(1-(5-chloro-3-methyl-6-nitro-1H-indole-2-carbonyl)piperidin-4-yl)-1-oxoinsoindolin-2-yl)piperidine-2,6-dione (Compound 84);
3-(5-(1-(3,5-dimethyl-6-nitro-1H-indole-2-carbonyl)piperidin-4-yl)-1-oxoinsoindolin-2-yl)piperidine-2,6-dione (Compound 85);
3-(5-(1-(5-hydroxy-3-methyl-6-nitro-1H-indole-2-carbonyl)piperidin-4-yl)-1-oxoinsoindolin-2-yl)piperidine-2,6-dione (Compound 86);
3-(5-(1-(6-chloro-3-((dimethylamino)methyl)-1H-indole-2-carbonyl)piperidin-4-yl)-1-oxoinsoindolin-2-yl)piperidine-2,6-dione (Compound 87);
3-(5-((1-(6-chloro-3-methyl-1H-indole-2-carbonyl)piperidin-4-yl)ethynyl)-6-fluoro-1-oxoinsoindolin-2-yl)piperidine-2,6-dione (Compound 88);
3-(5-((1-(6-chloro-3-methyl-1H-indole-2-carbonyl)piperidin-4-yl)ethynyl)-4-fluoro-1-oxoinsoindolin-2-yl)piperidine-2,6-dione (Compound 89);
3-(5-(1-(6-chloro-3-methyl-1H-indole-2-carbonyl)piperidin-4-yl)-6-fluoro-1-oxoinsoindolin-2-yl)piperidine-2,6-dione (Compound 90);
3-(5-(1-(6-chloro-3-methyl-1H-indole-2-carbonyl)piperidin-4-yl)-4-fluoro-1-oxoinsoindolin-2-yl)piperidine-2,6-dione (Compound 91);
5-(1-(6-chloro-3-methyl-1H-indole-2-carbonyl)piperidin-4-yl)-2-(2,6-dioxopiperidin-3-yl)isoindoline-1,3-dione (Compound 92);
3-(6-fluoro-5-(1-(3-methyl-6-nitro-1H-indole-2-carbonyl)piperidin-4-yl)-1-oxoinsoindolin-2-yl)piperidine-2,6-dione (Compound 93);
3-(4-fluoro-5-(1-(3-methyl-6-nitro-1H-indole-2-carbonyl)piperidin-4-yl)-1-oxoinsoindolin-2-yl)piperidine-2,6-dione (Compound 94);
2-(2,6-dioxopiperidin-3-yl)-5-(1-(3-methyl-6-nitro-1H-indole-2-carbonyl)piperidin-4-yl)isoindoline-1,3-dione (Compound 95);
5-((1-(6-chloro-3-methyl-1H-indole-2-carbonyl)piperidin-4-yl)ethynyl)-2-(2,6-dioxopiperidin-3-yl)isoindoline-1,3-dione (Compound 96);
3-(5-(1-(5-methoxy-3-methyl-6-nitro-1H-indole-2-carbonyl)piperidin-4-yl)-1-oxoinsoindolin-2-yl)piperidine-2,6-dione (Compound 97);
3-(5-(1-(6-chloro-7-fluoro-3-methyl-1H-indole-2-carbonyl)piperidin-4-yl)-1-oxoinsoindolin-2-yl)piperidine-2,6-dione (Compound 98);
3-(5-(1-(6-chloro-7-fluoro-3-methyl-1H-indole-2-carbonyl)piperidin-4-yl)-1-oxoinsoindolin-2-yl)piperidine-2,6-dione (Compound 99);
3-(5-(1-(6-chloro-3,7-dimethyl-1H-indole-2-carbonyl)piperidin-4-yl)-1-oxoinsoindolin-2-yl)piperidine-2,6-dione (Compound 100);
3-(5-(1-(6-chloro-5-hydroxy-3-methyl-1H-indole-2-carbonyl)piperidin-4-yl)-1-oxoinsoindolin-2-yl)piperidine-2,6-dione (Compound 101);
3-(5-(1-(6-chloro-5-fluoro-3-methyl-1H-indole-2-carbonyl)piperidin-4-yl)-1-oxoinsoindolin-2-yl)piperidine-2,6-dione (Compound 102);
3-(5-(1-(6-chloro-3,5-dimethyl-1H-indole-2-carbonyl)piperidin-4-yl)-1-oxoinsoindolin-2-yl)piperidine-2,6-dione (Compound 103);
3-(5-(1-(3-methyl-6-morpholino-1H-indole-2-carbonyl)piperidin-4-yl)-1-oxoinsoindolin-2-yl)piperidine-2,6-dione (Compound 104);
3-(5-(1-(6-chloro-3-(hydroxymethyl)-1H-indole-2-carbonyl)piperidin-4-yl)-1-oxoinsoindolin-2-yl)piperidine-2,6-dione (Compound 105);
3-(5-(1-(3-methyl-6-(piperazin-1-ylmethyl)-1H-indole-2-carbonyl)piperidin-4-yl)-1-oxoinsoindolin-2-yl)piperidine-2,6-dione chloride (Compound 106);
3-(5-(1-(4,6-dichloro-3-methyl-1H-indole-2-carbonyl)piperidin-4-yl)-1-oxoinsoindolin-2-yl)piperidine-2,6-dione (Compound 107);
3-(5-(1-(6-chloro-5-methoxy-3-methyl-1H-indole-2-carbonyl)piperidin-4-yl)-4-fluoro-1-oxoinsoindolin-2-yl)piperidine-2,6-dione (Compound 108);
3-(5-(1-(6-chloro-3-methyl-5-(piperazin-1-ylmethyl)-1H-indole-2-carbonyl)piperidin-4-yl)-1-oxoinsoindolin-2-yl)piperidine-2,6-dione dichloride (Compound 109);
3-(5-(1-(6-methoxy-3-(morpholinomethyl)-1H-indole-2-carbonyl)piperidin-4-yl)-1-oxoinsoindolin-2-yl)piperidine-2,6-dione (Compound 110);
3-(5-(1-(6-methoxy-3-(morpholinomethyl)-1H-indole-2-carbonyl)piperidin-4-yl)-1-oxoinsoindolin-2-yl)piperidine-2,6-dione (Compound 111);
3-(5-(1-(6-chloro-5-fluoro-3-methyl-1H-indole-2-carbonyl)piperidin-4-yl)-6-fluoro-1-oxoinsoindolin-2-yl)piperidine-2,6-dione (Compound 112);
3-(5-(1-(6-chloro-5-fluoro-3-methyl-1H-indole-2-carbonyl)piperidin-4-yl)-4-fluoro-1-oxoinsoindolin-2-yl)piperidine-2,6-dione (Compound 113);
3-(5-(1-(6-chloro-3-((4-methylpiperazin-1-yl)methyl)-1H-indole-2-carbonyl)piperidin-4-yl)-1-oxoinsoindolin-2-yl )piperidine-2,6-dione (Compound 114);
3-(5-(1-(6-chloro-5-hydroxy-3-methyl-1H-indole-2-carbonyl)piperidin-4-yl)-4-fluoro-1-oxoinsoindolin-2-yl)piperidine-2,6-dione (Compound 115);
3-(4-fluoro-5-(1-(3-methyl-6-morpholino-1H-indole-2-carbonyl)piperidin-4-yl)-1-oxoinsoindolin-2-yl)piperidine-2,6-dione (Compound 116);
3-(5-(1-(6-chloro-7-fluoro-3-methyl-1H-indole-2-carbonyl)piperidin-4-yl)-4-fluoro-1-oxoinsoindolin-2-yl)piperidine-2,6-dione (Compound 117);
2-(4-(2-(2,6-dioxopiperidin-3-yl)-4-fluoro-1-oxoindolin-5-yl)piperidine-1-carbonyl)-3-methyl-1H-indole-6-carbonitrile (Compound 118);
6-chloro-2-(4-(2-(2,6-dioxopiperidin-3-yl)-4-fluoro-1-oxoindolin-5-yl)piperidine-1-carbonyl)-3-methyl-1H-indole-5-carbonitrile (Compound 119);
2-(4-(2-(2,6-dioxopiperidin-3-yl)-4-fluoro-1-oxoindolin-5-yl)piperidine-1-carbonyl)-5-fluoro-3-methyl-1H-indole-6-carbonitrile (Compound 120);
5-(1-(6-chloro-3-methyl-1H-indole-2-carbonyl)piperidin-4-yl)-2-(2,6-dioxopiperidin-3-yl)-4-fluoroisoindoline-1,3-dione (Compound 121);
2-(4-(2-(2,6-dioxopiperidin-3-yl)-4-fluoro-1-oxoindolin-5-yl)piperidine-1-carbonyl)-7-fluoro-3-methyl-1H-indole-6-carbonitrile (Compound 122);
(3-(5-(1-(6-chloro-3-methyl-1H-indole-2-carbonyl)piperidin-4-yl)-4-fluoro-1-oxoinsoindolin-2-yl)-2,6-dioxopiperidin-1-yl)methyl pivalate (Compound 123);
3-(4-fluoro-5-(1-(7-fluoro-3-methyl-6-morpholino-1H-indole-2-carbonyl)piperidin-4-yl)-1-oxoinsoindolin-2-yl)piperidine-2,6-dione (Compound 124);
3-(4-fluoro-5-(1-(5-fluoro-3-methyl-6-morpholino-1H-indole-2-carbonyl)piperidin-4-yl)-1-oxoinsoindolin-2-yl)piperidine-2,6-dione (Compound 125);
3-(4-fluoro-5-(1-(7-fluoro-3-methyl-6-(4-methylpiperazin-1-yl)-1H-indole-2-carbonyl)piperidin-4-yl)-1-oxoinsoindolin-2-yl)piperidine-2,6-dione (Compound 126); and
3-(4-fluoro-5-(1-(5-fluoro-3-methyl-6-(4-methylpiperazin-1-yl)-1H-indole-2-carbonyl)piperidin-4-yl)-1-oxoinsoindolin-2-yl)piperidine-2,6-dione (Compound 127);
a stereoisomer thereof, a solvate thereof, a prodrug thereof, or a pharmaceutically acceptable salt thereof.

9. A pharmaceutical composition for prevention or treatment of a disease associated with regulation of IKZF2 protein level, the composition comprising, as an active ingredient, the compound, stereoisomer, solvate, prodrug, or pharmaceutically acceptable salt thereof according to any one of claims 1 to 8.

10. The pharmaceutical composition of claim 9, wherein the disease associated with regulation of IKZF2 protein level is cancer.

11. The pharmaceutical composition of claim 10, wherein the cancer is a solid cancer or hematological cancer.

12. The pharmaceutical composition of claim 11, wherein the solid tumor is selected from the group consisting of non-small cell lung cancer (NSCLC), melanoma, triple-negative breast cancer (TNBC), nasopharyngeal carcinoma (NPC), microsatellite stable colorectal cancer (mssCRC), thymoma, carcinoid, gastrointestinal stromal tumor (GIST), prostate cancer, breast carcinoma, lymphoma, leukemia, melanoma, bladder carcinoma, colon cancer, cutaneous melanoma, hepatocellular carcinoma, endometrial cancer, ovarian cancer, cervical cancer, lung cancer, renal cancer, glioblastoma multiforme, glioma, thyroid cancer, parathyroid cancer, nasopharyngeal cancer, tongue cancer, pancreatic cancer, esophageal cancer, cholangiocarcinoma, gastric cancer, soft tissue sarcoma, rhabdomyosarcoma (RMS), synovial sarcoma, osteosarcoma, sarcomatoid carcinoma, and Ewing's sarcoma.

13. The pharmaceutical composition of claim 11, wherein the hematological cancer is selected from the group consisting of acute myeloid leukemia (AML), chronic myeloid leukemia (CML), Bcr-ABL translocation-related CML, myelodysplastic syndrome (MDS), acute B-lymphoblastic leukemia (B-ALL), acute T-lymphoblastic leukemia (T-ALL), chronic lymphocytic leukemia (CLL), multiple myeloma (MM), myeloproliferative neoplasms (MPN), Richter's syndrome, hairy cell leukemia (HCL), blastic plasmacytoid dendritic cell neoplasm (BPDCN), non-Hodgkin's lymphoma (NHL), mantle cell lymphoma (MCL), small lymphocytic lymphoma (SLL), Hodgkin's lymphoma, systemic mastocytosis, and Burkitt's lymphoma.

14. A pharmaceutical composition for prevention or treatment of cancer, wherein the composition comprises the compound represented by Chemical Formula 1 of claim 1 and is used in combination with an anticancer agent drug.

15. The pharmaceutical composition of claim 14, wherein the anticancer agent drug is any one selected from the group consisting of paclitaxel, docetaxel, cabazitaxel, larotaxel, BMS-184476, BMS-188797, BMS275183, milataxel, ortataxel, TL-310, DHA-paclitaxel, nab-paclitaxel, EndoTAG+paclitaxel, XRP9881, polymer-micelle paclitaxel, RPR-109881A, nitrogen mustard N-oxide, cyclophosphamide, ifosfamide, melphalan, busulfan, mitobronitol, carbazilquinone, thiotepa, ranimustine, nimustine, temozolomide, carmustine, methotrexate, 6-mercaptopurine riboside, mercaptopurine, 5-fluorouracil, tegafur, doxifluridine, carmofur, cytarabine, cytarabine ocfosfate, enocitabine, S-1, gemcitabine, fludarabine, pemetrexed disodium, actinomycin D, doxorubicin, daunorubicin, neocarcinostatin, bleomycin, peplomycin, mitomycin C, aclarubicin, pirarubicin, epirubicin, zinostatin stimalamer, idarubicin, sirolimus, valrubicin, vincristine, vinblastine, vindesine, etoposide, sobuzoxane, docetaxel, paclitaxel, vinorelbine, cisplatin, carboplatin, nedaplatin, oxaliplatin, irinotecan, topotecan, camptothecin, gefitinib, imatinib, erlotinib, cetuximab, bevacizumab, rituximab, bevacizumab, alemtuzumab, trastuzumab, goserelin, leuprolide, tamoxifen, Imlygic, Avastin, bevacizumab, ramucirumab, aflibercept, cetuximab, panitumumab, regorafenib, sunitinib, sorafenib, pazopanib, vandetanib, axitinib, cediranib, vatalanib, motesanib, tucatinib, nintedanib, semaxanib, apatinib, lenvatinib, and cabozantinib.

16. A pharmaceutical composition for prevention or treatment of cancer, wherein the composition comprises the compound represented by Chemical Formula 1 of claim 1 and is used in combination with an immunomodulating cell therapeutic or an immunomodulation drug.

17. The pharmaceutical composition of claim 16, wherein the immunomodulating cell therapeutic or the immunomodulation drug is at least selected from a PD-1 inhibitor, a PD-L1 inhibitor, a LAG-3 inhibitor, a CTLA-4 antagonist, an A2A antagonist, a GITR agonist, a TIM-3 inhibitor, a STING agonist, and a TLR7 agonist.

18. The pharmaceutical composition of claim 17, wherein the PD-1 inhibitor is at least one selected from selected from the group consisting of nivolumab (BMS), pembrolizumab (Merck), BCD-100 (BIOCAD), cemiplimab (Regeneron Pharmaceuticals Inc.), sintilimab (Eli Lilly/InnoCare Biologics Inc.'s IBI-308), spartalizumab (Novartis AG's PDR-001), camrelizumab (Incyte Corporation/Jiangsu Hengrui's SHR-1210), tislelizumab (Beigene Ltd.), AGEN-2034 (Agenus Inc.), MEDI-0680 (AMP-514; Amplimmune/MedImmune LLC), toripalimab (Shanghai Junshi Biosciences Co., Ltd.'s JS-001), dostarlimab (Tesaro Inc.'s TSR-042), ABBV-181 (AbbVie Inc.), AK-104 (Akeso Biopharma Inc.), AK-105 (Akeso Biopharma Inc.), BAT-1306 (Bio-Thera Solutions Ltd.), BI754091 (Boehringer Ingelheim GmbH), CBT-501, xenolimab (CBT Pharmaceuticals Inc./Xeno), GLS-010 (Harbin Gloria/WuXi/Arcus), LZM-009 (Livzon Pharmaceutical Group Inc.), MGA-012 (Incyte Corporation/MacroGenics), MGD-013 (MacroGenics Inc.), PF-06801591 (Pfizer Inc.), Sym-021 (Symphogen A/S), CS-1003 (CStone Pharmaceuticals Co., Ltd.), HLX-10 (Henlius Biotech/Shanghai Henlius Biotech Co., Ltd.), AK-103 (Akeso Biopharma Inc.), AM-0001 (Armo Biosciences Inc.), TILT-123 (TILT Biotherapeutics Ltd.), BH-2922 (Beijing Hanmi Pharmaceutical), BH-2941 (Beijing Hanmi Pharmaceutical), BH-2950 (Beijing Hanmi Pharmaceutical), CX-188 (CytomX Therapeutics Inc.), ENUM244C8 (Enumeral Biomedical Holdings), ENUM-388D4 (Enumeral Biomedical Holdings), HAB-21 (Suzhou Steinway Biotech Inc.), HEISCOIII-003 (Sichuan Haisco Pharmaceutical), IKT-202 (Icelle K.Alex Therapeutics), JS-003 (Shanghai Junshi Biosciences), JTX-4014 (Jounce Therapeutics Inc.), MCLA-134 (Merus NV), MGD-019 (MacroGenics Inc.), MT-17000 (Molecular Templates Inc.), PEGMP-7 (D5 Pharma Inc.), PRS-332 (Pieris Pharmaceuticals Inc.), RXI-762 (RXi Pharmaceuticals Corporation), STI-1110 (Les Laboratoires Servier/Sorrento), VXM-10 (Vaximm AG), XmAb-20717 (Xencor Inc.), XmAb-23104 (Xencor Inc.), AK-112 (Akeso Biopharma Inc.), HLX-20 (Henlius Biotech/Shanghai Henlius Biotech Co., Ltd.), SSI-361 (Livzon Biopharma Ltd.), AT-16201 (AIMM Therapeutics BV), and SNA-01 (Fountain Biopharma Inc.).

19. The pharmaceutical composition of claim 17, wherein the PD-L1 inhibitor is at least one selected from the group consisting of atezolizumab (Genentech Inc.), avelumab (Merck KGaA/Pfizer), durvalumab (AstraZeneca Pharmaceuticals LP/MedImmune), BGB-A333 (Beigene Ltd.), CX-072 (CytomX Therapeutics Inc.), GNS-1480 (Yuhan Corporation/Genosco), AMP-224 (MedImmune LLC), CA-170 (Curis Inc./Origin), CK-301 (Checkpoint Therapeutics/TG Therapeutics), CS-1001 (CStone Pharmaceuticals Co., Ltd.), FAZ-053 (Novartis AG), envafolimab (ASC22 or KN-035; Suzhou Alphamab/3DMed), LY-3300054 (Eli Lilly and Company), M-7824 (Merck KGaA), HTI-1088 (HTI-131, SHR-1316; Atria and Jiangsu Hengrui Medicine Co.), MSB-2311 (Mapspace Biosciences (Suzhou) Co., Ltd.), STI-A1014 (Lee's Pharmaceutical/Sorrento), AK106 (Akeso Biopharma Inc.), AVA-004 (Avacta Life Sciences Ltd.), BBI-801 (Boston Biomedical Inc.), CA-327 (Origin/Curis), CBA-0710 (Sorrento Therapeutics Inc.), CBT-502 (CBT Pharmaceuticals/Chiatai Tianqing Pharmaceutical), FPT-155 (Five Prime Therapeutics Inc.), FS-118 (F-star Biotechnology Ltd.), IKT-201 (Icelle K.Alex Therapeutics), IKT-703 (Icelle K.Alex Therapeutics), IO-103 (IO Biotech ApS), JS-003 (Shanghai Junshi Biosciences), KD-033 (Cardamon Corp./Zhenghua Pharmaceutical), KY-1003 (Kymab Ltd.), MCLA-145 (Merus NV/Incyte), MT-5050 (Molecular Templates Inc.), and SNA-02 (Fountain Biopharma Inc.).

20. The pharmaceutical composition of claim 17, wherein the CTLA-4 antagonist comprises at least one selected from the group consisting of ipilimumab, tremelimumab, and MEDI5752.
